# EUROPEAN PATENT SPECIFICATION

(11) **EP 4 003 961 B1**
(45) Date of publication and mention of the grant of the patent: **24.06.2026**
(21) Application number: 20760536.1
(22) Date of filing: 24.07.2020
(51) Int. Cl.: C07D 215/233, C07D 215/38

(54) **SMALL MOLECULE INHIBITORS OF ACETYL COENZYME A SYNTHETASE SHORT CHAIN 2 (ACSS2)**
INHIBITOREN KLEINER MOLEKÜLE VON ACETYL-COENZYM A SYNTHETASE-KURZKETTEN 2 (ACSS2)
INHIBITEURS À PETITES MOLÉCULES DE L'ACÉTYL-COENZYME A SYNTHÉTASE À CHAÎNE COURTE 2 (ACSS2)

(30) Priority: 25.07.2019 GB 201910624; 25.07.2019 IN 201911030106
(43) Date of publication of application: 01.06.2022
(73) Proprietor: Curadev Pharma Pvt. Ltd., Noida 201305 (IN)
(72) Inventor: BASU, Sourav, Noida Uttar Pradesh 201305 (IN); YADAV, B., Dharmendra, Noida Uttar Pradesh 201305 (IN); GHOSH, Rajib, Noida Uttar Pradesh 201305 (IN); SHRIVASTAVA, Ritesh, Noida Uttar Pradesh 201305 (IN); MIDDYA, Sandip, Noida Uttar Pradesh 201305 (IN); PRYDE, David, Sandwich Kent CT13 9ND (GB); BANERJEE, Monali, Noida Uttar Pradesh 201305 (IN); SURYA, Arjun, Noida Uttar Pradesh 201305 (IN)
(74) Representative: Petty, Catrin Helen
(86) International application number: PCT/IB2020/057007
(87) International publication number: WO 2021/014415

(56) References cited:
- EP-A1- 0 507 594
- EP-A1- 1 072 263
- EP-A1- 2 727 920
- WO-A1-01/53274
- WO-A1-03/045920
- WO-A1-2007/093402
- WO-A1-2011/119693
- WO-A1-2017/028798
- WO-A1-2019/036657
- WO-A1-2019/067528
- WO-A1-2019/149260
- WO-A2-03/045313
- WO-A2-2004/014860
- WO-A2-2011/082270
- WO-A2-2012/080284
- US-A- 2 650 226
- US-A- 6 057 320
- TROND ULVEN ET AL: "6-Acylamino-2-aminoquinolines as Potent Melanin-Concentrating Hormone 1 Receptor Antagonists. Identification, Structure?Activity Relationship, and Investigation of Binding Mode", JOURNAL OF MEDICINAL CHEMISTRY, vol. 48, no. 18, 1 September 2005 (2005-09-01), US, pages 5684 - 5697, XP055759090, ISSN: 0022-2623, DOI: 10.1021/jm050103y
- VLADIMIR A. PETROW: "6. Novel types of styrylquinolinium compounds", JOURNAL OF THE CHEMICAL SOCIETY, 1 January 1945 (1945-01-01), pages 18, XP055743286, ISSN: 0368-1769, DOI: 10.1039/jr9450000018
- BATT D G ET AL: "Immunosuppressive structure-activity relationships of Brequinar and related cinchoninic acid derivatives", BIOORGANIC AND MEDICINAL CHEMISTRY LETTERS,, vol. 5, no. 14, 20 July 1995 (1995-07-20), pages 1549 - 1554, XP004135444, ISSN: 0960-894X, DOI: 10.1016/0960-894X(95)00252-O
- SESTILI I ET AL: "A new synthetic approach of N-(4-amino-2-methylquinolin-6-yl)-2-(4-ethylphenoxymethyl)benzamide (JTC-801) and its analogues and their pharmacological evaluation as nociceptin receptor (NOP) antagonists", EUROPEAN JOURNAL OF MEDICINAL CHEMISTRY, ELSEVIER, AMSTERDAM, NL, vol. 39, no. 12, 1 December 2004 (2004-12-01), pages 1047 - 1057, XP004663680, ISSN: 0223-5234, DOI: 10.1016/J.EJMECH.2004.09.009
- PETER NICKEL ET AL: "2-, 3-, 4-Mono-, Di- and Trimethylderivate von 6-(4-Diäthylamino-1-methylbutylamino)-5,6-dimethoxychinolin", JUSTUS LIEBIGS ANNALEN DER CHEMIE, vol. 2, 1 January 1976 (1976-01-01), pages 367 - 382, XP055743510
- JIA-CHEN XING ET AL: "Divergent Synthesis of Functionalized Quinolines from Aniline and Two Distinct Amino Acids", JOURNAL OF ORGANIC CHEMISTRY, vol. 82, no. 17, 1 January 2017 (2017-01-01), pages 9210 - 9216, XP055743514
- JOHN D. WILLIAMS ET AL: "Small molecule inhibitors of anthrax lethal factor toxin", BIOORGANIC & MEDICINAL CHEMISTRY, vol. 22, no. 1, 1 January 2014 (2014-01-01), NL, pages 419 - 434, XP055758696, ISSN: 0968-0896, DOI: 10.1016/j.bmc.2013.11.009
- BHAVIN MARVANIA ET AL: "The synthesis and biological evaluation of new DNA-directed alkylating agents, phenyl N-mustard-4-anilinoquinoline conjugates containing a urea linker", EUROPEAN JOURNAL OF MEDICINAL CHEMISTRY, vol. 83, 1 August 2014 (2014-08-01), NL, pages 695 - 708, XP055334281, ISSN: 0223-5234, DOI: 10.1016/j.ejmech.2014.06.066
- MADAPA S ET AL: "Search for new pharmacophores for antimalarial activity. Part I: Synthesis and antimalarial activity of new 2-methyl-6-ureido-4-quinolinamides", BIOORGANIC & MEDICINAL CHEMISTRY, ELSEVIER, NL, vol. 17, no. 1, 1 January 2009 (2009-01-01), pages 203 - 221, XP025875990, ISSN: 0968-0896, [retrieved on 20081118], DOI: 10.1016/J.BMC.2008.11.021
- UPADHAYAYA RAM SHANKAR ET AL: "New antiprotozoal agents: Their synthesis and biological evaluations", BIORGANIC & MEDICINAL CHEMISTRY LETTERS, ELSEVIER, AMSTERDAM , NL, vol. 23, no. 9, 22 February 2013 (2013-02-22), pages 2750 - 2758, XP028546930, ISSN: 0960-894X, DOI: 10.1016/J.BMCL.2013.02.054
- HIRONOBU MAEZAKI ET AL: "Discovery of potent, selective, and orally bioavailable quinoline-based dipeptidyl peptidase IV inhibitors targeting Lys554", BIOORGANIC & MEDICINAL CHEMISTRY, ELSEVIER, NL, vol. 19, no. 15, 10 June 2011 (2011-06-10), pages 4482 - 4498, XP028241209, ISSN: 0968-0896, [retrieved on 20110618], DOI: 10.1016/J.BMC.2011.06.032
- L V GYUL 'BUDAGYAN ET AL: "2-DICH LOROMETHYL-4-METHYL-2,3-DIHYDROFURO[3,2-c] - QUINOLINE DERIVATIVES", CHEMISTRY OF HETEROCYCLIC COMPOUNDS, vol. 9, 1 January 1973 (1973-01-01), pages 73 - 75, XP055758844
- L V GYUL 'BUDAGYAN ET AL: "QUINALDINE DERIVATIVES XXVI.* SYNTHESES OF SUBSTITUTED 3,4-DIHYDRO-2H- P YRANO [3,2-c ]QUINOLINES", CHEMISTRY OF HETEROCYCLIC COMPOUNDS, vol. 8, 1 January 1972 (1972-01-01), pages 487 - 490, XP055758846
- A K MALLAMS ET AL: "The Reaction between /3-Keto Esters and Arylamines in the Presence of Polyphosphoric Acid. II.1 Ethyl Acetoacetate and Its -Alkyl Derivatives and Arylamines", J. ORG. CHEM. CHEM. IND, 1 January 1961 (1961-01-01), pages 944 - 1649, XP055759136, Retrieved from the Internet <URL:https://pubs.acs.org/doi/pdf/10.1021/jo01035a026>
- HEINRICH JENSCH: "Die Bedeutung von Kern-Methylgruppen in einigen chemotherapeutisch wirksamen Verbindungen der 4-Aminochinolin-Reihe", JUSTUS LIEBIGS ANNALEN DER CHEMIE, vol. 568, no. 1, 20 April 1950 (1950-04-20), DE, pages 73 - 82, XP055666712, ISSN: 0075-4617, DOI: 10.1002/jlac.19505680107
- GYUL'BUDAGYAN ET AL: "QUINALDINE DERIVATIVES XXII. 3- (m-DICHLORALLYL) -4.6-DIAMINOQUINALDINE", ARMYANSKII KHIMICHESKII ZHURNAL/ AIKAKAN HIMIAKAN AMSAGIR/ ARMENIAN CHEMICAL JOURNAL, AKADEMIYA NAUK ARMENII, EREVAN, AI, vol. 24, 1 January 1971 (1971-01-01), pages 537, XP009524556, ISSN: 0515-9628
- GYUL'BUDAGYAN ET AL: "New derivatives of 4-quinaldinol - Communication VI. 3- (t-Chlorallyl) -6-amno-4-quina.1dinol and its transformations", INVESTI AKADEMIINAUK ARMANSKOJ SSR. HIMICESKIE NAUKI, EREVAN, AM, vol. 15, 1 January 1962 (1962-01-01), pages 199 - 203, XP009524560, ISSN: 0367-6846
- GYUL'BUDAGYAN: "New derivatives of 4-quinaldinol -Communication V. 2-Methyl-3- (l-alkoxybenzyl) -4-hydroxy-6-aminoquinolines and their derivatives", INVESTI AKADEMIINAUK ARMANSKOJ SSR. HIMICESKIE NAUKI, EREVAN, AM, vol. 15, 1 January 1962 (1962-01-01), pages 191 - 197, XP009524561, ISSN: 0367-6846
- NICKEL ET AL: "Antimalarial 6-aminoquinolines XI / Gegen Malaria wirksame 6-Aminochinoline", ARZNEIMITTEL FORSCHUNG. DRUG RESEARCH, ECV EDITIO CANTOR VERLAG, AULENDORF, DE, vol. 28, no. 5, 1 January 1978 (1978-01-01), pages 723 - 731, XP009524575, ISSN: 0004-4172

## Description

The present invention relates to small molecules which are inhibitors of the enzymatic activity of the acetyl coenzyme A synthetase short chain 2 (ACSS2) protein. Accordingly, the small molecules may be for use in the treatment of diseases, such as cancer, cardiac disorders, metabolic disorders, neurological disorders, fibrotic disease, ageing disorders, bacterial and viral infections and so on. The invention extends to the compounds *per se* pharmaceutical compositions. This disclosure also provides methods of making the compounds.

Acetyl CoA synthetases (ACSS 1 to 3) are a family of cellular enzymes that carry out the first enzymatic step in the conversion of acetate to the multifunctional metabolite acetyl coenzyme A (acetyl-CoA) through ligation of acetate with CoA in an ATP-driven process (Knowles, S.E.; Jarrett, I.G.; Filsell, O.H.; Ballard, F.J., Biochem. J., 1974, 142, 401-411). ACSS1 and ACSS3 are predominantly expressed in the mitochondria of cells, while ACSS2 is expressed in both nuclear and cytoplasmic compartments (Fujino, T.; Kondo, J.; Ishikawa, M.; Morikawa, K.; Yamamoto, T.T., J. Biol. Chem., 2001, 276, 11423-11426; Luong, A.; Hannah, V.C.; Brown, M.S.; Goldstein, J.L., J. Biol. Chem., 2000, 27.5, 26458-26466; Ariyannur, P.S.; Moffett, J.R.; Madhavarao, C.N.; Arun, P. et. al., J. Comp. Neurol., 2010, 518, 2952-2977).

Acetyl-CoA fulfils a central role in cellular metabolism and is involved in multiple cellular processes (Pietrocola, F.; Galluzzi, L.; Bravo-San Pedro, J. M. et. al., Cell. Metab., 2015, 21, 805-821). In well-nourished mammalian cells, acetyl-CoA enters the citric acid cycle by condensing with oxaloacetate to form citrate and therefrom a range of other metabolites (Srere, P.A., J. Biol. Chem., 1959, 234, 2544-2547). It is a key intermediate of carbon sources and is an essential building block for the synthesis of fatty acids, amino acids and sterols.

Cell growth and proliferation are closely co-ordinated with metabolism and the availability of acetyl-CoA. One of the hallmarks of cancer is rapid, uncontrolled cellular proliferation, which requires increased production of energy and biomass via ATP and lipid production. This involves changes in both the way extracellular nutrients are captured and how they are metabolised. Targeting the strategies adopted by cancer cells to increase rates of metabolism and enhance proliferative capacity under nutrient-limited conditions is an attractive anti-cancer therapeutic approach. It is especially compelling to target sources of the key cytosolic regulator of lipid, cholesterol and amino acid synthesis, namely acetyl-CoA.

In nutrient-limited conditions, such as in cancer cells, aerobic glycolysis takes place and the pyruvate formed from glucose metabolism is preferentially converted by reduction into lactate instead of being taken on to synthesise acetyl-CoA (the 'Warburg Effect'). In response, both acetate uptake and ACSS2 are upregulated and there is a significant shift in the sourcing of acetyl-CoA from other nutrients, a process which relies heavily on ACSS2 for acetyl-CoA synthesis.

Acetate is taken up and metabolized to biomass by proliferating hypoxic and lipid depleted tumor cells (Corbet, C.; Feron, O., Curr. Op. Clin. Nutr. Metab. Care, 2015, 18, 346-353). The propensity of certain tumors for acetate uptake has been exploited for over a decade to detect primary tumors and identify distant metastases by using ¹¹C-acetate guided positron emission tomography (PET) imaging to detect or grade gliomas, hepatocellular carcinomas, non-small cell lung cancer and metastases in prostate cancer patients. This reinforces early studies demonstrating that tumor cells take up significant amounts of acetate often in preference to glucose as a nutrient to satisfy their increased demand for acetyl-CoA (Yoshimoto, M.; Waki, A.; Yonekura, Y.; Sadato, N. et. al., Nucl. Med. Biol., 2001, 28, 117-122).

There is an increasing body of clinical evidence that places acetate and ACSS2 at a critical metabolic node in tumor cells under nutritional and hypoxic stress. Genome analysis reveals that ACSS2 copy number is associated with higher and more invasive stages of breast cancer and metastatic prostate cancer (Schug, Z.T.; Peck, B.; Jones, D.T.; Zhang, Q.; Grosskurth, S. et. al., Cancer Cell, 2015, 27, 57-71). Immunohistochemistry (IHC) using anti-ACSS2 antibodies on human breast, ovarian, kidney and lung tumor samples showed significant expression compared to matched normal samples which showed little or no ACSS2 expression. Survival analyses of patients with grade-2/3 gliomas (Mashimo, T.; Pichumani, K.; Vemireddy, V.; Hatanpaa, K.J., Cell, 2014, 159, 1603-1614) or triple negative breast cancer (Comerford, S.A.; Huang, Z.; Du, X.; Wang, Y. et. al., Cell, 2014, 159, 1591-1602) reveal that high ACSS2 expression is associated with shorter overall survival. These clinical findings show a strong correlation between acetate uptake, ACSS2 expression and cancer progression. This suggests that inhibiting ACSS2 activity could benefit patients with acetate metabolizing tumors.

Hypoxic tumor cells express high levels of cytosolic ACSS2. Knockdown of ACSS2 by RNA interference in tumor cells enhanced tumor cell death under long term hypoxia in vitro and slowed tumor growth in vivo (Yoshii, Y.; Furukawa, T.; Yoshii, H.; Mori, T. et. al., Cancer Sci., 2009, 100, 821-827), supporting a role for ACSS2 in tumor progression and providing the rationale for a pharmacological inhibitor. Interfering with the metabolism of acetate by curtailing ACSS2 activity would deprive resilient tumor cells of a critical nutrient source and could arrest or terminate the growth of intractable tumors.

ACSS2 contributes acetyl-CoA for histone acetyltransferases to acetylate lysine residues on histones (Takahashi, H.; McCaffery, J.M.; Irizarry, R.A.; Boeke, J.D., Mol. Cell, 2006, 23, 207-217) and thereby regulate transcription of growth genes through epigenetic modification of chromatin (Kaelin, W.G.; McKnight, S.L., Cell, 2013, 153, 56-69). Aberrant regulation of chromatin can affect diverse cellular processes reliant on acetylation such as glucose homeostasis, neuronal gene transcription, autophagy and mitochondrial respiration and is linked to conditions such as neurodegeneration, neurological disorders, immunodeficiency and metabolic disease (Mirabella, A.C.; Foster, B.M.; Bartke, T., Chromosoma, 2016, 125, 75-93). Given the extent to which cancer cells exploit metabolic adaptations to meet their elevated energy and biomass demands, it has been proposed that cancer cells may also produce major shifts in their epigenetic footprint to favour tumor growth and survival (Lu, C.; Thompson, C.B., Cell Metab., 2012, 16, 9-17).

The heterodimeric stress-responsive transcription factor Hypoxia Inducible Factor 2α (HIF-2α) is regulated through acetylation by Creb binding protein (CBP), and this acetylation is in turn regulated by acetyl-CoA production by ACSS2 (Chen, R.; Xu, M.; Nagati, J.S.; Hogg, R.T.; Das, A. et. al., PLoS One, 2015, 10, e0116515). Knockdown of ACCS2 or HIF-2α in tumor cells impairs cell proliferation, cell migration and invasion during hypoxia and leads to significant reduction in tumor burden in mice carrying HT1080 flank tumors.

ACSS2 is post-translationally modified by the NAD-dependent deacetylase sirtuins. Sirtuins play a central role in energy homeostasis and ageing and it has therefore been proposed that the regulation of ACSS2 and acetate metabolism may also play a central role in ageing (Shimazu, T.; Hirschey, M.D.; Huang, Y.; Ho, L.T.Y.; Verdin, E., Mech. Ageing Develop., 2010, 131, 511-516).

The causes of ageing are multifactorial but are manifest in a progressive decline in metabolic performance. As individuals age, there is an accompanying build-up of cellular damage and changes to the endogenous repair and detoxification processes. Healthy ageing depends on the efficient removal of damaged cellular material that is mediated in part by autophagy (Eisenberg, T.; Schroeder, S.; Andryushkova, A. et. al., Cell Metab., 2014, 19, 431-444). Knockdown of ACCS2 in mammalian cells results in a strong induction of autophagy and maintenance of lifespan, while nutrient starvation of cells achieves the same effect (Marino, G.; Pietrocola, F.; Eisenberg, T.; Kong, Y. et. al., Mol. Cell, 2014, 53, 710-725).

Human cytomegalovirus (HCMV) induces a robust increase in lipid synthesis to boost the chances of a productive infection. It has recently been shown that in ACSS2 knockout human fibroblasts both HCMV induced lipogenesis and viral growth were sharply reduced compared to normal controls (Vysochan, A.; Sengupta, A.; Weljie, A.M.; Alwine, J.C.; Yu, Y., PNAS, 2017, 114, E1528-E1535) suggesting that impairment of ACSS2 may have some utility as an antiviral therapy in some types of infections. Martinez-Micaelo et. al. have shown that ACSS2 is a nutrient-sensing protein and a key regulator of metabolic homeostasis (Martinez-Micaelo, N.; Gonzalez-Abuin, N.; Terra, X.; Ardevol, A.; Pinent, M. et. al., Disease Mod. Mechan., 2016, 9, 1231-1239). ACSS2 gene expression was correlated with hepatic concentrations of TCA-involved metabolites and with glucose plasma levels. Phosphoprotein analysis of mice fed with a high fat diet (Shaik, A.A.; Qiu, B.; Wee, S.; Choi, H. et. al., Nature, 2016, 6, 25844) showed an array of phosphorylation changes on several enzymes involved in lipid and glucose homeostasis, including reduced phosphorylation of ACSS2, indicating a role in obesity for this protein. Huang et. al. observed significant reductions in body weight and hepatic steatosis in a diet-induced obesity model following knockdown of ACSS2. ACSS2 deficiency appears to reduce dietary lipid absorption, lipid transport to the liver (Huang, Z.; Zhang, M.; Plec, A.A.; Estill, S.J. et. al., PNAS, 2018, 115, E9499-E9506) and controls systemic lipid metabolism according to acetate availability. These studies indicate that a selective inhibitor of ACSS2 could have therapeutic benefit in obesity and fatty liver disease.

This developing knowledge has stimulated considerable research into possible therapeutic applications of ACSS2 inhibition. For instance, WO 2019/067528 A1 describes compositions and methods for inhibiting ACSS2.

However, there remains a need in the art for improved therapies for treating diseases, such as cancer, neurological disorders and metabolic disorders, which can be refractory to traditional therapeutic approaches. There is a need to develop improved compositions and methods in this field. In particular, there is a need for compounds that inhibit the human ACSS2 protein, as well as methods for treating diseases that can benefit from such modulation.

The present invention has arisen from the inventors work in attempting to identify ACSS2 protein inhibitors.

The invention is as defined in the claims.

In a first aspect of the invention, there is provided a compound of formula (I): 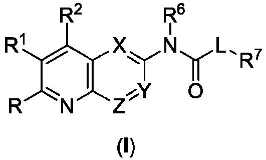
, wherein X is CR³;
Y is CR⁴;
Z is CR⁵ or N;
R is selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl;
R¹ is selected from the group consisting of mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl, wherein when R¹ is an optionally substituted alkyl, an optionally substituted alkenyl or an optionally substituted alkynyl, the alkyl, alkenyl or alkynyl is unsubstituted or substituted with one or more of a halogen, an optionally substituted C₆-C₁₂ aryl or an optionally substituted 5 to 10 membered heteroaryl;
R² is H, halogen, COOR⁹, CN, CONR⁹R¹⁰, NR⁹R¹⁰, NR⁹SO₂R¹⁰, SO₂NR⁹R¹⁰, NR⁹COR¹⁰, optionally substituted C₁-C₁₀ alkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R³ is H, halogen, optionally substituted C₁-C₁₀ alkyl, optionally substituted mono or bicyclic C₃-C₆ cycloalkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R⁴ and R⁵ are each independently selected from the group consisting of H and halogen; R⁶ is H;
L is NR⁸ and R⁷ is optionally substituted C₃-C₁₀ alkyl, optionally substituted C₂-C₁₀ alkenyl, optionally substituted C₂-C₁₀ alkynyl, mono or bicyclic optionally substituted C₆-C₁₂ aryl, mono or bicyclic optionally substituted 5 to 10 membered heteroaryl, optionally substituted mono or bicyclic C₃-C₆ cycloalkyl, or optionally substituted mono or bicyclic 3 to 8 membered heterocycle; or L is absent and R⁷ is optionally substituted C₃-C₁₀ alkyl or optionally substituted mono or bicyclic 3 to 8 membered heterocycle, wherein when L is absent and R⁷ is optionally substituted C₃-C₁₀ alkyl, the alkyl, is unsubstituted or substituted with one or more of OH, oxo or halogen;
R⁸ is H; and
R⁹ and R¹⁰ are each independently selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, optionally substituted C₂-C₁₀ alkynyl, optionally substituted C₁-C₁₀ alkoxy and NH₂;
or a pharmaceutically acceptable complex, salt, solvate, tautomeric form or polymorphic form thereof,
wherein, unless otherwise specified:
   an optionally substituted alkyl is unsubstituted or substituted with one or more of halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle; an optionally substituted alkenyl is unsubstituted or substituted with one or more of optionally substituted C₁-C₆ alkyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted alkynyl group is unsubstituted or substituted with one or more of optionally substituted C₁-C₆ alkyl, optionally substituted C₂-C₆ alkenyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted alkoxy group is unsubstituted or substituted with one or more of halogen, OH, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted aryloxy, optionally substituted heteroaryloxy, optionally substituted C₆-C₁₂ aryl, optionally substituted 5 to 10 membered heteroaryl, C₃-C₆ cycloalkyl and 3 to 8 membered heterocycle;
   an optionally substituted aryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted aryloxy is the group aryl-O- where aryl is an optionally substituted C₆-C₁₂ aryl group;
   an optionally substituted cycloalkyl or cycloalkenyl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted heteroaryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted heterocycle is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle; and
   wherein the compound is not:

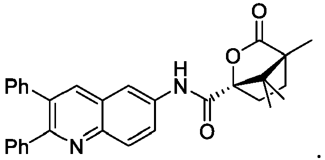

The inventors have found that the compounds of formula **(I)** are useful in therapy or as a medicament.

This disclosure also provides a conjugate of a compound of formula **(I).**

Also disclosed in the present application is a conjugate of formula **(II):** 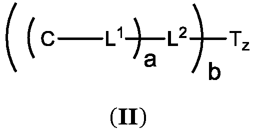
wherein, C is a compound of formula **(I);**
L¹ and L² are linkers;
T is a targeting moiety; and
a is an integer between 1 and 5;
b is an integer between 1 and 10; and
z is an integer between 1 and 5.

It may be appreciated that a hydrogen may be removed from the compound of formula (I) and L¹ may be bonded to the position where the hydrogen would otherwise be present.

Such conjugates may be designed to specifically target certain cell types or tumor types via the targeting moiety, which directs the compound of formula **(I)** to just those cells or tumors and delivers the ACSS2 inhibitor in a cell-specific manner. The principle of this targeted delivery will be known to those skilled in the art as being closely related to ADC (antibody-drug conjugate) technology, for example as described in Polakis, P., Pharmacol. Revs., 2016, 68, 3-19. The linker will then be designed to cleave and the active compound would then diffuse into the cell and contact the ACSS2 protein.

Hence, in a second aspect, there is provided a compound of formula **(I),** or a pharmaceutically acceptable complex, salt, solvate, tautomeric form or polymorphic form thereof, for use in therapy.

The inventors have also found that compounds of formula **(I)** and conjugates of formula **(II)** are useful in modulating the acetyl coenzyme A synthetase short chain 2 (ACSS2) protein.

The compound of formula **(I)** or the conjugate of formula **(II)** may inhibit, or inactivate, the ACSS2 protein. The compound of formula **(I)** or the conjugate of formula **(II)** may inhibit, or inactivate, ACSS2 enzymatic activity as evidenced by a reduction of one or more biological effects selected from the group consisting of production of acetyl-CoA, acetate incorporation into lipids, acetate incorporation into histones and incorporation of acetate into tumor cells.

By inhibiting the ACSS2 protein, it is possible to treat, ameliorate or prevent cancer, bacterial infection, viral infection, parasitic infection, neurodegenerative disease, cardiovascular disease, fatty liver disease, a metabolic disorder and promote healthy ageing.

Advantageously, the compounds and conjugates of the invention selectively inhibit only one subtype of the human ACCS family of proteins. Compounds of the invention are potent inhibitors of ACCS2, but do not inhibit ACSS1 or ACSS3.

By inhibiting the ACSS2 protein, it is possible to treat, ameliorate or prevent cancer, bacterial infection, viral infection, parasitic infection, fungal infection, neurodegenerative disease, neurological disorder, cerebrovascular disease, cardiovascular disease, non-alcoholic fatty liver disease, obesity and promote healthy ageing.

Accordingly, in a third aspect there is provided a compound of formula **(I):** 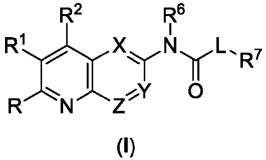
, wherein X is CR³;
Y is CR⁴;
Z is CR⁵ or N;
R is selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl;
R¹ is selected from the group consisting of mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl, wherein when R¹ is an optionally substituted alkyl, an optionally substituted alkenyl or an optionally substituted alkynyl, the alkyl, alkenyl or alkynyl is unsubstituted or substituted with one or more of a halogen, an optionally substituted C₆-C₁₂ aryl or an optionally substituted 5 to 10 membered heteroaryl;
R² is H, halogen, COOR⁹, CN, CONR⁹R¹⁰, NR⁹R¹⁰, NR⁹SO₂R¹⁰, SO₂NR⁹R¹⁰, NR⁹COR¹⁰, optionally substituted C₁-C₁₀ alkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R³ is H, halogen, optionally substituted C₁-C₁₀ alkyl, optionally substituted mono or bicyclic C₃-C₆ cycloalkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R⁴ and R⁵ are each independently selected from the group consisting of H and halogen; R⁶ is H;
L is NR⁸ and R⁷ is optionally substituted C₃-C₁₀ alkyl, optionally substituted C₂-C₁₀ alkenyl, optionally substituted C₂-C₁₀ alkynyl, mono or bicyclic optionally substituted C₆-C₁₂ aryl, mono or bicyclic optionally substituted 5 to 10 membered heteroaryl, optionally substituted mono or bicyclic C₃-C₆ cycloalkyl, or optionally substituted mono or bicyclic 3 to 8 membered heterocycle; or L is absent and R⁷ is optionally substituted C₃-C₁₀ alkyl or optionally substituted mono or bicyclic 3 to 8 membered heterocycle, wherein when L is absent and R⁷ is optionally substituted C₃-C₁₀ alkyl, the alkyl, is unsubstituted or substituted with one or more of OH, oxo or halogen;
R⁸ is H; and
R⁹ and R¹⁰ are each independently selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, optionally substituted C₂-C₁₀ alkynyl, optionally substituted C₁-C₁₀ alkoxy and NH₂;
or a pharmaceutically acceptable complex, salt, solvate, tautomeric form or polymorphic form thereof,
wherein, unless otherwise specified:
   an optionally substituted alkyl is unsubstituted or substituted with one or more of halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle; an optionally substituted alkenyl is unsubstituted or substituted with one or more of optionally substituted C₁-C₆ alkyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted alkynyl group is unsubstituted or substituted with one or more of optionally substituted C₁-C₆ alkyl, optionally substituted C₂-C₆ alkenyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted alkoxy group is unsubstituted or substituted with one or more of halogen, OH, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted aryloxy, optionally substituted heteroaryloxy, optionally substituted C₆-C₁₂ aryl, optionally substituted 5 to 10 membered heteroaryl, C₃-C₆ cycloalkyl and 3 to 8 membered heterocycle;
   an optionally substituted aryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted aryloxy is the group aryl-O- where aryl is an optionally substituted C₆-C₁₂ aryl group;
   an optionally substituted cycloalkyl or cycloalkenyl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted heteroaryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   an optionally substituted heterocycle is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
   or a pharmaceutically acceptable salt, solvate, tautomeric form or polymorphic form thereof, or a pharmaceutical composition comprising a compound of formula (I), or a pharmaceutically acceptable salt, solvate, tautomeric form or polymorphic form thereof, and a pharmaceutically acceptable vehicle, for use in treating, ameliorating or preventing a disease selected from cancer, bacterial infection, viral infection, parasitic infection, fungal infection, neurodegenerative disease, neurological disorder, cerebrovascular disease, cardiovascular disease, non-alcoholic fatty liver disease and obesity or for use in promoting healthy ageing.

Preferably, the disease is cancer.

Also disclosed in the present application is a method of inhibiting the ACSS2 protein in a subject, the method comprising administering, to a subject in need of such treatment, a therapeutically effective amount of a compound of formula **(I),** or a pharmaceutically acceptable complex, salt, solvate, tautomeric form or polymorphic form thereof, or a conjugate of formula **(II).**

Preferably, the method comprises inhibiting the ACSS2 protein.

Also disclosed in the present application is a method of treating, ameliorating or preventing a disease selected from cancer, bacterial infection, viral infection, parasitic infection, fungal infection, neurodegenerative disease, neurological disorder, cerebrovascular disease, cardiovascular disease, metabolic disorder, non-alcoholic fatty liver disease and obesity or promoting healthy ageing; the method comprising administering, to a subject in need of such treatment, a therapeutically effective amount of a compound of formula **(I),** or a pharmaceutically acceptable complex, salt, solvate, tautomeric form or polymorphic form thereof, or a conjugate of formula **(II).**

It may be appreciated that the term "preventing" can mean "reducing the likelihood of".

The neurodegenerative disorder may be amyotrophic lateral sclerosis, Parkinson's disease, Alzheimer's disease or Huntington's disease. The neurological disorder may be anxiety, depression, autism or post-traumatic stress disorder.

The parasitic infection may be malaria.

The metabolic disorder may be obesity or fatty liver disease, for example non-alcoholic steatohepatitis.

Promoting healthy ageing may include restoring or enhancing autophagy and autophagic protein clearance.

In one preferred embodiment, the disease is cancer. The cancer may be selected from the group consisting of colorectal cancer, aero-digestive squamous cancer, gastrointestinal stromal tumors, lung cancer, brain cancer, neuroblastoma, glial tumors, astrocytoma, glioblastoma, liver cancer, stomach cancer, sarcoma, leukaemia, lymphoma, multiple myeloma, ovarian cancer, uterine cancer, breast cancer, melanoma, prostate cancer, bladder cancer, pancreatic carcinoma or renal carcinoma. In some embodiments, the cancer may have upregulated ACSS2 expression and /or ACSS2 activity in a tissue compared to that of a healthy subject.

In an alternative preferred embodiment, the disease is a viral infection. The viral infection may be a hepatitis C virus (HCV) infection or human cytomegalovirus (HCMV) infection.

The following definitions are used in connection with the compounds of the present invention unless the context indicates otherwise.

Throughout the description and the claims of this specification the word "comprise" and other forms of the word, such as "comprising" and "comprises," means including but not limited to, and is not intended to exclude for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions.

"Optional" or "optionally" means that the subsequently described event, operation or circumstances can or cannot occur, and that the description includes instances where the event, operation or circumstance occurs and instances where it does not.

The term "halo" or "halogen" includes fluoro (-F), chloro (-Cl), bromo (-Br) and iodo (-I).

The term "polyfluoroalkyl" may denote a C₁-C₃ alkyl group in which two or more hydrogen atoms are replaced by fluorine atoms. The term may include perfluoroalkyl groups, *i.e.* a C₁-C₃ alkyl group in which all the hydrogen atoms are replaced by fluorine atoms. Accordingly, the term C₁-C₃ polyfluoroalkyl includes, but is not limited to, difluoromethyl, trifluoromethyl, 2,2,2-trifluoroethyl, pentafluoroethyl, 3,3,3-trifluoropropyl, 2,2,3,3,3-pentafluoropropyl, and 2,2,2-trifluoro-1-(trifluoromethyl)ethyl.

A complex of the compound of formula **(I)** may be understood to be a multi-component complex, wherein the drug and at least one other component are present in stoichiometric or non-stoichiometric amounts. The complex may be other than a salt or solvate. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallisation, by recrystallisation from solvents, or by physically grinding the components together - see Chem Commun, 17, 1889-1896, by O. Almarsson and M. J. Zaworotko (2004**).** For a general review of multi-component complexes, see J Pharm Sci, 64 (8), 1269-1288, by Haleblian (August 1975).

The term "pharmaceutically acceptable salt" may be understood to refer to any salt of a compound provided herein which retains its biological properties and which is not toxic or otherwise undesirable for pharmaceutical use. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, adepic, aspartic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, benzoic, glutamic, hydroxynaphthoic, salicylic, stearic, cyclohexylsulfamic, quinic, muconic acid and the like acids; or (2) base addition salts formed when an acidic proton present in the parent compound either (a) is replaced by a metal ion, e.g., an alkali metal ion, an alkaline earth ion or an aluminium ion, or alkali metal or alkaline earth metal hydroxides, such as sodium, potassium, calcium, magnesium, aluminium, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like.

Pharmaceutically acceptable salts may include, sodium, potassium, calcium, magnesium, ammonium, tetraalkylammonium and the like, and when the compound contains a basic functionality, salts of non-toxic organic or inorganic acids, such as hydrohalides, e.g. hydrochloride, hydrobromide and hydroiodide, carbonate or bicarbonate, sulfate or bisulfate, borate, phosphate, hydrogen phosphate, dihydrogen phosphate, pyroglutamate, saccharate, stearate, sulfamate, nitrate, orotate, oxalate, palmitate, pamoate, acetate, trifluoroacetate, trichloroacetate, propionate, hexanoate, cyclopentylpropionate, glycolate, glutarate, pyruvate, lactate, malonate, succinate, tannate, tartrate, tosylate, sorbate, ascorbate, malate, maleate, fumarate, tartarate, camsylate, citrate, cyclamate, benzoate, isethionate, esylate, formate, 3-(4-hydroxybenzoyl)benzoate, picrate, cinnamate, mandelate, phthalate, laurate, methanesulfonate (mesylate), methylsulphate, naphthylate, 2-napsylate, nicotinate, ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, tert-butylacetate, lauryl sulfate, gluceptate, gluconate, glucoronate, hexafluorophosphate, hibenzate, benzoate, glutamate, hydroxynaphthoate, salicylate, stearate, cyclohexylsulfamate, quinate, muconate, xinofoate and the like.

Hemisalts of acids and bases may also be formed, for example, hemisulphate salts. The skilled person will appreciate that the aforementioned salts include ones wherein the counterion is optically active, for example D-lactate, or racemic, for example DL-tartrate.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula **(I)** may be prepared by one or more of three methods:
(i) by reacting the compound of formula **(I)** with the desired acid or base;
(ii) by removing an acid- or base-labile protecting group from a suitable precursor of the compound of formula **(I)** using the desired acid or base; or
(iii) by converting one salt of the compound of formula **(I)** to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The term "solvate" may be understood to refer to a compound provided herein or a salt thereof, that further includes a stoichiometric or non-stoichiometric amount of solvent bound by non-covalent intermolecular forces. Where the solvent is water, the solvate is a hydrate. Pharmaceutically acceptable solvates in accordance with the invention include those wherein the solvent of crystallization may be isotopically substituted, e.g. D₂O, d₆-acetone and d₆-DMSO.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

The compounds of the invention may exist in a continuum of solid states ranging from fully amorphous to fully crystalline, including polymorphs of said crystalline material. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as -COO⁻Na⁺, -COO⁻K⁺, or -SO₃⁻Na⁺) or non-ionic (such as -N⁻N⁺(CH₃)₃) polar head group. For more information, see Crystals and the Polarizing Microscope by N. H. Hartshorne and A. Stuart, 4th Edition (Edward Arnold, 1970).

R and R¹ may each independently be selected from the group consisting of mono or bicyclic optionally substituted C₆-C₁₂ aryl, mono or bicyclic optionally substituted 5 to 10 membered heteroaryl, optionally substituted C₁-C₆ alkyl, optionally substituted mono or bicyclic C₃-C₆ cycloalkyl, optionally substituted mono or bicyclic C₃-C₆ cycloalkenyl, optionally substituted C₂-C₆ alkenyl and optionally substituted C₂-C₆ alkynyl.

When R and/or R¹ is an optionally substituted C₆-C₁₂ aryl, the aryl is preferably optionally substituted phenyl. The phenyl may be unsubstituted or substituted with one or more of halogen, optionally substituted C₁-C₆ alkyl, optionally substituted C₁-C₆ alkoxy, COR, NR⁹R¹⁰, CN, R⁹COR¹⁰, optionally substituted C₆-C₁₂ aryl or optionally substituted 5 to 10 membered heteroaryl, wherein R⁹ and R¹⁰ are each H or an optionally substituted C₁-C₆ alkyl. More preferably, the phenyl is unsubstituted or substituted with one or more of fluorine, C₁-C₆ alkoxy, COR⁹, NR⁹R¹⁰, CN, R⁹COR¹⁰ or C₆-C₁₂ aryl, wherein R⁹ and R¹⁰ are each H or a C₁-C₆ alkyl. Most preferably, the phenyl is unsubstituted or substituted with one or more of fluorine, OH, OCH₃, OCH₂CH₃, OCH₂CH(CH₃)₂, COCH₃, N(CH₃)₂, NH₂, CN, NHC(O)CH₃ or phenyl.

When R and/or R¹ is an optionally substituted 5 to 10 membered heteroaryl, the heteroaryl is preferably an optionally substituted 5 or 6 membered heteroaryl, and more preferably an optionally substituted pyridinyl or an optionally substituted pyrazolyl. The heteroaryl may be unsubstituted or substituted with optionally substituted C₁-C₆ alkyl, optionally substituted C₁-C₆ alkoxy or OH. More preferably, the heteroaryl is unsubstituted or substituted with one or more of C₁-C₆ alkyl, C₁-C₆ alkoxy or OH. Most preferably, the heteroaryl is unsubstituted or substituted with one or more of methyl, OCH₃ or OH.

When R and/or R¹ is an optionally substituted mono or bicyclic C₃-C₆ cycloalkyl, the cycloalkyl is preferably unsubstituted.

When R and/or R¹ is an optionally substituted C₁-C₆ alkyl, optionally substituted C₂-C₆ alkenyl or optionally substituted C₂-C₆ alkynyl, the alkyl, alkenyl or alkynyl is preferably an optionally substituted C₁-C₃ alkyl, optionally substituted C₂-C₃ alkenyl or optionally substituted C₂-C₃ alkynyl, and more preferably an optionally substituted C₁-C₂ alkyl, optionally substituted C₂ alkenyl or optionally substituted C₂ alkynyl. The alkyl, alkenyl or alkynyl may be unsubstituted or substituted with one or more of a halogen, an optionally substituted C₆-C₁₂ aryl or an optionally substituted 5 to 10 membered heteroaryl. Preferably, the alkyl, alkenyl or alkynyl is unsubstituted or substituted with one or more of fluorine or a C₆-C₁₂ aryl. Most preferably, the alkyl, alkenyl or alkynyl is unsubstituted or substituted with one or more of fluorine or phenyl.

R² may be H, COOR⁹, CONR⁹R¹⁰, CN, NR⁹COR¹⁰, NR⁹R¹⁰, NR⁹SO₂R¹⁰, optionally substituted C₁-C₆ alkyl, optionally substituted mono or bicyclic 3 to 8 membered heterocycle, optionally substituted C₁-C₆ alkoxy, mono or bicyclic optionally substituted C₆-C₁₂ aryl or a mono or bicyclic optionally substituted 5 to 10 membered heteroaryl.

When R² is COOR⁹, CONR⁹R¹⁰, CN, NR⁹COR¹⁰, NR⁹R¹⁰ or NR⁹SO₂R¹⁰, R⁹ and R¹⁰ may independently be H or an optionally substituted C₁-C₆ alkyl, an optionally substituted mono or bicyclic C₃-C₆ cycloalkyl, mono or bicyclic optionally substituted C₆-C₁₂ aryl or a mono or bicyclic optionally substituted 5 to 10 membered heteroaryl. More preferably, R⁹ and R¹⁰ are independently H or an optionally substituted C₁-C₃ alkyl, a C₃-C₆ cycloalkyl, optionally substituted phenyl or an optionally substituted 5 or 6 membered heteroaryl. R⁹ and R¹⁰ may be H, optionally substituted methyl, cyclopropyl or optionally substituted pyrazolyl. When R⁹ or R¹⁰ is an optionally substituted alkyl or cycloalkyl, the alkyl or cycloalkyl may be substituted with a mono or bicyclic optionally substituted C₆-C₁₂ aryl or a mono or bicyclic optionally substituted 5 to 10 membered heteroaryl, preferably with an optionally substituted phenyl or a mono or bicyclic optionally substituted 5 or 6 membered heteroaryl, more preferably with phenyl or an optionally substituted pyrazolyl. When R⁹ or R¹⁰ is an optionally substituted heteroaryl, the heteroaryl may be substituted with a C₁-C₆ alkyl, more preferably a C₁-C₃ alkyl, and most preferably methyl. Accordingly, R² may be H, COOH, COOCH₃, CONH₂, CONHCH₃, CON(CH₃)₂, 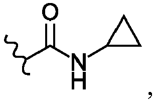 NHCOCH₃, NH₂, N(CH₃)₂, 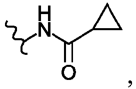 NHSO₂CH₃,

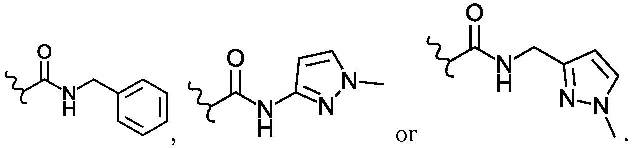

When R² is an optionally substituted C₁-C₆ alkyl or an optionally substituted C₁-C₆ alkoxy, R² may be an optionally substituted C₁-C₃ alkyl or an optionally C₁-C₃ alkoxy and is preferably CH₃ or OCH₃.

When R² is a mono or bicyclic optionally substituted 5 to 10 membered heteroaryl, the heteroaryl may be an optionally substituted 5 or 6 membered heteroaryl, and may be an optionally substituted oxazolyl. The heteroaryl may be unsubstituted.

In a preferred embodiment, X is CR³, Y is CR⁴ and Z is CR⁵.

In a still further embodiment, X is CR³, Y is CR⁴ and Z is N.

R³ may be H, halogen, optionally substituted C₁-C₆ alkyl, optionally substituted mono or bicyclic C₃-C₆ cycloalkyl, optionally substituted C₁-C₆ alkoxy, mono or bicyclic optionally substituted C₆-C₁₂ aryl, or mono or bicyclic optionally substituted 5 to 10 membered heteroaryl. Preferably, R³ is H, halogen, optionally substituted C₁-C₆ alkyl, mono or bicyclic optionally substituted C₆-C₁₂ aryl, optionally substituted mono or bicyclic C₃-C₆ cycloalkyl or mono or bicyclic optionally substituted 5 to 10 membered heteroaryl. More preferably, R³ is H, F, Br, cyclopropyl or an optionally substituted 5 or 6 membered heteroaryl. Most preferably, R³ is H.

When R³ is a halogen it may be F, Cl, Br or I, and more preferably is Br.

When R³ is optionally substituted C₁-C₆ alkyl it is preferably an optionally substituted C₁-C₃ alkyl, and more preferably is an optionally substituted methyl. Preferably, the C₁-C₆ alkyl is unsubstituted.

When R³ is optionally substituted C₃-C₆ cycloalkyl it is preferably an optionally substituted cyclopropyl. Preferably, the C₃-C₆ cycloalkyl is unsubstituted.

When R³ is an optionally substituted 5 to 10 membered heteroaryl, the heteroaryl is preferably an optionally substituted 5 or 6 membered heteroaryl, and more preferably an optionally substituted pyridinyl or pyrazolyl. The heteroaryl may be unsubstituted or substituted with optionally substituted C₁-C₆ alkyl, optionally substituted C₁-C₆ alkoxy or OH. More preferably, the heteroaryl is unsubstituted or substituted with one or more of C₁-C₆ alkyl, C₁-C₆ alkoxy or OH. Most preferably, the heteroaryl is unsubstituted or substituted with one or more of methyl, OCH₃ or OH.

R⁴ and R⁵ may each independently be selected from the group consisting of H and halogen. Preferably, R⁴ and R⁵ are H.

When R⁴ and/or R⁵ is a halogen it may be F, Cl, Br or I, and more preferably is F.

When L is NR⁸, R⁷ may be H, optionally substituted C₃-C₁₀ alkyl or optionally substituted mono or bicyclic C₃-C₆ cycloalkyl. More preferably, R⁷ may be an optionally substituted C₃-C₁₀ alkyl or an optionally substituted mono or bicyclic C₃-C₆ cycloalkyl. Even more preferably, R⁷ is an optionally substituted C₃-C₆ alkyl, and most preferably is an optionally substituted C₃-C₅ alkyl.

When L is NR⁸, R⁷ may be H, optionally substituted C₃-C₆ alkyl, optionally substituted C₂-C₆ alkenyl, optionally substituted C₂-C₆ alkynyl, mono or bicyclic optionally substituted C₆-C₁₂ aryl, mono or bicyclic optionally substituted 5 to 10 membered heteroaryl, optionally substituted mono or bicyclic C₃-C₆ cycloalkyl, or optionally substituted mono or bicyclic 3 to 8 membered heterocycle. More preferably, R⁷ is optionally substituted C₃-C₆ alkyl, optionally substituted C₂-C₆ alkenyl, optionally substituted C₂-C₆ alkynyl, optionally substituted phenyl, optionally substituted 5 or 6 membered heteroaryl, optionally substituted C₃-C₆ cycloalkyl, or optionally substituted 3 to 6 membered heterocycle.

When L is NR⁸ and R⁷ is an optionally substituted alkyl, an optionally substituted alkenyl or an optionally substituted alkynyl, the alkyl, alkenyl or alkynyl may be unsubstituted or substituted with one or more substituents selected from the group consisting of halogen, -OH, oxo, optionally substituted C₁-C₆ alkoxy, NR⁹R¹⁰, C(O)R⁹, OC(O)R⁹, COOR⁹, OP(O)(OH)₂ and NR⁹C(O)R¹⁰. R⁹ and R¹⁰ may each be H, an optionally substituted C₁-C₆ alkyl, an optionally substituted C₂-C₆ alkenyl or optionally substituted C₂-C₆ alkynyl. More preferably, the alkyl, alkenyl or alkynyl may be unsubstituted or substituted with one or more substituents selected from the group consisting of F, -OH, oxo, C₁-C₃ alkoxy, C(O)R⁹, OP(O)(OH)₂ and NHC(O)R¹⁰, wherein R⁹ and R¹⁰ are each H or a C₁-C₆ alkyl. Most preferably, the alkyl, alkenyl or alkynyl may be unsubstituted or substituted with one or more substituents selected from the group consisting of F, -OH, oxo, OCH₃, C(O)CH₃, OP(O)(OH)₂ and NHC(O)CH₃.

When R⁷ is an optionally substituted heteroaryl, optionally substituted cycloalkyl or optionally substituted heterocycle, the heteroaryl, cycloalkyl or heterocycle may be unsubstituted or substituted with one or more substituents selected from the group consisting of halogen, -OH, oxo, optionally substituted C₁₋₆ alkyl, optionally substituted C₁-C₆ alkenyl, optionally substituted C₁-C₆ alkynyl, optionally substituted C₁-C₆ alkoxy, NR⁹R¹⁰, C(O)R⁹, OC(O)R⁹, COOR⁹, OP(O)(OH)₂ and NR⁹C(O)R¹⁰. R⁹ and R¹⁰ may each be H, an optionally substituted C₁-C₆ alkyl, an optionally substituted C₂-C₆ alkenyl or optionally substituted C₂-C₆ alkynyl. When the heteroaryl, cycloalkyl or heterocycle is substituted, either directly or indirectly, with an alkyl, alkenyl and/or alkynyl, the or each alkyl, alkenyl or alkynyl may be unsubstituted or substituted with one or more substituents selected from the group consisting of halogen, -OH and oxo. Preferably, the heteroaryl, cycloalkyl or heterocycle is unsubstituted or substituted with one or more substituents selected from the group consisting of F, -OH, oxo, optionally substituted C₁₋₃ alkyl, optionally substituted C₁-C₃ alkenyl, optionally substituted C₁-C₃ alkynyl, C₁-C₃ alkoxy, C(O)R⁹, OP(O)(OH)₂ and NHC(O)R¹⁰, wherein R⁹ and R¹⁰ are each H or a C₁-C₆ alkyl. Most preferably, the heteroaryl, cycloalkyl or heterocycle may be unsubstituted or substituted with one or more substituents selected from the group consisting of F, -OH, oxo, CH₃, CH₂OH, OCH₃, C(O)CH₃, OP(O)(OH)₂ and NHC(O)CH₃.

When R⁷ is an optionally substituted aryl, the aryl may be unsubstituted or substituted with one or more substituents selected from the group consisting of halogen, -OH, optionally substituted C₁₋₆ alkyl, optionally substituted C₁-C₆ alkenyl, optionally substituted C₁-C₆ alkynyl, optionally substituted C₁-C₆ alkoxy, NR⁹R¹⁰, C(O)R⁹, OC(O)R⁹, COOR⁹, OP(O)(OH)₂ and NR⁹C(O)R¹⁰. R⁹ and R¹⁰ may each be H, an optionally substituted C₁-C₆ alkyl, an optionally substituted C₂-C₆ alkenyl or optionally substituted C₂-C₆ alkynyl. When the aryl is substituted, either directly or indirectly, with an alkyl, alkenyl and/or alkynyl, the or each alkyl, alkenyl or alkynyl may be unsubstituted or substituted with one or more substituents selected from the group consisting of halogen, -OH and oxo. Preferably, the aryl is unsubstituted or substituted with one or more substituents selected from the group consisting of F, -OH, optionally substituted C₁₋₃ alkyl, optionally substituted C₁-C₃ alkenyl, optionally substituted C₁-C₃ alkynyl, C₁-C₃ alkoxy, C(O)R⁹, OP(O)(OH)₂ and NHC(O)R¹⁰, wherein R⁹ and R¹⁰ are each H or a C₁-C₆ alkyl. Most preferably, the aryl may be unsubstituted or substituted with one or more substituents selected from the group consisting of F, -OH, CH₃, CH₂OH, OCH₃, C(O)CH₃, OP(O)(OH)₂ and NHC(O)CH₃.

In some embodiments, R⁷ may be butyl, pentyl, cyclohexyl,

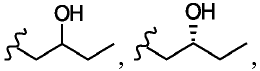

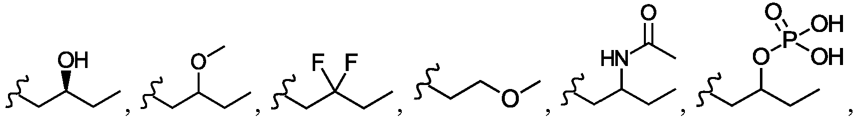

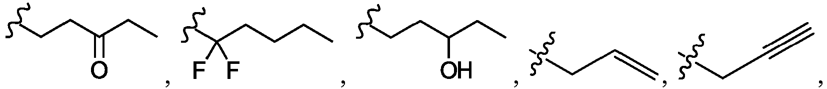

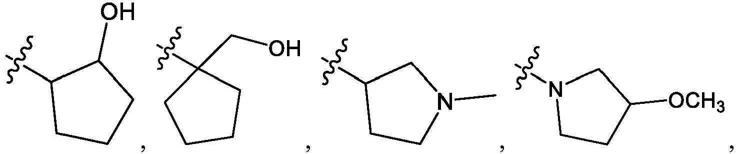

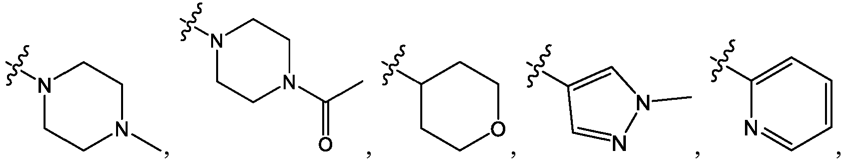

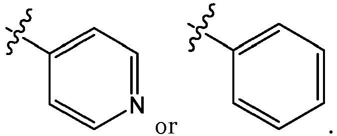

In some embodiments, L is absent. In alternative embodiments, L is NR⁸. R⁸ may be H or an optionally substituted C₁-C₆ alkyl. More preferably, R⁸ is H.

Compounds of formula (I) may include one or more stereogenic centers and so may exist as optical isomers, such as enantiomers and diastereomers. All such isomers and mixtures thereof are included within the scope of the present invention.

Stereogenic centres may arise within any of the groups R and R¹-R⁹.

It will be understood that the above compounds may exist as enantiomers and as diastereoisomeric pairs. These isomers also represent further embodiments of the invention.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

Mixtures of stereoisomers may be separated by conventional techniques known to those skilled in the art; see, for example, "Stereochemistry of Organic Compounds" by E. L. Eliel and S. H. Wilen (Wiley, New York, 1994).

The structure (-L¹-)ₐ-L²- may be referred to as "the linker".

L¹ may be absent or may be:

-A-W-D-

wherein:
A is absent or is selected from the group consisting of -L³-, -X⁴L³-, -L³X⁴-, -C(O)X⁴, -L³C(O)X⁴, 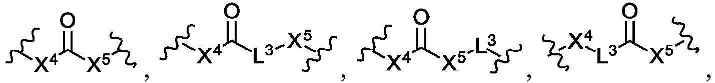 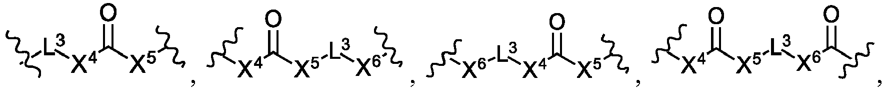 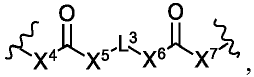 -X⁴L⁴-, -L⁴X⁴-, -X⁴L³L⁴-, -L⁴L³X⁴-, -X⁴L³L⁴L⁵-, -L⁵L⁴L³X⁴-, - L³L⁴-, -L⁴L³-, -L³X⁴L⁴-, -L⁴X⁴L³-, -L³L⁴L⁶-, -L³X⁴L⁴X⁵L⁶-, 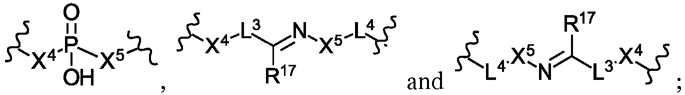 W is either absent or is selected from the group consisting of -L⁷NH-, -L³L⁷NH-, -L⁷NHC(O)-, -L³L⁷NHC(O)-, -L⁷L⁸NH-, -L³L⁷L⁸NH-, -L⁷L⁸NHC(O)-, and - L³L⁷L⁸NHC(O)-;
D is either absent or has formula -(D¹)_{q}- or -(D¹)_{q}C(O)-, wherein (D¹)_{q} is either linear or cyclic;
the or each L³ and L⁶ are each independently an optionally substituted C₁-C₂₅ alkylene or an optionally substituted C₂-C₂₅ alkylyne;
L⁴ and L⁵ are each independently selected from the group consisting of an optionally substituted mono or bicyclic C₆-C₁₂ aryl; an optionally mono or bicyclic 5 to 10 membered heteroaryl; an optionally C₃-C₁₂ cycloalkyl; and an optionally mono or bicyclic 3 to 12 membered heterocycle;
L⁷ and L⁸ are each independently an optionally substituted mono or bicyclic C₆-C₁₂ aryl; or an optionally substituted mono or bicyclic 5 to 10 membered heteroaryl, wherein the aryl or heteroaryl is optionally further substituted with at least one -OR¹⁸ group;
the or each of X⁴, X⁵, X⁶ and X⁷ is independently O, S or NR¹⁶;
R¹⁶ is selected from the group consisting of H, halogen, CN, hydroxyl, COOH, CONR⁹R¹⁰, NR⁹R¹⁰, NHCOR⁹, optionally substituted C₁-C₆ alkyl, C₁-C₃ polyfluoroalkyl, optionally substituted C₁-C₆ alkylsulfonyl, optionally substituted mono or bicyclic C₃-C₆ cycloalkyl, optionally substituted C₂-C₆ alkenyl, optionally substituted C₂-C₆ alkynyl, optionally substituted C₁-C₆ alkoxy, optionally substituted C₁-C₆ alkoxycarbonyl, mono or bicyclic optionally substituted C₅-C₁₀ aryl, mono or bicyclic optionally substituted 5 to 10 membered heteroaryl, optionally substituted mono or bicyclic 3 to 8 membered heterocycle, optionally substituted aryloxy, optionally substituted heteroaryloxy, and optionally substituted heterocyclyloxy;
R¹⁷ is hydrogen or an optionally substituted C₁₋₆ alkyl;
R¹⁸ is an optionally substituted C₃-C₆ cycloalkyl, or an optionally substituted mono or bicyclic 3 to 8 membered heterocycle;
each D¹ independently has general formula 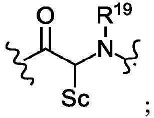
Sc is a side chain of a natural or unnatural amino acid and R¹⁹ is H, or Sc and R¹⁹ together with the atoms to which they are attached form a ring; and
q is an integer between 2 and 20.

L² may be absent or may be:

-G-(S-)_{z}

wherein, G is either absent or is (-G¹)ₐ-G²-(G³-)_{z},
wherein, the or each G¹ is independently either absent or selected from the group consisting of -L³-, -(X⁴L³)ₚ-, -(L³X⁴)ₚ-, -L⁴-, -X⁴-, -X⁸-, -X⁴C(O)-, -C(O)X⁴-, 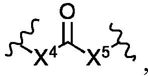 -L3X4C(O)-, -L3C(0)X4, 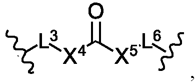 -L⁹-, -L⁹L3⁻, -L⁹L³C(O)⁻, - C(O)L³-, -C(O)L⁹-, -C(O)L³X⁴L⁶-, -C(O)L³X⁴C(O)L⁶-,
   -C(O)L⁹L³-, -C(O)L³C(O)-, -C(O)L⁹C(O)-, -C(O)L⁹L³C(O)-, a poly(ethylene glycol) (PEG) chain of between 1 and 25 units and a cyclodextrin;
G² is either absent or is selected from the group consisting of 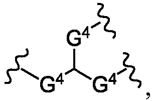 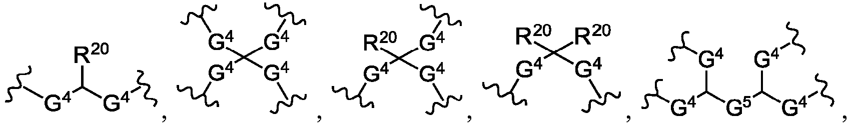 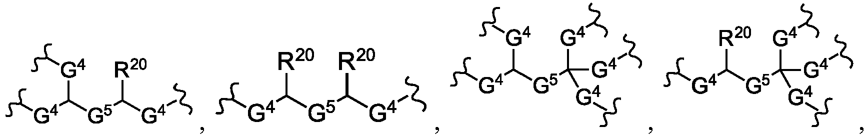 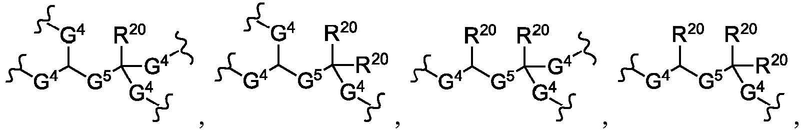 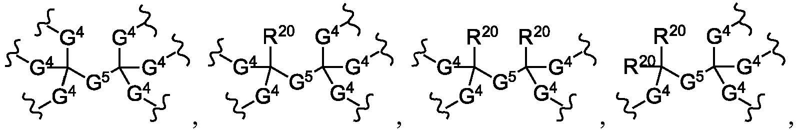 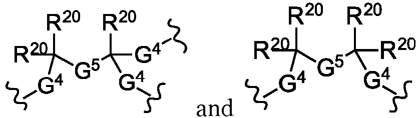 wherein a wavy line indicates either the attachment of G² to G¹ or, in examples where G¹ is absent, to L¹, or the attachment of the G² to G³ or, in examples where G³ is absent, to S, and each G², in examples where it is present, is attached to at least one G¹ or, in examples where G¹ is absent, to at least one group L¹, and each G², in examples where it is present, is attached to at least one G³ or, in examples where G³ is absent, to at least one group S;
the or each G³ is independently either absent or selected from the group consisting of - L3-, -(X⁴L³)ₚ-, -(L³X⁴)ₚ-, , -L⁴-, -X⁴-, -X⁸-, -X⁴C(O)-, -C(O)X⁴-, 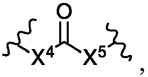 -L³X⁴C(O)-, -L³C(O)X⁴, 1-L³X⁴C(O)L⁶-, -L³C(O)X⁴L⁶-, 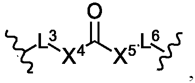 -L⁹-, -X⁴L⁹-, -L⁹L³-, - L⁹L³C(O)-, -C(O)L³-, -C(O)L⁹-, -C(O)L³X⁴L⁶-, -C(O)L³X⁴C(O)⁶-, -C(O)L⁹L³-, - C(O)L³C(O)-, -C(O)L⁹C(O)-, -C(O)L⁹L³C(O)-, a poly(ethylene glycol) (PEG) chain of between 1 and 25 units and a cyclodextrin;
the or each G⁴ is independently either absent or selected from the group consisting of -L³-, -(X⁴L³)ₚ-, -(L³X⁴)ₚ-, -X⁴-, -X⁸-, -X⁴C(O)-, -C(O)X⁴-, 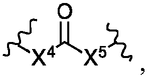 -L³X⁴C(O)-, -C(O)X⁴L³-,-L³C(O)X⁴-, -X⁴C(O)L³-, -X⁴L³C(O)X⁵-, -X⁴C(O)L³X⁵-, -L³X⁴ L⁶C(O)X⁵-, -X⁴C(O)L³X⁵L³- 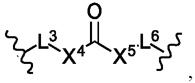 -L⁹-, -L⁹L³-, -L⁹L³C(O)-, -C(O)L³-, -C(O)L⁹-, -C(O)L³X⁴L⁶-, -C(O)L³X⁴C(O)L⁶-, -C(O)L⁹L³, -C(O)L³C(O)-, -C(O)L⁹C(O)-, -C(O)L⁹L³C(O)-, a poly(ethylene glycol) (PEG) chain of between 1 and 25 units and a cyclodextrin; G⁵ is either -L³-, -(X⁴L³)ₚ-, -(L³X⁴)ₚ-, -X⁴-, -X⁸-, -X⁴C(O)-, -C(O)X₄-, 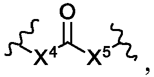 -L³X⁴C(O)-, -L³C(O)X⁴, -L³X⁴C(O)L⁶-, -L³C(O)X⁴L⁶-, 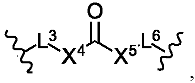 -L⁹-, -X⁴L⁹-, - L⁹L³-, -L⁹L³C(O)-, -C(O)L³-, -C(O)L⁹-, -C(O)L³X⁴L⁶-, -C(O)L³X⁴C(O)L⁶-, -C(O)L⁹L³-, - C(O)L³C(O)-, -C(O)L⁹C(O)-,
-C(O)L⁹L³C(O)-, a poly(ethylene glycol) (PEG) chain of between 1 and 25 units and a cyclodextrin;
S is either absent or is selected from the group consisting of -X⁴-, -X⁴-, -X⁸-, -C(X⁹)-, -X⁴C(X⁹)-, -X⁴C(X⁹)L³-, -X⁴C(X⁹)L³C(O)-, -X⁸L³-, -X⁴X⁸L³-, X⁸L³C(O)-, -L³-, -L⁴-, -L⁴L³-, -L⁴C(O)-, -C(O)L⁴C(O)-, -L³C(O)L⁴C(O)-, -L⁴L³L⁵-, L⁴L³L⁵C(O)-, 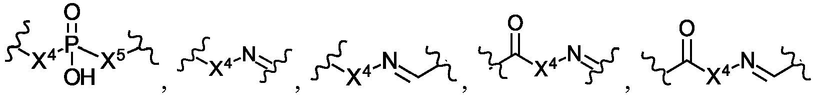 and 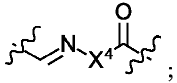
L³ to L⁸ and X⁴ to X⁷ are as defined above,
L⁹ is a poly(ethylene glycol) (PEG) chain between 1 and 25 units long;
X⁸ is -S(O)- or -SO₂-;
X⁹ is O or S;
R²⁰ is an optionally substituted C₁-C₆ alkyl, an optionally substituted C₂-C₆ alkenyl, an optionally substituted C₂-C₆ alkynyl, -L⁹H, -C(O)L³H, -C(O)L⁹H, -X⁴L³H, -X⁴L⁹H, -X⁴C(O)L³H, -X⁴C(O)L⁹H, -C(O)X⁴L³H or -C(O)X⁴L⁹H; and
p is an integer between 1 and 25.
a may be 1, 2, 3, 4 or 5. Preferably, a is an integer between 1 and 3.
z may be 1, 2, 3, 4 or 5. Preferably, z is an integer between 1 and 3.
-L⁹- may be

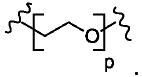

Preferably, at least one of L¹ and L² is present.

Preferably, 3 or less of A, W, D, G and S are absent, and more preferably 2 or less or 1 or less of A, W, D, G and S are absent. In some examples, none of A, W, D, G and S are absent.

A may be -L³-. L³ may be an optionally substituted C₁-C₆ alkylene. Preferably, L³ is an optionally substituted C₁-C₂ alkylene or an optionally substituted C₁ alkylene.

A may be -L³X⁴-. L³ may be an optionally substituted C₁-C₆ alkylene, and is preferably -CH₂CH₂- or -CH₂CH₂CH₂-. Accordingly, A may be -CH₂CH₂O-, -CH₂CH₂NH-, -CH₂CH₂S-, -CH₂CH₂CH₂O-, -CH₂CH₂CH₂NH- or -CH₂CH₂CH₂S-.

A may be -C(O)X⁴ or -L³C(O)X⁴. X⁴ may be O. L³ may be an optionally substituted C₁-C₆ alkylene, and is preferably a C₁-C₃ alkylene, and most preferably is -CH₂-. Accordingly A may be -C(O)O- or -CH₂C(O)O-.

A may be 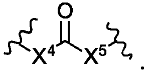 X⁴ may be -O- or -NR¹⁶-. X₅ may be -O- or NR¹⁶-.

Accordingly, A may be 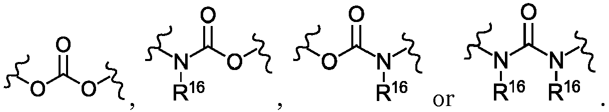 R₁₆ may be an optionally substituted C₁-C₆ alkyl or hydrogen. The optionally substituted C₁-C₆ alkyl may be substituted with an optionally substituted C₁-C₆ alkoxy, which may be substituted with an -OH. Accordingly, R¹⁶ may be methyl or -CH₂CH₂OCH₂CH₂OH.

Accordingly, A may be

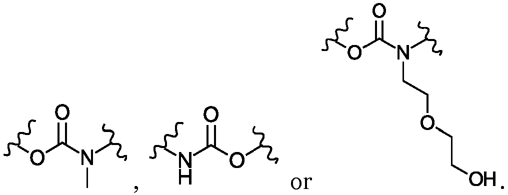

A may be 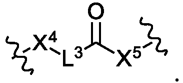 X⁴ may be -O-, -S- or -NH-. X₅ may be -O- or -NR¹⁶-.

Accordingly, A may be 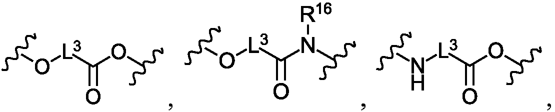 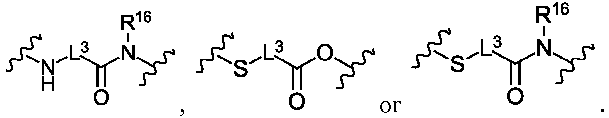 L³ may be an optionally substituted C₁-C₁₀ alkylene, and is preferably an optionally substituted C₁-C₆ alkylene.

A may be 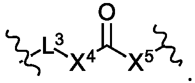 X⁴ may be -O- or -NR¹⁶-. X₅ may be -O- or NR¹⁶-. L³ may be an optionally substituted C₁-C₆ alkylene. Accordingly, A may be

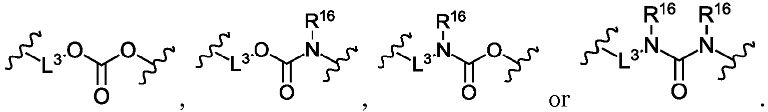

Preferably, L³ is an optionally substituted C₁-C₂ alkylene or an optionally substituted C₁ alkylene.

A may be 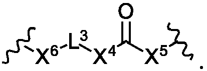 X⁶ may be -O-, -S- or -NH-. X⁴ may be -O- or -NR¹⁶-. X₅ may be -O- or -NR¹⁶-. A may be

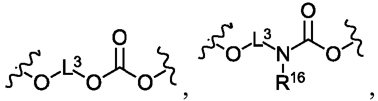

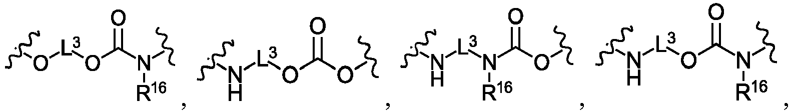

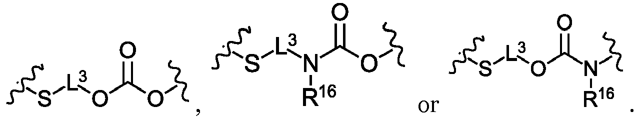

A may be 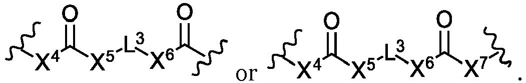 X⁴ may be -O-. X⁵ may be -O- or NR¹⁶-. L³ may an optionally substituted C₁-C₆ alkylene, and is preferably - CH₂CH₂-. X⁶ may be -O- or NR¹⁶-. X⁷ may be -O-. Accordingly, A may be: 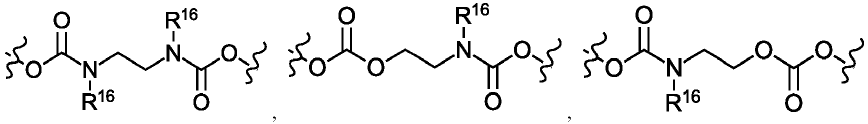 or

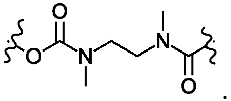

A may be 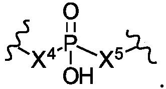 Preferably, X⁴ is -O-. Preferably, X₅ is -O-.

A may be -X⁴L³L4- or -X⁴L³L⁴L⁵-. X⁴ may be -O-. L³ may be an optionally substituted C₁-C₆ alkylene, and is preferably a C₁-C₂ alkylene, and more preferably is -CH₂-. L⁴ may be an optionally substituted 3 to 12 membered heterocycle, preferably L⁴ may is an optionally substituted 3 to 8 membered heterocycle, and most preferably L⁴ is an optionally substituted 5 or 6 membered heterocycle. L⁴ may be 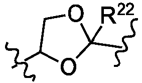 or 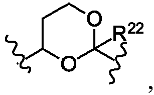 where R²² may be a hydrogen, a C₁-C₆ alkyl or a mono or bicyclic C₆-C₁₂ aryl. Accordingly, L⁴ may be 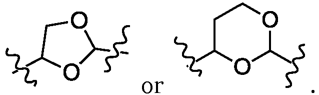 L⁵ may be an optionally substituted mono or bicyclic C₆-C₁₂ aryl. Preferably, L⁵ is an optionally substituted phenyl, and in some examples is an unsubstituted phenyl. Accordingly, A may be

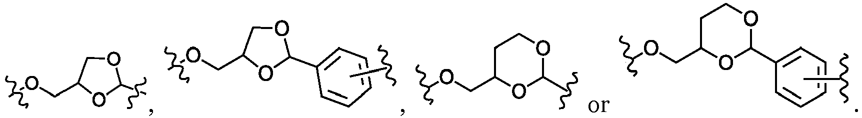

A may be -L³X⁴L⁴X⁵L⁶-. L³ and L⁶ may independently be an optionally substituted C₁-C₆ alkylene, and are preferably independently a C₁-C₂ alkylene. L³ may be -CH₂CH₂-. L⁶ may be -CH₂-. X⁴ may be -O-. X⁵ may be -O-. L⁴ may be an optionally substituted 3 to 12 membered heterocycle. L⁴ is preferably an optionally substituted 6 to 12 membered bicyclic heterocycle, and more preferably an optionally substituted 6 to 12 membered spirocyclic heterocycle. Accordingly, L⁴ may be 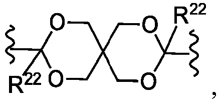 where R²² may be a hydrogen, a C₁-C₆ alkyl or a mono or bicyclic C₆-C₁₂ aryl. Accordingly, L⁴ may be 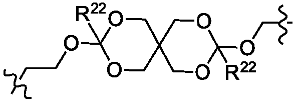 and is preferably 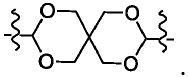 Accordingly, A may be

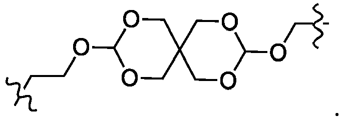

A may be 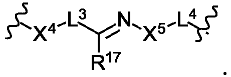 X⁴ is preferably -O-. L³ may be an optionally substituted C₁-C₆ alkylene, and preferably is a C₁-C₂ alkylene, and more preferably is - CH₂-. R¹⁷ may be an optionally substituted C₁₋₆ alkyl, and is preferably a C₁₋₃ alkyl and more preferably is a methyl. X₅ is preferably -NH- or -O-. L⁴ may be an optionally substituted mono or bicyclic C₆-C₁₂ aryl. Preferably, L⁴ is an optionally substituted phenyl, and in some examples is an unsubstituted phenyl. Accordingly, A may be

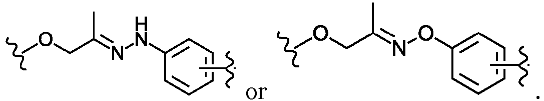

As explained above, W is -L⁷NH-, -L³L⁷NH-, -L⁷NHC(O)-, -L³L⁷NHC(O)-, -L⁷L⁸NH-, -L³L⁷L⁸NH-, -L⁷L⁸NHC(O)-, or -L³L⁷L⁸NHC(O)-.

L³ may an optionally substituted C₁-C₆ alkylene or C₁-C₆ alkylyne, preferably C₁-C₃ alkylene or C₁-C₃ alkylyne, and most preferably is -CH₂- or -CH₂CHCH-.

Preferably, L⁷ and L⁸ are each independently a mono or bicyclic C₆-C₁₂ aryl; or a mono or bicyclic 5 to 10 membered heteroaryl, wherein the aryl or heteroaryl is optionally substituted with one -OR¹⁸ group.

L⁷ may be a phenyl, napthalenyl or a 2H-chromen-2-only group, wherein each group may be further substituted with one -OR¹⁸ group.

L⁸ is preferably a phenyl.

R¹⁸ is preferably an optionally substituted mono or bicyclic 3 to 8 membered heterocycle. More preferably, R¹⁸ is an optionally substituted 6 membered heterocycle, and most preferably an optionally substituted tetrahydropyranyl. Preferably, the heterocycle is substituted with between 1 and 9 substituents, more preferably between 2 and 7 or between 3 and 5 substituents, and most preferably with 4 substituents. The substituents may be selected from C₁-C₆ alkoxy, OH and COOH. Preferably, the C₁-C₆ alkoxy is a C₁-C₄ alkoxy, more preferably a C₁-C₂ alkoxy, and most preferably -CH₂OH. Preferably, the heterocycle is substituted with between 1 and 9 OH groups, more preferably between 2 and 5 OH groups, and most preferably with 3 OH groups. Preferably, the heterocycle is substituted with between 1 and 9 C₁-C₆ alkoxy and/or COOH groups, more preferably between 1 and 5 C₁-C₆ alkoxy and/or COOH groups, and most preferably with 1 C₁-C₆ alkoxy or COOH group.

R¹⁸ may be 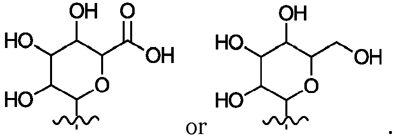 More preferably, R¹⁸ is

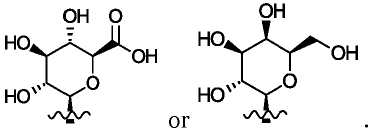

Accordingly, W may be:

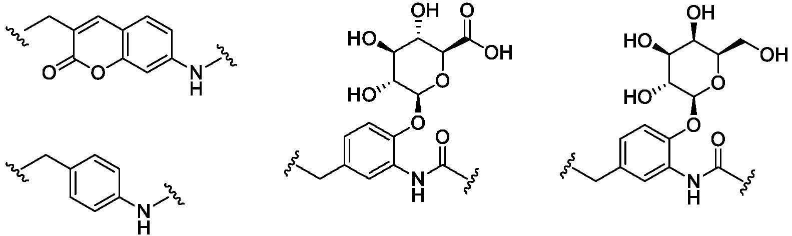

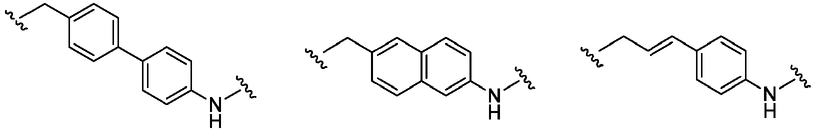

D¹ may have general formula

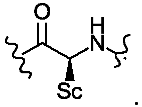

Sc may be H, an optionally substituted C₁-C₆ alkyl, an optionally substituted mono or bicyclic C₆-C₁₂ aryl, an optionally mono or bicyclic 5 to 10 membered heteroaryl, an optionally C₃-C₁₂ cycloalkyl, or an optionally mono or bicyclic 3 to 12 membered heterocycle. Preferably, Sc is H, an optionally substituted C₁-C₆ alkyl, a mono or bicyclic C₆-C₁₂ aryl, a mono or bicyclic 5 to 10 membered heteroaryl, a C₃-C₁₂ cycloalkyl, or a mono or bicyclic 3 to 12 membered heterocycle.

In examples where Sc is an optionally substituted C₁-C₆ alkyl, the alkyl may be substituted with at least one of NR⁹R¹⁰, NHC(NH)NH₂, OH, COOH, CONR⁹R¹⁰, SeH, SR⁹, an optionally substituted C₆-C₁₂ aryl, an optionally substituted 5 to 10 membered heteroaryl, an optionally substituted C₃-C₆ cycloalkyl or an optionally substituted 3 to 8 membered heterocycle. When the alkyl is substituted with NR⁹R¹⁰ then R¹⁰ may be H. R⁹ may also be H. Alternatively, R⁹ may be C(O)NH₂. Accordingly, the alkyl may be substituted with NHC(O)NH₂. When the alkyl is substituted with CONR⁹R¹⁰ then R¹⁰ may be H. R⁹ may also be H. Alternatively, R⁹ may be C(O)NH₂. When the alkyl is substituted with SR⁹, R⁹ may be H or a C₁-C₆ alkyl, preferably R⁹ is H or methyl. When the alkyl is substituted with an optionally substituted C₆-C₁₂ aryl, the optionally substituted C₆-C₁₂ aryl is preferably optionally substituted phenyl. The phenyl may optionally be substituted with an -OH. When the alkyl is substituted with an optionally substituted 5 to 10 membered heteroaryl, the optionally substituted 5 to 10 membered heteroaryl is preferably imidazolyl or 1H-indolyl.

In examples where Sc is NC(O)R⁹, R⁹ may be a C₁-C₆ alkyl, and preferably is methyl.

Accordingly, in some examples, Sc is H or a C₁-C₆ alkyl optionally substituted with at least one substituent selected from the group consisting of NH₂, NHC(NH)NH₂, OH, COOH, CONR⁹H, SeH, SH, SCH₃, a phenyl optionally substituted with an OH, imidazolyl and 1H-indolyl.

Preferably, Sc is a C₁-C₆ alkyl optionally substituted with NHC(O)NH₂ or COOH. More preferably, Sc is methyl, isopropyl, -CH₂CH₂CH₂NHC(O)NH² or -CH₂CH₂COOH.

Alternatively, D¹ may be 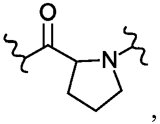 or more preferably 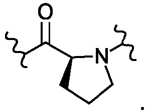 q may be an integer between 1 and 10, more preferably between 2 and 7, and most preferably between 3 and 5.

Accordingly, D may be:

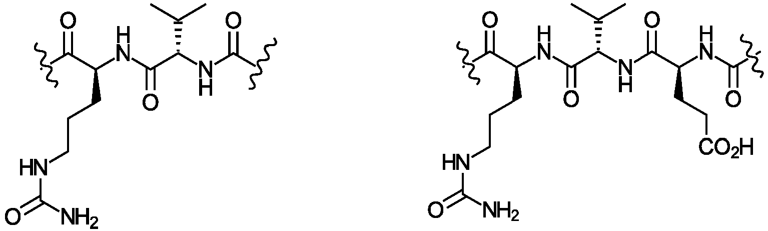

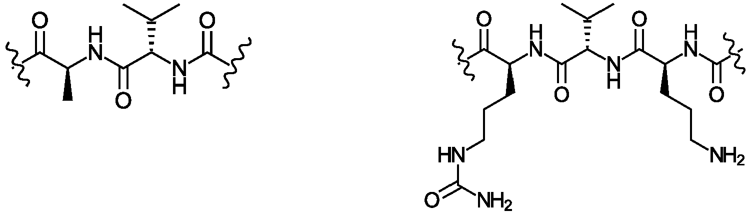

G¹ and G³ may each independently be -L³-, -(X⁴L³)ₚ- or -(L³X⁴-)ₚ. p may be 1 or 2. L³ may be an optionally substituted C₁-C₁₅ alkylene, more preferably an optionally substituted C₁-C₁₀ alkylene, and most preferably optionally substituted C₁-C₆ alkylene. L³ may be substituted with one or more optionally substituted C₁-C₆ alkyl. Preferably, the C₁-C₆ alkyl is unsubstituted. Accordingly, G¹ and G³ may each independently be substituted with one or more methyl groups. G¹ and G³ may each independently be -CH₂-, -(CH₂)₂-, -(CH₂)₃-, -(CH₂)₄-, -(CH₂)₅-, -CH₂C(Me)H-, CH₂CMe₂-, -CH₂CMe₂S- -CH₂O-, -CH₂CH₂O-, -CH₂CH₂OCH₂CH₂O- or -(CH₂)₅NH-. In examples where G² and G³ are absent, G may be -CH₂-, -(CH₂)₂-, -(CH₂)₃-, -(CH₂)₄-, -(CH₂)₅-, -CH₂C(Me)H-, CH₂CMe₂-, -CH₂CMe₂S- or -(CH₂)₅NH-. In some examples where G¹ and G³ are present, but G² is absent, G may be -CH₂OCH₂CH₂OCH₂CH₂O-.

G¹ and G³ may each independently be 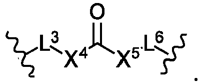 L³ and L⁶ may independently be an optionally substituted C₁-C₁₀ alkylene, and more preferably an optionally substituted C₁-C₆ alkylene. X⁴ may be NH. X⁵ may be NH. Accordingly, G¹ and/or G³ may be 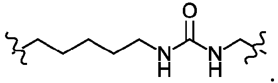 In examples where G² and G³ are absent, G may be

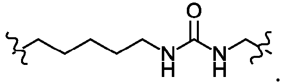

G¹ and G³ may each independently be may be -L³X⁴C(O)- or -C(O)L³X⁴C(O)L⁶-. Preferably, L³ is an optionally substituted C₁-C₁₅ alkylene, more preferably an optionally substituted C₁-C₁₀ alkylene, and most preferably an optionally substituted C₁-C₆ alkylene. The alkylene may be substituted with an optionally substituted C₁-C₆ alkyl or -COOH. The alkyl may be substituted with NH₂. Preferably X⁴ is -NH-. L⁶ is preferably, an optionally substituted C₁-C₁₅ alkylene, more preferably an optionally substituted C₁-C₁₀ alkylene, and most preferably an optionally substituted C₁-C₆ alkylene. The alkylene may be substituted with an optionally substituted C₁-C₆ alkyl or -COOH. The alkyl may be substituted with NH₂. Alternatively, the alkylene may be unsubstituted. Accordingly, G¹ and G³ may each independently be-(CH₂)₅NHC(O)-, 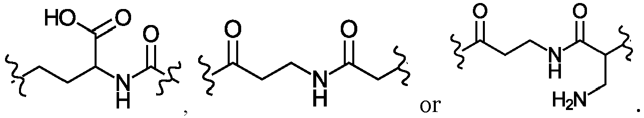 More preferably, G¹ and G³ may each independently be -(CH₂)₅NHC(O)-, 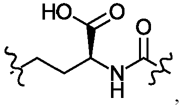 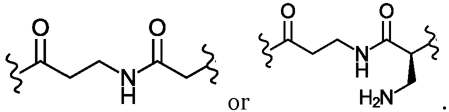 In examples where G² and G³ are absent, G may be-(CH₂)₅NHC(O)-,

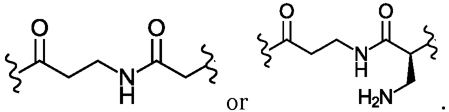

G¹ and G³ may each independently be an optionally substituted C₃-C₆ cycloalkyl, a mono or bicyclic optionally substituted C₆-C₁₂ aryl, a mono or bicyclic optionally substituted 5 to 10 membered heteroaryl or a mono or bicyclic optionally substituted 5 to 10 membered heterocycle.

In some examples G may be -L³X⁴C(O)-. G² may be absent. G³ may be -L⁴-.

Accordingly, G may be 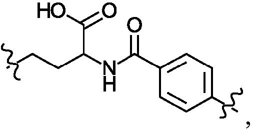 and is preferably

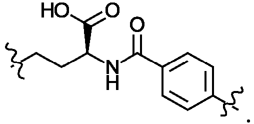

G¹ and G³ may each independently be-O-, -S-, -NR⁹-, -S(O)-, -SO₂-, -C(O)L³-,-C(O)L³C(O)-, -OC(O)-, -C(O)O-, -OC(O)O-, -L³OC(O) -, -L³C(O)O-, -(OL³)ₚ-, -(L³O)ₚ-, - C(O)NR⁹-, -NR⁹C(O)O- or -NR⁹C(O)NR¹⁰-. In some examples, G¹ and G³ may each independently be -C(O)L³- or -C(O)L³C(O)- where L³ is an optionally substituted C₁-C₆ alkylene, and more preferably an optionally substituted C₄-C₅ alkylene. In examples where G² and G³ are absent, G may be -C(O)L³- or -C(O)L³C(O)- where L³ is an optionally substituted C₁-C₆ alkylene, and more preferably an optionally substituted C₄-C₅ alkylene.

G¹ and/or G³ may be -L⁴-. Accordingly, G¹ and/or G³ may be an optionally substituted mono or bicyclic C₆-C₁₂ aryl. G¹ and/or G³ may be an optionally substituted phenyl. In examples where G² and G³ are absent, G may be

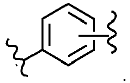

G¹ and/or G³ may be a poly(ethylene glycol) (PEG) chain of between 1 and 25 units. The PEG chain may be a cyclic PEG chain, branched PEG chain or a linear PEG chain.

G¹ and/or G³ may be a cyclodextrin. The cyclodextrin may be α, β or γ cyclodextrin.

G¹ and/or G³ may be -C(O)L⁹L³-, -L⁹L³C(O)-, -C(O)L⁹L³C(O)- or -L⁹L³-. L³ may be an optionally substituted C₁-C₆ alkylene, and is more preferably methylene or ethylene. G¹ and/or G³ may be 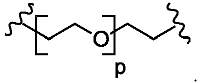 p may be an integer between 1 and 15, more preferably between 2 and 10 or between 3 and 5. In examples where G² and G³ are

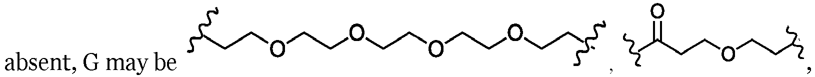

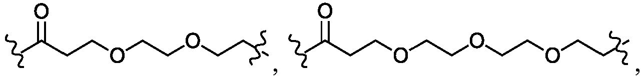

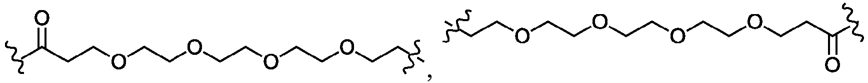

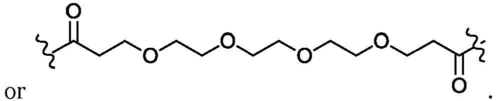

G² may be 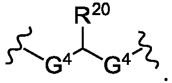 Each G⁴ may be absent, -L³- or -X⁴C(O)-. In one example, one G⁴ is absent and one G⁴ is -X⁴C(O)-. X⁴ may be -NH-. Accordingly, G² may be 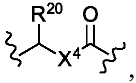 preferably is 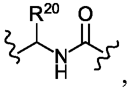 and more preferably is

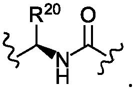

Alternatively, G² may be 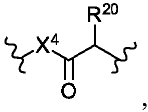 preferably is 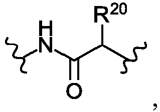 and more preferably is 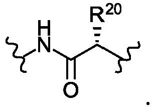 In one example, R²⁰ may be -L⁹H, -C(O)L⁹H, -X⁴L⁹H, - X⁴C(O)L⁹H or -C(O)X⁴L⁹H. Preferably R²⁰ is -C(O)X⁴L⁹H. X⁴ may be -NH-. L⁹- may be 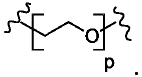 p may be an integer between 2 and 10, more preferably between 3 and 5, and most preferably 4. Accordingly, G² may be 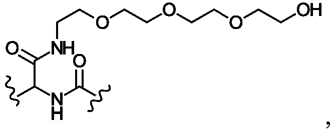 and more preferably is

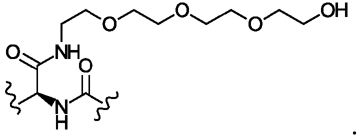

G¹ and G³ may each independently be an optionally substituted C₁-C₁₀ alkylene, more preferably an optionally substituted C₁-C₆ alkylene. G¹ may be ethylene. G³ may be pentylene. Accordingly, G may be 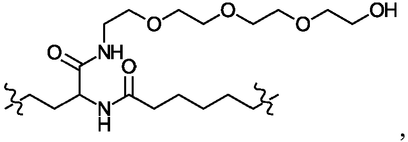 and preferably is

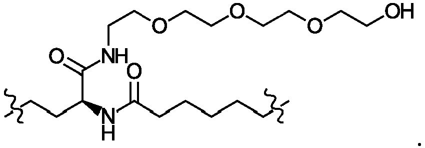

In an alternative example, R²⁰ may be an optionally substituted C₁-C₆ alkyl, an optionally substituted C₂-C₆ alkenyl or an optionally substituted C₂-C₆ alkynyl. More preferably, R²⁰ is an optionally substituted C₁-C₃ alkyl, and most preferably is optionally substituted methyl. Preferably, the alkyl, alkenyl or C₂-C₆ alkynyl is substituted with - NR⁹R¹⁰. Preferably, R⁹ and R¹⁰ are H. Accordingly, G² may be 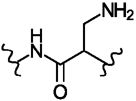 and more preferably is

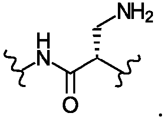

G¹ may be an optionally substituted C₁-C₁₀ alkylene, more preferably an optionally substituted C₁-C₆ alkylene. G¹ may be ethylene. G³ may be absent. Accordingly, G may be 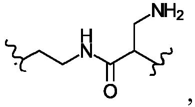 and preferably is

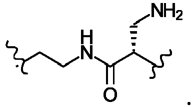

In an alternative example, G² may be 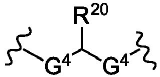 and one G⁴ is absent if -L³-. -L³-may be an optionally substituted C₁-C₁₂ alkylene, more preferably an optionally substituted C₁-C₆ alkylene, and most preferably methylene or ethylene. Accordingly, G² may be 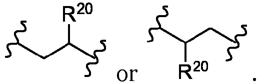 R²⁰ may be an optionally substituted C₁-C₆ alkyl, and in some examples is methyl. Accordingly, G² may be 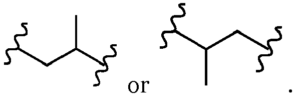 G¹ and G³ may be absent. Accordingly, in some examples, G may be 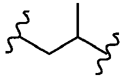 or

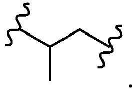

In some examples, G² may be 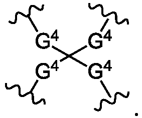 Each G⁴ may independently be absent, or selected from the group consisting of -L³X⁴C(O)-, -C(O)X⁴L³-,-L³C(O)X⁴, -X⁴C(O)L³-, -X⁴L³C(O)X₅-, -X⁴C(O)L³X⁵-, -L³X⁴L⁶C(O)X⁵- and -X⁴C(O)L³X⁵L³-. At least one G⁴ group may be -X⁴C(O)L³-. X⁴ may be -NH-. -L³- may be an optionally substituted C₁-C₁₂ alkylene, more preferably an optionally substituted C₁-C₆ alkylene, and most preferably methylene or ethylene. At least one G⁴ group may be -L³X⁴L⁶C(O)X⁵-. Preferably, at least two or at least three G⁴ groups are -L³X⁴L⁶C(O)X⁵-. Each X⁴ may be -NH-. Each X^{%} may be -NH-. Each -L³- and -L⁶- may independently be an optionally substituted C₁-C₁₂ alkylene, more preferably an optionally substituted C₁-C₆ alkylene, and most preferably methylene or ethylene. Accordingly, G² may be:

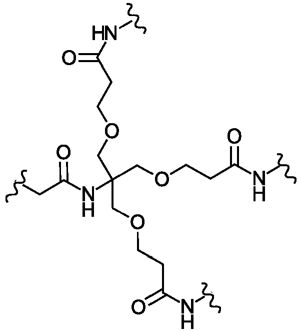

Each G¹ and G³ may independently be absent, -L³-, -L⁹-, -X⁴L⁹-, -L⁹L³-,-L³X⁴C(O)-, - L³C(O)X⁴, -L³X⁴C(O)L⁶- or -L³C(O)X⁴L⁶-.

The G group may comprise at least one G¹ group. Accordingly, a may be 1, 2 or 3. Preferably, a is 1. G¹ may be -L⁹- or -X⁴L⁹-. Preferably, G¹ is -X⁴L⁹-. Preferably, X⁴ is - O-. Preferably, -L⁹- is 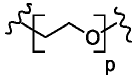 and p is an integer between 1 and 10, more preferably between 2 and 5, most preferably p is 3. Accordingly, G¹ may be

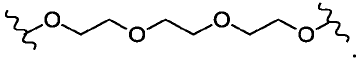

The G group may comprise at least one, at least two or at least three G³ groups. Accordingly, z may be 1, 2 or 3. Preferably, z is 3. G³ may be -L³X⁴C(O)-, -L³C(O)X⁴, - L³X⁴C(O)L⁶- or -L³C(O)X⁴L⁶-. Preferably, G³ is -L³X⁴C(O)L⁶-. -L³- and -L⁶- may independently be an optionally substituted C₁-C₁₂ alkylene, more preferably an optionally substituted C₁-C₆ alkylene, and most preferably a C₂-C₅ alkylene. Preferably, X⁴ is -NH-. Accordingly, each G³ may be

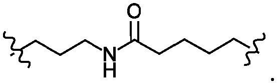

Accordingly, in some examples, G may be: 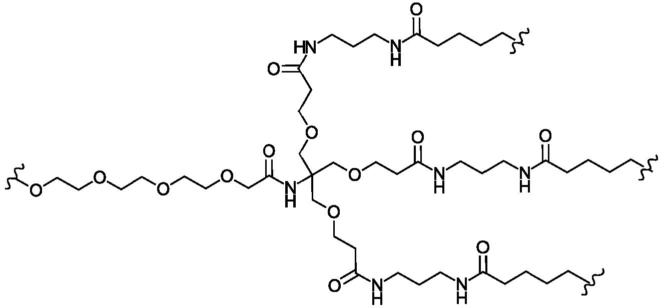 G² may be 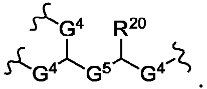 Each G⁴ may independently be absent, -L³-, -X⁴-, -X⁸-, - X⁴C(O)- or -C(O)X⁴-. Preferably, at least one, and more preferably at least two G⁴ groups are -L³-. -L³- may be an optionally substituted C₁-C₁₂ alkylene, more preferably an optionally substituted C₁-C₆ alkylene, and most preferably methylene or ethylene. Preferably, at least one G⁴ group is -X⁴C(O)-. Preferably X⁴ is -NH-. Preferably, G⁵ is - X⁴C(O)- or -C(O)X⁴-. Preferably, X⁴ is -NH-. Accordingly, G² may be 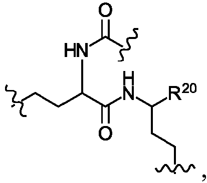 and more preferably is 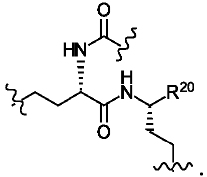 R²⁰ may be - X⁴C(O)L³H, -X⁴C(O)L⁹H, -C(O)X⁴L³H or -C(O)X⁴L⁹H. Preferably, X⁴ is -NH-.

Preferably, R²⁰ is -C(O)X⁴L⁹H. Preferably, -L⁹- is 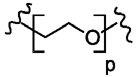 and p is an integer between 1 and 10, more preferably between 2 and 5, and most preferably p is 3.

Accordingly, G² may be 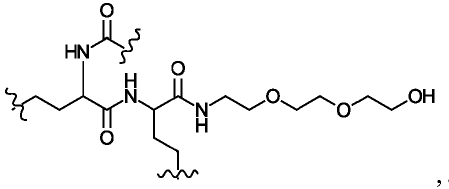 and more preferably is

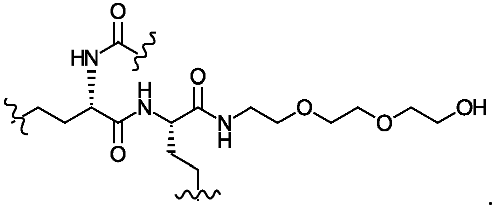

G¹ may be absent. G³ may be -L³-, -L⁹-, or -L⁹L³-. Preferably, -L⁹- is 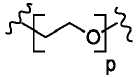 and p is an integer between 1 and 10, more preferably between 2 and 5, and most preferably p is 4. -L³- may be an optionally substituted C₁-C₁₂ alkylene, more preferably an optionally substituted C₁-C₆ alkylene, and most preferably ethylene.

Accordingly, G may be 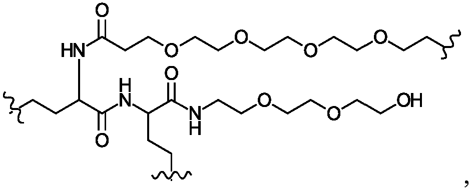 and is more preferably

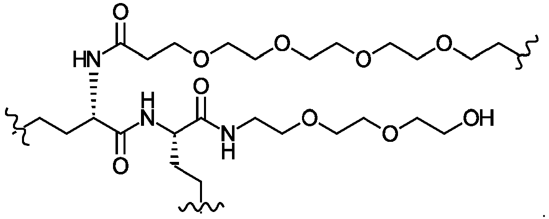

S may be -L³-. L³ may an optionally substituted C₁-C₁₀ alkylene, more preferably an optionally substituted C₁-C₆ alkylene. Preferably, the alkylene is unsubstituted.

S may be -X⁴L³-. X⁴ may be -NH-. L³ may be a C₁-C₁₂ optionally substituted alkylene, more preferably a C₁-C₆ optionally substituted alkylene and most preferably methylene or ethylene. Accordingly, S may be -NHCH₂-.

S may be an optionally substituted mono or bicyclic C₆-C₁₂ aryl, an optionally substituted mono or bicyclic 5 to 10 membered heteroaryl, an optionally substituted C₃-C₁₂ cycloalkyl, or an optionally substituted mono or bicyclic 3 to 12 membered heterocycle. Preferably S is an optionally substituted mono or bicyclic 5 to 10 membered heteroaryl or an optionally substituted mono or bicyclic 3 to 12 membered heterocycle. More preferably, S is an optionally substituted mono or bicyclic 5 membered heteroaryl or an optionally substituted mono or bicyclic 5 membered heterocycle. In some examples, G is a succinimidyl group, a triazolyl group or a tetrazolyl group. The triazolyl group may be a 1,2,3-trazolyl group. Accordingly, S may be 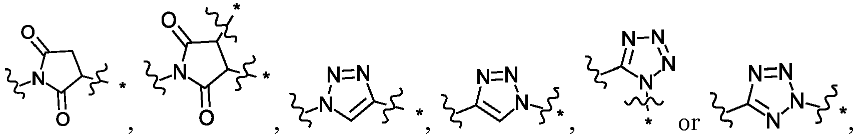 wherein a wavy line and asterisk indicate the attachment of the group S to the targeting moiety T. It may be appreciated that where two attachments sites are shown then S may be attached to the same targeting moiety at two separate points.

S may be -O-, -NH-, -S- or -C(O)-.

S may be L³. L³ may be an optionally substituted C₁-C₁₅ alkylene, more preferably an optionally substituted C₁-C₁₀ alkylene, and most preferably an optionally substituted C₁-C₆ alkylene. In some examples, the alkylene is unsubstituted.

S may be -X⁴C(X⁹)L³-, -X⁴C(X⁹)-, -X⁴C(X⁹)L³C(O)-, -X⁸L³-, -X⁴X⁸L³- or -X⁸L₃C(O)-. X⁴ may be NH. X⁹ may be O or S. L³ may be an optionally substituted C₁-C₆ alkylene, and more preferably an optionally substituted C₁-C₂ alkylene. The alkylene may be substituted with COOH or a C₁-C₆ alkyl which is optionally substituted with COOH or SO₂R⁹. Accordingly, S may be 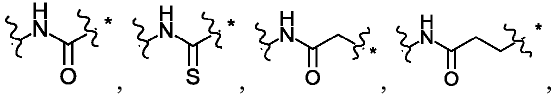 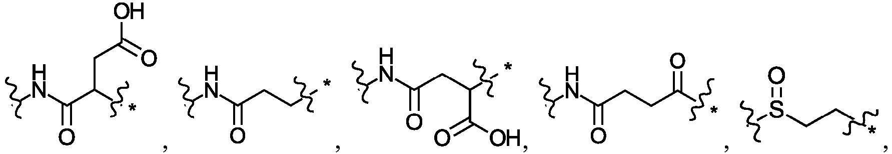 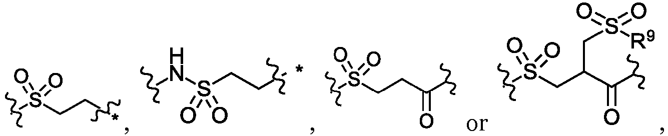 wherein a wavy line and asterisk indicate the attachment of the group S to the targeting moiety T.

S may be 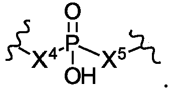 Preferably X⁴ and X⁵ are O.

S may be

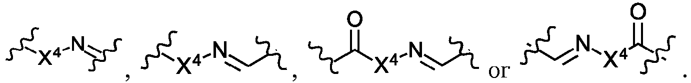

Preferably, X⁴ is O or NH. Accordingly, S may be 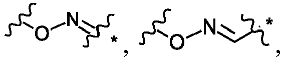 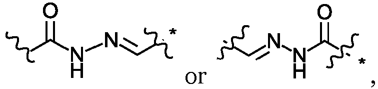 wherein a wavy line and asterisk indicate the attachment of the group S to the targeting moiety T.

S may be -L⁴L³-. L³ may be an optionally substituted C₁-C₆ alkylene, and more preferably a C₁-C₂ alkylene. L⁴ may be an optionally substituted mono or bicyclic C₆-C₁₂ aryl or an optionally substituted mono or bicyclic 5 to 10 membered heteroaryl, and preferably is a phenyl or a 6 membered heteroaryl. Accordingly, S may be 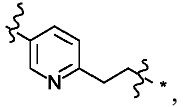 wherein a wavy line and asterisk indicate the attachment of the group S to the targeting moiety T.

S may be -L⁴L³L⁵C(O)-. L³ may be an optionally substituted C₁-C₆ alkylene, more preferably a C₁-C₂ alkylene, and most preferably methylene. L⁴ may be an optionally substituted C₃-C₁₂ cycloalkyl or an optionally substituted mono or bicyclic 3 to 12 membered heterocycle, more preferably is an optionally substituted C₃-C₆ cycloalkyl or an optionally substituted mono or bicyclic 3 to 6 membered heterocycle, even more preferably is an optionally substituted mono or bicyclic 5 membered heterocycle, and most preferably is a succinimidyl. L⁵ may be an optionally substituted C₃-C₁₂ cycloalkyl or an optionally substituted mono or bicyclic 3 to 12 membered heterocycle, more preferably is an optionally substituted C₃-C₆ cycloalkyl or an optionally substituted mono or bicyclic 3 to 6 membered heterocycle, and most preferably is a cyclohexyl.

Accordingly, S may be 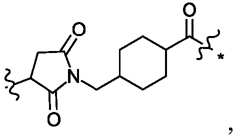 wherein a wavy line and asterisk indicate the attachment of the group S to the targeting moiety T.

S may be -L³C(O)L⁴C(O)-. L³ may be an optionally substituted C₁-C₆ alkylene, more preferably a C₁-C₂ alkylene, and most preferably methylene. L⁴ may be an optionally substituted C₃-C₁₂ cycloalkyl or an optionally substituted mono or bicyclic 3 to 12 membered heterocycle, more preferably is an optionally substituted C₃-C₆ cycloalkyl or an optionally substituted mono or bicyclic 3 to 6 membered heterocycle, most preferably is a mono or bicyclic 6 membered heterocycle. Accordingly, S may be 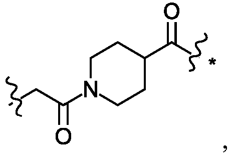 wherein a wavy line and asterisk indicate the attachment of the group S to the targeting moiety T.

In a preferred example, A may be absent, -L³X⁴-, -C(O)X⁴-, -L³C(O)X⁴,

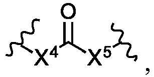

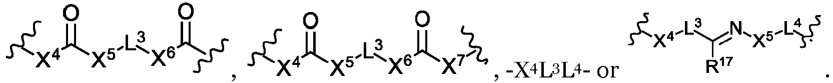

In examples where A is -L³X⁴-, L³ may be an optionally substituted C₁-C₁₂ alkylene, and is preferably a C₁-C₆ alkylene, and more preferably is -CH₂-, -CH₂CH₂- or-CH₂CH₂CH₂-. Preferably X⁴ is O. Accordingly, A may be -CH₂O-, -CH₂CH₂O- or - CH₂CH₂CH₂O-.

In examples where A is -C(O)X⁴- or -L³C(O)X⁴, X⁴ may be -O-. L³ may be an optionally substituted C₁-C₆ alkylene, and is preferably a C₁-C₃ alkylene, and most preferably is - CH₂-. Accordingly A may be -C(O)O- or -CH₂C(O)O-.

In examples where A is 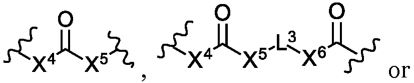 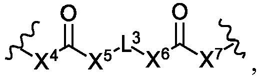 A may be

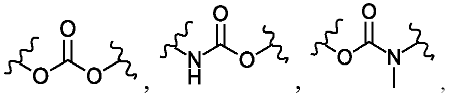

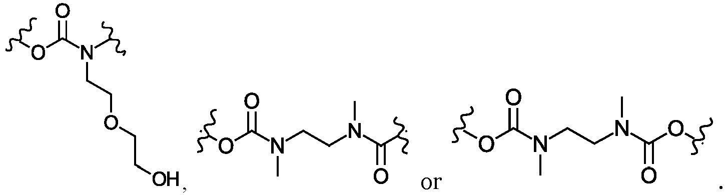

In examples where A is -X⁴L³L⁴-, X⁴ is preferably -O-. L³ may be an optionally substituted C₁-C₆ alkylene, and is preferably a C₁-C₂ alkylene, and more preferably is - CH₂-. L⁴ may be an optionally substituted 3 to 12 membered heterocycle, preferably L⁴ may is an optionally substituted 3 to 8 membered heterocycle, and most preferably L⁴ is an optionally substituted 5 or 6 membered heterocycle. L⁴ may be

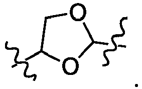

Accordingly, A may be

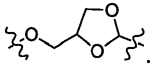

In examples where A is 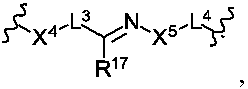 X⁴ is preferably -O-. L³ may be an optionally substituted C₁-C₆ alkylene, and preferably is a C₁-C₂ alkylene, and more preferably is -CH₂-. R¹⁷ may be an optionally substituted C₁₋₆ alkyl, and is preferably a C₁₋₃ alkyl and more preferably is a methyl. X₅ is preferably -NH- or -O-. L⁴ may be an optionally substituted mono or bicyclic C₆-C₁₂ aryl. Preferably, L⁴ is an optionally substituted phenyl, and in some examples is an unsubstituted phenyl. Accordingly, A may be

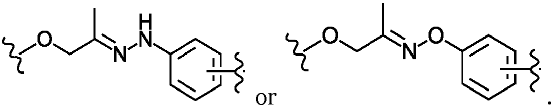

In a preferred example, W is absent or is -L³L⁷NH-. More preferably W is

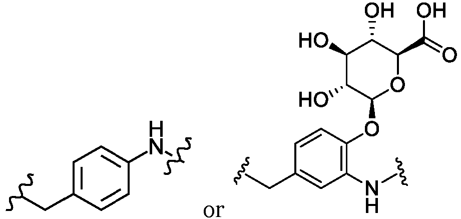

In a preferred example, D is absent or is -(D¹)_{q}C(O)-, where q is an integer between 2 and 10, and more preferably between 3 and 4. Preferably, D¹ may have general formula 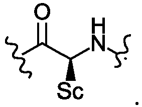 Preferably, each Sc group is an optionally substituted C₁-C₆ alkyl.

Preferably, the alkyl is optionally substituted with NHC(O)NH₂ or COOH. Accordingly, D may be

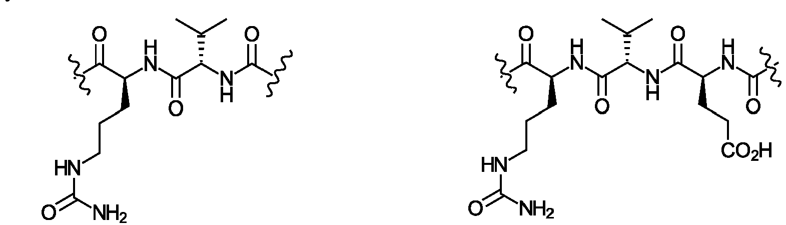

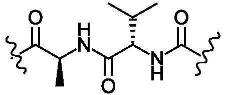

In a preferred example, S is -L³-, -X⁴-, -X⁴L³-, -C(X₉)-, -L₄-, -X₄C(X₉)L₃-,-X⁸L₃-, - X⁴X⁸L³-, 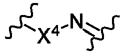 or -L⁴L³-.

In examples where S is -X⁴L³-, X⁴ may be -NH-. L³ may be a C₁-C₁₂ optionally substituted alkylene, more preferably a C₁-C₆ optionally substituted alkylene and most preferably methylene or ethylene.

In examples where S is -L⁴-, S may be an optionally substituted mono or bicyclic 5 to 10 membered heteroaryl or an optionally substituted mono or bicyclic 3 to 12 membered heterocycle. More preferably, S is an optionally substituted mono or bicyclic 5 membered heteroaryl or an optionally substituted mono or bicyclic 5 membered heterocycle.

In examples where S is -X⁴C(X⁹)L³-,-X⁸L³- or -X⁴X⁸L³-, X⁴ may be NH. X⁹ may be O. C⁸ may be -SO₂-. L³ may be an optionally substituted C₁-C₆ alkylene, and more preferably an optionally substituted C₁-C₂ alkylene. The alkylene may be unsubstituted or substituted with COOH or a C₁-C₆ alkyl which is optionally substituted with COOH.

In examples where S is -L⁴L³-, L³ may be an optionally substituted C₁-C₆ alkylene, and more preferably a C₁-C₂ alkylene. L⁴ may be an optionally substituted mono or bicyclic C₆-C₁₂ aryl or an optionally mono or bicyclic 5 to 10 membered heteroaryl, and preferably is a phenyl or a 6 membered heteroaryl.

Accordingly, in a preferred example, S may be -(CH₂)₅-, -NH-, -S-, -C(O)-, -NHCH₂-, 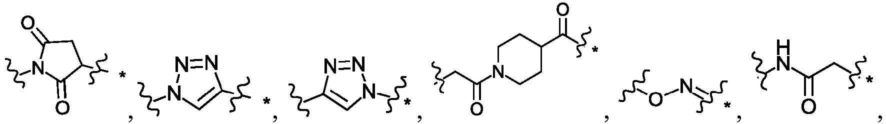 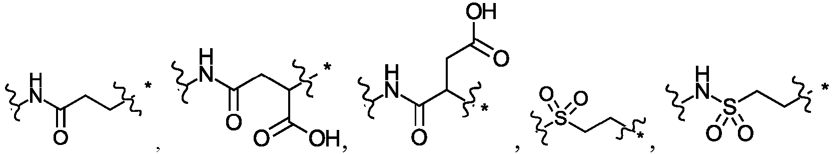 or

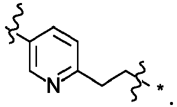

A, W, D, G and S may all be present. In some examples, a is 1 and z is 1. Accordingly, the linker may be: 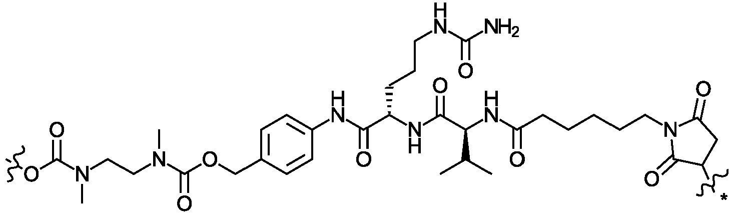 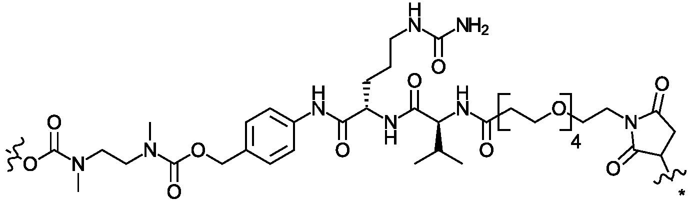 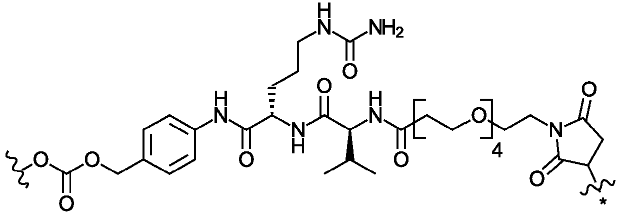 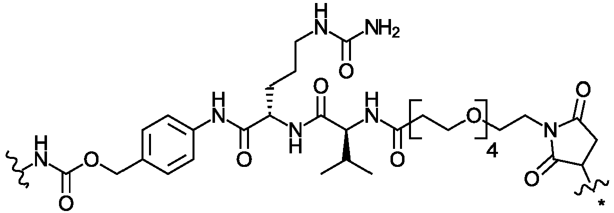 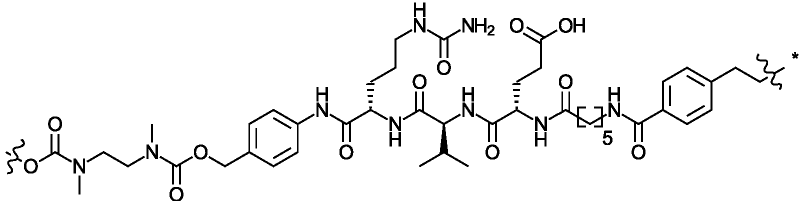 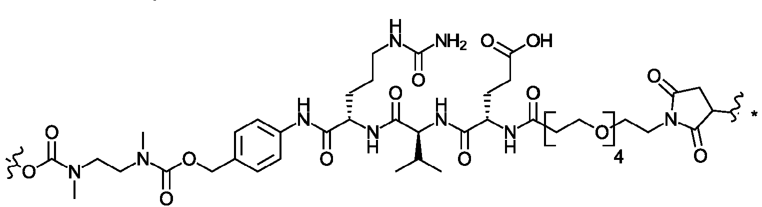 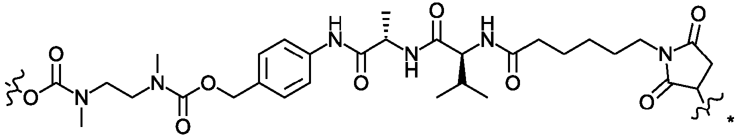 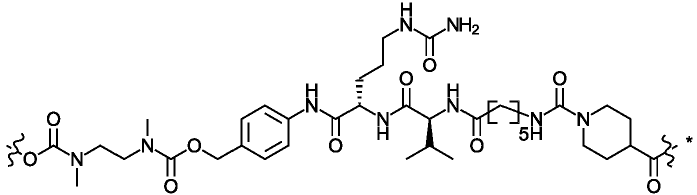 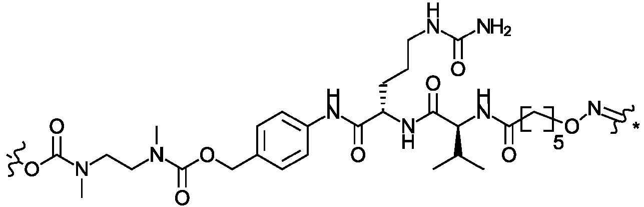 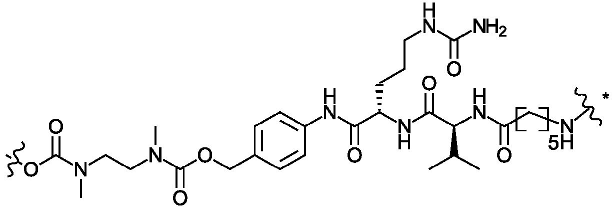 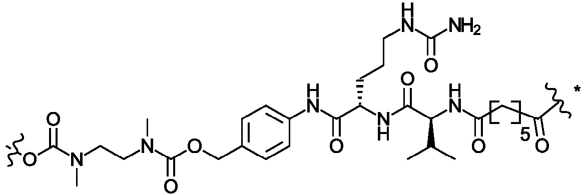 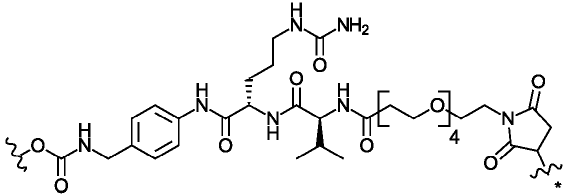 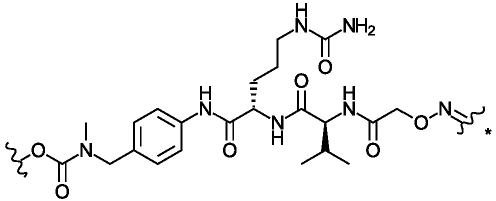 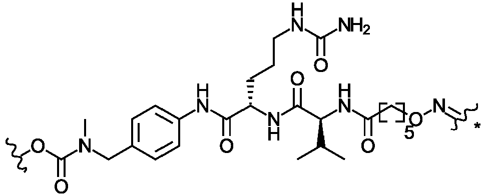 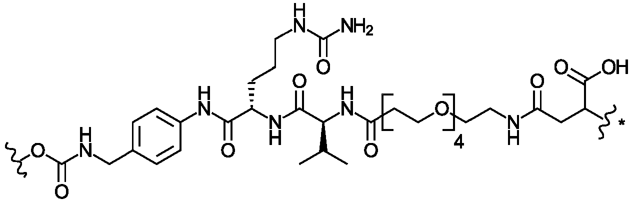 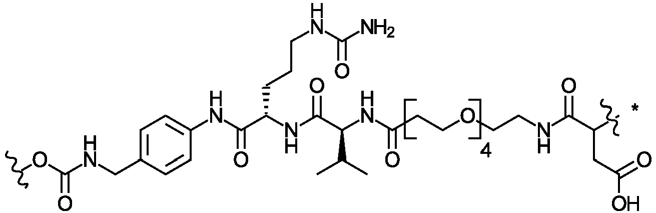 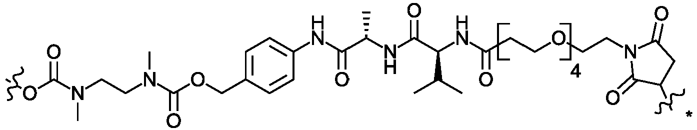 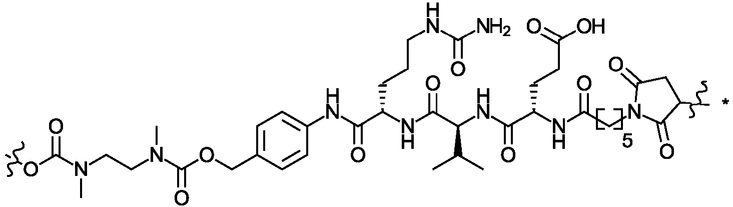 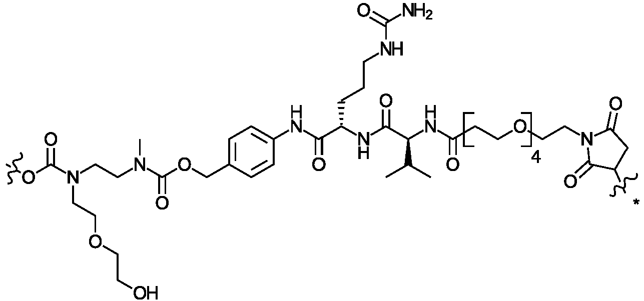 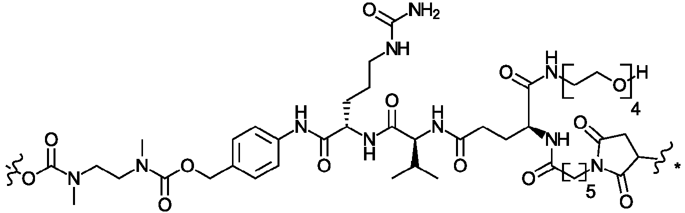 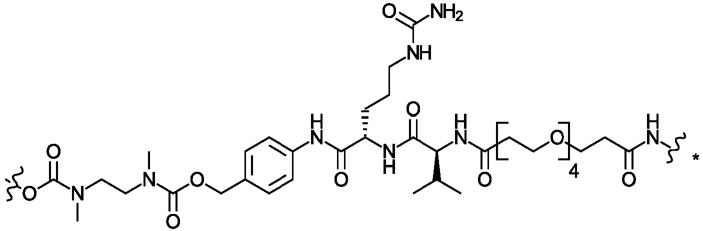 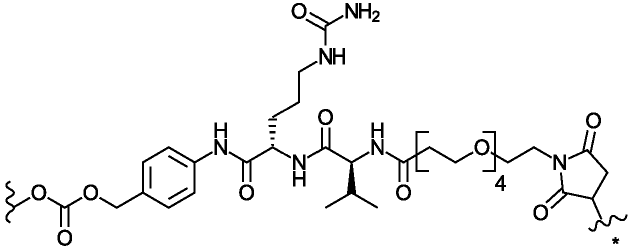 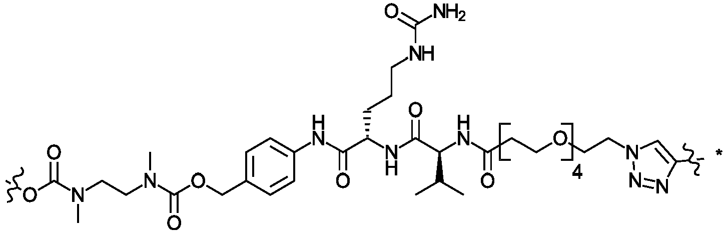 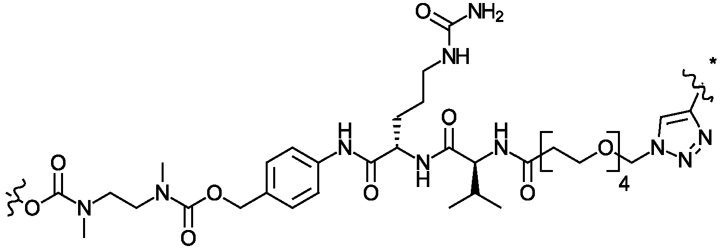 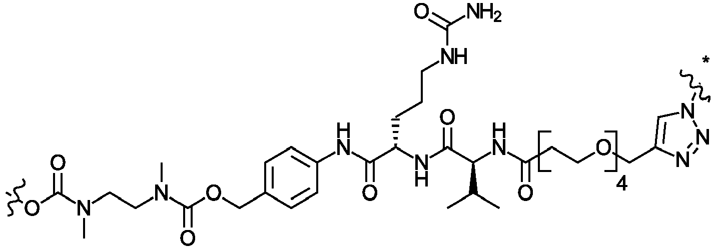 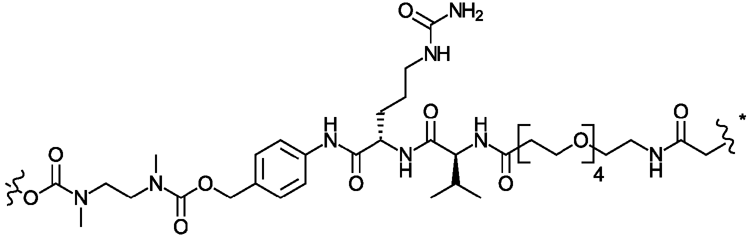 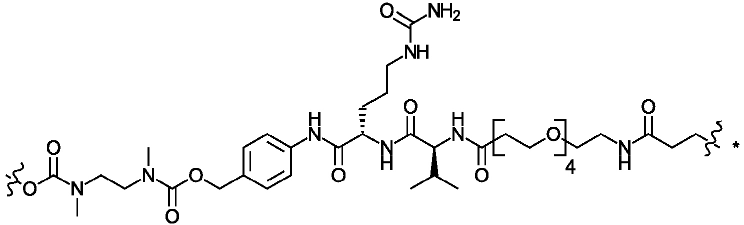 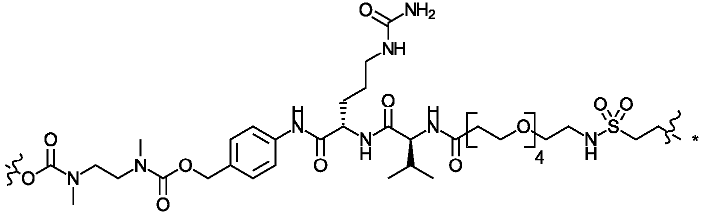 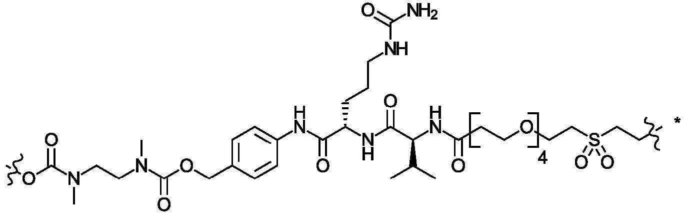 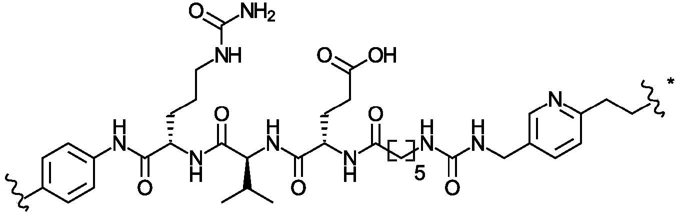 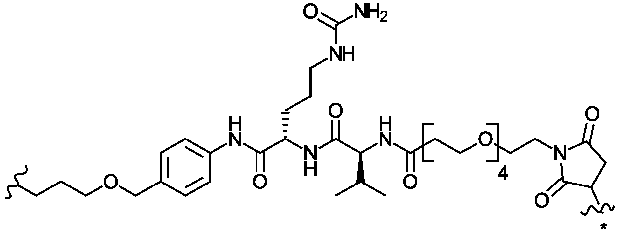 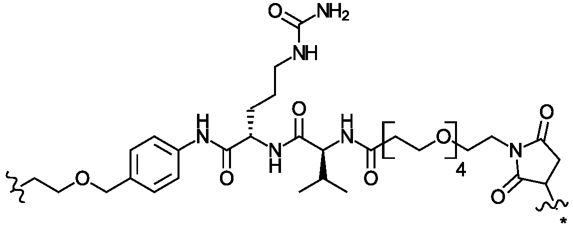 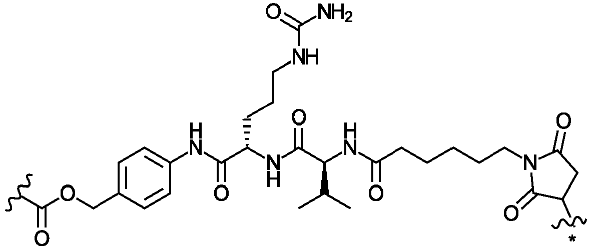 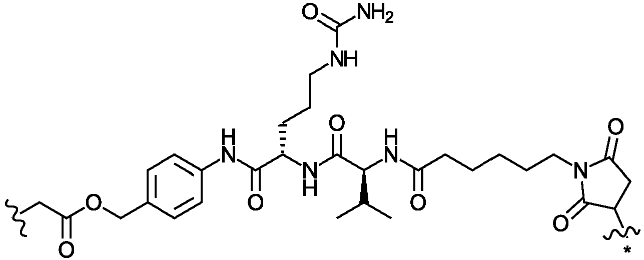 or 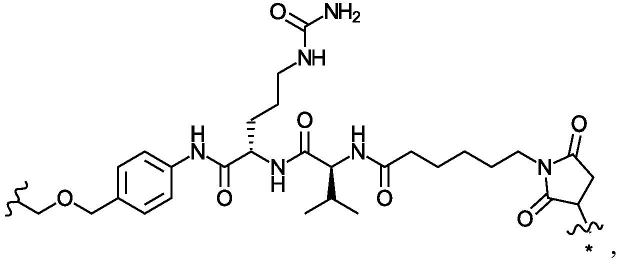 wherein a wavy line and asterisk indicates the attachment of the linker to the targeting moiety T, and a wavy line and no asterix indicates the attachment of the linker to the active compound C.

W, D, G and S may all be present. A may be absent. In some examples, a is 1 and z is 1. Accordingly, the linker may be: 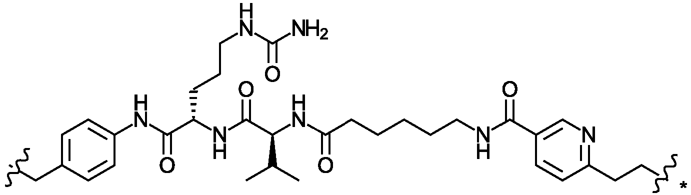 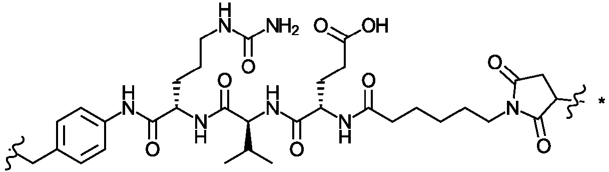 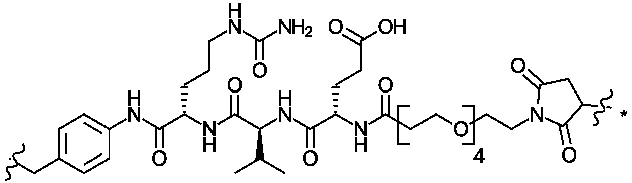 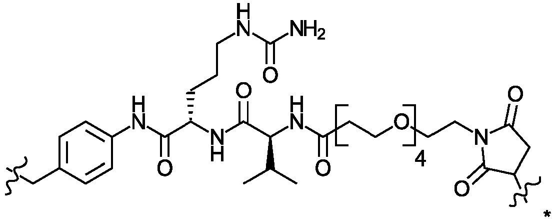 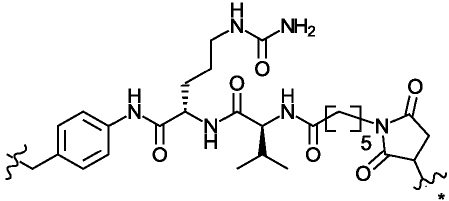 or 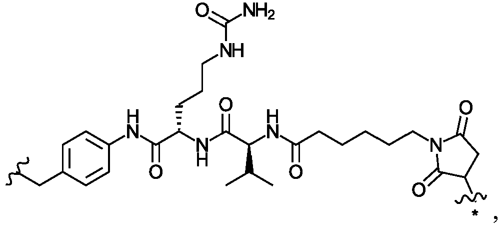 wherein a wavy line and asterisk indicates the attachment of the linker to the targeting moiety T, and a wavy line and no asterix indicates the attachment of the linker to the active compound C.

A, G and S may all be present. D may be absent. W may be absent. In some examples, a is 1 and z is 1. Accordingly, the linker may be: 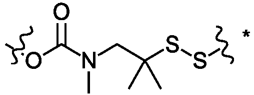 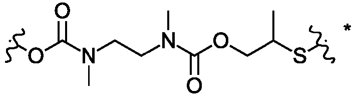 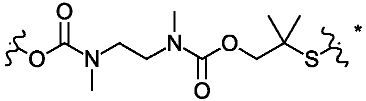 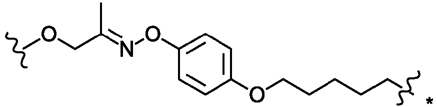 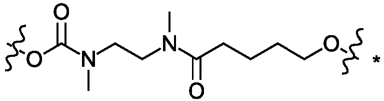 or 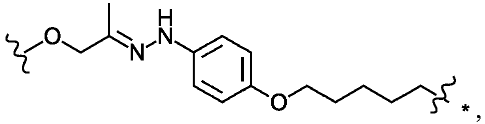 wherein a wavy line and asterisk indicates the attachment of the linker to the targeting moiety T, and a wavy line and no asterix indicates the attachment of the linker to the active compound C.

A, W, G and S may all be present. D may be absent. In some examples, a is 1 and z is 1. Accordingly, the linker may be: 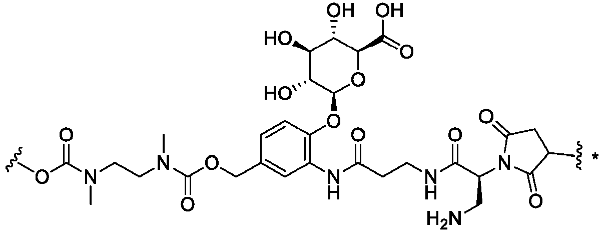 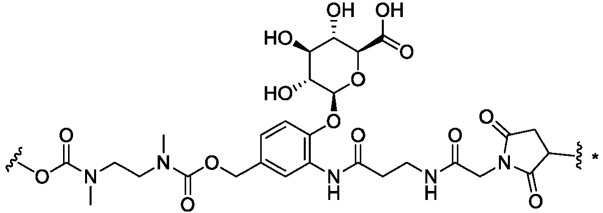 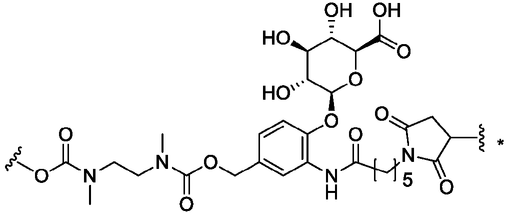 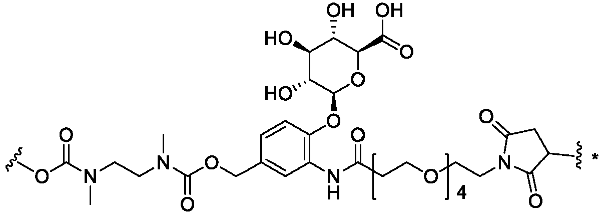 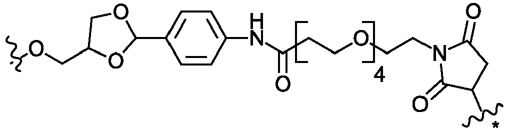 or 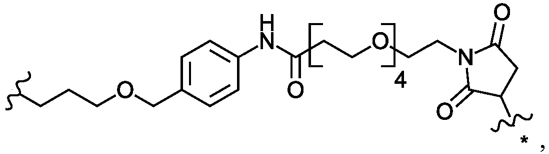 wherein a wavy line and asterisk indicates the attachment of the linker to the targeting moiety T, and a wavy line and no asterix indicates the attachment of the linker to the active compound C.

A and G may both be present. D may be absent. W may be absent. S may be absent. In some examples, a is 1 and z is 1. Accordingly, the linker may be: 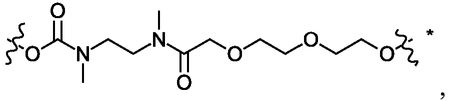 wherein a wavy line and asterisk indicates the attachment of the linker to the targeting moiety T, and a wavy line and no asterix indicates the attachment of the linker to the active compound C.

In some examples, a is 1 and z is 2 or 3. Accordingly, a may be 1 and z may be 3. In some examples, G and S may both be present. A may be absent. D may be absent. W may be absent. Accordingly, the linker may be: 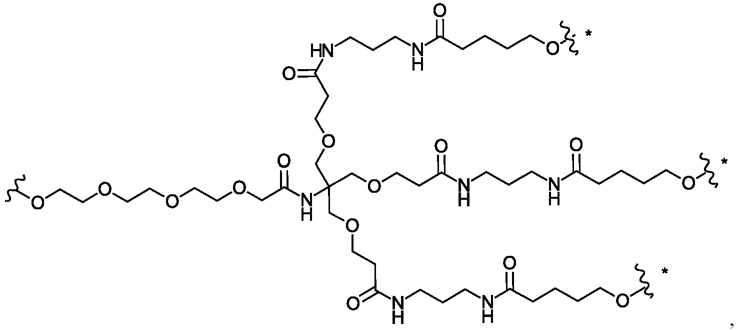 wherein a wavy line and asterisk indicates the attachment of the linker to the targeting moiety T, and a wavy line and no asterix indicates the attachment of the linker to the active compound C.

In some examples, a is 2 or 3 and z is a. Accordingly, a may be 2 and z may be a. In some examples, W, D, G and S may all be present. A may be absent. Accordingly, the linker may be: 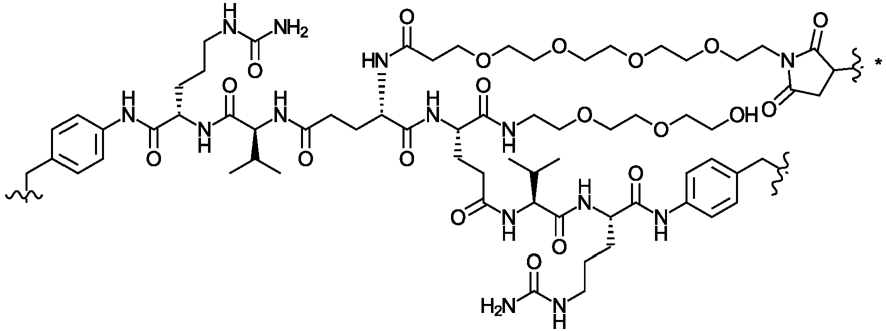 wherein a wavy line and asterisk indicates the attachment of the linker to the targeting moiety T, and a wavy line and no asterix indicates the attachment of the linker to the active compound C.

The linker will be known to those skilled in the art as either 'stable' linkers which are resistant to degradation in cells and in the systemic circulation or 'cleavable' or 'conditionally labile' linkers which are designed to degrade under intracellular conditions and/or in the systemic circulation following a defined trigger event, which may be a change in pH or a metabolic process such as ester or amide hydrolysis. Conjugates of the present invention may comprise two or more cleavage elements which may be selected from acid-induced cleavage, peptidase-induced cleavage (for example, a peptide linker cleaved by an intracellular protease, such as a lysosomal protease or an endosomal protease, see Trout et. al., 1982, PNAS USA, 79, 626-629), esterase-induced cleavage, glycosidase-induced cleavage, glucuronidase-induced cleavage, phosphodiesterase-induced cleavage, phosphatase-induced cleavage, lipase-induced cleavage or disulfide bond cleavage. Certain intracellular compartments, such as endosomes and lysosomes, have an acidic pH (pH 4.5), and provide conditions suitable to cleave acid-labile linkers. Specific hydrolysis processes have been described, such as the protease cleavage of a dipeptide e.g. the valine-citrulline dipeptide moiety (Ducry et. al., Bioconj. Chem., 2010, 21, 5-13) contained in the clinically precedented ADC brentuximab vedotin, a phenylalanine-lysine dipeptide, maleimidocaproyl or a maleimidocaproyl-valine-citrulline linker. The self-immolative group para-aminobenzyloxycarbonyl (PABC) may also form part of the linker structure in which, in response to a suitable trigger event, will eliminate from the conjugate to release the parent structure (Carl et. al., J. Med. Chem., 1981, 24, 479 and Chakravarty et. al., J. Med. Chem., 1983, 26, 638), for example in the maleimidocaproyl-valine-citrulline-PABC linker. Other linkers include those linkers that are cleaved at a specific pH or pH range such as a hydrazone e.g. the hydrazone moiety in gemtuzumab ozogamicin.

A non-cleavable linker may be protease insensitive. Non-cleavable linkers include that contained in the clinically precedented ADC trastuzumab emtansine and will require the conjugate to be degraded intracellularly to release the active drug C. See for example; Wong, Chemistry of Protein Conjugation and Cross-Linking, CRC Press Inc., Boca Raton, 1991**.**

The linker may be dendritic in nature, in that more than one small molecule C may be covalently attached through a branched, multifunctional unit to the targeting moiety (US2oo6/116422, US2005/271615). Dendritic linkers can increase the molar ratio of drug to targeting group which is related to the potency of the conjugate. Thus, where a targeting group contains for example just a single thiol group, a multitude of small molecules may be attached through a dendritic or branched linker.

The linker may be attached to a targeting moiety T in a variety of ways at any suitable available position on the targeting moiety through a reactive group thereon. Examples of suitable reactive groups include a surface lysine, an oxidised carbohydrate and a cysteine residue. Suitable reactive groups will be known by the skilled person. For instance, a variety of antibody-drug conjugate (ADC) linkage technologies are known in the art, including via alkylation, reductive amination, transesterification, amidation and thiol Michael additions. The resulting linkages include hydrazone, disulphide, maleimide, succinimide and peptide-based functional groups. For example thiol groups, or cysteine residues may be bonded to the linker or spacer group via a maleimide group. Alternative conjugation chemistries include lysine-reactive groups, such as succinyl or HOBt esters, pentafluorophenyl esters, β-lactam amides, isocyanates, and isothiocyanates; azide reactive groups, such as alkynes and strained alkynes; cysteine reactive groups, such as maleimides, α-haloacetamides, pyridyl disulfides and vinyl sulfoxides; and ketone reactive groups, such as hydroxylamines, hydrazines and acyl hydrazides.

In some examples, the number of drug/linker moieties conjugated per antibody molecule ranges from 1 to 10. The drug antibody ratio (DAR) is typically from 1 to 10, and may be from 2 to 5 or 2 to 3. Accordingly, b may be 1, 2, 3, 4, 5, 6, 7, 8, 9 or 10.

Such conjugates may be designed to specifically target certain cell types or tumour types via the targeting moiety. Accordingly, the targeting moiety may be configured to direct the compound of formula (I) to a specific cell or tumour type, and thereby deliver the STING modulator in a cell-specific manner. The conjugate can therefore be used accordingly in a therapeutic setting. The principle of this targeted delivery will be known to those skilled in the art as being closely related to ADC technology, for example as described in Polakis, P., Pharmacol. Revs., 2016, 68, 3-19 and Beck et. al., Nat. Revs. Drug Disc., 2017, 16, 315-337. The conjugate may then be taken up inside a cell or tumour through receptor-mediated endocytosis. The target antigen or receptor may be part of a cell or tumour or can be an extracellular matrix protein within the microenvironment of the cell or tumour. Once inside a cell or tumour, one or more specific peptide sequences within the conjugate may be hydrolytically cleaved by one or more cell or tumour proteases. For example, a tumour-associated protease, cathepsin B, C or D, or a plasmin protease to cleave the linker and release the active compound either in the target cells or in the tumour microenvironment of the target cells. The active drug is then free to migrate within the cell or microenvironment and thereby contact and subsequently modulate the STING protein. In some examples, the active drug may be cleaved from the targeting moiety outside of cells or tumours and the active drug subsequently acts at the cell surface or penetrates the cell or tumour.

T is a targeting moiety and may comprise an antibody, an antibody fragment, a nucleic acid based molecule, a carbohydrate, a peptide, a modified peptide or a small molecule.

In one example, T may be configured to target a tumour antigen. Accordingly, T may be configured to target the Human Epidermal Growth Factor Receptor (EGFR), a plasminogen activator, a cytotoxic T-lymphocyte associated antigen (CTLA) such as CTLA-4, vascular endothelial growth factor (VEGF), neurotrophic factors such as BDNF, fibroblast growth factor receptor (FGFR), a nerve growth factor, platelet-derived growth factor (PDGF), transforming growth factor (TGF), tissue factor (TF), EpCAM, CEACAM5, CEACAM6, colon-specific antigen p, FLT3, PSA, PSMA, PSCA, STEAP, BCMA, CEA, folate receptor, cathepsin D, estrogen receptor, progesterone receptor, NCA-95, NCA-90, A3, A33, Ep-CAM, the CD33/CD30/CD37/CD52/CD66e, CD56/CD74/CD79/CD22 receptors, the SLC34A2 gene product, SLC44A4, the mesothelin protein, the integrin α_{V}β3, PD-1, PD-L1, EGP-1, EGP-2, the EphA2 tyrosine kinase, the mucin cell-surface antigens e.g. MUC16, the hLewis Y antigen, carbonic anhydrase IX, 5T4, EFNA4, DLL4, Axl, B7, ALK, Fyn3, HLA, HIF, IGF, CC49, AFP, NaPi2b, brc-abl, caspase-8, guanylyl cyclase C, CD19, CD20, CD21, CD22, CD40, CD79a, CD79b, CD98, CD123, PTK7, CDK4, RANTES, CD44, CD48, CD133, CD70, CD72, CD74, CD166, c-kit, cMet, ErbB2/Her2, ErbB3/Her3, ErbB4/Her4, OX40, p53, α-fetoprotein, R1, PAP, PAX3, PAX5, Ras, Rho, ROR2, nectin-4, E-cadherin, P-cadherin, cadherin-6, LRRC15, BMPR1B, E16, Sema 5b, ETBR, MSG783, Trop2, TRPM4, ENPP3, SLITRK6, LIV-1, CRIPTO, FcRH1, IRTA2, TENB2, FcRH2, NCA, MDP, IL30Rα, ERK, gpNMB, LYPD3, GEDA, CXCR5, HLA-DOB, P2X5, LY64 or LY75.

In one example, T is configured to target Her2. It may be appreciated that HER2 may also be called Erbb2, and is a biomarker for breast cancer, gastric cancer, ovarian cancer and/or lung cancer.

In one example, T is an antibody, or a fragment thereof. Certain antibodies have been applied in the field of immune oncology previously. Exemplary anti-PD1 antibodies include lambrolizumab (MK-3475, Merck), nivolumab (BMS-936558, Bristol-Myers Squibb), AMP-224 (Merck) and pidilizumab (CT-011, Curetech Ltd.). Known anti-PDL1 antibodies include MDX-1105 (Medarex), MEDI4736 (Medimmune), MPDL4280A (Genentech) and BMS-936559 (Bristol-Myers Squibb). Exemplary anti-CTLA4 antibodies include ipilimumab (Yervoy, Bristol-Myers Squibb) and tremelimumab (Pfizer). Exemplary anti-ErbB2/Her2 antibodies include trastuzumab (Roche), pertuzumab (Genentech), margetuximab (Macrogenics) and HT-19 (Mersana Therapeutics). In one example, T is trastuzumab or a fragment or derivative thereof.

As an example, conjugates which comprise an anti-HER2 antibody can be specifically targeted to HER2-positive cancer cells or tumours. Trastuzumab (Herceptin or Herclon) is a humanized monoclonal antibody that binds to the juxtamembrane portion of the extracellular domain of the HER2 receptor (Hudis et. al., N. Engl. J. Med., 2007, 352, 39-51; Cho et. al., Nature, 2003, 421, 756-760). Trastuzumab gained US FDA approval in September 1998 for the treatment of metastatic breast cancer in patients whose tumours overexpress HER2 and who received one or more chemotherapy regimens for their metastatic disease.

The invention extends to both whole antibodies, as well as to antigen-binding fragments or regions of the corresponding full-length antibody.

The antibody or antigen-binding fragment thereof may be monovalent, divalent or polyvalent. Monovalent antibodies are dimers (HL) comprising a heavy (H) chain associated by a disulphide bridge with a light chain (L). Divalent antibodies are tetramer (H2L3) comprising two dimers associated by at least one disulphide bridge. Polyvalent antibodies may also be produced, for example by linking multiple dimers. The basic structure of an antibody molecule consists of two identical light chains and two identical heavy chains which associate non-covalently and can be linked by disulphide bonds. Each heavy and light chain contains an amino-terminal variable region of about 110 amino acids, and constant sequences in the remainder of the chain. The variable region includes several hypervariable regions, or Complementarity Determining Regions (CDRs), that form the antigen-binding site of the antibody molecule and determine its specificity for the antigen or variant or fragment thereof (e.g. an epitope). On either side of the CDRs of the heavy and light chains is a framework region, a relatively conserved sequence of amino acids that anchors and orients the CDRs. Antibody fragments may include a bi-specific antibody (BsAb) or a chimeric antigen receptor (CAR).

The constant region consists of one of five heavy chain sequences (µ, γ, ζ, α, or ε) and one of two light chain sequences (κ or λ). The heavy chain constant region sequences determine the isotype of the antibody and the effector functions of the molecule.

Preferably, the antibody or antigen-binding fragment thereof is isolated or purified.

In one example, the antibody or antigen-binding fragment thereof comprises a polyclonal antibody, or an antigen-binding fragment thereof. The antibody or antigen-binding fragment thereof may be generated in a rabbit, mouse or rat.

In one example, the antibody or antigen-binding fragment thereof comprises a monoclonal antibody or an antigen-binding fragment thereof. Preferably, the antibody is a human antibody. As used herein, the term "human antibody" can mean an antibody, such as a monoclonal antibody, which comprises substantially the same heavy and light chain CDR amino acid sequences as found in a particular human antibody exhibiting immunospecificity. An amino acid sequence, which is substantially the same as a heavy or light chain CDR, exhibits a considerable amount of sequence identity when compared to a reference sequence. Such identity is definitively known or recognizable as representing the amino acid sequence of the particular human antibody. Substantially the same heavy and light chain CDR amino acid sequence can have, for example, minor modifications or conservative substitutions of amino acids.

The term "human monoclonal antibody" can include a monoclonal antibody with substantially or entirely human CDR amino acid sequences produced, for example by recombinant methods such as production by a phage library, by lymphocytes or by hybridoma cells.

The term "humanised antibody" can mean an antibody from a non-human species (e.g. mouse or rabbit) whose protein sequences have been modified to increase their similarity to antibodies produced naturally in humans.

The antibody may be a recombinant antibody. The term "recombinant human antibody" can include a human antibody produced using recombinant DNA technology.

The term "antigen-binding region" can mean a region of the antibody having specific binding affinity for its target antigen or a variant or fragment thereof. Preferably, the fragment is an epitope. The binding region may be a hypervariable CDR or a functional portion thereof. The term "functional portion" of a CDR can mean a sequence within the CDR which shows specific affinity for the target antigen. The functional portion of a CDR may comprise a ligand which specifically binds to the target antigen or a fragment thereof.

The term "CDR" can mean a hypervariable region in the heavy and light variable chains. There may be one, two, three or more CDRs in each of the heavy and light chains of the antibody. Normally, there are at least three CDRs on each chain which, when configured together, form the antigen-binding site, i.e. the three-dimensional combining site with which the antigen binds or specifically reacts. It has however been postulated that there may be four CDRs in the heavy chains of some antibodies.

The definition of CDR also includes overlapping or subsets of amino acid residues when compared against each other. The exact residue numbers which encompass a particular CDR or a functional portion thereof will vary depending on the sequence and size of the CDR. Those skilled in the art can routinely determine which residues comprise a particular CDR given the variable region amino acid sequence of the antibody.

The term "functional fragment" of an antibody can mean a portion of the antibody which retains a functional activity. A functional activity can be, for example antigen binding activity or specificity. A functional activity can also be, for example, an effector function provided by an antibody constant region. The term "functional fragment" is also intended to include, for example, fragments produced by protease digestion or reduction of a human monoclonal antibody and by recombinant DNA methods known to those skilled in the art. Human monoclonal antibody functional fragments include, for example individual heavy or light chains and fragments thereof, such as VL, VH and Fd; monovalent fragments, such as Fv, Fab, and Fab'; bivalent fragments such as F(ab')₂; single chain Fv (scFv); and Fc fragments.

The term "VL fragment" can mean a fragment of the light chain of a human monoclonal antibody which includes all or part of the light chain variable region, including the CDRs. A VL fragment can further include light chain constant region sequences.

The term "VH fragment" can means a fragment of the heavy chain of a human monoclonal antibody which includes all or part of the heavy chain variable region, including the CDRs.

The term "Fd fragment" can mean the heavy chain variable region coupled to the first heavy chain constant region, i.e. VH and CH-1. The "Fd fragment" does not include the light chain, or the second and third constant regions of the heavy chain.

The term "Fv fragment" can mean a monovalent antigen-binding fragment of a human monoclonal antibody, including all or part of the variable regions of the heavy and light chains, and absent of the constant regions of the heavy and light chains. The variable regions of the heavy and light chains include, for example, the CDRs. For example, an Fv fragment includes all or part of the amino terminal variable region of about 110 amino acids of both the heavy and light chains.

The term "Fab fragment" can mean a monovalent antigen-binding fragment of a human monoclonal antibody that is larger than an Fv fragment. For example, a Fab fragment includes the variable regions, and all or part of the first constant domain of the heavy and light chains. Thus, a Fab fragment additionally includes, for example, amino acid residues from about 110 to about 220 of the heavy and light chains.

The term "Fab' fragment" can mean a monovalent antigen-binding fragment of a human monoclonal antibody that is larger than a Fab fragment. For example, a Fab' fragment includes all of the light chain, all of the variable region of the heavy chain, and all or part of the first and second constant domains of the heavy chain. For example, a Fab' fragment can additionally include some or all of amino acid residues 220 to 330 of the heavy chain.

The term "F(ab')₂ fragment" can mean a bivalent antigen-binding fragment of a human monoclonal antibody. An F(ab')₂ fragment includes, for example, all or part of the variable regions of two heavy chains-and two light chains, and can further include all or part of the first constant domains of two heavy chains and two light chains.

The term "single chain Fv (scFv)" can mean a fusion of the variable regions of the heavy (VH) and light chains (VL) connected with a short linker peptide.

The term "bispecific antibody (BsAb)" can mean a bispecific antibody comprising two scFv linked to each other by a shorter linked peptide.

One skilled in the art knows that the exact boundaries of a fragment of an antibody are not important, so long as the fragment maintains a functional activity. Using well-known recombinant methods, one skilled in the art can engineer a polynucleotide sequence to express a functional fragment with any endpoints desired for a particular application. A functional fragment of the antibody may comprise or consist of a fragment with substantially the same heavy and light chain variable regions as the human antibody.

The antigen-binding fragment thereof may comprise or consist of any of the fragments selected from a group consisting of VH, VL, Fd, Fv, Fab, Fab', scFv, F (ab')₂ and Fc fragment.

The antigen-binding fragment thereof may comprise or consist of any one of the antigen binding region sequences of the VL, any one of the antigen binding region sequences of the VH, or a combination of VL and VH antigen binding regions of a human antibody. The appropriate number and combination of VH and VL antigen binding region sequences may be determined by those skilled in the art depending on the desired affinity and specificity and the intended use of the antigen-binding fragment. Functional fragments or antigen-binding fragments of antibodies may be readily produced and isolated using methods well known to those skilled in the art. Such methods include, for example, proteolytic methods, recombinant methods and chemical synthesis. Proteolytic methods for the isolation of functional fragments comprise using human antibodies as a starting material. Enzymes suitable for proteolysis of human immunoglobulins may include, for example, papain, and pepsin. The appropriate enzyme may be readily chosen by one skilled in the art, depending on, for example, whether monovalent or bivalent fragments are required. For example, papain cleavage results in two monovalent Fab' fragments that bind antigen and an Fc fragment. Pepsin cleavage, for example, results in a bivalent F (ab') fragment. An F (ab')₂ fragment of the invention may be further reduced using, for example, DTT or 2-mercaptoethanol to produce two monovalent Fab' fragments.

Functional or antigen-binding fragments of antibodies produced by proteolysis may be purified by affinity and column chromatographic procedures. For example, undigested antibodies and Fc fragments may be removed by binding to protein A. Additionally, functional fragments may be purified by virtue of their charge and size, using, for example, ion exchange and gel filtration chromatography. Such methods are well known to those skilled in the art.

The antibody or antigen-binding fragment thereof may be produced using techniques well known in the art. For example, by recombinant methodology (see US Pat. No. 4,816,567), hydridoma technology (Kohler et. al., Nature, 1975, 256, 495), phage display technologies (for example, see Clackson et. al., Nature, 1991, 352, 624 and Marks et. al., J. Mol. Biol., 1991, 222, 581), synthetic technologies or combinations of such technologies. Preferably, one initially isolates a polynucleotide encoding desired regions of the antibody heavy and light chains. Such regions may include, for example, all or part of the variable region of the heavy and light chains. Preferably, such regions can particularly include the antigen binding regions of the heavy and light chains, preferably the antigen binding sites, most preferably the CDRs.

The polynucleotide encoding the antibody or antigen-binding fragment thereof according to the invention may be produced using methods known to those skilled in the art. The polynucleotide encoding the antibody or antigen-binding fragment thereof may be directly synthesized by methods of oligonucleotide synthesis known in the art. Alternatively, smaller fragments may be synthesized and joined to form a larger functional fragment using recombinant methods known in the art. Antibodies of use may be commercially obtained from a wide variety of known sources e.g. the American Type Culture Collection (ATCC, Manassas, Va.). A large number of antibodies against a wide variety of disease targets and tumor-associated antigens have been deposited at the ATCC and/or have published variable region sequences and are available for use in the claimed methods and compositions.

Cysteine-engineered antibodies have been designed as Fab antibody fragments (ThioFab) and expressed as full-length IgG monoclonal (thioMab) antibodies (US. Pat. 7,521,541). ThioFab and ThioMab antibodies have been conjugated through linkers at the newly introduced cysteine thiols to prepare site-specific antibody-drug conjugates (US. Pat. 7521541, US2008/0050310, WO2008/052187).

Polytherics have described a method for bridging a pair of sulfhydryl groups contained in antibody proteins derived from reduction of a native disulfide hinge (Badescu et. al., Bioconjugate Chem., 2014, 25, 1124-1136) to synthesise homogenous drug-loaded ADCs. Similar methods have been described by Concortis (US Patent 0105540, April 26 2015), Thiologics (Schumacher et. al., Org Biomol. Chem., 2014, 12, 7261-7269) and Igenica (Behrens et. al., Mol. Pharm., 2015, 12, 3986-3998). Related methods have been described in Frigerio et. al., Curr. Top. Med. Chem., 2018, 18, 1-32.

Other recent methods that have been used to target homogeneous drug-loaded ADCs include the incorporation of unnatural amino acids such as selenocysteine (Hofer, T. et. al., Biochem., 2009, 48, 12047-12057) or formyl glycine (Drake, P.M. et. al., Bioconj. Chem., 2014, 25, 1331-1341) groups into antibodies. Glycoengineering has been used to introduce sialic acid residues at specific sites (Zhou, Q. et. al., Bioconj. Chem., 2014, 25, 510-520) and transglutaminases used to enzymatically conjugate primary amine-containing linker/payloads to glutamine residues (Dorywalska, M. et. al., Bioconj. Chem., 2015, 26, 650-659). These, and other, methods are described in Sochaj, A.M. et. al., Biotech. Adv., 2015, 33, 775-784.

As used herein, the term "immunospecificity" can mean the binding region is capable of immunoreacting with the target antigen, or a variant or fragment thereof, by specifically binding therewith. The antibody or antigen-binding fragment thereof can selectively interact with an antigen with an affinity constant of approximately 10⁻⁵ to 10⁻¹³ M⁻¹, preferably 10⁻⁶ to 10⁻⁹ M⁻¹, even more preferably, 10⁻¹⁰ to 10⁻¹² M⁻¹.

The term "immunoreact" can mean the binding region is capable of eliciting an immune response upon binding with the target antigen, or an epitope thereof.

The term "epitope" can mean any region of an antigen with the ability to elicit, and combine with, a binding region of the antibody or antigen-binding fragment thereof.

In one example, T comprises a nucleic acid based molecule. The nucleic acid based molecule may be an aptamer. The nucleic acid based molecule may target the CD33/CD34 antigen as described in Zaimy, M.A. et. al., Cancer Gene Ther., 2016, 23, 315-320 or PSMA tumor antigens such as A9, A10 and A9L described by Lupold, S.E. et. al., Cancer Res., 2002, 62, 4029-4033; Dassie, J.P. et. al., Nat. Biotech., 2009, 27, 839-849; Rockey, W.M. et. al., Nucleic Acid Ther., 2011, 21, 299-314, or any other tumor antigen known to those skilled in the art, for example as described in Orava, E., Biochem. Biophys. Acta, 2010, 1798, 2190-2200.

Aptamers are nucleic acid or peptide molecules that assume a specific, sequence-dependent shape and bind to specific target ligands based on a lock-and-key fit between the aptamer and ligand. Typically, aptamers may comprise either single- or double-stranded DNA molecules (ssDNA or dsDNA) or single-stranded RNA molecules (ssRNA). Peptide aptamers consist of a short variable peptide domain, attached at both ends to a protein scaffold. Aptamers may be used to bind both nucleic acid and non-nucleic acid targets.

Suitable aptamers may be selected from random sequence pools, from which specific aptamers may be identified which bind to the selected antigen with high affinity. Methods for the production and selection of aptamers having desired specificity are well known to those skilled in the art, and include the SELEX (systematic evolution of ligands by exponential enrichment) process. Briefly, large libraries of oligonucleotides are produced, allowing the isolation of large amounts of functional nucleic acids by an iterative process of in *vitro* selection and subsequent amplification through polymerase chain reaction. Preferred methodologies for producing aptamers include those disclosed in WO 2004/042083.

In one example, T comprises a peptide or a modified peptide. The peptide or modified peptide may comprise the RGD sequence motif, as described in Mousavizadeh, A., Colloids Surfaces B., 2017, 158, 507-517 to include linear RGD peptide sequences or cyclised versions thereof as described in Belvisi, L et. al., Curr. Top., Med Chem., 2016, 16, 314-329. Examples of an RGD ligand which the targeting moiety may target and bind are as follows:

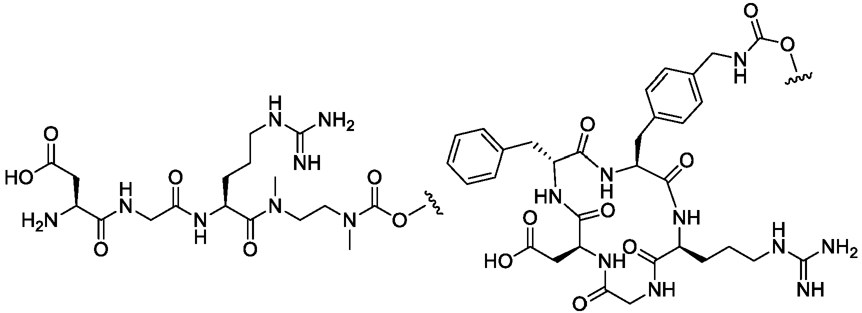

The peptide or modified peptide may comprise transferrin, or modified versions of transferrin, which has been described as showing promise for the targeted delivery of xenobiotics (Kratz et. al., Cancer Chemother. Pharmacol., 1998, 41, 155-160), including crossing the blood-brain barrier (Fishman et. al., J. Cell Biol., 1987, 101, 423-427). The peptide or modified peptide may also comprise albumin, or modified versions of albumin, in which the albumin protein may be conjugated to a suitable linker via Cys34 or other suitable residue as described in Larsen et. al., Mol Cell Ther., 2016, 4, 3.

In an alternative example, T comprises a carbohydrate or a modified carbohydrate molecule which can target a tumour-associated carbohydrate antigen receptor on target tumours and cells. For example, glycosphingolipids, gangliosides, sialic acids and mucins are indicative of malignant transformation and an aberrant glycosylation pattern on cancer cells (as reviewed in Feng, D. et. al., ACS Chem. Biol. 2016, 11, 850-863; Hakomori, S., Ann. Rev. Immunol., 1984, 2, 103-126; Dube, D.H. and Bertozzi, C.R., Nat. Rev. Drug Disc., 2005, 4, 477-488) and targeting ligands based on carbohydrate molecules have been designed against them, for example mannose, galactose or cerebrosidase derivatives. In a related method, cell-surface receptors on tissues of interest may also be targeted; a recent example includes derivatives of N-acetyl-galactosamine (GalNAc) which have been developed to target the asialoglycoprotein receptor on hepatocytes (reviewed in D'Souza, A. et. al., J. Controlled Rel., 2015, 203, 126-139 and a recent example in Sanhueza, C.A. et. al., JACS, 2017, 139, 3528-3536). Examples of carbohydrates which may be used as the targeting moiety are as follows:

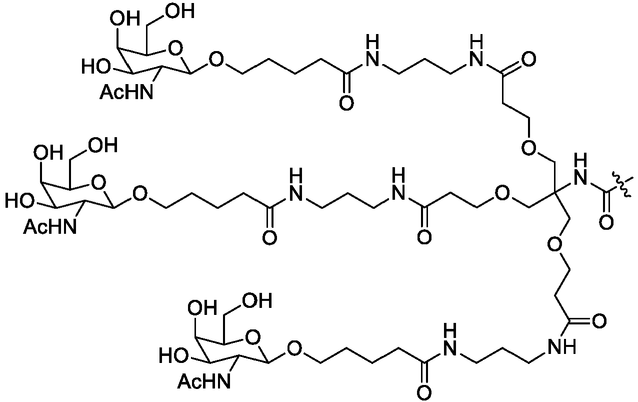

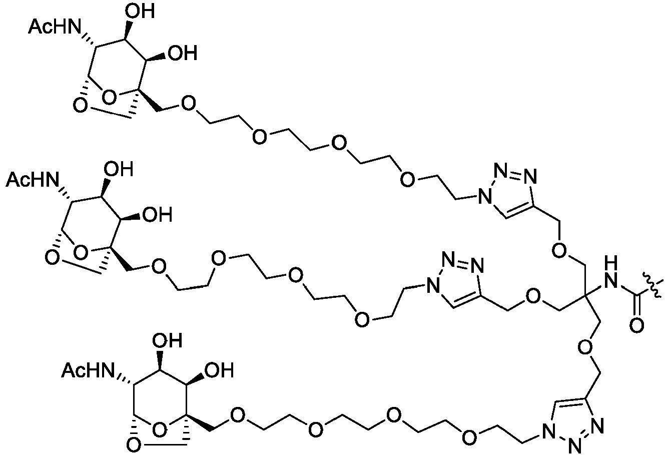

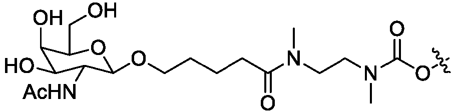

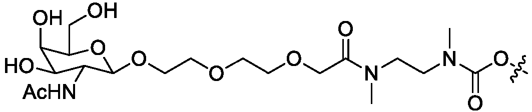

Accordingly, T may be 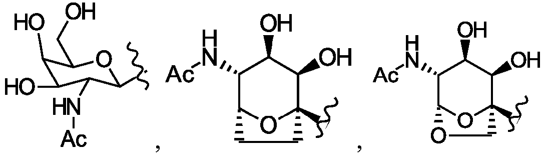 or

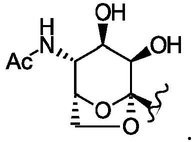

In another example, T comprises a small molecule ligand with affinity for a cell or tumour-surface receptor. For example, folic acid or derivatives thereof may be used to target folate receptors α, β or γ (FRα, FRβ and FRy). FRα in particular is known to be expressed in multiple endothelial tumour types such as breast, lung and kidney (see Fernandez, M. *et. al.,* **2018,** 4, 790-810 for a recent review) and conjugates of folate derivatives and toxins have been described previously (Vlahov, I. and Leamon, C.P., Bioconjugate Chem., 2012, 23, 1357-1369).

Linkers may be joined to a compound of formula (I) through a C atom, an O atom, a N atom or a S atom.

Linkers may be cleavable, non-cleavable, hydrophilic or hydrophobic. A cleavable linker can be sensitive to enzymes and may be cleaved by enzymes such as proteases. For example, a cleavable linker can be a valine-citrulline linker or a valine-alanine linker. For example:

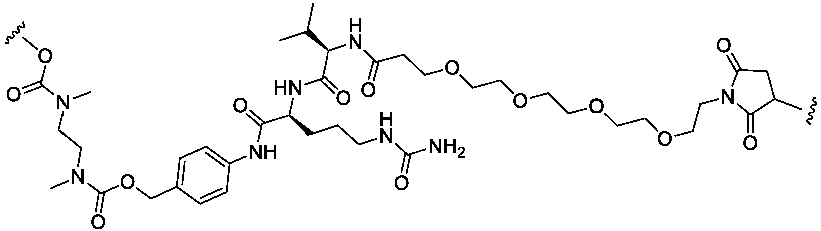

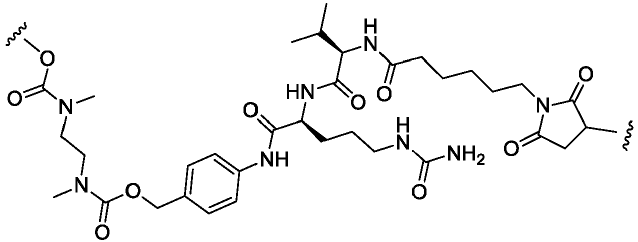

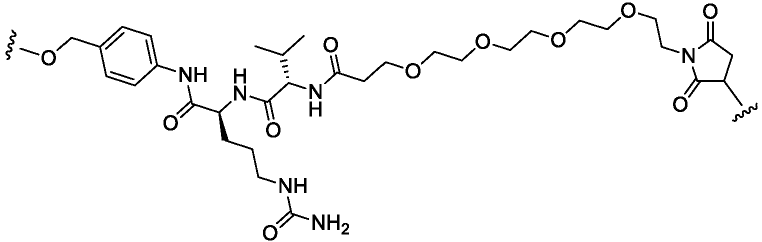

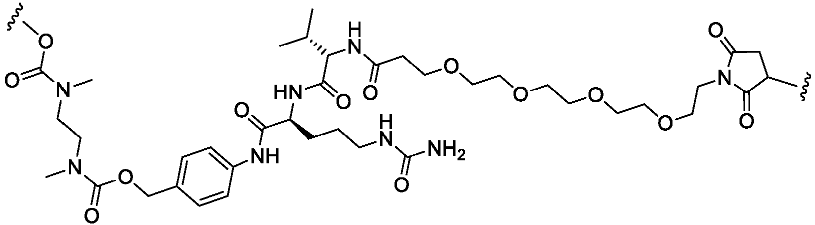

A non-cleavable linker may be protease insensitive.

The inventors have found that compounds of the current invention may be functionalised in various locations with a variety of linkers and spacers to provide conjugate molecules. Said linkers may include self-immolating groups (for example a p-aminobenzyl ether or amine and/or a valine-citrulline unit) that are designed to release the parent ACSS2 inhibitor upon a hydrolytic event, for example following amide, peptide or carbamate hydrolysis.

The term 'ACSS2' refers to acetyl coenzyme A synthetase short chain 2, and ATP-dependent enzyme that catalyses the synthesis of acetyl coenzyme A from acetate. ACSS2 is responsible for acetate incorporation in cells, including the uptake of acetate into lipids and histones.

It will be appreciated that an 'antagonist', or 'inhibitor' as it relates to a ligand and ACSS2, comprises a molecule, combination of molecules, or a complex, that inhibits, counteracts, downregulates, and/or desensitizes ACSS2 activity. 'Antagonist' encompasses any reagent that inhibits a constitutive activity of ACSS2. A constitutive activity is one that is manifest in the absence of a ligand/ACSS2 interaction. 'Antagonist' also encompasses any reagent that inhibits or prevents a stimulated (or regulated) activity of ACSS2.

Preferably, the compound of formula **(I)** or the conjugate of formula **(II)** is an inhibitor of the ACSS2 protein.

It will be appreciated that the compounds described herein or a pharmaceutically acceptable salt, solvate, tautomeric form or polymorphic form thereof may be used in a medicament which may be used in a monotherapy (i.e. use of the compound alone).

Alternatively, the compounds or a pharmaceutically acceptable salt, solvate, tautomeric form or polymorphic form thereof may be used as an adjunct to, or in combination with, known therapies.

Accordingly, in one example, a second therapeutic agent may be administered with a compound of Formula **(I)** or a conjugate of formula **(II).** The compound of Formula **(I)** or the conjugate of formula **(II)** may be administered before, after, and/or together with the second therapeutic agent. The second therapeutic agent may comprise an antiviral agent, an anti-inflammation agent, conventional chemotherapy, an anticancer vaccine and/or hormonal therapy. Alternatively, or additionally, the second therapeutic agent may comprise a B7 costimulatory molecule, interleukin-2, interferon-g, GM-CSF, a CTLA-4 antagonist (such as Ipilimumab and tremilimumab), an IDO inhibitor or IDO/TDO inhibitor (such as Epacadostat and GDC-0919), a PD-1 inhibitor (such as Nivolumab, Pembrolizumab, Pidilizumab, AMP-224, and MDX-1106), a PD-L1 inhibitor (such as Durvalumab, Avelumab and Atezolizumab), an OX-40 ligand, a LAG3 inhibitor, a CD40 ligand, a 41BB/CD137 ligand, a CD27 ligand, Bacille Calmette-Guerin (BCG), liposomes, alum, Freund's complete or incomplete adjuvant, a TLR agonist (such as Poly I:C, MPL, LPS, bacterial flagellin, imiquimod, resiquimod, loxoribine and a CpG dinucleotide) and/or detoxified endotoxins.

Methods for co-administration with an additional therapeutic agent are well known in the art (Hardman et. al. (eds.), Goodman and Gilman's The Pharmacological Basis of Therapeutics, 10th ed., 2001, McGraw-Hill New York, NY; Poole and Peterson (eds.), Pharmacotherapeutics for Advanced Practice: A Practical Approach, 2001, Lippincott, Williams and Wilkins, Philadelphia, PA; Chabner and Longo (eds.), Cancer Chemotherapy and Biotherapy, 2001, Lippincott, Williams and Wilkins, Philadelphia, PA).

In one example, the disease is cancer and a chemotherapeutic agent may be administered with a compound of Formula **(I)** or a conjugate of formula **(II).** The chemotherapeutic agent may be selected from a group further consisting of a cancer vaccine, a targeted drug, a targeted antibody, an antibody fragment, an antimetabolite, an antineoplastic, an antifolate, a toxin, an alkylating agent, a DNA strand breaking agent, a DNA minor groove binding agent, a pyrimidine analogue, a ribonucleotide reductase inhibitor, a tubulin interactive agent, an anti-hormonal agent, an immunomodulator, an anti-adrenal agent, a cytokine, radiation therapy, a cell therapy, cell depletion therapy such as B-cell depletion therapy and a hormone therapy. Alternatively or additionally, the chemotherapeutic agent may comprise abiraterone, altretamine, anhydrovinblastine, auristatin, bexarotene, bicalutamide, bleomycin, cachectin, cemadotin, chlorambucil, cyclophosphamide, docetaxol, doxetaxel, carboplatin, cysplatin, cytarabine, dactinomycin, daunorubicin, decitabine, doxorubicin, etoposide, 5-fluorouracil, finasteride, flutamide, hydroxyurea, streptozocin, mitomycin, methotrexate, taxanes, tamoxifen, vinblastine, vincristine and/or vindesine.

The compound of Formula **(I)** or the conjugate of formula **(II)** may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising the compounds described herein may be used in a number of ways. Suitable modes of administration include oral, intra-tumoral, parenteral, topical, inhaled/intranasal, rectal/intravaginal, and ocular/aural administration.

Formulations suitable for the aforementioned modes of administration may be formulated to be immediate and/or modified release. Modified release formulations include delayed-, sustained-, pulsed-, controlled-, targeted and programmed release.

The compounds of the invention may be administered orally. Oral administration may involve swallowing, so that the compound enters the gastrointestinal tract, or buccal or sublingual administration may be employed by which the compound enters the blood stream directly from the mouth. Formulations suitable for oral administration include solid formulations such as tablets, capsules containing particulates, liquids, or powders, lozenges (including liquid-filled), chews, multi- and nano-particulates, gels, solid solution, liposome, films, ovules, sprays, liquid formulations and buccal/mucoadhesive patches.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkylsubstituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet. Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated. The formulation of tablets is discussed in "Pharmaceutical Dosage Forms: Tablets", Vol. 1, by H. Lieberman and L. Lachman (Marcel Dekker, New York, 1980).

Suitable modified release formulations for the purposes of the invention are described in US Patent No. 6,106,864. Details of other suitable release technologies such as high energy dispersions and osmotic and coated particles are to be found in "Pharmaceutical Technology On-line", 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) and conjugates of formula (II) used in the preparation of parenteral solutions may be increased by the use of appropriate formulation techniques, such as the incorporation of solubility-enhancing agents. Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(dl-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject^{™}, Bioject^{™}, etc.) injection.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as L-leucine, mannitol, or magnesium stearate.

The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (I) or the conjugate of formula (II), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

In the case of dry powder inhalers and aerosols, the dosage unit is determined by means of a valve which delivers a metered amount. Units in accordance with the invention are typically arranged to administer a metered dose or "puff" containing from 1µg to 100mg of the compound of formula (I) or the conjugate of formula (II). The overall daily dose will typically be in the range 1µg to 200mg which may be administered in a single dose or, more usually, as divided doses throughout the day.

The compounds of the invention may be administered rectally or vaginally, for example, in the form of a suppository, pessary, microbicide, vaginal ring or enema. Cocoa butter is a traditional suppository base, but various alternatives may be used as appropriate.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

The compounds of the invention may also be administered directly to a site of interest by injection of a solution or suspension containing the active drug substance. The site of interest may be a tumour and the compound may by administer via intratumoral injection. Typical injection solutions are comprised of propylene glycol, sterile water, ethanol and sodium chloride. Alternative solvents which may be used instead of propylene glycol include glycerol and polyethylene glycol.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

It will be appreciated that the amount of the compound that is required is determined by its biological activity and bioavailability, which in turn depends on the mode of administration, the physiochemical properties of the compound, and whether it is being used as a monotherapy, or in a combined therapy. The frequency of administration will also be influenced by the half-life of the compound within the subject being treated. Optimal dosages to be administered may be determined by those skilled in the art, and will vary with the particular compound in use, the strength of the pharmaceutical composition, the mode of administration, and the advancement of the disease. Additional factors depending on the particular subject being treated will result in a need to adjust dosages, including subject age, weight, gender, diet, and time of administration.

Generally, for administration to a human, the total daily dose of the compounds of the invention is typically in the range 100µg to 10g, such as 1mg to 1g, for example 10mg to 500mg. For example, oral administration may require a total daily dose of from 25mg to 250mg. The total daily dose may be administered in single or divided doses and may, at the physician's discretion, fall outside of the typical range given herein. These dosages are based on an average human subject having a weight of about 60kg to 70kg. The physician will readily be able to determine doses for subjects whose weight falls outside this range, such as infants and the elderly.

The compound may be administered before, during or after onset of the disease to be treated.

Known procedures, such as those conventionally employed by the pharmaceutical industry (e.g. in *vivo* experimentation, clinical trials, etc.), may be used to form specific formulations comprising the compounds according to the invention and precise therapeutic regimes (such as daily doses of the compounds and the frequency of administration). The inventors believe that they are the first to describe a pharmaceutical composition for treating a disease, based on the use of the compounds of the invention.

Hence, in a further example of the invention, there is provided a pharmaceutical composition comprising a compound according to the first aspect, or a pharmaceutically acceptable salt, solvate, tautomeric form or polymorphic form thereof, and a pharmaceutically acceptable vehicle.

This disclosure also provides a process for making the composition according to the further aspect, the process comprising contacting a therapeutically effective amount of a compound of the first aspect, or a pharmaceutically acceptable salt, solvate, tautomeric form or polymorphic form thereof, and a pharmaceutically acceptable vehicle.

A "subject" may be a vertebrate, mammal, or domestic animal. Hence, compounds, compositions and medicaments according to the invention may be used to treat any mammal, for example livestock (e.g. a horse), pets, or may be used in other veterinary applications. Most preferably, however, the subject is a human being.

A "therapeutically effective amount" of compound is any amount which, when administered to a subject, is the amount of drug that is needed to treat the target disease, or produce the desired effect, i.e. inhibit the ACSS2 protein.

For example, the therapeutically effective amount of compound used may be from about 0.01 mg to about 800 mg, and preferably from about 0.01 mg to about 500 mg. It is preferred that the amount of compound is an amount from about 0.1 mg to about 250 mg, and most preferably from about 0.1 mg to about 20 mg.

A "pharmaceutically acceptable vehicle" as referred to herein, is any known compound or combination of known compounds that are known to those skilled in the art to be useful in formulating pharmaceutical compositions.

In one example, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents (i.e. the compound according to the first aspect) according to the invention. In tablets, the active compound may be mixed with a vehicle having the necessary compression properties in suitable proportions and compacted in the shape and size desired. The powders and tablets preferably contain up to 99% of the active compound. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another example, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The compound according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The compound may be prepared as a sterile solid composition that may be dissolved or suspended at the time of administration using sterile water, saline, or other appropriate sterile injectable medium.

The compound and compositions of the invention may be administered in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The compounds used according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

The scope of the invention includes all pharmaceutically acceptable isotopically-labelled compounds of the invention wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of the invention, for example those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection. Substitution with isotopes such as deuterium, i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds of formula **(I)** or conjugates of formula **(II)** can generally be prepared by conventional techniques known to those skilled in the art or by processes analogous to those described in the accompanying Examples and Preparations using an appropriate isotopically-labelled reagent in place of the non-labelled reagent previously employed.

All features described herein (including any accompanying claims, drawings and abstract), and/or all of the steps of any method or process so disclosed, may be combined with any of the above aspects in any combination, except combinations where at least some of such features and/or steps are mutually exclusive.

### General Schemes

### General Scheme 1:

Compounds of formula **(III)** may be prepared from compounds of formula **(IV)** and **(V)** using a urea bond forming reaction, as shown below.

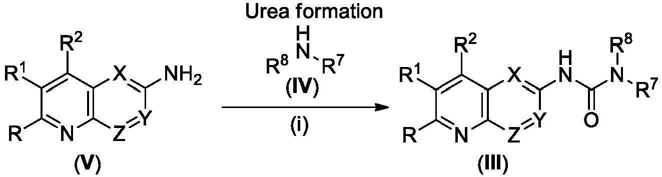

Typical reaction conditions for the activation of the aromatic amine of the compound of formula **(V)** employ 4-nitrophenyl chloroformate to generate an intermediate which can be attacked by a suitable nucleophile such as amine **(IV)** to give a urea as a compound of formula **(III).** Preferred organic bases include DIPEA or TEA in a suitable organic solvent such as DCM, DMF, DMA or MeCN. The reaction may be shaken or stirred at room temperature.

Compounds of formula **(IV)** and **(V)** are commercially available or may be synthesized by those skilled in the art. In particular, methods of synthesizing compounds of formula **(V)** are described in General Schemes 3 to 10.

### General Scheme 2:

Compounds of formula **(VI)** may be prepared from compounds of formula **(V)** and **(VII)** using an amide bond forming reaction, as shown below.

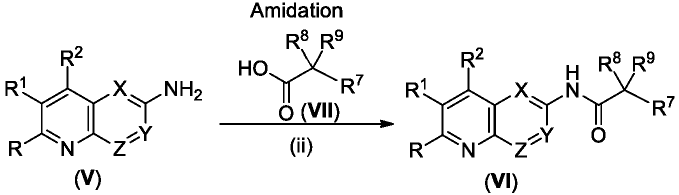

Typical conditions employ activation of the carboxylic acid of the compound of formula **(VII)** using a suitable organic base and a suitable coupling agent. Preferred coupling agents are either EDCI with HOBt, T₃P, HATU, HBTU or BOP. Preferred organic bases include DIPEA or TEA in a suitable organic solvent such as DCM, DMF, DMA or MeCN. The reaction may be shaken or stirred at room temperature.

Compounds of formula **(V)** and **(VII)** are commercially available or may be synthesized by those skilled in the art. In particular, methods of synthesizing compounds of formula **(V)** or derivatives thereof, are described in General Schemes 3-10.

### General Scheme 3:

Compounds of formula **(V)** may be prepared from nitro compounds of formula **(VIII)** by simple reduction.

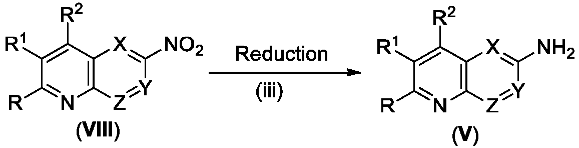

Typical reduction conditions may include Pd catalyzed or Ra-Ni catalyzed hydrogenation methods, or reduction in the presence of a transition metal reagent, for example Fe-AcOH, Zn-AcOH or SnCl₂-H₂O-HCl in a suitable solvent such as methanol or ethanol.

Compounds of formula **(VIII)** are commercially available or may be synthesized by those skilled in the art. In particular, methods of synthesizing compounds of formula **(VIII)** are described in General Schemes 6, 7, 9 and 11-14 shown below.

### General Scheme 4:

Compounds of formula **(V)** may be prepared from haloaromatic compounds of formula **(IX)** by a direct ammonolysis reaction.

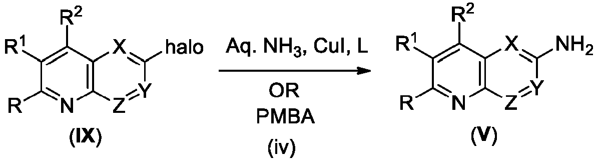

Typical amination conditions use liquid or aqueous solutions of ammonia in the presence of a suitable catalyst, typically CuI in the presence of a suitable activating ligand such as dimethylethylenediamine (DMEDA), amino acids such as proline, pyridine-based ligands such as phenanthroline, diols and diketones. Alternatively, the halide may be aminated with para-methoxybenzylamine and then deprotected using methods known to those skilled in the art, for example using TFA, ceric ammonium nitrate or a Pd-catalyzed hydrogenation reaction.

Compounds of formula **(IX)** are commercially available or may be synthesized by those skilled in the art. In particular, methods of synthesizing compounds of formula **(IX)** are described in General Schemes 8 and 10 shown below.

### General Scheme 5:

The amines of formula **(V)** may be further functionalized at the X position according to the below scheme.

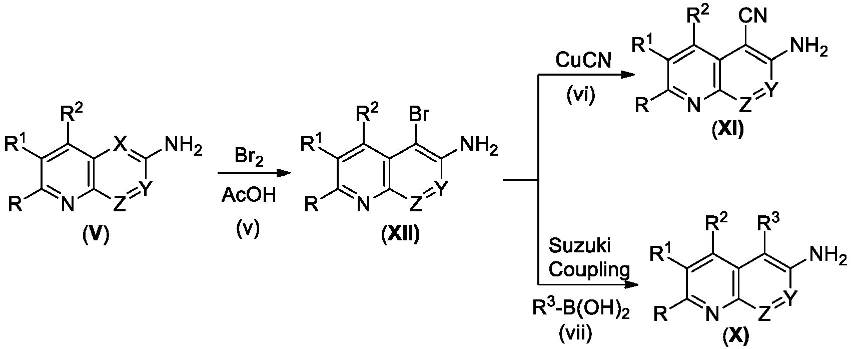

Compounds of formula **(V)** may be halogenated, for example brominated using bromine in AcOH to provide the bromides of formula **(XII).** Said bromides may then be reacted with a suitable cyano source such as CuCN or Zn(CN)₂ in a suitable polar solvent such as DMF, DMA, NMP or pyridine, optionally in the presence of a catalyst such as tetrakistriphenylphosphine palladium(o) usually at reflux temperature to give the nitriles of formula **(XI).** Alternatively, the bromides of formula **(XII)** may undergo a Suzuki reaction with a suitable boronic acid R³-B(OH)₂ or boronate ester in the presence of a transition metal catalyst, typically Pd for example Pd(OAc)₂, Pd₂dba₃ or Pd(dppf)Cl₂, a suitable base such as KO^{t}Bu, K₃PO₄, K₂CO₃, Cs₂CO₃ or Na₂CO₃ and a suitable solvent system such as THF-water, ethanol-water, DMF, dioxane or toluene. Reactions are typically heated to give the R³ functionalized amines **(X).**

### General Scheme 6:

Nitro compounds of formula **(XIII)** may be synthesized starting from benzopyridones of formula **(XX).**

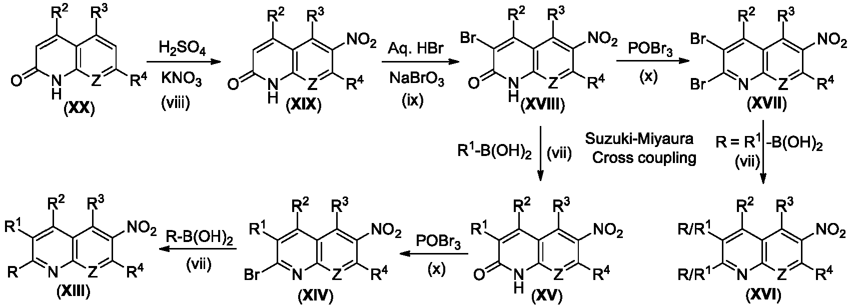

Benzopyridones of formula **(XX)** may undergo a nitration reaction, for example in mixtures of sulfuric acid and fuming nitric acid, typically at room temperature to give nitro compounds of formula **(XIX).** These nitro compounds may then be halogenated, for example brominated with sodium bromate and a strong acid such as aqueous HBr under heating to provide bromo derivatives of formula **(XVIII).** Treatment of a compound of formula **(XVIII)** with POBr₃ converts a pyridine into the corresponding bromopyridine to give dibromo compounds of formula **(XVII).** Compounds of formula **(XVII)** may then undergo a Suzuki reaction as described in General Scheme 5 with boronates or boronic acids R¹-B(OH)₂ and/or R-B(OH)₂ to provide a quinoline of formula **(XVI).** Alternatively, the bromopyridones of formula **(XVIII)** may also be reacted in a Suzuki reaction with boronates or a boronic acid of formula R¹-B(OH)₂ to provide the functionalized pyridine of formula **(XV)** which may then be further brominated to a bromopyridine **(XIV)** and subjected to a Suzuki reaction with a boronate or boronic acid R-B(OH)₂ to provide a quinoline of formula **(XIII).**

Compounds of formula **(XX)** are commercially available or may be synthesized by those skilled in the art.

### General Scheme 7:

Nitro-quinolines may also be built up according to the below scheme, starting from a nitro aniline of formula **(XXII)** and an acetic acid derivative of formula **(XXVI).**

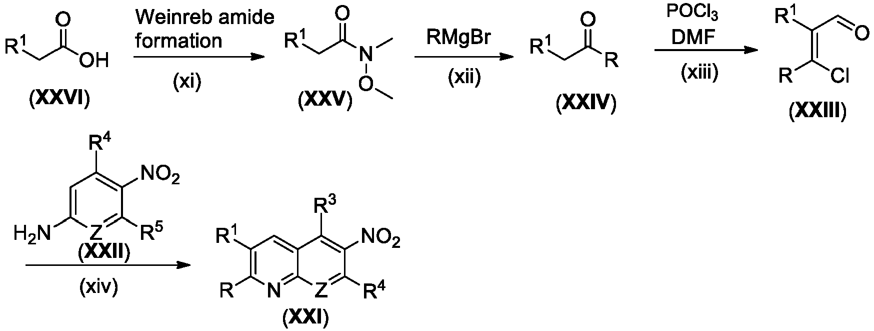

An acid of formula **(XXVI)** may be converted into a Weinreb amide of formula **(XXV)** with, for example, N,O-diMe-hydroxylamine using a suitable organic base and a suitable coupling agent. Preferred coupling agents are either EDCI with HOBt, T₃P, HATU, HBTU or BOP. Preferred organic bases include DIPEA or TEA in a suitable organic solvent such as DCM, DMF, DMA or MeCN. The reaction may be shaken or stirred at room temperature. The Weinreb amides may then be reacted with a suitable nucleophilic species, typically a Grignard or organolithium reagent in a suitable solvent such as THF or diethyl ether, to give a ketone of formula **(XXIV).** Treatment of a ketone of formula **(XXIV)** with POCl₃ in a suitable solvent such as DMF or DCM provides vinylogous aldehydes of formula **(XXIII),** which when combined with aniline of formula **(XXII)** in a suitable solvent such as DMF, DMA or DMSO can provide a functionalized quinoline of formula **(XXI).**

Compounds of formula **(XXVI)** and **(XXII)** are commercially available or may be synthesized by those skilled in the art.

### General Scheme 8:

Bromoquinolines of formula **(XXVII)** may be prepared starting from a nitro carbonyl compound of formula **(XXIX),** for example a ketone or an aldehyde.

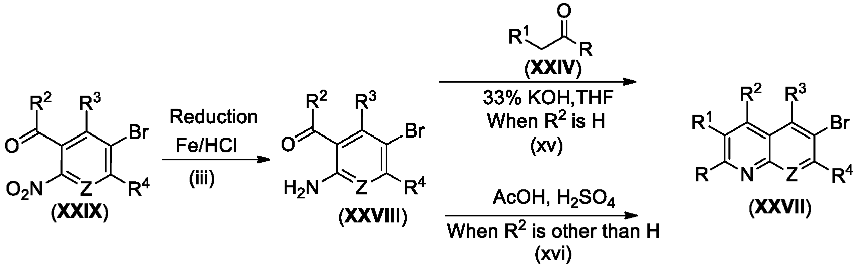

The nitro carbonyl compound of formula **(XXIX)** may be subjected to a reduction of the nitro group, for example using a transition metal catalyst such as Fe in a suitable acid, for example HCl. Alternative reduction methods include any of those described in General Scheme 3. The product of the reduction is an aniline compound of formula **(XXVIII)** which may be condensed in a Friedlander quinoline synthesis with a ketone of formula **(XXIV)** using either a strong base such as KOH or NaOH in a suitable solvent such as THF, dioxane or ethanol, or alternatively using a strong acid such as sulfuric acid or para-toluene sulfonic acid in a suitable solvent such as AcOH depending on the nature of the group R². The product of the condensation reaction is a quinoline of formula **(XXVII).**

### General Scheme 9:

An alternative synthesis of a nitro-quinoline of formula **(XXX)** starts from a bromo-nitro benzaldehyde of formula **(XXXII).** The halide may be displaced using a nitrogen nucleophile such as NaN₃ or KN₃ in a suitable solvent such as DMF, DMA or NMP typically at elevated temperature to give an azide compound of formula **(XXXI).**

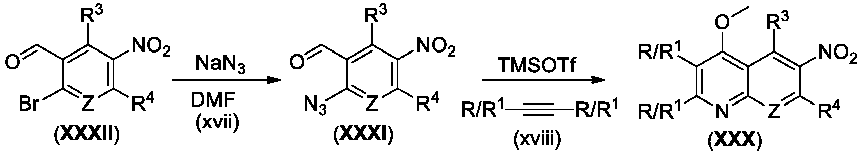

The azide compound of formula **(XXXI)** may then undergo reaction with an alkyne and suitable reagent such as TMSOTf or AgNTf₂ in a suitable solvent such as MeOH to give a functionalized quinoline compound of formula **(XXX).**

### General Scheme 10:

A flexible method of making halo-quinolines is shown in the below scheme, starting from an oxindole of formula **(XXXVIII).** An oxindole compound of formula **(XXXVIII)** may be reacted with a ketone of formula **(XXIV)** in the presence of a strong base such as NaOH or KOH, typically with heating or under reflux conditions to provide the key intermediate bromo-quinoline carboxylic acid of formula **(XXXVII).**

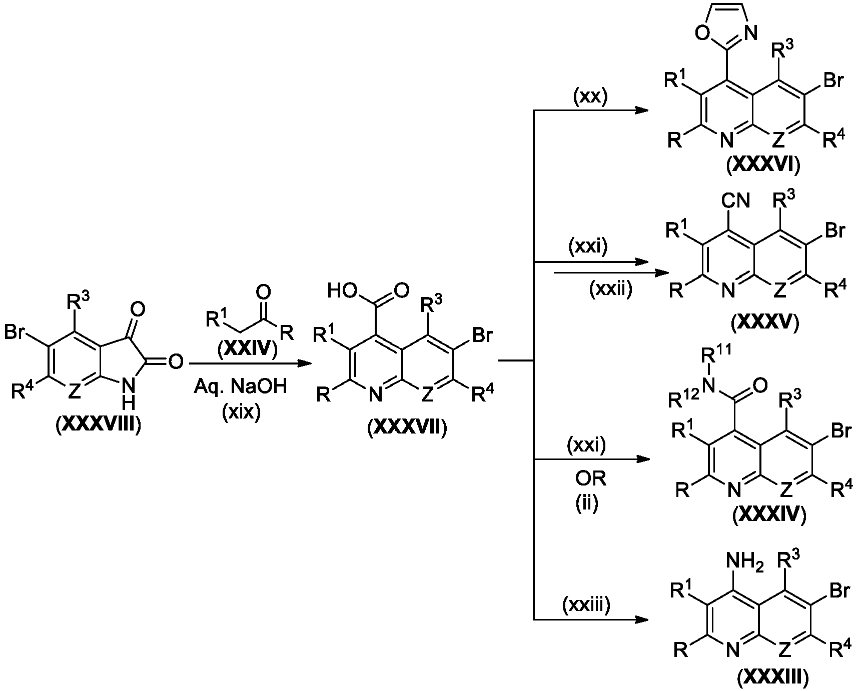

The acid function of **(XXXVII)** may then be elaborated into several different functional groups at the R² position according to the above scheme. For example, the acid of formula **(XXXVII)** may be converted into an α-acylaminocarbonyl compound which can then undergo a cyclodehydration reaction to form an oxazole compound of formula **(XXXVI).** Typically, this transformation will involve activation of the carboxylic acid followed by treating with an aminocarbonyl reagent such as 2-aminoacetaldehyde or a derivative thereof. Preferred activating agents include DCC, CDI, T₃P, HATU, HBTU or treatment of the acid with oxalyl chloride and a catalytic amount of DMF. Preferred organic bases include DIPEA or TEA in a suitable organic solvent such as DCM, DMF, DMA or MeCN. The reaction may be shaken or stirred at room temperature. Subsequently, the intermediate α-acylaminocarbonyl compound is then treated with a strong acid such as H₂SO₄ to provide the oxazoles of formula **(XXXVI).**

Alternatively, an acid of formula **(XXXVII)** may be converted to a nitrile of formula **(XXXV)** in a 2-step process that first converts the acid into a primary carboxamide. Typical conditions employ activation of the carboxylic acid of the compound of formula **(XXXVII)** using a suitable organic base and a suitable activating agent, and then treating the activated acid with an ammonia source. Preferred activating agents are DCC, CDI, T₃P, HATU, HBTU or treatment of the acid with oxalyl chloride and a catalytic amount of DMF. Preferred organic bases include DIPEA or TEA in a suitable organic solvent such as DCM, DMF, DMA or MeCN. Preferred ammonia sources are NH₄OH and NH₄OAc. The reaction may be shaken or stirred at room temperature. The primary carboxamides thus formed may subsequently be dehydrated to provide the nitriles of formula **(XXXV).** Typical conditions involve treating the carboxamide with a suitable reagent, preferably SOCl₂, Burgess' Reagent, POCl₃ or TFAA at temperatures between room temperature and reflux to provide the nitriles of formula **(XXXV).**

Alternatively, an acid of formula **(XXXVII)** may be converted to an amide of formula **(XXXIV)** using the methods described in General Scheme 2. Typical conditions employ activation of the carboxylic acid of the compound of formula **(XXXVII)** using a suitable organic base and a suitable coupling agent. Preferred coupling agents are either EDCI with HOBt, T₃P, HATU, HBTU or BOP. Preferred organic bases include DIPEA or TEA in a suitable organic solvent such as DCM, DMF, DMA or MeCN. The reaction may be shaken or stirred at room temperature.

Alternatively, an acid of formula **(XXXVII)** may be converted to an amine of formula **(XXXIII)** using a suitable azido reagent such as diphenyl phosphorazide in a suitable solvent such as DMF, DMA, MeCN or NMP in the presence of a suitable base such as DIPEA or TEA. Reactions are typically carried out at room temperature. The intermediate acyl azide thus formed is then typically treated with water under heating or reflux conditions to provide an amine of formula **(XXXIII).**

### General Synthetic Procedures

### General Purification and Analytical Methods

All final compounds were purified by either Combi-flash or prep-HPLC purification, and analyzed for purity and product identity by UPLC or LCMS according to one of the following methods.

### Prep-HPLC

Preparative HPLC was carried out on a Waters auto purification instrument using either an YMC Triart C18 column (250 x 20 mm, 5 µm) or a Phenyl Hexyl column (250 x 21.2 mm, 5 µm) or a Kinetex C18 column (250 x 21.2 mm, 10 µm) operating at between ambient temperature and 50 °C with a flow rate of 16.0 - 50.0 mL/min. Mobile phase 1: A = 20mM Ammonium Bicarbonate in water, B = Acetonitrile; Gradient Profile: Mobile phase initial composition of 80% A and 20% B, then 60% A and 40% B after 3 min., then 30% A and 70% B after 20 min., then 5% A and 95% B after 21 min., held at this composition for 1 min. for column washing, then returned to initial composition for 3 min.

Mobile phase 2: A = 10mM Ammonium Acetate in water, B = Acetonitrile; Gradient Profile: Mobile phase initial composition of 90% A and 10% B, then 70% A and 30% B after 2 min., then 20% A and 80% B after 20 min., then 5% A and 95% B after 21 min., held at this composition for 1 min. for column washing, then returned to initial composition for 3 min.

Mobile phase 3: A = 0.1% Formic acid in water, B = Acetonitrile; Gradient Profile: Mobile phase initial composition of 90% A and 10% B, then 70% A and 30% B after 2 min., then 20% A and 80% B after 20 min., then 5% A and 95% B after 21 min., held at this composition for 1 min. for column washing, then returned to initial composition for 3 min.

### LCMS method

General 5 min method: Zorbax Extend C18 column (50 x 4.6 mm, 5 µm) or Luna C18 column (50 x 4.6 mm, 5 µm) operating at ambient temperature and a flow rate of 1.2 mL/min. Mobile phase: A = 10 mM Ammonium Acetate in water, B = Acetonitrile; Gradient profile: from 90 % A and 10 % B to 70 % A and 30% B in 1.5 min, and then to 10 % A and 90 % B in 3.0 min, held at this composition for 1.0 min, and finally back to initial composition for 2.0 min.

### UPLC method

UPLC was carried out on a Waters auto purification instrument using a Zorbax Extend C18 column (50 x 4.6 mm, 5 µm) or Kinetex Evo C18 column (100 x 2.1 mm, 1.7 µm) at ambient temperature and a flow rate of 0.3 to 1.5ml/min.

Mobile phase 1: A = 5 mM Ammonium Acetate in water, B = 5 mM Ammonium Acetate in 90/10 Acetonitrile/water; Gradient profile from 95% A and 5% B to 65% A and 35% B in 2 min., then to 10% A and 90% B in 3.0 min., held at this composition for 4.0 min. and finally back to the initial composition for 5.0 min.

Mobile phase 2: A = 0.05 % formic acid in water, B = Acetonitrile; Gradient profile from 98 % A and 2 % B over 1 min., then 90 % A and 10 % B for 1 min., then 2 % A and 98 % B for 2 min. and then back to the initial composition for 3 min.

### General Procedure 1: Urea formation

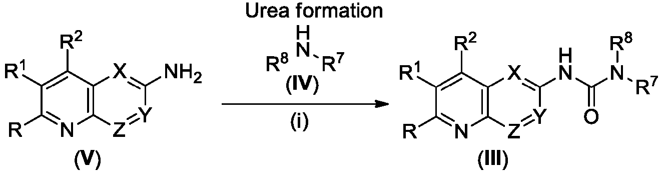

To a stirred solution of aromatic amine **(V)** (1.0 eq.) in a suitable solvent, such as DCM, DMF, MeCN or THF (8 mL/mmol) was added p-nitrophenyl chloroformate (1.1 eq.) at 0-5 °C and the whole stirred for 1-3 h at RT. TEA or DIPEA (6 eq.) and amine R⁸-NH-R⁷ **(IV)** (2.0 eq.) were then added dropwise and the whole further stirred for 1-5 h at RT. Progress of the reaction was monitored by TLC/LCMS and after completion the reaction mass was diluted with EtOAc and washed with a dilute solution of a suitable inorganic base such as NaHCO₃ or 1N NaOH and finally with brine. The organic layer was dried over anhydrous Na₂SO₄ and evaporated in vacuo to give a residue which was purified by column chromatography or combi-flash or prep-HPLC to afford a compound of formula **(III)** (yield 4-80%) as an off white to white solid. A similar procedure can be followed to synthesize all ureas of formula **(III).**

### General Procedure 2: Amidation

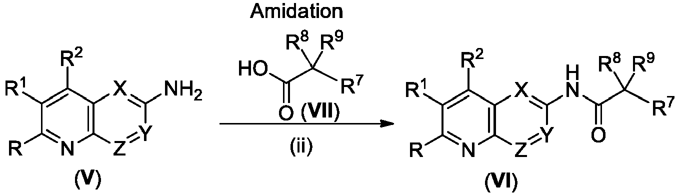

To a stirred solution of carboxylic acid **(VII)** (1.5 eq.) in a suitable solvent, such as DCM, DCE, DMA, THF, MeCN or DMF (5 mL/mmol) was added a suitable organic base, such as TEA or DIPEA (1.5 eq.) and a coupling reagent, such as T₃P, HATU, EDC.HCI, HOBT, BOP, HBTU or HATU (1.5 eq.). Stirring was continued for 10 min. and then the aromatic amine **(V)** (1.0 eq.) was added. The whole was stirred at RT for 1-5 h. Progress of the reaction was confirmed by TLC/LCMS and after completion the reaction mixture was diluted with water and extracted with MTBE or EtOAc or DCM, washed with brine, dried over anhydrous Na₂SO₄ and evaporated in vacuo to give the crude product which was purified by column chromatography or combi-flash or prep-HPLC to afford a compound of formula **(VI)** (25-90 %) as an off white to white solid. A similar procedure can be followed to synthesize all amides of formula **(VI).**

### General Procedure 3: Nitro group Reduction

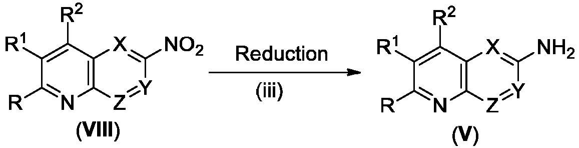

### Option A:

To a stirred solution of a compound of formula **(VIII)** (1.0 eq.) in a suitable solvent such as DMF, EtOH, MeOH or THF (4 mL/mmol) was added aq. HCl (0.12 mL/mmol, 36% w/w in water) followed by SnCl₂.2H₂O or SnCl₂ (4.0 eq.) at RT and the resulting reaction mixture was stirred at RT to 100 °C for 1-5 h. Progress of the reaction was monitored by TLC/LCMS and after completion the reaction mixture was diluted with EtOAc and washed with a saturated solution of NaHCO₃ or 0.5N NaOH solution and finally with brine. The organic layer was dried over anhydrous Na₂SO₄ and concentrated in vacuo to afford the crude product which was purified by column chromatography to give a compound of formula **(V)** (yield 70-80%) as an off white to yellow solid.

### Option B:

To a stirred solution of a compound of formula **(VIII)** (1.0 eq.) in a suitable solvent, such as EtOAc, EtOH or MeOH (7 mL/mmol) was added 10% Pd-C (68 mg/mmol, 50% w/w in water) under an inert atmosphere and the resulting reaction mixture was stirred under H₂ gas balloon pressure at RT for 2-12 h. Progress of the reaction was monitored by TLC and UPLC-MS which showed complete conversion of the nitro group to its corresponding amino group. The H₂ gas balloon was removed and the reaction mixture filtered under an inert atmosphere. The obtained filtrate was evaporated in vacuo to give a compound of formula **(V)** as crude which was used in the next step without any further purification.

### General Procedure 4: Amination

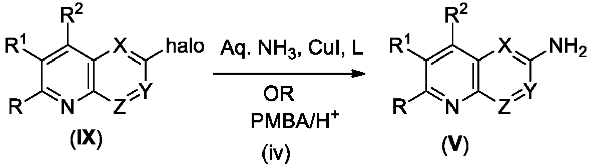

### Option A: Amination by Aq. NH3

A solution of a compound of formula **(IX)** (1.0 eq.), CuI (0.05 eq.), a suitable ligand such as BINAP, N1,N2-bis(4-phenoxyphenyl)oxalamide, trans-4-hydroxy-L-proline or L-proline (0.05 eq.) and a suitable base, such as KHCO₃, K₃PO₄ or K₂CO₃ (1.0 eq.) and DMSO (11 mL/mmol) in a sealed tube was purged with argon for 30 min. then aq. NH₃ (0.5 mL/mmol, 36% w/w in water) was added and the whole stirred at 60-100 °C for 4-16 h. After completion of the reaction (monitored by TLC and LCMS) the reaction mixture was diluted with water and extracted with EtOAc. The organic layer was washed with brine, dried over anhydrous Na₂SO₄ and concentrated in vacuo to give the title compound as a crude brown solid.

### Option B: Amination using p-methoxy benzyl amine

To a stirred solution of a compound of formula **(IX)** (1.0 eq.) in a suitable solvent, such as DMF or DMSO (4 mL/mmol) was added p-methoxy benzyl amine (6 eq.) followed by a suitable base, such as KHCO₃, K₃PO₄ or K₂CO₃ (2.5 eq.) under an inert atmosphere. CuI (1.0 eq.) and a suitable ligand, such as BINAP, N1,N2-bis(4-phenoxyphenyl)oxalamide, trans-4-hydroxy-L-proline or L-proline( 0.5 eq.) were then added. The resulting reaction mixture was transferred to a sealed tube and heated at 60-100 °C for 4-16 h. Progress of the reaction was monitored by LCMS and after completion the reaction mixture was poured into ice-water. The aqueous reaction mixture was extracted with EtOAc and washed with brine. The organic layer was dried over anhydrous Na₂SO₄ and concentrated in vacuo to give a residue which was stirred with a suitable acid, such as HCl or TFA at RT. The resulting reaction mixture was quenched in ice-water, basified with solid K₂CO₃ and extracted with EtOAc. The combined organic layers were washed with brine, dried and concentrated in vacuo to give the crude product which was purified by column chromatography/combi-flash to afford a compound of formula **(V)** (yield 60-90%) as a light brown to yellow solid.

### General Procedure 5: Bromination

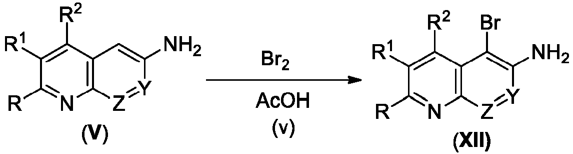

To a stirred solution of a compound of formula (V) (1.0 eq.) in AcOH (8 mL/mmol) was added Br₂ (1.0 eq.) in AcOH (2mL/mmol) at 0-5 °C. The whole was stirred at RT for 1-4 h. Progress of the reaction was monitored by LCMS and after completion the reaction mixture was quenched with a saturated aqueous solution of sodium metabisulfite. The aqueous mass was basified with solid NaHCO₃ and extracted with EtOAc, washed with brine, dried and concentrated to give the crude product which was purified by column chromatography to afford a compound of formula **(XII)** (80-95% yield) as an off white solid.

### General Procedure 6: Cyanation

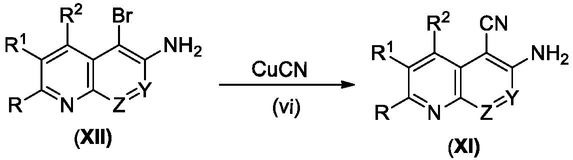

To a stirred solution of a compound of formula **(XII)** (1.0 eq.) in NMP (3 mL/mmol) was added CuCN (4 eq.) and the whole stirred in a sealed tube at 100-120 °C for 10-16 h. Progress of the reaction was monitored by TLC/LCMS and after completion the reaction mixture was diluted with water and extracted with MTBE. The organic layer was washed with a saturated solution of NH₄Cl and finally with brine, dried and concentrated in vacuo to give the crude product which was purified by column chromatography to afford a compound of formula **(XI)** (60-80% yield) as an off white to light brown solid.

### General Procedure 7: Suzuki-Miyaura Cross Coupling Reaction

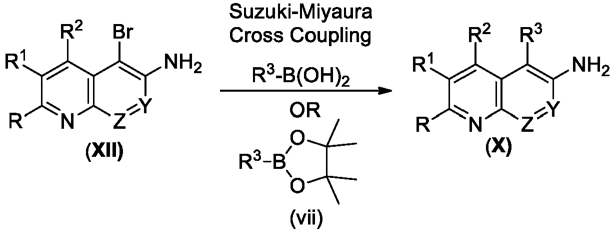

A solution of a compound of formula **XII** (4.805 mmol, 1.0 eq.), R³-B(OH)₂ (2.0 eq.) and a suitable base, such as K₂CO₃, TEA, NaHCO₃ or K₃PO₄ (3.0 eq.) in a mixture of solvents 1,4-dioxane (4 mL/mmol) and water (0.5 mL/mmol) was degassed with argon for 30 min. then a suitable transition metal catalyst, such as Pd(OAc)₂, Pd(PPh₃)₄, Pd(PPh₃)₂Cl₂ or Pd₂(dba)₃ (0.1 eq.) and a suitable ligand, such as BINAP or PCy₃ (0.2 eq.) were added. The resulting reaction mixture was stirred at 80-100 °C for 4-16 h under an inert atmosphere. Progress of the reaction was monitored by TLC/LCMS and after completion the reaction mixture was diluted with EtOAc, filtered and the filtrate was partitioned by the addition of water. The layers were separated and the aqueous layer was extracted with EtOAc. The combined organic layers were washed with brine, dried over anhydrous Na₂SO₄ and concentrated in vacuo to afford the crude product which was purified by column chromatography to give a compound of formula (yield 50-80%) as a pale yellow solid

### General Procedure 8: Nitration

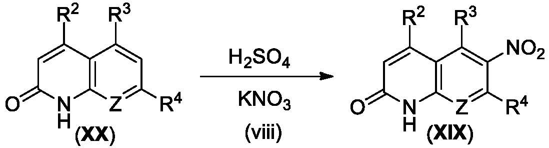

To a stirred solution of a compound of formula **(XX)** (1.0 eq.) in H₂SO₄ (3 mL/mmol) was added KNO₃ (1.1 eq.) portionwise at -10-0 °C . The whole was stirred at 0-5 °C for 30 min. Completion of the reaction was confirmed by TLC/LCMS. The reaction mixture was quenched with ice-water and extracted with EtOAc. The combined organic layers were washed with brine, dried over anhydrous Na₂SO₄ and concentrated in vacuo to afford a compound of formula **(XIX)** as a crude yellow solid.

### General Procedure Q: Bromination by Aq. HBr

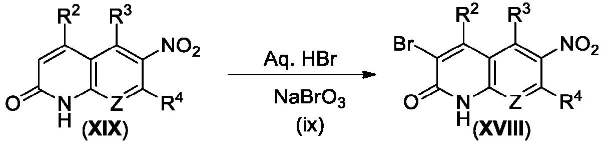

To a stirred solution of a compound of formula **(XIX)** (1.0 eq.) in water (0.6 mL/mmol) was added NaBrO₃ (1.2 eq.) at -10-5 °C, then aq. 48% HBr (3.5 mL/mmol) was added dropwise at the same temperature. The whole was then refluxed at 90-100 °C for 2-6 h. Progress of the reaction was monitored by TLC/LCMS and after completion the reaction mixture was quenched with ice-water and stirred at 0-5 °C for 1 h. The resulting solid precipitate was filtered off and washed with water to give a solid material which was slurry washed with hexane to afford a compound of formula **(XVIII)** (yield 65-80%) as a yellow solid.

### General Procedure 10: Bromination by POBr₃

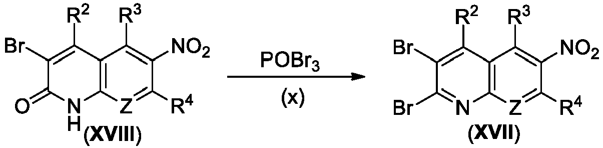

A solution of a compound of formula **(XVIII)** (1.0 eq.) in POBr₃ (2.0 eq.) was heated at 120-140 °C for 10-16 h. Progress of the reaction was monitored by TLC/LCMS and after completion the reaction mixture was quenched with cold water and basified with aqueous NaHCO₃ to pH 7-8. Then the aqueous mass was extracted with EtOAc and washed with brine. The combined organic layers were dried over anhydrous Na₂SO₄ and concentrated under reduced pressure to give a compound of formula **(XVII)** as a crude brown solid.

### General Procedure 11: Weinreb amide formation

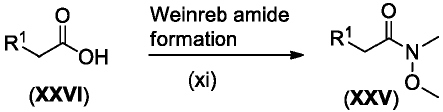

To a stirred solution of a compound of formula **(XXVI)** (1.0 eq.) in DCM (1.3 mL/mmol) was added a catalytic amount of DMF (2 drops) and oxalyl chloride (1.5 eq.) dropwise at 0-5 °C. The resulting reaction mixture was stirred at RT for 1-2 h and then commercially available N,O-dimethylhydroxylamine hydrochloride (1.0 eq.) added, followed by TEA (4.0 eq.) at 0-5 °C and the mixture was further stirred at RT for 30 min. Progress of the reaction was monitored by TLC and LCMS, and after completion the reaction mixture was diluted with water and extracted with DCM. The organic layer was dried over anhydrous Na₂SO₄ and concentrated in vacuo to give the crude product which was purified by column chromatography or combi-flash to afford a compound of formula **(XXV)** (yield 65-80%) as an off white solid.

### General Procedure 12: Grignard Reaction

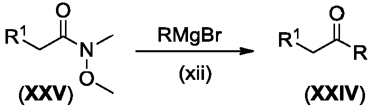

To a stirred solution of a compound of formula **(XXV)** (1.0 eq.) in a suitable solvent, such as dry diethyl ether or dry THF (3.2 mL/mmol) was added R-MgBr (0.67 mL/mmol, 3M solution in diethyl ether) at -78 °C. The mixture was warmed to RT and stirred at RT for 2-3 h. The reaction was monitored by TLC and LCMS which now showed completion of the reaction. The reaction mixture was quenched with a saturated solution of ammonium chloride and extracted with EtOAc. The organic layer was washed with brine, dried over anhydrous Na₂SO₄ and concentrated in vacuo to give the crude product which was purified by column chromatography or combi-flash to afford a compound of formula **(XXIV)** (yield 80-90%) as a white solid.

### General Procedure 13: Vilsmeier-Haack Reaction

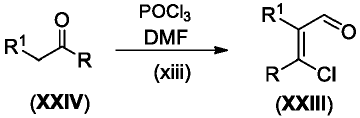

To a stirred DMF (0.24 mL/mmol) was added POCl3 (3.0 eq.) dropwise at 0-5 °C and the whole was stirred at RT for 1-2 h, before a compound of formula **(XXIV)** (1.0 eq.) was added slowly at RT. The resulting reaction mixture was stirred at 55-60 °C for 10-16 h. Progress of the reaction was monitored by TLC/LCMS and after completion the reaction mixture was poured into crushed ice and stirred at RT for 1-2 h. The obtained solid precipitate was collected by filtration, dissolved in EtOAc, dried over anhydrous Na₂SO₄ and the solvent removed in vacuo to afford a compound of formula **(XXIII)** (yield 85-95%) as a crude off white solid which was used in the next step without any further purification.

### General Procedure 14: Condensation

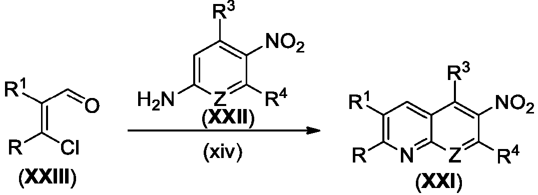

A stirred solution of a compound of formula **(XXII)** (1.0 eq.) and a compound of formula **(XXIII)** (1.0 eq.) in AcOH (0.36 mL/mmol) was refluxed for 10-16 h. TLC showed formation of new polar spot. The mass of the product was confirmed by LCMS. The reaction mixture was diluted with cold water and the product was extracted with EtOAc. The organics were washed with water followed by brine, dried over anhydrous Na₂SO₄ and concentrated under reduced pressure to give the crude product which was purified by silica gel column chromatography or combi-flash to afford a compound of formula **(XXI)** (yield 30-45%) as a yellow solid.

### General Procedure 15: Condensation

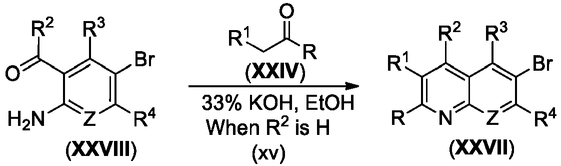

To a stirred solution of a compound of formula **(XXIV)** (1.0 eq.) and a compound of formula **(XXVIII)** (1.0 eq.) in ethanol (8 mL/mmol) was added 33% aq. KOH solution (0.81 mL/mmol) or piperidine at RT. The resulting reaction mixture was refluxed at 70-75 °C for 1-4 h. The reaction was monitored by TLC and LCMS and after completion the solvents were evaporated to give a residue which was diluted with EtOAc and washed with brine. The organic layer was dried over anhydrous Na₂SO₄ and concentrated in vacuo to give the crude product which was purified by column chromatography or combi-flash to afford a compound of formula **(XXVII)** (yield 30-50%) as a white solid.

### General Procedure 16: Condensation

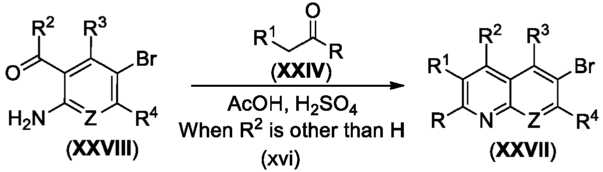

To a stirred solution of a compound of formula **(XXVIII)** (1.0 eq.) in glacial acetic acid (4.4 mL/mmol) was added a compound of formula **(XXIV)** (1.0 eq.) and conc. H₂SO₄ (0.1 mL/mmol). The whole was heated at 110-120 °C for 4-6 h. The reaction was monitored by TLC and LCMS and after completion the solvents were partially evaporated under reduced pressure to give a residue which was dissolved in water and extracted with EtOAc. The organic layer was washed with a saturated solution of NaHCO₃ followed by brine. The organic layer was dried over anhydrous Na₂SO₄ and concentrated in vacuo to give the crude product which was purified by column chromatography or combi-flash to afford a compound of formula **(XXVII)** (yield 40-50%) as a light yellow solid.

### General Procedure 17: Azide formation

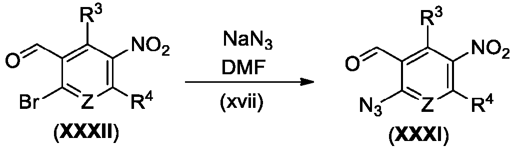

To a stirred solution of a compound of formula **(XXXII)** (1.0 eq.) in a suitable solvent, such as HMPA or DMF was added NaN₃ (1.5 eq.) at 45-50 °C and stirring was continued at the same temperature for 30 min. The reaction was monitored by TLC and after complete consumption of the bromide reagent the reaction mixture was quenched with ice-water and stirred for another 30 min. to give a solid precipitate which was filtered off. The filtrate was then extracted with MTBE, washed with brine, dried over anhydrous Na₂SO₄ and concentrated in vacuo to afford a compound of formula **(XXXI)** (yield 75-85 %) as a yellow solid.

### General Procedure 18: Condensation

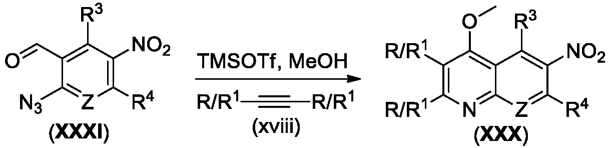

To a stirred solution of a compound of formula **(XXXI)** (1.0 eq.) in dry DCM (2.5 mL/mmol) was added MeOH (0.16 mL/mmol) followed by an acetylene derivative (1.0 eq.) at RT. The reaction mixture was cooled to 0-5 °C and TMSOTf (4.0 eq.) then added dropwise. The resulting reaction mixture was stirred at RT for 10-16 h. Progress of the reaction mixture was monitored by TLC/LCMS and after completion the reaction mixture was diluted with EtOAc and washed with brine. The organic layer was dried over anhydrous Na₂SO₄ and concentrated in vacuo to afford the crude product which was purified by column chromatography or combi-flash to give a compound of formula **(XXX)** (yield 80-90%) as a yellow solid.

### General Procedure 19: Pfitzinger Reaction

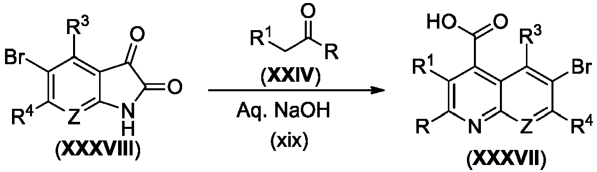

A stirred solution of a compound of formula **(XXXVIII)** (1.0 eq.) in 2N NaOH solution (4.5 mL/mmol) was refluxed at 85-90 °C for 30 min. The reaction mixture was then cooled to RT and a compound of formula **(XXIV)** (1.1 eq.) was added at RT. The whole was refluxed at 85-90 °C for 10-16 h. Progress of the reaction was monitored by TLC/LCMS and after completion the reaction mixture was filtered and the obtained solid was dissolved in water and acidified with 2N HCl solution to give a solid precipitate which was filtered and washed with water to afford a compound of formula **(XXXVII)** (25-45%) as a white solid.

### General Procedure 20: Oxazole formation

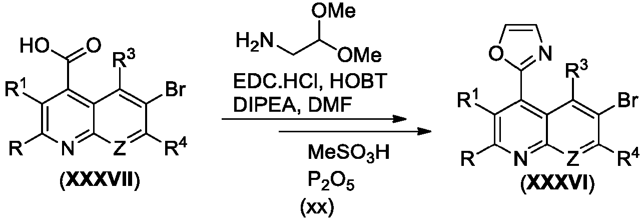

To a stirred solution of a compound of formula **(XXXVII)** (1.0 eq.) in a suitable solvent, such as DCM, THF or DMF (2.5 mL/mmol) was added an organic base, such as TEA or DIPEA (4.0 eq.), and a coupling agent such as EDC.HCl (1.5 eq.) and HOBT (1.5 eq.) at RT and the whole stirred at RT for 10-15 min. 2,2-dimethoxyethanamine (1.2 eq.) was then added and the whole allowed to stir at RT for 10-12 h. Progress of the reaction mixture was monitored by TLC/LCMS and after completion the reaction mixture was diluted with water and extracted with EtOAc. The combined organics were washed with brine, dried over anhydrous Na₂SO₄ and concentrated in vacuo to give the crude product which was purified by column chromatography to afford a corresponding amide intermediate (40-50%) as an off white solid.

The obtained amide intermediate (1.0 eq.) was dissolved in methanesulfonic acid (5 mL/mmol) at RT and then P₂O₅ (3.0 eq.) added. The whole was heated at 120-130 °C for 3-5 h. Progress of the reaction was monitored by TLC and after complete consumption of amide; the reaction mixture was poured into ice-water and extracted with EtOAc. The combined organics were washed with brine, dried over anhydrous Na₂SO₄ and concentrated in vacuo to give the crude product which was purified by column chromatography to afford a compound of formula **(XXXVI)** (yield 35-40%) as an off white solid.

### General Procedure 21: Amide/cyanide via acid chloride formation

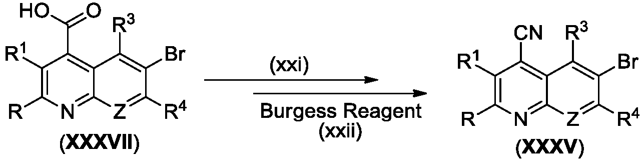

To a stirred solution of a compound of formula **(XXXVII)** (1.0 eq.) in a mixture of solvents DCM (4 mL/mmol) and DMF (0.015 mL/mmol) was added oxalyl chloride (2.0 eq.) dropwise at -5 to 0 °C and the mixture stirred at RT for 1-2 h. Formation of the corresponding acid chloride was confirmed by LCMS. Then aq. NH₃ or another amine (3.0 eq., 33% in water) was added dropwise at 0-5 °C. The resulting reaction mixture was stirred at RT for 2-3 h. Progress of the reaction was confirmed by TLC/LCMS and after completion the obtained solid was filtered and washed with hexane. The filtrate was extracted to recover the dissolved material with 10% MeOH in DCM and washed with brine. The combined organic layers were dried over anhydrous Na₂SO₄ and evaporated in vacuo to give the corresponding amide intermediate as crude.

### General Procedure 22: Burgess Reaction

The obtained amide intermediate (1.0 eq.) was dissolved in dry THF (12 mL/mmol), then Burgess Reagent (2.0 eq.) added at 0-5 °C. The whole was heated at 50-60 °C for 1-2 h under an inert atmosphere. After completion of the reaction (monitored by TLC/LCMS) the solid obtained was filtered off and the filtrate was diluted with water and extracted with 5% MeOH in DCM. The combined organic layers were washed with brine, dried over anhydrous Na₂SO₄ and concentrated in vacuo to give a compound of formula **(XXXV)** (yield 70-80%) as a white solid.

### General Procedure 23: Curtius Reaction

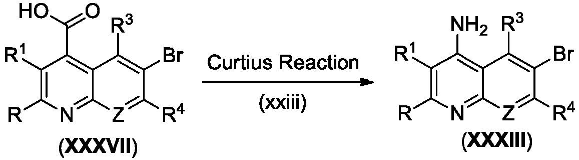

To a stirred solution of a compound of formula **(XXXVII)** (1.0 eq.) in DMF (6 mL/mmol) was added DIPEA or TEA (1.5 eq.), and diphenyl phosphorazide (2.0 eq.) at 0-5 °C under a N₂ atmosphere. The whole was stirred at RT for 1-3 h then water (6 mL/mmol) was added and the whole refluxed at 100-110 °C for 2-6 h. Progress of the reaction was monitored by LCMS and after completion the reaction mixture was diluted with water to give a solid precipitate which was filtered and washed with water followed by hexane. The obtained solid was dried in a vacuum oven to afford a compound of formula **(XXXIII)** (yield 80-85%) as a pale yellow solid.

### Examples

Nuclear magnetic resonance (NMR) spectra were in all cases consistent with the proposed structures. Characteristic chemical shifts (δ) are given in parts-per-million downfield from tetramethylsilane (for ¹H-NMR) and upfield from trichloro-fluoromethane (for ¹⁹F NMR) using conventional abbreviations for designation of major peaks: e.g. s, singlet; d, doublet; t, triplet; q, quartet; m, multiplet; br, broad. The following abbreviations have been used for common solvents: CDCl₃, deuterochloroform; d₆-DMSO, deuterodimethylsulphoxide; and CD₃OD, deuteromethanol.

Mass spectra, MS (m/z), were recorded using electrospray ionisation (ESI). Where relevant and unless otherwise stated the m/z data provided are for isotopes ¹⁹F, ³⁵Cl, ⁷⁹Br and ¹²⁷I.

All chemicals, reagents and solvents were purchased from commercial sources and used without further purification. All reactions were performed under an atmosphere of nitrogen unless otherwise noted.

Flash column chromatography was carried out using pre-packed silica gel cartridges in a Combi-Flash platform. Prep-HPLC purification was carried out according to the General purification and analytical methods described above. Thin layer chromatography (TLC) was carried out on Merck silica gel 60 plates (5729). All final compounds were >95% pure as judged by the LCMS or UPLC analysis methods described in the General purification and analytical methods above unless otherwise stated.

### Example 45: 1-(2,2-Difluorobutyl)-3-(3-(2-fluorophenyl)-2-phenylquinolin-6-yl)urea

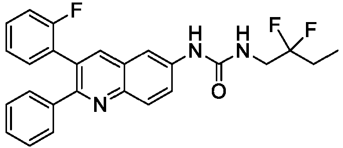

Example 45 was prepared according to the methods described in General Procedures 1, 3, 13, 14 and the methods described below.

### Preparation 1: 3-(2-Fluorophenyl)-2-phenylquinolin-6-amine

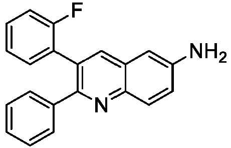

### Step 1: 2-(2-Fluorophenyl)-1-phenylethanone

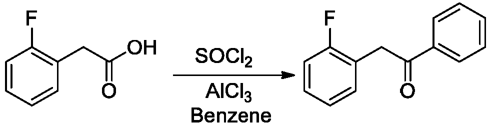

To a stirred solution of commercially available 2-(2-fluorophenyl)acetic acid (35.0 g, 222.93 mmol) in benzene (350 mL) was added thionyl chloride (105 mL) at 0-5 °C and the resulting reaction mixture was allowed to slowly warmup to RT and was then refluxed for 4 h. The solvent was removed under high vacuum and the leftover residue was diluted with benzene (105 mL). AlCl₃ (32.615 g, 245.22 mmol) was then added carefully portionwise at 0-5 °C, and the reaction mixture then allowed to slowly warm to RT and stir at this temperature for 3 h. The reaction was monitored by TLC/ LCMS and after completion the reaction mixture was poured into crushed ice, extracted with EtOAc and then washed with water followed by brine. The organic layer was dried over anhydrous Na₂SO₄ and concentrated under reduced pressure to give the title compound (35 g, yield 73.3%) as a white solid. LCMS m/z: 215 [M+H].

### Step 2: 3-Chloro-2-(2-fluorophenyl)-3-phenylacrylaldehyde

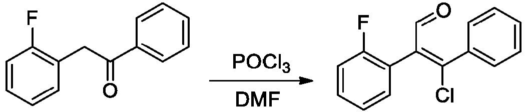

To a stirred DMF (55 mL, 700.93 mmol) was added POCl3 (65.4 mL, 700.93 mmol) dropwise at 0-5 °C and the whole was stirred at RT for 1 h, before 2-(2-fluorophenyl)-1-phenylethanone (Preparation 1, Step 1) (50.0 g, 233.65 mmol) was added slowly at RT. The resulting reaction mixture was stirred at 60 °C for overnight. Progress of the reaction was monitored by TLC/LCMS and after completion the reaction mixture was poured into crushed ice and stirred at RT for 1 h. The obtained solid precipitate was collected by filtration, dissolved in EtOAc, dried over anhydrous Na₂SO₄ and the solvent removed in vacuo to afford the title compound (55.0 g, yield 90%) as a crude off white solid which was used in the next step without any further purification. LCMS m/z: 261 [M+H].

### Step 3: 3-(2-Fluorophenyl)-6-nitro-2-phenylquinoline

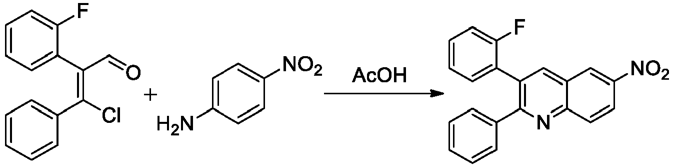

A stirred solution of commercially available p-nitroaniline (26.54 g, 192.307 mmol) and 3-chloro-2-(2-fluorophenyl)-3-phenylacrylaldehyde (Preparation 1, Step 2) (50.0 g, 192.307 mmol) in AcOH (70 mL) was refluxed for 16 h. TLC showed formation of new polar spot. The mass of the product was confirmed by LCMS. The reaction mixture was diluted with cold water and the product was extracted with EtOAc. The organics were washed with water followed by brine, dried over anhydrous Na₂SO₄ and concentrated under reduced pressure to give the crude product which was purified by silica gel column chromatography to afford the titled compound (21 g, yield 31.7%) as a yellow solid. LCMS m/z: 245 [M+H].

### Step 4: 3-(2-Fluorophenyl)-2-phenylquinolin-6-amine

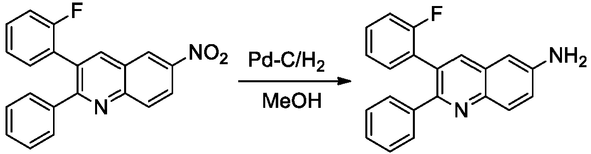

To a stirred solution of 3-(2-fluorophenyl)-6-nitro-2-phenylquinoline (Preparation 1, Step 3) (500 mg, 1.453 mmol) in MeOH (10 mL) was added 10% Pd-C (100 mg, 50% w/w in water) under an inert atmosphere and the resulting reaction mixture was stirred under H₂ gas balloon pressure at RT for 2 h. Progress of the reaction was monitored by TLC and UPLC-MS which showed complete conversion of the nitro group into its corresponding amino group. The H₂ gas balloon was removed and the reaction mixture filtered under an inert atmosphere. The obtained filtrate was evaporated in vacuo to give the title compound (450 mg) as crude which was used in the next step without any further purification. LCMS m/z: 315 [M+H].

### Preparation 2: 1-(2,2-Difluorobutyl)-3-(3-(2-fluorophenyl)-2-phenylquinolin-6-yl)urea (Example 45)

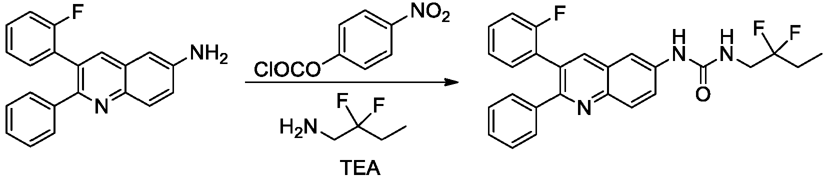

To a stirred solution of 3-(2-fluorophenyl)-2-phenylquinolin-6-amine (Preparation 1, Step 4) (404 mg, 1.286 mmol) in THF (10 mL) was added p-nitrophenyl chloroformate (285 mg, 1.415) at 0-5 °C and the whole stirred for 1 h at RT. TEA (781 mg, 7.718 mmol) and 2,2-difluorobutan-1-amine (280.06 mg, 2.573 mmol) were then added dropwise and further stirred for 3 h at RT. Progress of the reaction was monitored by TLC/LCMS and after completion the reaction mas was diluted with EtOAc and washed with 1N NaOH solution and finally with brine. The organic layer was dried over anhydrous Na₂SO₄ and evaporated in vacuo to give a residue which was purified by prep-HPLC to afford the title compound (25 mg, yield 4%) as a white solid. LCMS m/z: 450.21 [M+H]; Purity 99.86%; ¹H NMR (500 MHz; DMSO-d₆): δ 0.99 (t, *J =* 7.5 Hz. 3H), 1.86-1.97 (m, 2H), 3.59-3.66 (m, 2H), 6.71 (t, *J =* 6.2 Hz, 1H), 7.09-7.13 (m, 1H), 7.25-7.30 (m 4H), 7.35-7.42 (m 3H), 7.46-7.49 (m, 1H), 7.76-7.78 (m, 1H), 7.99 (d, *J =* 9.05 Hz, 1H), 8.15 (d, *J =* 2.2 Hz, 1H), 8.28 (s, 1H), 9.08 (s, 1H).

### Example 15: (R)-1-(3-(2-Fluorophenyl)-2-phenylquinolin-6-yl)-3-(2-hydroxybutyl)urea

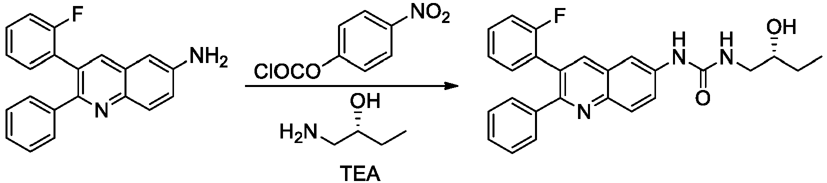

Example 15 was prepared according to the methods described for the preparation of Example 45 and the method described below.

To a stirred solution of 3-(2-fluorophenyl)-2-phenylquinolin-6-amine (Preparation 1, Step 4) (9.0 g, 28.66 mmol) in THF (90 mL) was added p-nitrophenyl chloroformate (6.4 g, 31.526 mmol) at 0-5 °C and the whole stirred at RT for 3 h. TEA (11.58 g, 114.64 mmol) and (R)-1-aminobutan-2-ol (2.55 g, 28.66 mmol) were then added dropwise and further stirred for 2 h at RT. Progress of the reaction was monitored by TLC/LCMS and after completion the reaction mixture was diluted with water and extracted with EtOAc. The organic layer was washed with 1N NaOH solution to remove any p-nitrophenol and with 1N HCl to remove unreacted amine, and finally with brine solution. The organic layer was dried over anhydrous Na₂SO₄ and concentrated in vacuo to give the crude product which was purified by trituration with acetone and hexane to afford the title compound (7.0 g, yield 57%) as a white solid. LCMS m/z: 430.34 [M+H]; Purity 99.46%; ¹H NMR (500 MHz; DMSO-d₆): δ 0.90 (t, *J =* 7.4 Hz, 3H), 1.34-1.46 (m 2H), 2.99-3.03 (m, 1H), 3.23-3.28 (m, 1H), 3.44 (t, *J =* 6.5 Hz, 1H), 4.80 (d, *J =* 5.15 Hz, 1H), 6.34 *(t, J* = 5.55 Hz, 1H), 7.11 (t, *J =* 8.95 Hz, 1H), 7.25-7.29 (m, 4H), 7.35-7.46 (m, 3H), 7.46-7.49 (m, 1H), 7.74 (d, *J =* 6.9 Hz, 1H), 7.97 (d, *J =* 9.05 Hz, 1H), 8.13 (d, *J =* 2.1 Hz, 1H), 8.25 (s, 1H), 9.06 (s, 1H).

### Example 72: (R)-1-(2-Hydroxybutyl)-3-(2-methyl-3-phenylquinolin-6-yl)urea

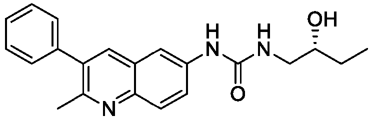

Example 72 was prepared according to the methods described in General Procedures 1, 4, 11, 14, 15 and the methods described below.

### Preparation 3: 2-Methyl-3-phenylquinolin-6-amine

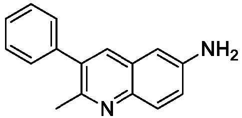

### Step 1: N-Methoxy-N-methyl-2-phenylacetamide

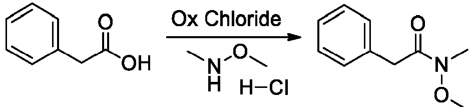

To a stirred solution of commercially available phenyl acetic acid (4.18 g, 30.753 mmol) in DCM (40 mL) was added a catalytic amount of DMF (2 drops) and oxalyl chloride (5.855 g, 46.135 mmol) dropwise at 0-5 °C. The resulting reaction mixture was stirred at RT for 2 h and then commercially available N,O-dimethylhydroxylamine hydrochloride (3.0 g, 30.75 mmol) added, followed by TEA (12.45 g, 123.03 mmol) at 0-5 °C and the mixture was further stirred at RT for 30 min. Progress of the reaction was monitored by TLC and LCMS, and after completion the reaction mixture was diluted with water and extracted with DCM. The organic layer was dried over anhydrous Na₂SO₄ and concentrated in vacuo to give the crude product which was purified by column chromatography to afford the title compound (4.0 g, yield 72.7%) as an off white solid. LCMS m/z: 180 [M+H].

### Step 2: 1-Phenylpropan-2-one

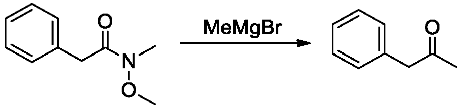

To a stirred solution of N-methoxy-N-methyl-2-phenylacetamide (Preparation 3, Step 1) (1.0 g, 5.586 mmol) in dry THF (18 mL) was added methyl magnesium bromide (1.332 g, 3.72 mL, 3M solution in diethyl ether) at -78 °C under an inert atmosphere and the reaction mixture was maintained at the same temperature for 1 h. LCMS indicated that the reaction was ~ 60% complete. The mixture was warmed to RT and stirred at RT for 2 h. The reaction was monitored by TLC and LCMS which now showed completion of the reaction. The reaction mixture was quenched with a saturated solution of ammonium chloride and extracted with EtOAc. The organic layer was washed with brine, dried over anhydrous Na₂SO₄ and concentrated in vacuo to give the crude product which was purified by column chromatography to afford the title compound (0.7 g, yield 93%) as a white solid. LCMS m/z: 135 [M+H].

### Step 3: 6-Bromo-2-methyl-3-phenylquinoline

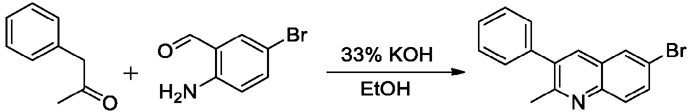

To a stirred solution of 1-phenylpropan-2-one (Preparation 3, Step 2) (1.098 g, 8.146 mmol) and commercially available 2-amino-5-bromobenzaldehyde (1.646 g, 8.146 mmol) in ethanol (65 mL) was added a 33% aq. KOH solution (6.584 mL) at RT. The resulting reaction mixture was refluxed at 70 °C for 1 h. The reaction was monitored by TLC and LCMS and after completion the solvents were evaporated to give a residue which was diluted with EtOAc and washed with brine. The organic layer was dried over anhydrous Na₂SO₄ and concentrated in vacuo to give the crude product which was purified by column chromatography to afford the title compound (0.94 g, yield 38%) as a white solid. LCMS m/z: 298 [M+H].

### Step 4: N-(4-Methoxybenzyl)-2-methyl-3-phenylquinolin-6-amine

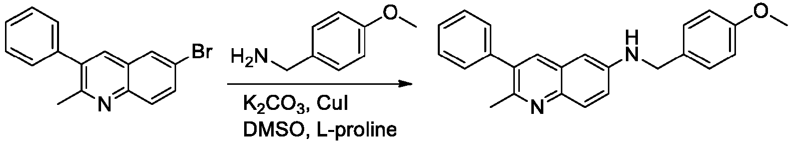

To a stirred solution of 6-bromo-2-methyl-3-phenylquinoline (Preparation 3, Step 3) (921 mg, 3.099 mmol) in DMSO (12 mL) was added p-methoxy benzyl amine (2.44 mL, 18.594 mmol) followed by K₂CO₃ (1070 mg, 7.747 mmol) under an inert atmosphere, then CuI (590 mg, 3.099 mmol) and L-proline (178.68 mg, 1.549 mmol) were added. The resulting reaction mixture was transferred to a sealed tube and heated at 90 °C for 12 h. Progress of the reaction was monitored by LCMS and after completion the reaction mixture was poured into ice-water. The aqueous reaction mixture was extracted with EtOAc and washed with brine. The organic layer was dried over anhydrous Na₂SO₄ and concentrated in vacuo to give a crude product which was purified by column chromatography to afford the title compound (951 mg, yield 87%) as a yellow solid. LCMS m/z: 355 [M+H].

### Step 5: 2-Methyl-3-phenylquinolin-6-amine

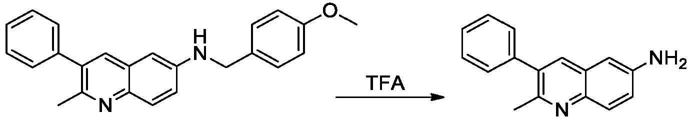

A solution of N-(4-methoxybenzyl)-2-methyl-3-phenylquinolin-6-amine (Preparation 3, Step 4) (920 mg, 2.137 mmol) in TFA (10 mL) was stirred at RT for 12 h. Progress of the reaction was monitored by TLC/LCMS and after completion the reaction mixture was neutralized with an aqueous solution of NaHCO₃, extracted with EtOAc and washed with a saturated solution of NaHCO₃ followed by brine. The combined organics were dried over anhydrous Na₂SO₄ and concentrated in vacuo to give the title compound (644 mg, purity 90%) as a crude solid which was used in the next step without any further purification. LCMS m/z: 235 [M+H].

### Preparation 4: (R)-1-(2-Hydroxybutyl)-3-(2-methyl-3-phenylquinolin-6-yl)urea (Example 72)

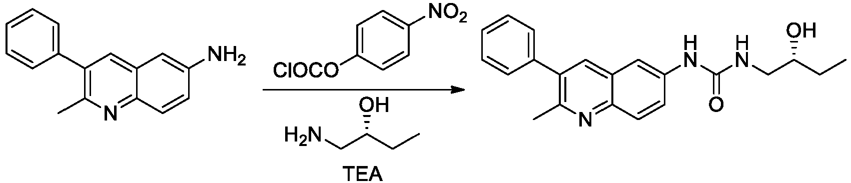

To a stirred solution of 2-methyl-3-phenylquinolin-6-amine (Preparation 3, Step 5) (644 mg, 2.751 mmol) in THF (6 mL) was added p-nitrophenyl chloroformate (609.8 mg, 3.026 mmol) and the whole stirred at RT for 1 h. After complete consumption of the amine by TLC, TEA (1.535 mL, 11.00 mmol) and (R)-1-aminobutan-2-ol (269.68 mg, 3.025 mmol) were added dropwise and the combined mixture was further stirred at RT for 3 h. Progress of the reaction was monitored by TLC/LCMS and after completion the reaction mixture was diluted with EtOAc, washed with 1N NaOH solution thrice followed by brine and 1N HCl solution and again with brine. The organic layer was dried over anhydrous Na₂SO₄ and concentrated in vacuo to give the crude product which was purified by crystallization with acetone and hexane to afford the title compound (393 mg, yield 41%) as a white solid. LCMS m/z: 350.22 [M+H]; Purity 99.71%; ¹H NMR (400 MHz; DMSO-d₆): δ 0.88 (t, *J =* 7.44 Hz, 3H), 1.28-1.44 (m, 2H), 2.50 (s, 3H), 2.93-2.99 (m, 1H), 3.18-3.32 (m, 1H), 3.40 (d, *J =* 4.84 Hz, 1H), 4.76 (d, *J* = 4.96 Hz, 1H), 6.25 (t, *J =* 5.72 Hz, 1H), 7.40-7.50 (m, 5H), 7.61-7.64 (m, 1H), 7.82 (d, *J* = 9.0 Hz, 1H), 7.95 (s, 1H), 7.99 (s, 1H), 8.90 (s, 1H).

### Example 101: (R)-1-(2-Hydroxybutyl)-3-(4-methoxy-2,3-diphenylquinolin-6-yl)urea

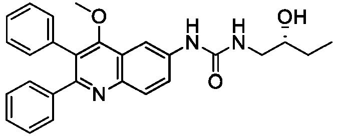

Example 101 was prepared according to the methods described in General Procedures 1, 3, 17, 18 and the method described below.

### Preparation 5: 4-Methoxy-2,3-diphenylquinolin-6-amine

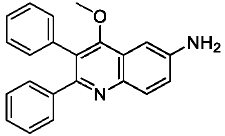

### Step 1: 2-Azido-5-nitrobenzaldehyde

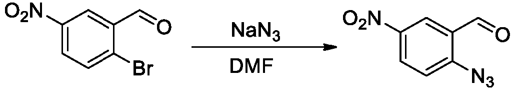

To a stirred solution of commercially available 2-bromo-5-nitrobenzaldehyde (2.0 g, 8.684 mmol) in DMF was added NaN₃ (0.869 g, 13.378 mmol) at 50 °C and the reaction mixture maintained at this temperature for 30 min. The reaction was monitored by TLC and after complete consumption of the starting material; the reaction mixture was quenched with ice-water and stirred for another 30 min. to give a solid precipitate which was filtered off. The filtrate was then extracted with MTBE, washed with brine, dried over anhydrous Na₂SO₄ and concentrated in vacuo to afford the title compound (1.35 g, yield 80.8 %) as a yellow solid. LCMS m/z: 193 [M+H].

### Step 2: 4-Methoxy-6-nitro-2,3-diphenylquinoline

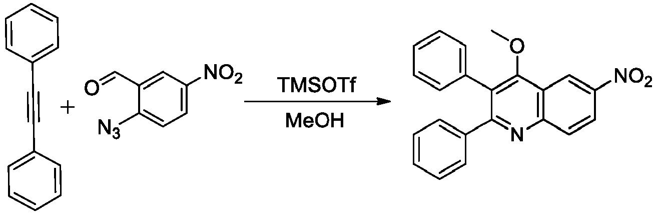

To a stirred solution of 2-azido-5-nitrobenzaldehyde (Preparation 5, Step 1) (1.35 g, 5.862 mmol) in dry DCM was added MeOH (0.94 mL, 23.44 mmol) followed by diphenyl acetylene (1.044 g, 5.86 mmol) at RT. The reaction mixture was cooled to 0-5 °C and TMSOTf (5.22 g, 23.44 mmol) then added dropwise. The resulting reaction mixture was stirred at RT for 12 h. Progress of the reaction mixture was monitored by TLC/LCMS and after completion the reaction mixture was diluted with EtOAc and washed with brine. The combined organic layers were dried over anhydrous Na₂SO₄ and concentrated in vacuo to afford the crude product which was purified by column chromatography to give the title compound (2.2 g, yield 88%) as a yellow solid. LCMS m/z: 357.25 [M+H].

### Step 3: 4-Methoxy-2,3-diphenylquinolin-6-amine

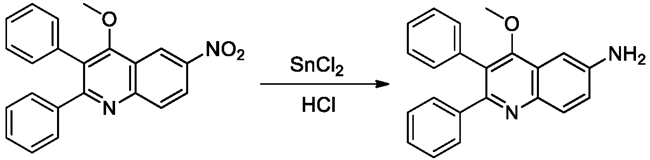

To a stirred solution of 4-methoxy-6-nitro-2,3-diphenylquinoline (Preparation 5, Step 2) (832 mg, 2.335 mmol) in THF (10 mL) was added aq. HCl (0.3 mL) followed by SnCl₂ (1742 mg, 9.338 mmol) at RT and the resulting reaction mixture was refluxed at 60 °C for 2 h. Progress of the reaction mixture was monitored by TLC/LCMS and after completion the reaction mixture was diluted with EtOAc and washed with 0.5N NaOH and brine. The organic layer was dried over anhydrous Na₂SO₄ and concentrated in vacuo to afford the crude product which was purified by column chromatography to give the title compound (605 mg, yield 79.5%) as a yellow solid. LCMS m/z: 327 [M+H].

### Preparation 6: (R)-1-(2-Hydroxybutyl)-3-(4-methoxy-2,3-diphenylquinolin-6-yl)urea (Example 101)

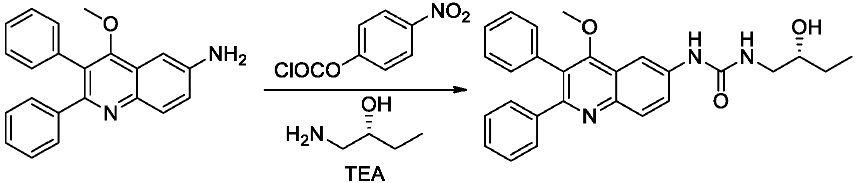

To a stirred solution of 4-methoxy-2,3-diphenylquinolin-6-amine (Preparation 5, Step 3) (605 mg, 1.855 mmol) in THF (6 mL) was added p-nitrophenyl chloroformate (411.29 mg, 2.041 mmol) and the whole stirred at RT for 1 h. After complete consumption of the amine by TLC, TEA (1.035 mL, 7.42 mmol) and (R)-1-aminobutan-2-ol (181.87 mg, 2.0405 mmol) were added dropwise and the combined mixture was further stirred at RT for 3 h. Progress of the reaction was monitored by TLC/LCMS and after completion the reaction mixture was diluted with EtOAc, washed with 1N NaOH solution thrice followed by brine and 0.02N HCl solution and again with brine. The organic layer was dried over anhydrous Na₂SO₄ and concentrated in vacuo to give the crude product which was purified by crystallization with acetone and hexane to afford the title compound (400 mg, yield 49%) as a white solid. LCMS m/z: 442.27 [M+H]; Purity 99.51%; ¹H NMR (400 MHz; DMSO-d₆): δ 0.88 (t, *J =* 7.4 Hz, 3H), 1.32-1.42 (m, 2H), 2.96-3.01 (m, 1H), 3.21-3.25 (m, 1H), 3.42 (d, *J =* 4.76 Hz, 1H). 3.49 (s, 3H), 4.75 (d, *J =* 5.16 Hz, 1H), 6.26 (t, *J =* 5.48 Hz, 1H), 7.16-7.33 (m, 10H), 7.67 (dd, *J'* = 2.36 Hz, *J"* = 9.04 Hz, 1H), 7.93 (d, *J =* 9.08 Hz, 1H), 8.29 (d, *J =* 2.44 Hz, 1H), 9.04 (s, 1H).

### Example 20: (R)-1-(2,3-Bis(2-fluorophenyl)quinolin-6-yl)-3-(2-hydroxybutyl)urea

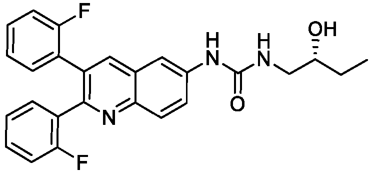

Example 20 was prepared according to the methods described in General Procedures 1, 3, 7, 8, 9, 10 and the method described below.

### Preparation 7: 2,3-Bis(2-fluorophenyl)quinolin-6-amine

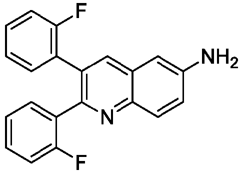

### Step 1: 6-Nitroquinolin-2(1H)-one

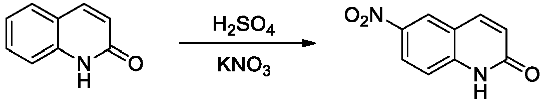

To a stirred solution of commercially available quinolin-2(1H)-one (5.0 g, 34.44 mmol) in H₂SO₄ (100 mL, 1.876 mmol) was added KNO₃ (3.83 g, 37.884 mmol) portionwise at -10 to 0 °C. The whole was stirred at 0-5 °C for 30 min. Completion of the reaction was confirmed by TLC/LCMS. The reaction mixture was quenched with ice-water and extracted with EtOAc. The combined organic layers were washed with brine, dried over anhydrous Na₂SO₄ and concentrated in vacuo to afford the title compound (7.4 g, crude) as a yellow solid. LCMS m/z: 188.97 [M-H].

### Step 2: 3-Bromo-6-nitroquinolin-2(1H)-one

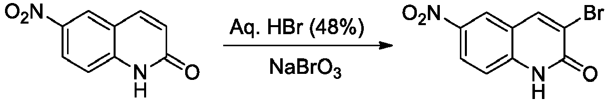

To a stirred solution of 6-nitroquinolin-2(1H)-one (Preparation 7, Step 1) (7.315 g, 38.46 mmol) in water (25.15 mL) was added NaBrO₃ (6.965 g, 46.16 mmol) at -10 to 5 °C, then aq. 48% HBr (133.8 mL) was added dropwise at the same temperature. The whole was then refluxed at 100 °C for 4 h. Progress of the reaction was monitored by TLC/LCMS and after completion the reaction mixture was quenched in ice-water and stirred at 0-5 °C for 1 h. The resulting solid precipitate was filtered off and washed with water to give a solid material which was slurry washed with hexane (500 mL) to afford the title compound (8.0 g, yield 77.3 %) as a yellow solid. LCMS m/z: 266.98 [M-H].

### Step 3: 2,3-Dibromo-6-nitroquinoline

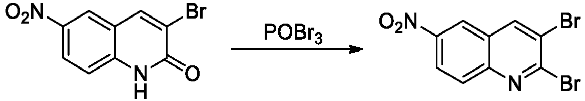

A solution of 3-bromo-6-nitroquinolin-2(1H)-one (Preparation 7, Step 2) (2.0 g, 7.435 mmol) in POBr₃ (4.263 g, 14.869 mmol) was heated at 140 °C for 12 h. Progress of the reaction was monitored by TLC/LCMS and after completion the reaction mixture was quenched with cold water and basified with aqueous NaHCO₃ to pH 7-8. The aqueous mixture was extracted with EtOAc and washed with brine. The combined organic layers were dried over anhydrous Na₂SO₄ and concentrated under reduced pressure to give the title compound (1.6 g crude) as a crude brown solid. LCMS m/z: 231 [M+H].

### Step 4: 2,3-Bis(2-fluorophenyl)-6-nitroquinoline

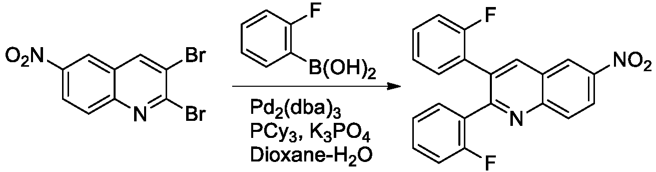

A solution of 2,3-dibromo-6-nitroquinoline (Preparation 7, Step 3) (1.6 g, 4.805 mmol), (2-fluorophenyl)boronic acid (1.7 g, 12.151 mmol) and K₃PO₄ (4.118 g, 19.399 mmol) in a mixture of solvents 1,4-dioxane (20 mL) and water (2 mL) was degassed with argon for 30 min. then Pd₂(dba)₃ (444 mg, 0.485 mmol) and PCy₃ (272 mg, 0.969 mmol) were added. The resulting reaction mixture was stirred at 100 °C for 16 h under an inert atmosphere. Progress of the reaction was monitored by TLC/LCMS and after completion the reaction mixture was diluted with EtOAc, filtered and the filtrate was partitioned with water. The layers were separated and the aqueous layer was extracted with EtOAc. The combined organic layers were washed with brine, dried over anhydrous Na₂SO₄ and concentrated in vacuo to afford the crude product which was purified by column chromatography to give the title compound (1.2 g, yield 68.73%) as a pale yellow solid. LCMS m/z: 363 [M+H].

### Step 5: 2,3-Bis(2-fluorophenyl)quinolin-6-amine

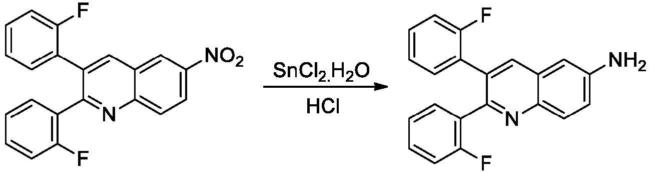

To a stirred solution of 2,3-bis(2-fluorophenyl)-6-nitroquinoline (Preparation 7, Step 4) (1.2 g, 3.312 mmol) in THF (15 mL) was added aq. HCl (0.2 mL) followed by SnCl₂.H₂O (2.98 g, 13.22 mmol) at RT and the resulting reaction mixture was then refluxed at 60 °C for 2 h. Progress of the reaction mixture was monitored by TLC/LCMS and after completion the reaction mixture was diluted with EtOAc and washed with a saturated aqueous solution of NaHCO₃ and brine. The organic layer was dried over anhydrous Na₂SO₄ and concentrated in vacuo to afford the crude product which was purified by column chromatography to give the title compound (1.0 g, yield 91%) as a light yellow solid. LCMS m/z: 333 [M+H].

### Preparation 8: (R)-1-(2,3-Bis(2-fluorophenyl)quinolin-6-yl)-3-(2-hydroxybutyl)urea (Example 20)

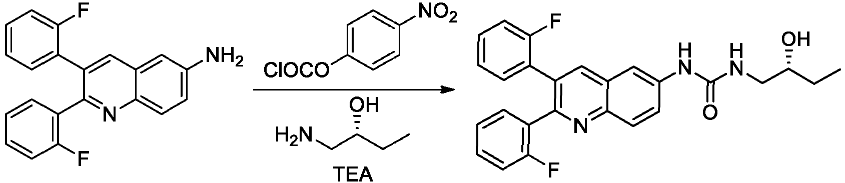

To a stirred solution of 2,3-bis(2-fluorophenyl)quinolin-6-amine (Preparation 7, Step 5) (1.0 g, 3.008 mmol) in THF (15 mL) was added p-nitrophenyl chloroformate (758 mg, 3.761 mmol) and the whole stirred at RT for 2 h. After complete consumption of the amine by TLC, TEA (1.257 g, 12.445 mmol) and (R)-1-aminobutan-2-ol (0.335 g, 3.764 mmol) were added dropwise and the combined mixture was further stirred at RT for 2 h. Progress of the reaction was monitored by TLC/LCMS and after completion the reaction mixture was diluted with EtOAc, washed with saturated solution of NaHCO₃ thrice followed by brine and 0.02N HCl solution and again with brine. The organic layer was dried over Na₂SO₄ and concentrated in vacuo to give the crude product which was purified by column chromatography to afford the title compound (0.6 g, yield 45%) as a white solid. LCMS m/z: 448.37 [M+H]; Purity 98.05%; ¹H NMR (500 MHz; DMSO-d₆): δ 0.90 *(t, J* = 7.4 Hz, 3H), 1.34-1.45 (m, 2H), 2.99-3.03 (m, 1H), 3.24-3.27 (m, 1H), 3.44 (d, *J =* 4.9 Hz, 1H), 4.81 (d, *J =* 5.1 Hz, 1H), 6.39 (t, *J =* 5.45 Hz, 1H), 7.04-7.20 (m, 4H), 7.30-7.36 (m, 3H), 7.44 (t, *J =* 7.35 Hz, 1H), 7.75-7.77 (m, 1H), 7.97 (d, *J =* 9.1 Hz, 1H), 8.17 (d, *J =* 2.05 Hz, 1H), 8.29 (s, 1H), 9.15 (s, 1H)

### Example 115: (R)-6-(3-(2-Hydroxybutyl)ureido)-3-methyl-N-(1-methyl-1H-pyrazol-3-yl)-2-phepylquinoline-4-carboxamide

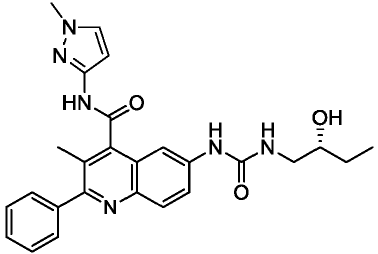

Example 115 was prepared according to the methods described in General Procedures 1, 4, 19, 23 and the method described below.

### Preparation 9: 6-Amino-3-methyl-N-(1-methyl-1H-pyrazol-3-yl)-2-phenylquinoline-4-carboxamide

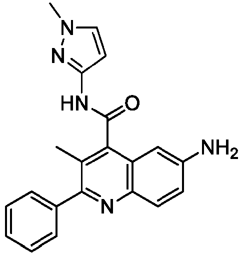

### Step 1: 6-Bromo-3-methyl-2-phenylquinoline-4-carboxylic acid

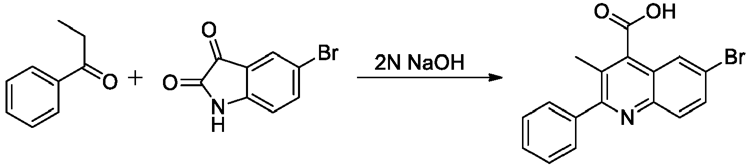

A stirred solution of commercially available 5-bromoisatin (3.0 g, 13.272 mmol) in 2N NaOH (60 mL) solution was refluxed at 90 °C for 30 min. The reaction mixture was then cooled to RT and commercially available propiophenone (1.959 g, 14.60 mmol) was added at RT. The whole was refluxed at 85-90 °C for 16 h. Progress of the reaction was monitored by TLC/LCMS and after completion the reaction mixture was filtered and the obtained solid was dissolved in water and acidified with 2N HCl solution to give a solid precipitate which was filtered and washed with water to afford the title compound (1.3 g, yield 28.6%) as a white solid. LCMS m/z: 342 [M+H].

### Step 2: 6-Bromo-3-methyl-N-(1-methyl-1H-pyrazol-3-yl)-2-phenylquinoline-4-carboxamide

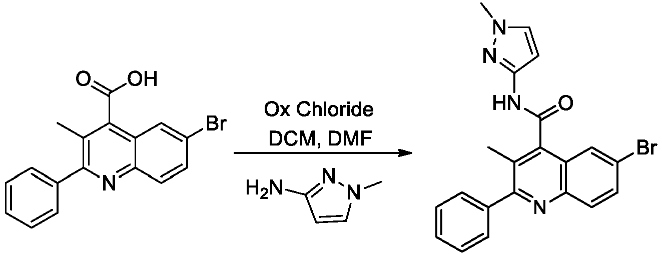

To a stirred solution of 6-bromo-3-methyl-2-phenylquinoline-4-carboxylic acid (Preparation 9, Step 1) (600 mg, 1.753 mmol) in a mixture of solvents DCM (10 mL) and DMF (10 mL) was added oxalyl chloride (0.229 mL, 2.63 mmol) dropwise at 0-5 °C. The whole was stirred at RT for 1 h under an inert atmosphere. After formation of the corresponding acid chloride (a small aliquot was quenched in MeOH and checked by LCMS), excess oxalyl chloride was evaporated in vacuo to give a residue. A pre-prepared mixture of 1-methyl-1H-pyrazol-3-amine (340 mg, 3.506 mmol) and TEA (0.977 mL, 7.01 mmol) in DCM (5 mL) was added dropwise into the concentrated acid chloride residue at low temperature and the resulting reaction mixture was further stirred at RT for 16 h. Progress of the reaction was monitored by TLC/LCMS and after completion the reaction mixture was diluted with DCM, washed with brine solution and dried over anhydrous Na₂SO₄. The organic layer was concentrated under reduced pressure to give the crude compound which was purified by combi-flash to afford the title compound (603 mg, yield 79.0%) as a yellow solid. LCMS m/z: 421.30 [M+H].

### Step 3: 6-((4-Methoxybenzyl)amino)-3-methyl-N-(1-methyl-1H-pyrazol-3-yl)-2-phenylquinoline-4-carboxamide

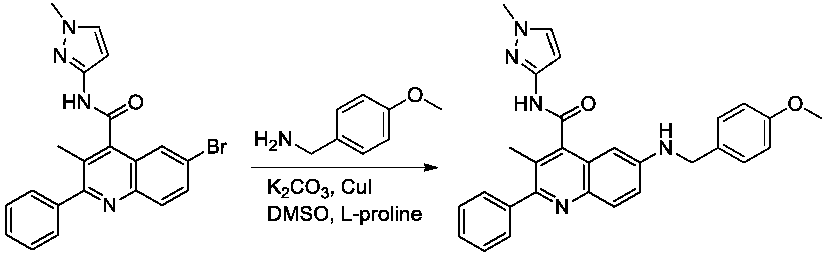

To a stirred solution of 6-bromo-3-methyl-N-(1-methyl-1H-pyrazol-3-yl)-2-phenylquinoline-4-carboxamide (Preparation 9, Step 2) (550 mg, 1.306 mmol) in DMSO (10 mL) was added p-methoxy benzyl amine (899 mg, 6.527 mmol) followed by K₂CO₃ (451 g, 2.263 mmol) under an inert atmosphere, then CuI (497 mg, 2.611 mmol) and L-proline ( 171 mg, 1.305 mmol) were added. The resulting reaction mixture was transferred to a sealed tube and heated at 90 °C for 14 h. Progress of the reaction was monitored by LCMS and after completion the reaction mixture was poured into ice-water. The aqueous reaction mixture was extracted with EtOAc and washed with brine. The organic layer was dried over anhydrous Na₂SO₄ and concentrated in vacuo to give a crude product which was purified by column chromatography to afford the title compound (484 mg, yield 77.5%) as a yellow solid. LCMS m/z: 478 [M+H].

### Step 4: 6-Amino-3-methyl-N-(1-methyl-1H-pyrazol-3-yl)-2-phenylquinoline-4-carboxamide

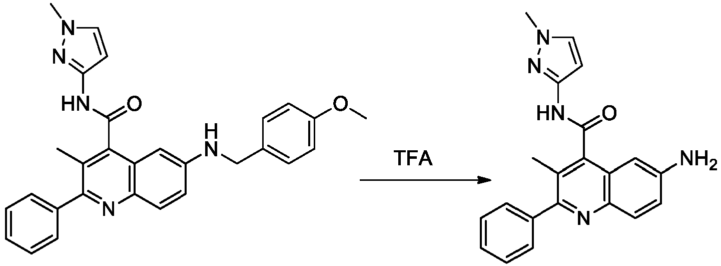

A solution of 6-((4-methoxybenzyl)amino)-3-methyl-N-(1-methyl-1H-pyrazol-3-yl)-2-phenylquinoline-4-carboxamide (Preparation 9, Step 3) (484 mg, 1.014 mmol) in TFA (5 mL) was stirred at RT for 14 h. Progress of the reaction was monitored by TLC/LCMS and after completion the reaction mixture was neutralized with an aqueous solution of NaHCO₃, extracted with 5% MeOH in DCM and washed with a saturated solution of NaHCO₃ followed by brine. The combined organics were dried over anhydrous Na₂SO₄ and concentrated in vacuo to give the title compound (450 mg, purity 91.69%) as a crude solid which was used in the next step without any further purification. LCMS m/z: 358.34 [M+H].

### Preparation 10: (R)-6-(3-(2-Hydroxybutyl)ureido)-3-methyl-N-(1-methyl-1H-pyrazol-3-yl)-2-phenylquinoline-4-carboxamide (Example 115)

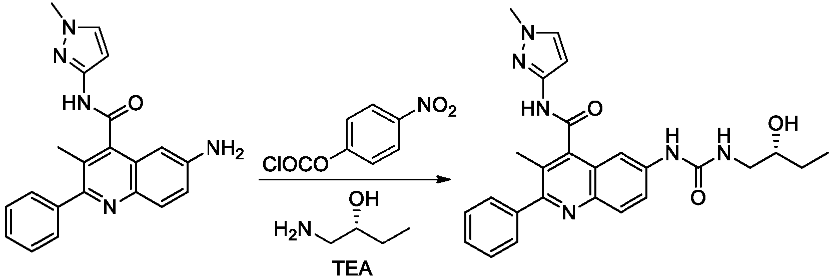

To a stirred solution of 6-amino-3-methyl-N-(1-methyl-1H-pyrazol-3-yl)-2-phenylquinoline-4-carboxamide (Preparation 9, Step 4) (212 mg, 0.593 mmol) in THF (5 mL) was added p-nitrophenyl chloroformate (132 mg, 0.653 mmol) and the whole stirred at RT for 1 h. After complete consumption of the amine by TLC, TEA (0.33 mL, 2.374 mmol) and (R)-1-aminobutan-2-ol (63.47 mg, 0.712 mmol) were added dropwise and the combined mixture was further stirred at RT for 3 h. Progress of the reaction was monitored by TLC/LCMS and after completion the reaction mixture was diluted with EtOAc, washed with a saturated solution of NaHCO₃ thrice followed by brine and 0.02N HCl solution and again with brine. The organic layer was dried over anhydrous Na₂SO₄ and concentrated in vacuo to give the crude product which was purified by prep-HPLC to afford the title compound (89.6 mg, yield 32%) as a white solid. LCMS m/z: 473.5 [M+H]; Purity 98.83%; ¹H NMR (500 MHz; DMSO-d₆): δ 0.88 (bs, 3H), 1.33-1.41 (m, 2H), 2.31 (s, 3H), 2.95 (bs, 1H), 3.20 (bs, 1H), 3.41 (s, 1H), 3.79 (s, 3H), 4.77 (s, 1H), 6.20 (s, 1H), 6.72 (s, 1H), 7.49-7.57 (m, 5H), 7.68 (s, 1H), 7.79-7.82 (m, 2H), 7.91 (d, *J =* 10.1 Hz, 1H), 9.07 (s, 1H)11.12 (s, 1H).

### Example 95: 1-(4-Amino-2,3-diphepylquinolin-6-yl)-3-(2-hydroxybutyl)urea

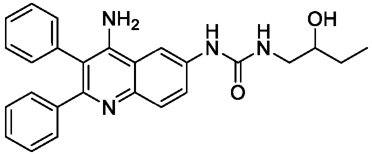

Example 95 was prepared according to the methods described in General Procedures 1, 4, 19, 22 and the method described below.

### Preparation 11: 2,3-Diphenylquinoline-4,6-diamine

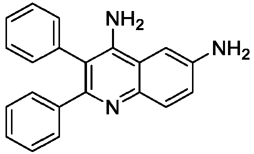

### Step 1: 6-Bromo-2,3-diphenylquinolin-4-amine

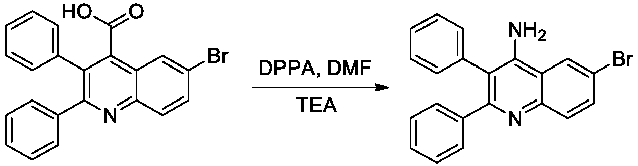

To a stirred solution of 6-bromo-2,3-diphenylquinoline-4-carboxylic acid (Synthesized according to method described in preparation 9, Step 1) (2.0 g, 4.948 mmol) in DMF (30 mL) was added TEA (1.03 mL, 7.42 mmol) at 0-5 °C under a N₂ atmosphere, and diphenyl phosphorazide (2.126 mL, 9.896 mmol) was added. The whole was stirred at RT for 2 h then water (30 mL) was added and refluxed at 100 °C for 4 h. Progress of the reaction was monitored by LCMS and after completion the reaction mixture was diluted with water to give a solid precipitate which was filtered and washed with water followed by hexane. The obtained solid was dried in a vacuum oven to afford the title compound (1.5 g, yield 81%) as a pale yellow solid. LCMS m/z: 375.40/377.35 [M+H].

### Step 2: N6-(4-Methoxybenzyl)-2,3-diphenylquinoline-4,6-diamine

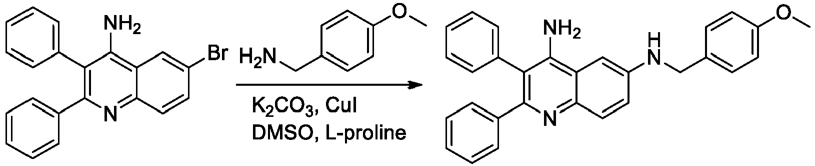

To a stirred solution of 6-bromo-2,3-diphenylquinolin-4-amine (Preparation 11, Step 1) (500 mg, 1.06 mmol) in DMSO (15 mL) was added p-methoxy benzyl amine (0.83 mL, 6.39 mmol) followed by K₂CO₃ (365.7 mg, 2.65 mmol) under an inert atmosphere, then CuI (100.9 mg, 0.53 mmol) and L-proline (48.8 mg, 0.424 mmol) were added. The resulting reaction mixture was transferred to a sealed tube and heated at 90 °C for 16 h. Progress of the reaction was monitored by LCMS and after completion the reaction mixture was poured into ice-water. The aqueous reaction mixture was extracted with EtOAc and washed with brine. The organic layer was dried over anhydrous Na₂SO₄ and concentrated in vacuo to give a crude product which was purified by column chromatography to afford the title compound (329 mg, yield 71.5%) as a yellow solid. LCMS m/z: 432.35 [M+H].

### Step 3: 2,3-Diphenylquinoline-4,6-diamine

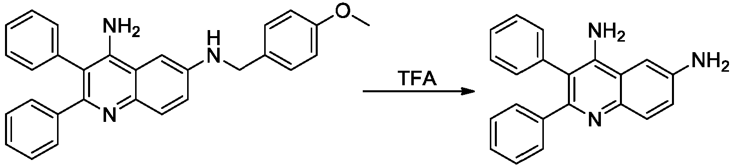

A solution of N6-(4-methoxybenzyl)-2,3-diphenylquinoline-4,6-diamine (Preparation 11, Step 2) (320 mg, 0.742 mmol) in TFA (7 mL) was stirred at RT for 16 h. Progress of the reaction was monitored by TLC/LCMS and after completion the reaction mixture was diluted with water and neutralized with solid NaHCO₃, extracted with EtOAc and washed with a saturated solution of NaHCO₃ followed by brine. The combined organics were dried over anhydrous Na₂SO₄ and concentrated in vacuo to give the title compound (400 mg, purity 88%) as a crude solid which was used in the next step without any further purification. LCMS m/z: 312.19 [M+H].

### Preparation 12: 1-(4-Amino-2,3-diphenylquinolin-6-yl)-3-(2-hydroxybutyl)urea (Example 95)

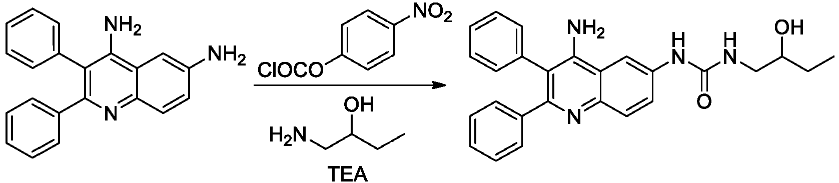

To a stirred solution of 2,3-diphenylquinoline-4,6-diamine (Preparation 11, Step 3) (80 mg, 0.257 mmol) in THF (3 mL) was added p-nitrophenyl chloroformate (64.78 mg, 0.321 mmol) and the whole stirred at RT for 1 h. After complete consumption of the amine by TLC, TEA (0.14 mL, 1.06 mmol) and 1-aminobutan-2-ol (0.03 mL, 0.312 mmol) were added dropwise and the combined mixture was further stirred at RT for 3 h. Progress of the reaction was monitored by TLC/LCMS and after completion the reaction mixture was diluted with EtOAc and washed with 0.5N NaOH solution followed by brine. The organic layer was dried over anhydrous Na₂SO₄ and concentrated in vacuo to give the crude product which was purified by prep-HPLC to afford the title compound (20 mg, yield 18.3%) as a white solid. LCMS m/z: 427.37 [M+H]; Purity 98.19%; ¹H NMR (500 MHz; DMSO-d₆): δ 0.90 *(t, J* = 7.4 Hz, 3H), 1.42-1.47 (m, 2H), 2.98-3.03 (m, 1H), 3.24-3.28 (m, 1H), 3.44-3.45 (m, 1H), 4.82 (d, *J* =4.95 Hz, 1H), 6.53 (s, 1H), 7.21 (d, *J =* 7.05 Hz, 2H), 7.36-7.39 (m, 10H), 7.87-7.92 (m, 2H), 8.34 (s, 1H), 8.99 (s, 1H).

### Example 94: N-(6-(3-(2-Hydroxybutyl)ureido)-2,3-diphenylquinolin-4-yl)methanesulfonamide

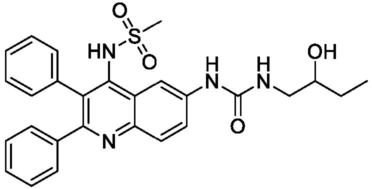

Example 94 was prepared according to the methods described in General Procedures 1, 4, 19, 22 and the method described below.

### Preparation 13: N-(6-Amino-2,3-diphenylquinolin-4-yl)methanesulfonamide

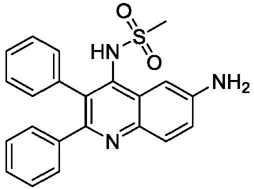

### Step 1: N-(6-Bromo-2,3-diphenylquinolin-4-yl)-N-(methylsulfonyl)methanesulfonamide

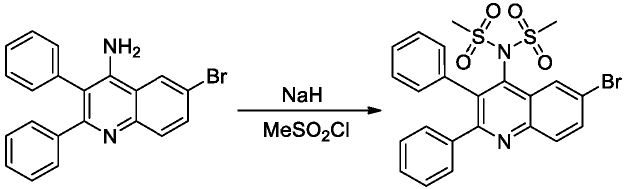

To a stirred solution of 6-bromo-2,3-diphenylquinolin-4-amine (Preparation 11, Step 1) (800 mg, 2.12 mmol) in dry DMF (10 mL) was added NaH (153 mg, 6.38 mmol, 60% suspension in mineral oil) at 0-5 °C and the whole stirred at RT for 20 min. Then MeSO₂Cl (0.5 mL, 6.36 mmol) was added dropwise at 0-5 °C and the whole stirred at RT for a further 2 h. Progress of the reaction was monitored by TLC/LCMS and after completion the reaction mixture was quenched with a saturated solution of NH₄Cl and extracted with MTBE and washed with water followed by brine. The organic layer was dried over anhydrous Na₂SO₄ and concentrated in vacuo to give the title compound (1.132 g crude) as a yellow gummy solid. LCMS m/z: 531.25 [M+H].

### Step 2: N-(6-Bromo-2,3-diphenylquinolin-4-yl)methanesulfonamide

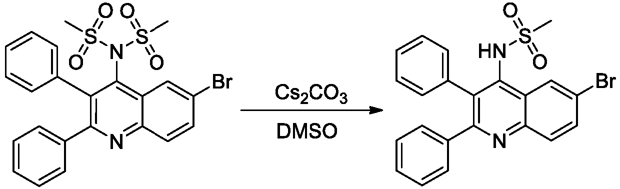

To a stirred solution of N-(6-bromo-2,3-diphenylquinolin-4-yl)-N-(methylsulfonyl)methane-sulfonamide (Preparation 13, Step 1) (1.132 g, 2.13 mmol) in DMSO (10 mL) was added Cs₂CO₃ (2.776 g, 8.5 mmol) and the mixture heated in a sealed tube at 120 °C for 16 h. The reaction was checked by TLC/LCMS and after completion the reaction mixture was quenched with ice-water and extracted with EtOAc, washed with brine, dried over anhydrous Na₂SO₄ and concentrated in vacuo to give the crude product which was purified by combi-flash to afford the title compound (900 mg, yield 93%) as a brown solid. LCMS m/z: 453.17 [M+H].

### Step 3: N-(6-((4-Methoxybenzyl)amino)-2,3-diphenylquinolin-4-yl)methanesulfonamide

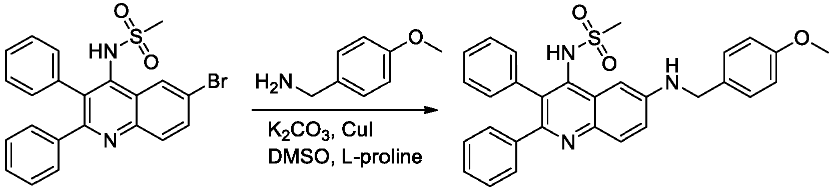

To a stirred solution of N-(6-bromo-2,3-diphenylquinolin-4-yl)methanesulfonamide (Preparation 13, Step 2) (383 mg, 0.84 mmol) in DMSO (15 mL) was added p-methoxy benzyl amine (0.667 mL, 5.06 mmol) followed by K₂CO₃ (289.8 mg, 2.1 mmol) under an inert atmosphere, then CuI (80 mg, 0.50 mmol) and L-proline (39 mg, 0.336 mmol) were added. The resulting reaction mixture was transferred to a sealed tube and heated at 90 °C for 16 h. Progress of the reaction was monitored by LCMS and after completion the reaction mixture was poured into ice-water. The aqueous reaction mixture was extracted with EtOAc and washed with brine. The organic layer was dried over anhydrous Na₂SO₄ and concentrated in vacuo to give a crude product which was purified by column chromatography to afford the title compound (300 mg, yield 69.7%) as a yellow solid. LCMS m/z: 510.37 [M+H].

### Step 4: N-(6-Amino-2,3-diphenylquinolin-4-yl)methanesulfonamide

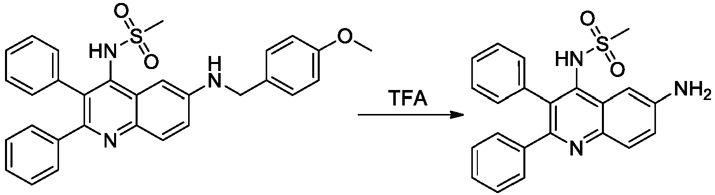

A solution of N-(6-((4-methoxybenzyl)amino)-2,3-diphenylquinolin-4-yl)methanesulfonamide (Preparation 13, Step 3) (293 mg, 0.575 mmol) in TFA (4 mL) was stirred at RT for 16 h. Progress of the reaction was monitored by TLC/LCMS and after completion the reaction mixture was diluted with water and neutralized with solid NaHCO₃, extracted with EtOAc and washed with a saturated solution of NaHCO₃ followed by brine. The combined organics were dried over anhydrous Na₂SO₄ and concentrated in vacuo to give the title compound (400 mg, crude) as a yellow gummy semisolid which was used in the next step without any further purification. LCMS m/z: 390.31 [M+H].

### Preparation 14: N-(6-(3-(2-Hydroxybutyl)ureido)-2,3-diphenylquinolin-4-yl)methanesulfonamide (Example 94)

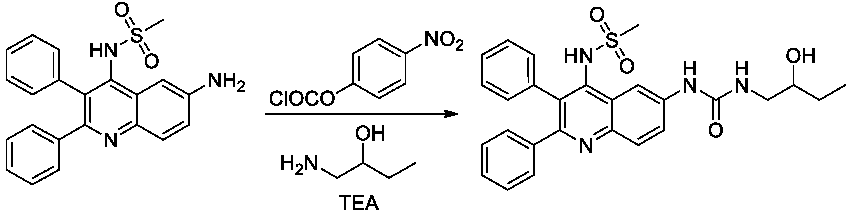

To a stirred solution of N-(6-amino-2,3-diphenylquinolin-4-yl)methanesulfonamide (Preparation 13, Step 4) (400 mg, 1.02 mmol) in THF (5 mL) was added p-nitrophenyl chloroformate (258.86 mg, 1.28 mmol) and the whole stirred at RT for 1 h. After complete consumption of the amine by TLC, TEA (0.58 mL, 4.207 mmol) and 1-aminobutan-2-ol (0.12 mL, 1.28 mmol) were added dropwise and the combined mixture was further stirred at RT for 3 h. Progress of the reaction was monitored by TLC/LCMS and after completion the reaction mixture was diluted with EtOAc and washed with 0.5N NaOH solution followed by brine. The organic layer was dried over anhydrous Na₂SO₄ and concentrated in vacuo to give the crude product which was purified by prep-HPLC to afford the title compound (150 mg, yield 29%) as a white solid. LCMS m/z: 505.37 [M+H]; Purity 98.16%; ¹H NMR (500 MHz; DMSO-d₆): δ 0.90 *(t, J* = 7.4 Hz, 3H), 1.34-1.46 (m, 2H), 2.24 (s, 3H), 2.98-3.03 (m, 1H), 3.24-3.35 (m, 1H), 3.45 (s, 1H), 4.81 (d, *J =* 5.1 Hz, 1H), 6.36 (d, *J =* 5.1 Hz, 1H), 7.22-7.34 (m, 10H), 7.97 (d, *J =* 9 Hz, 1H), 8.05 (d, *J =* 9.15 Hz, 1H), 8.17 (s, 1H), 9.16 (s, 1H), 9.56 (bs, 1H).

### Example 145: 1-(6,7-Diphenyl-1,8-naphthyridin-3-yl)-3-(2-hydroxybutyl)urea

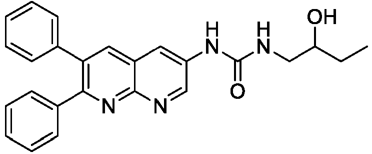

Example 145 was prepared according to the methods described in General Procedures 1, 4, 15 and the method described below.

### Preparation 16: 6,7-Diphenyl-1,8-naphthyridin-3-amine

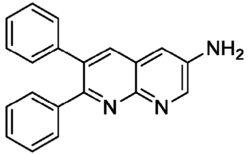

### Step-1: 6-Bromo-2,3-diphenyl-1,8-naphthyridine

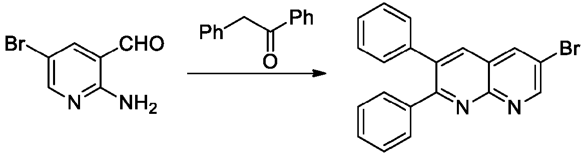

To a stirred solution of commercially available 2-amino-5-bromonicotinaldehyde (250 mg, 1.24 mmol) in piperidine (3 mL), 1,2-diphenylethan-1-one (244 mg. 1.24 mmol) was added at room temperature and the resulting reaction mixture was heated at 120 °C for 16 h in a sealed tube. The reaction was monitored by LCMS and after completion of the reaction, it was quenched with saturated sodium bicarbonate solution and extracted with EtOAc (3 x 50 mL) and washed with water (3 x 50 mL) followed by brine (3 x 50 mL), dried over anhydrous Na₂SO₄ and concentrated under reduced pressure to obtain the crude product which was purified by column chromatography on silica gel to give the title compound (200 mg, 45% yield) as an off white solid. LCMS m/z: 361 [M+H].

### Step-2: N-(4-Methoxybenzyl)-6,7-diphenyl-1,8-naphthyridin-3-amine

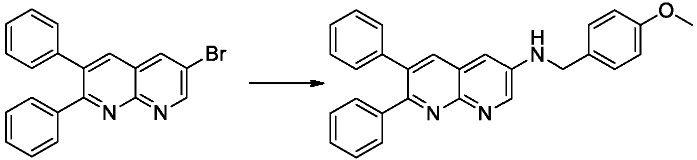

To a stirred solution of 6-bromo-2,3-diphenyl-1,8-naphthyridine (Preparation 16, Step 1) (400 mg, 1.108 mmol) in toluene (10 mL), p-methoxy benzyl amine (227.70 mg, 1.66 mmol), K₃PO₄ (704.70 mg, 3.32 mmol) and X-Phos (105.48 mg, 0.222 mmol) were added and the reaction mixture was degassed with N₂ gas for 10 min. After that Pd₂(dba)₃ (101.38 mg, 0.111 mmol) was added to the reaction mixture. The whole reaction mixture was capped and stirred at 100 °C for 16 h. The reaction was monitored by LCMS and after completion of the reaction the reaction mixture was quenched with water and extracted with EtOAc (2 x 50 mL). The combined organic layers were washed with brine solution (3 x 50 mL), dried over anhydrous Na₂SO₄ and evaporated in vacuo to give a crude product which was purified by column chromatography (silica gel, eluent: 60% EtOAc in hexane) to afford the title compound (300 mg, 65% yield) as a reddish gummy solid. LCMS m/z: 418.0 [M+H].

### Step-3: 6,7-Diphenyl-1,8-naphthyridin-3-amine

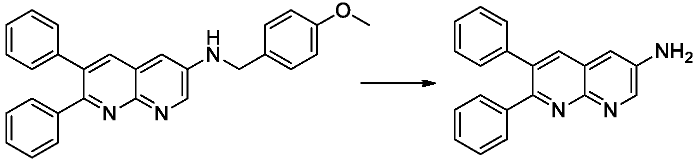

To a stirred solution of N-(4-methoxybenzyl)-6,7-diphenyl-1,8-naphthyridin-3-amine (Preparation 16, Step 2) (190 mg, 0.455 mmol) in DCM (1 mL) was added TFA (1.8 mL) and the whole stirred for 2 h. The reaction was monitored by LCMS and after completion of the reaction it was basified with saturated sodium bicarbonate solution and extracted with EtOAc (3 x 50 mL), washed with water (3 x 50 mL) followed by brine (3 x 50 mL), dried over anhydrous Na₂SO₄ and concentrated under reduced pressure to give the crude product which was purified by column chromatography (silica gel, eluent: 80% EtOAc in hexane) to afford the title compound (100 mg, 73% yield) as a yellowish gum. LCMS m/z: 297.9 [M+H].

### Preparation 17: 1-(6,7-Diphenyl-1,8-naphthyridin-3-yl)-3-(2-hydroxybutyl)urea (Example 145)

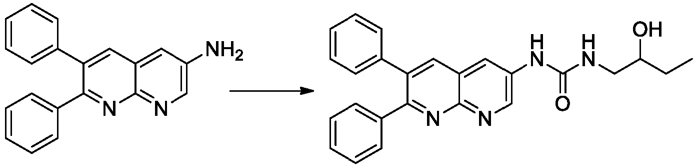

To a stirred solution of 6,7-diphenyl-1,8-naphthyridin-3-amine (Preparation 16, Step 3) (150 mg, 0.504 mmol) in THF (3 mL) was added p-nitrophenyl chloroformate (152.51 mg, 0.757 mmol) at 0-5 °C and the whole stirred at room temperature for 3 h. To the reaction mixture was added TEA (0.352 mL, 2.52 mmol) and 1-aminobutan-2-ol (0.053 mL, 0.555 mmol) at the same temperature and the whole stirred for another 6 h. The reaction was monitored by LCMS and after completion the solvent was evaporated to give the crude product. The crude was purified by reverse phase prep-HPLC to afford the title compound (23 mg, 11% yield) as an off white solid. LCMS m/z: 413.5 [M+H]; Purity 99.45%; ¹H NMR (400 MHz; DMSO-d₆): δ 0.89 (t, *J =* 7.4 Hz, 3H), 1.32-1.39 (m, 1H), 1.41-1.48 (m, 1H), 2.98-3.05 (m, 1H), 3.23-3.28 (m, 1H), 3.45 (d, *J =* 4.8 Hz, 1H), 4.78 (d, *J =* 5.08 Hz, 1H), 6.48 (d, *J =* 5.32 Hz, 1H), 7.26-7.39 (m, 10H), 8.35 (s, 1H), 8.57 (d, *J =* 2.72 Hz, 1H), 8.93 (d, *J =* 2.72 Hz, 1H), 9.28 (s, 1H).

### Preparation 18: 6-Chloro-2,3-diphenyl-1,5-naphthyridine

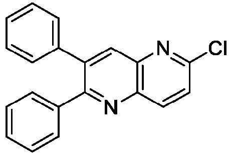

### Step-1: 3-Amino-6-chloropicolinamide

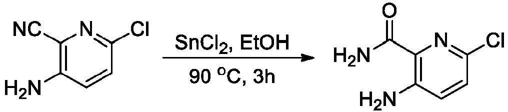

To a stirred solution of commercially available 3-amino-6-chloropicolinonitrile (4.0 g, 26.1 mmol) in EtOH (40.0 mL) was added SnCl2 (9.92 g, 52.3 mmol) and the reaction mixture was stirred at 90 °C for 16 h. Progress of the reaction was monitored by LCMS and after completion of the reaction, the excess solvent was concentrated under reduced pressure and the reaction mixture was quenched with 2M NaOH solution to make the pH 8-9 and the product was then extracted with EtOAc (3 x 50 mL). The combined organic layers were washed with brine solution (1 x 30 mL), dried over anhydrous Na₂SO₄ and filtered. The filtrate was concentrated under reduced pressure to afford the title compound (3.2 g, 71.2% yield) as a crude light yellow solid which was used in the next step without any further purification. LCMS m/z: 172.0 [M+H].

### Step-2: 3-Amino-6-chloropicolinic acid

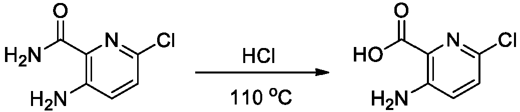

3-Amino-6-chloropicolinamide (Preparation 18, Step-1) (3.0 g, 17.5 mmol) was taken up in cone. HCl (15 mL) and heated to 110 °C for 5 h. The solvent was removed under reduced pressure to afford the title compound (3.0 g, crude) as a yellow solid. The crude was used in the next step without any further purification. LCMS m/z: 173.09 [M+H].

### Step-3: (3-Amino-6-chloropyridin-2-yl)methanol

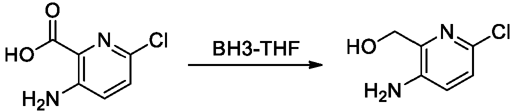

To a stirred solution of 3-amino-6-chloropicolinic acid (Preparation 18, Step-2) (3.0 g, 17.4 mmol) in THF (30.0 mL) was added BH3-THF (1M in THF) (69.5 mL, 69.5 mmol) and the reaction mixture was stirred at room temperature for 48 h. Progress of the reaction was monitored by LCMS and after completion the reaction mixture was diluted with HCl and H₂O, then neutralized with NaHCO3 and extracted with EtOAc (3 x 30 mL). The combined organic layers were dried over anhydrous Na₂SO₄ and filtered. The filtrate was concentrated to afford the title compound (2.5 g, crude) as a yellow sticky solid which was used in the next step without any further purification.

### Step-4: 3-Amino-6-chloropicolinaldehyde

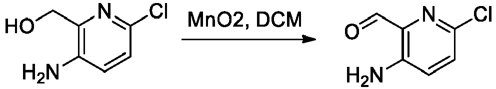

To a stirred solution of (3-amino-6-chloropyridin-2-yl)methanol (Preparation 18, Step-3) (2.5 g, 15.8 mmol) in DCM (50 mL) was added MnO₂ (2.75 g, 31.6 mmol). After 24 h another portion of MnO₂ (2 eq.) was added to the mixture and the whole was stirred for a further 24 h at RT. Progress of the reaction was monitored by LCMS and after completion the reaction mixture was filtered through Celite, and the solvent was removed *in vacuo* to afford the title compound (950 mg, 38.4% yield) as a yellow solid. LCMS m/z: 156.84 [M+H].

### Step-5: 6-Chloro-2,3-diphenyl-1,5-naphthyridine

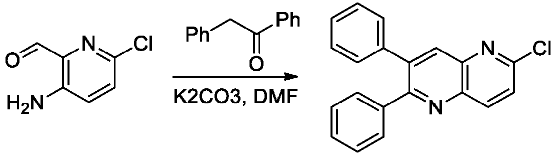

To a stirred solution of 3-amino-6-chloropicolinaldehyde (Preparation 18, Step-4) (100 mg, 0.64 mmol) in DMF (2.0 mL) was added K₂CO₃ (88.6 mg, 0.64 mmol) and 1,2-diphenylethan-1-one (126 mg, 0.64 mmol) and the reaction mixture was stirred at 90 °C for 16 h. Progress of the reaction was monitored by LCMS and after completion the reaction mixture was quenched with crushed ice and cold water (20 mL). The product was extracted using EtOAc (3 x 20 mL) and the combined organic layers were washed with brine solution (1 x 30 mL), dried over anhydrous Na₂SO₄ and filtered. The filtrate was concentrated under reduced pressure to give the crude product which was purified by preparative-TLC (20% EtOAc-Hexane) to afford the title compound (3 mg, 1.48% yield) as an off white solid. LCMS m/z: 316.9 [M+H]; ¹H NMR (400 MHz; DMSO-d₆): δ 8.53 (d, *J* =8.8 Hz, 1H), 8.36 (s, 1H), 7.87 (d, *J =* 8.8 Hz, 1H), 7.31-7.40 (m, 10H),

### Preparation 19: N-(4-Methoxybenzyl)-2,3-diphenyl-1,7-naphthyridin-6-amine

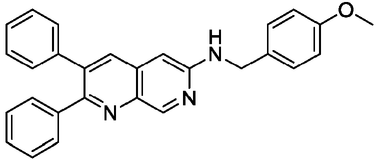

### Step-1: 6-Chloro-2,3-diphenyl-1,7-naphthyridine

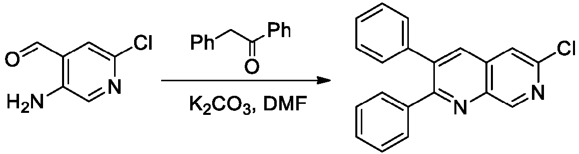

To a stirred solution of commercially available 5-amino-2-chloroisonicotinaldehyde (300 mg, 1.92 mmol) in DMF (6.0 mL) were added K₂CO₃ (265.7 mg, 1.92 mmol) and 1,2-diphenylethan-1-one (377 mg, 1.92 mmol) and the reaction mixture was stirred at 90 °C for 16 h. Progress of the reaction was monitored by LCMS and after completion the reaction mixture was quenched with crushed ice and cold water (20 mL). The product was extracted using EtOAc (3 x 20 mL) and the combined organic layers were washed with brine solution (3 x 20 mL), dried over anhydrous Na₂SO₄ and filtered. The filtrate was concentrated under reduced pressure to afford the crude product which was purified by preparative-TLC (20% EtOAc-Hexane) to afford the title compound (240 mg, 34.9% yield) as an off white solid. LCMS m/z: 316.9 [M+H].

### Step-2: N-(4-Methoxybenzyl)-2,3-diphenyl-1,7-naphthyridin-6-amine

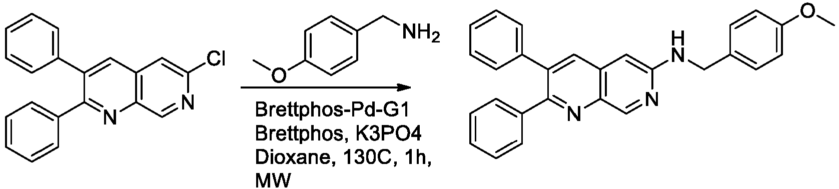

To a degassed solution of 6-chloro-2,3-diphenyl-1,7-naphthyridine (Preparation 19, Step-1) (100 mg, 0.32 mmol) in toluene (1 mL) were added p-methoxy benzyl amine (0.08 mL, 0.63 mmol), K₃PO₄ (201 mg, 0.95 mmol), Brett-phos (57.23 mg, 0.063 mmol) and Brettphos-Pd-G1 (50.43 mg, 0.063 mmol). The whole reaction mixture was capped and stirred at 130 °C for 1 h in the microwave. After completion of the reaction (monitored by LCMS) the reaction mixture was quenched with water and extracted with EtOAc (2 x 20 mL). The combined organic layers were washed with brine solution (1 x 10mL), dried over anhydrous Na₂SO₄ and evaporated to give the crude product which was purified by column chromatography (silica gel, eluent: 50% EtOAc in hexane) followed by prep-TLC to afford the title compound (10 mg, crude) as a brown solid. LCMS m/z: 418.0 [M+H].

| **Ex** | **Structure** | **IUPAC Name** | **1H-NMR** | **LCMS [M+H]** | **Purity (%)** |
|---|---|---|---|---|---|
| 1 | 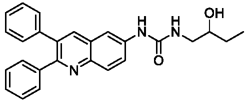 | 1-(2,3-diphenylquinol in-6-yl)-3-(2-hydroxybutyl) urea | (500 MHz; DMSO-d₆): δ 0.90 (t, *J* = 7.3 Hz, 3H), 1.33-1.46 (m, 2H), 2.99-3.03 (m, 1H), 3.25 (t, *J* = 7.85 Hz, 1H), 3.44 (d, *J* = 4.4 Hz, 1H), 4.81 (d, *J* = 4.85 Hz, 1H), 6.35 (s, 1H), 7.26-7.36 (m, 10H), 7.72 (d, *J* = 8.85 Hz, 1H), 7.95 (d, *J* = 9.0 Hz, 1H), 8.13 (s, 1H), 8.20 (s, 1H), 9.05 (s, 1H) | 412.19 | 97.58 |
| 2 | 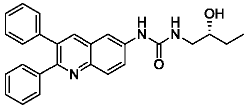 | (R)-1-(2,3-diphenylquinol in-6-yl)-3-(2-hydroxybutyl) urea | (500 MHz; DMSO-d₆): δ 0.90 (t, *J* = 7.4 Hz, 3H), 1.32-1.42 (m, 1H), 1.44-1.47 (m, 1H), 2.99-3.04 (m, 1H), 3.21-3.26 (m, 1H), 3.42-3.46 (m, 1H), 4.80 (d, *J* = 5.1 Hz, 1H), 6.47 (s, 1H), 7.25-7.36 (m, 10H), 7.73 (dd, *J'* = 2.3 Hz, *J"* = 9.05 Hz, 1H), 7.95 (d, *J* = 9.1 Hz, 1H), 8.14 (s, 1H), 8.19 (s, 1H), 9.26 (s, 1H) | 412.17 | 96.82 |
| 3 | 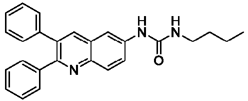 | 1-butyl-3-(2,3-diphenylquinol in-6-yl)urea | (500 MHz; DMSO-d₆): δ 0.92 (t, *J* = 7.3 Hz, 3H), 1.30-1.38 (m, 2H), 1.42-1.48 (m, 2H), 3.12-3.16 (m, 2H), 6.31 (t, *J* = 5.55 Hz, 1H), 7.25-7.36 (m, 10H), 7.72-7.74 (m, 1H), 7.95 (d, *J* = 9.05 Hz, 1H), 8.13 (s, 1H), 8.22 (s, 1H), 8.87 (s, 1H) | 396.20 | 98.79 |
| 4 | 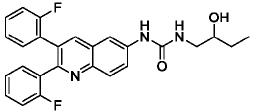 | 1-(2,3-bis(2-fluorophenyl) quinolin-6-yl)-3-(2-hydroxybutyl) urea | (500 MHz; DMSO-d₆): δ 0.91 (t, *J* = 7.4 Hz, 3H), 1.35-1.45 (m, 2H), 2.99-3.03 (m, 1H), 3.24-3.27 (m, 1H), 3.45-3.46 (m, 1H), 4.81 (d, *J* = 5.1 Hz, 1H), 6.34 (t, *J* = 5.55 Hz, 1H), 7.02-7.20 (m, 4H), 7.30-7.36 (m, 3H), 7.42-7.45 (m, 1H), 7.75-7.77 (m, 1H), 7.98 (d, *J* = 9.05 Hz, 1H), 8.16 (d, *J* = 2.15 Hz, 1H), 8.29 (s, 1H), 9.07 (s, 1H) | 448.15 | 99.15 |
| 5 | 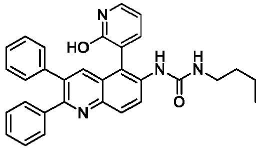 | 1-butyl-3-(5-(2-hydroxypyridin -3-yl)-2,3-diphenylquinol in-6-yl)urea | (500 MHz; DMSO-d₆): δ 0.90 (t, *J* = 7.3 Hz, 3H), 1.28-1.33 (m, 2H), 1.38-1.42 (m, 2H), 3.05-3.10 (m, 2H), 6.41 (t, *J* = 6.6 Hz, 1H), 6.84 (t, *J* = 5.3 Hz, 1H), 7.11-7.13 (m, 2H), 7.28-7.36 (m, 8H), 7.52-7.59 (m, 4H), 8.00 (d, *J =* 9.3 Hz, 1H), 8.59 (d, *J =* 9.3 Hz, 1H), 11.99 (s, 1H) | 489.20 | 98.58 |
| 6 | 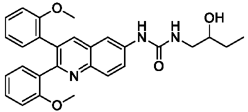 | 1-(2,3-bis(2-methoxyphenyl )quinolin-6-yl)-3-(2-hydroxybutyl) urea | (500 MHz; DMSO-d₆): δ 0.91 (t, *J* = 7.4 Hz, 3H), 1.34-1.45 (m, 2H), 3.00-3.02 (m, 1H), 1.20-1.24 (m, 1H), 3.33 (s, 3H), 3.42 (bs, 4H), 4.78 (d, *J* = 5.15 Hz, 1H), 6.29-6.31 (m, 1H), 6.73 (d, *J* = 8.3 Hz, 1H), 6.83-6.85 (m, 2H), 6.93 (t, *J* = 7.3 Hz, 1H), 7.1 (bs, 1H), 7.18-7.23 (m, 2H), 7.30 (dd, *J' =* 1.5 hz, *J"* = 7.45 Hz, 1H), 7.67 (dd, *J' =* 2.3 Hz, *J"* = 9.05 Hz, 1H), 7.89 (d, *J* = 9.05 Hz, 1H), 8.00 (s, 1H), 8.07 (d, *J* = 2.25 Hz, 1H), 8.96 (s, 1H) | 472.23 | 99.36 |
| 7 | 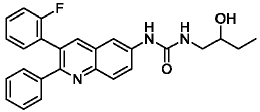 | 1-(3-(2-fluorophenyl)-2-phenylquinolin -6-yl)-3-(2-hydroxybutyl) urea | (500 MHz; DMSO-d₆): δ 0.91 (t, *J* = 7.3 Hz, 3H), 1.33-1.47 (m, 2H), 2.98-3.03 (m, 1H), 3.23-3.28 (m, 1H), 3.45 (bs, 1H), 4.80 (d, *J* = 4.55 Hz, 1H), 6.36 (t, *J* = 5.2 Hz, 1H), 7.11 (t, *J* = 9.2 Hz, 1H), 7.25-7.29 (m, 4H), 7.35-7.42 (m, 3H), 7.46-7.49 (m, 1H), 7.75 (t, *J* = 7.55 Hz, 1H), 7.98 (d, *J* = 9.0 Hz, 1H), 8.13 (s, 1H), 8.24 (s, 1H), 9.07 (s, 1H) | 430.18 | 99.27 |
| 8 | 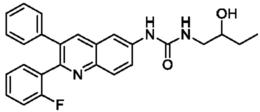 | 1-(2-(2-fluorophenyl)-3-phenylquinolin -6-yl)-3-(2-hydroxybutyl) urea | (500 MHz; DMSO-d₆): δ 0.91 (t, *J* = 7.4 Hz, 3H), 1.35-1.46 (m, 2H), 2.99-3.03 (m, 1H), 3.24-3.29 (m, 1H), 3.45-3.46 (m, 1H), 4.80 (d, *J* = 5.1 Hz, 1H), 6.34 (t, *J* = 5.5 Hz, 1H), 7.04 (t, *J* = 9.3 Hz, 1H), 7.23-7.30 (m, 6H), 7.37-7.39 (m, 1H), 7.51-7.54 (m, 1H), 7.72-7.74 (m, 1H), 7.96 (d, *J* = 9.05 Hz, 1H), 8.17 (d, *J* = 2.1 Hz, 1H), 8.25 (s, 1H), 9.05 (s, 1H) | 430.18 | 98.15 |
| 9 | 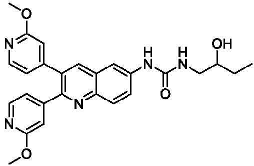 | 1-(2,3-bis(2-methoxypyridin-4-yl)quinolin-6-yl)-3-(2-hydroxybutyl) urea | (500 MHz; DMSO-d₆): δ 0.90 (t, *J* = 7.4 Hz, 3H), 1.33-1.47 (m, 2H), 2.98-3.03 (m, 1H), 3.23-3.28 (m, 1H), 3.45-3.46 (m, 1H), 3.84 (s, 3H), 3.86 (s, 3H), 4.80 (d, *J* = 5.1 Hz, 1H), 6.35 (t, *J* = 5.5 Hz, 1H), 6.81-6.85 (m, 3H), 6.93 (d, *J* = 5.25 Hz, 1H), 7.75-7.78 (m, 1H), 7.99 (d, *J* = 9.1 Hz, 1H), 8.10-8.11 (m, 2H), 8.18 (d, *J* = 2.1 Hz, 1H), 8.34 (s, 1H), 9.10 (s, 1H) | 474.24 | 99.76 |
| 10 | 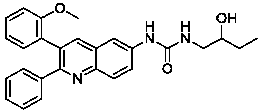 | 1-(2-hydroxybutyl)-3-(3-(2-methoxyphenyl )-2-phenylquinolin -6-yl)urea | (500 MHz; DMSO-d₆): δ 0.90 (t, *J* = 7.4 Hz, 3H), 1.34-1.46 (m, 2H), 2.98-3.03 (m 1H), 3.23-3.25 (m, 1H), 3.27 (s, 3H), 3.44-3.45 (m, 1H), 4.80 (d, *J =* 5.1 Hz, 1H), 6.32 (t, *J =* 5.5 Hz, 1H), 6.87 (d, *J* = 8.05 Hz, 1H), 7.2 (t, *J* = 7.4 Hz, 1H), 7.20-7.25 (m, 3H), 7.31-7.34 (m, 4H), 7.69-7.71 (m, 1H), 7.94 (d, *J* = 9.05 Hz, 1H), 8.08 (s, 2H), 9.00 (s, 1H) | 442.22 | 99.73 |
| 11 | 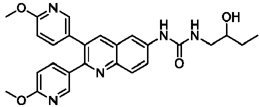 | 1-(2,3-bis(6-methoxypyridin-3-yl)quinolin-6-yl)-3-(2-hydroxybutyl) urea | (500 MHz; DMSO-d₆): δ 0.90 (t, *J* = 7.3 Hz, 3H), 1.34-1.46 (m, 2H), 2.98-3.03 (m, 1H), 3.23-3.28 (m, 1H), 3.44-3.45 (m, 1H), 3.86 (s, 3H), 3.88 (s, 3H), 4.80 (d, *J* = 5.0 Hz, 1H), 6.33 (t, *J* = 5.25 Hz, 1H), 6.78-6.82 (m, 2H), 7.55 (d, *J* = 8.05 Hz, 1H), 7.68-7.71 (m, 2H), 7.96 (d, *J* = 9.05 Hz, 1H), 8.16 (d, *J* = 9.25 Hz, 3H), 8.25 (s, 1H), 9.04 (s, 1H) | 474.22 | 99.06 |
| 12 | 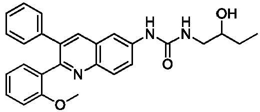 | 1-(2-hydroxybutyl)-3-(2-(2-methoxyphenyl )-3-phenylquinolin -6-yl)urea | (500 MHz; DMSO-d₆): δ 0.91 (t, *J* = 7.4 Hz, 3H), 1.33-1.46 (m, 2H), 2.98-3.03 (m, 1H), 3.20 (s, 3H), 3.24-3.28 (m, 1H), 3.45-3.46 (m, 1H), 4.79 (d, *J* = 4.9 Hz, 1H), 6.31 (t, *J =* 5.45 Hz, 1H), 6.79 (d, *J =* 8.3 Hz, 1H), 7.02 (t, *J =* 7.4 Hz, 1H), 7.18-7.24 (m, 5H), 7.29-7.33 (m, 1H), 7.42 (d, *J* = 7.35 Hz, 1H), 7.68-7.70 (m, 1H), 7.91 (d, *J* = 9.0 Hz, 1H), 8.12 (s, 2H), 8.99 (s, 1H) | 442.22 | 98.90 |
| 13 | 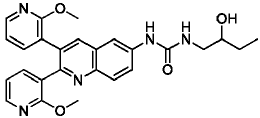 | 1-(2,3-bis(2-methoxypyridin -3-yl)quinolin-6-yl)-3-(2-hydroxybutyl)urea | (500 MHz; DMSO-d₆): δ 0.90 (t, J = 7.4 Hz, 3H), 1.35-1.45 (m, 2H), 3.99-3.02 (m, 1H), 3.25-3.27 (m, 1H), 3.44 (s, 4H), 3.58 (s, 3H), 4.79 (d, J = 5.1 Hz, 1H), 6.33 (t, J = 5.45 Hz, 1H), 6.94-6.97 (m, 1H), 7.02-7.04 (m, 1H), 7.53 (s, 1H), 7.70-7.72 (m, 1H), 7.76-7.78 (m, 1H), 7.93 (d, J = 9.0 Hz, 1H), 8.06-8.10 (m, 2H), 8.13-8.15 (m, 2H), 9.03 (s, 1H) | 474.25 | 98.07 |
| 14 | 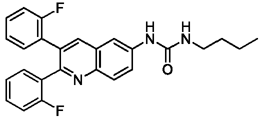 | 1-(2,3-bis(2-fluorophenyl) quinolin-6-yl)-3-butylurea | (500 MHz; DMSO-d₆): δ 0.92 (t, *J* = 7.3 Hz, 3H), 1.32-1.37 (m, 2H), 1.43-1.47 (m, 2H), 3.12-3.16 (m, 2H), 6.32 (t, *J* = 5.55 Hz, 1H), 7.04 (t, *J* = 9.4 Hz, 1H), 7.10-7.20 (m, 3H), 7.29-7.38 (m, 3H), 7.43 (t, *J* = 7.5 Hz, 1H), 7.77-7.79 (m, 1H), 7.97 (d, *J* = 9.05 Hz, 1H), 8.16 (d, *J* = 2.15 Hz, 1H), 8.28 (s, 1H), 8.91 (s, 1H) | 432.2 | 98.88 |
| 15 | 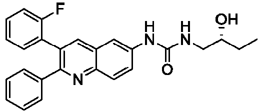 | (R)-1-(3-(2-fluorophenyl)-2-phenylquinolin -6-yl)-3-(2-hydroxybutyl) urea | (500 MHz; DMSO-d₆): δ 0.90 (t, *J* = 7.4 Hz, 3H), 1.34-1.46 (m 2H), 2.99-3.03 (m, 1H), 3.23-3.28 (m, 1H), 3.44 (t, *J* = 6.5 Hz, 1H), 4.80 (d, *J* = 5.15 Hz, 1H), 6.34 (t, *J* = 5.55 Hz, 1H), 7.11 (t, *J* = 8.95 Hz, 1H), 7.25-7.29 (m, 4H), 7.35-7.46 (m, 3H), 7.46-7.49 (m, 1H), 7.74 (d, *J* = 6.9 Hz, 1H), 7.97 (d, *J* = 9.05 Hz, 1H), 8.13 (d, *J* = 2.1 Hz, 1H), 8.25 (s, 1H), 9.06 (s, 1H). | 430.19 | 99.58 |
| 16 | 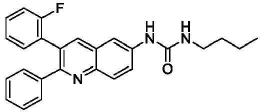 | 1-butyl-3-(3-(2-fluorophenyl)-2-phenylquinolin -6-yl)urea | (500 MHz; DMSO-d₆): δ 0.92 (t, *J* = 7.3 Hz, 3H), 1.31-1.38 (m, 2H), 1.43-1.49 (m, 2H), 3.12-3.15 (m, 2H), 6.30 (t, *J* = 5.5 Hz, 1H), 7.11 (t, *J* = 9.2 Hz, 1H), 7.25-7.30 (m, 4H), 7.35-7.42 (m, 3H), 7.45-7.48 (m, 1H), 7.76 (dd, *J' =* 2.25 Hz, *J" =* 9.05 Hz, 1H), 7.97 (d, *J* = 9.05 Hz, 1H), 8.15 (s, 1H), 8.25 (s, 1H), 8.87 (s, 1H) | 414.21 | 98.18 |
| 17 | 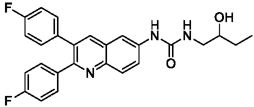 | 1-(2,3-bis(4-fluorophenyl) quinolin-6-yl)-3-(2-hydroxybutyl) urea | (500 MHz; DMSO-d₆): δ 0.90 (t, *J* = 7.4 Hz, 3H), 1.33-1.47 (m, 2H), 2.98-3.03 (m, 1H), 3.23-3.28 (m, 1H), 3.42-3.48 (m, 1H), 4.80 (d, *J* = 5.1 Hz, 1H), 6.33 (t, *J* = 5.5 Hz, 1H), 7.12-7.21 (m, 4H), 7.28-7.31 (m, 2H), 7.36-7.39 (m, 2H), 7.70-7.73 (m, 1H), 7.95 (d, *J* = 9.1 Hz, 1H), 8.13 (s, 1H), 8.21 (s, 1H), 9.04 (s, 1H) | 448.19 | 98.18 |
| 18 | 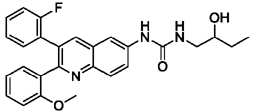 | 1-(3-(2-fluorophenyl)-2-(2-methoxyphenyl)quinolin-6-yl)-3-(2-hydroxybutyl) urea | (500 MHz; DMSO-d₆): δ 0.90 (t, *J* = 7.4 Hz, 3H), 1.34-1.46 (m, 2H), 2.98-3.03 (m, 1H), 3.24-3.29 (m, 1H), 3.35 (s, 3H), 3.45 (d, *J* = 4.8 Hz, 1H), 4.80 (d, *J =* 5.1 Hz, 1H), 6.32 (t, *J* = 5.45 Hz, 1H), 6.75 (d, *J* = 8.3 Hz, 1H), 6.99 (t, *J =* 7.4 Hz, 1H), 7.05 (t, *J =* 7.4 Hz, 1H), 7.12 (t, *J* = 8.9 Hz, 2H), 7.26-7.30 (m, 2H), 7.41 (d, *J* = 7.35 Hz, 1H), 7.71-7.73 (m, 1H), 7.93 (d, *J* = 9.05 Hz, 1H), 8.12 (s, 1H), 8.16 (s, 1H), 9.02 (s, 1H) | 460.22 | 98.08 |
| 19 | 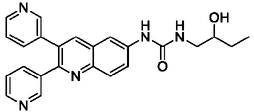 | 1-(2,3-di(pyridin-3-yl)quinolin-6-yl)-3-(2-hydroxybutyl) urea | (500 MHz; DMSO-d₆): δ 0.91 (t, *J* = 7.3 Hz, 3H), 1.33-1.47 (m, 2H), 2.99-3.04 (m, 1H), 3.24-3.35 (m, 1H), 3.46 (bs, 1H), 4.81 (d, *J* = 5.0 Hz, 1H), 6.36 (t, *J* = 5.35 Hz, 1H), 7.34-7.40 (m, 2H), 7.70-7.77 (m, 3H), 8.01 (d, *J =* 9.05 Hz, 1H), 8.19 (d, *J =* 1.9 Hz, 1H), 8.37 (s, 1H), 8.50-8.53 (m, 4H), 9.11 (s, 1H) | 414.20 | 95.66 |
| 20 | 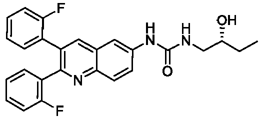 | (R)-1-(2,3-bis(2-fluorophenyl) quinolin-6-yl)-3-(2-hydroxybutyl) urea | (500 MHz; DMSO-d₆): δ 0.90 (t, *J* = 7.4 Hz, 3H), 1.34-1.45 (m, 2H), 2.99-3.03 (m, 1H), 3.24-3.27 (m, 1H), 3.44 (d, *J* = 4.9 Hz, 1H), 4.81 (d, *J* = 5.1 Hz, 1H), 6.39 (t, *J* = 5.45 Hz, 1H), 7.04-7.20 (m, 4H), 7.30-7.36 (m, 3H), 7.44 (t, *J* = 7.35 Hz, 1H), 7.75-7.77 (m, 1H), 7.97 (d, *J* = 9.1 Hz, 1H), 8.17 (d, *J* = 2.05 Hz, 1H), 8.29 (s, 1H), 9.15 (s, 1H) | 448.19 | 98.38 |
| 21 | 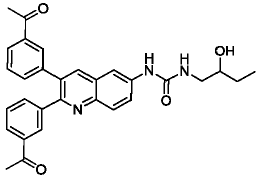 | 1-(2,3-bis(3-acetylphenyl) quinolin-6-yl)-3-(2-hydroxybutyl) urea | (500 MHz; DMSO-d₆): δ 0.91 (s, 3H), 1.36-1.44 (m, 2H), 2.44 (s, 3H), 2.51 (s, 3H), 3.02 (bs, 1H), 3.25-3.27 (m, 1H), 3.46 (bs, 1H), 4.80 (s, 1H), 6.34 (s, 1H), 7.44-7.48 (m, 3H), 7.59 (d, *J* = 6.7 Hz, 1H), 7.75 (d, *J* = 8.75 Hz, 1H), 7.88 (t, *J* = 6.7 Hz, 2H), 7.93 (s, 1H), 7.98-8.02 (m, 2H), 8.20 (s, 1H), 8.37 (s, 1H), 9.06 (s, 1H) | 496.26 | 99.40 |
| 22 | 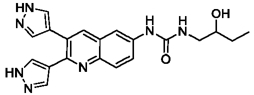 | 1-(2,3-di(1H-pyrazol-4-yl)quinolin-6-yl)-3-(2-hydroxybutyl) urea | (500 MHz; DMSO-d₆): δ 0.90 (t, *J* = 7.4 Hz, 3H), 1.32-1.47 (m, 2H), 2.97-3.02 (m, 1H), 3.21-3.26 (m, 1H), 3.43 (d, *J* = 5.1 Hz, 1H), 4.79 (d, *J* = 4.9 Hz, 1H), 6.37 (s, 1H), 7.55-7.62 (m, 4H), 7.83 (d, *J* = 9.05 Hz, 2H), 7.99 (s, 1H), 8.03 (s, 1H), 9.00 (s, 1H), 12.88 (s, 1H), 13.03 (s, 1H) | 392.17 | 98.09 |
| 23 | 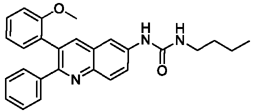 | 1-butyl-3-(3-(2-methoxyphenyl )-2-phenylquinolin -6-yl)urea | (500 MHz; DMSO-d₆): δ 0.92 (t, *J* = 7.3 H, 3H), 1.31-1.38 (m, 2H), 1.43-1.48 (m, 2H), 3.12-3.16 (m, 2H), 3.34 (s, 3H), 6.28 (t, *J =* 5.55 Hz, 1), 6.88 (t, *J =* 8.1 Hz, 1H), 7.02 (t, *J =* 7.4 Hz, 1H), 7.21-7.25 (m, 3H), 7.31-7.34 (m, 4H), 7.71-7.73 (m, 1H), 7.93 (d, *J* = 9.0 Hz, 1H), 8.08 (s, 2H), 8.81 (s, 1H) | 426.26 | 99.92 |
| 24 | 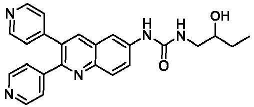 | 1-(2,3-di(pyridin-4-yl)quinolin-6-yl)-3-(2-hydroxybutyl) urea | (500 MHz; DMSO-d₆): δ 0.90 (t, *J* = 5.5 Hz, 3H), 1.36-1.44 (m, 2H), 3.01-3.02 (m, 1H), 3.25-3.28 (m, 1H), 3.45 (bs, 1H), 4.81 (s, 1H), 6.36 (s, 1=H), 7.34 (t, *J* = 5.95 Hz, 4H), 7.78 (s, 1H), 8.02 (s, 1H), 8.21 (s, 1H), 8.38 (s, 1H), 8.54 (bs, 4H), 9.13 (s, 1H) | 414.21 | 98.35 |
| 25 | 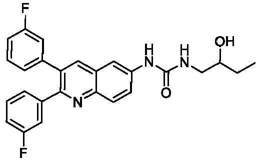 | 1-(2,3-bis(3-fluorophenyl) quinolin-6-yl)-3-(2-hydroxybutyl) urea | (500 MHz; DMSO-d₆): δ 0.88 (d, *J* = 3.6 Hz, 3H), 1.36 (s, 1H), 1.34 (s, 1H), 3.01 (s, 1H), 3.23 (s, 2H), 4.90 (s, 1H), 6.34 (s, 1H), 7.08-7.15 (m, 6H), 7.33-7.37 (m, 2H), 7.73 (s, 1H), 7.98 (s, 1H), 8.13 (s, 1H), 8.25 (s, 1H), 9.07 (s, 1H) | 448.19 | 98.05 |
| 26 | 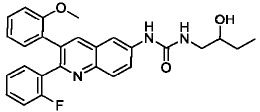 | 1-(2-(2-fluorophenyl)-3-(2-methoxyphenyl )quinolin-6-yl)-3-(2-hydroxybutyl) urea | (500 MHz; DMSO-d₆): δ 0.90 (t, *J* = 7.4 Hz, 3H), 1.33-142 (m, 1H), 1.43-1.47 (m, 1H), 2.98-3.03 (m, 1H), 3.23-3.28 (m, 1H), 3.38 (s, 3H), 3.45 (d, *J =* 4.9 Hz, 1H), 4.80 (d, *J =* 5.0 Hz, 1H), 6.33 (t, *J =* 5.4 Hz, 1H), 6.82 (d, *J =* 8.6 Hz, 1H), 6.95 (t, *J* = 7.4 Hz, 1H), 7.02-7.05 (m, 1H), 7.08-7.11 (m, 1H), 7.25-7.32 (m, 4H), 7.00-7.72 (m, 1H), 7.93 (d, *J* = 9.05 Hz, 1H), 8.12 (s, 2H), 9.02 (s, 1H) | 460.22 | 99.31 |
| 27 | 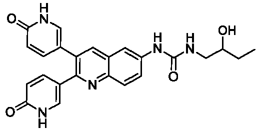 | 1-(2,3-bis(6-oxo-1,6-dihydropyridin -3-yl)quinolin-6-yl)-3-(2-hydroxybutyl) urea | (500 MHz; DMSO-d₆): δ 0.91 (t, *J* = 7.4 Hz, 3H), 1.33-1.45 (m, 2H), 2.99-3.02 (m, 1H), 3.23-3.27 (m, 1H), 3.43-3.46 (m, 1H), 4.79 (d, *J* = 4.4 Hz, 1H), 6.30-6.35 (m, 3H), 7.33 (d, *J* = 9.25 Hz. 1H), 7.45 (s, 1 H), 7.50 (s, 1H), 7.62-7.66 (m, 2H), 7.89 (d, *J* = 8.95 Hz, 1H), 8.08 (s, 1H), 8.15 (s, 1H), 8.99 (s, 1H), 11.77 (bs, 2H) | 446.18 | 98.56 |
| 28 | 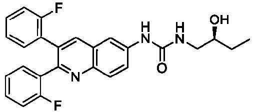 | (S)-1-(2,3-bis(2-fluorophenyl) quinolin-6-yl)-3-(2-hydroxybutyl) urea | (500 MHz; DMSO-d₆): δ 0.90 (t, *J* = 7.4 Hz, 3H), 1.34-1.39 (m, 1H), 1.42-1.45 (m, 1H), 2.99-3.02 (m, 1H), 3.25-3.27 (m, 1H), 3.45 (d, *J* = 4.85 Hz, 1H), 4.80 (d, *J* = 5.1 Hz, 1H), 6.36 (t, *J* = 5.45 Hz, 1H), 7.04 (t, *J* = 9.35 Hz, 1H), 7.10-7.20 (m, 3H), 7.30-7.36 (m, 3H), 7.42-7.45 (m, 1H), 7.75-7.77 (m, 1H), 7.98 (d, *J* = 9.05 Hz, 1H), 8.16 (d, *J* = 2.15 Hz, 1H), 8.29 (s, 1H), 9.09 (s, 1H) | 448.22 | 99.35 |
| 29 | 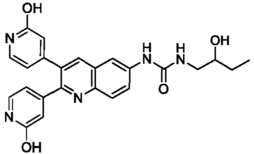 | 1-(2,3-bis(2-hydroxypyridin -4-yl)quinolin-6-yl)-3-(2-hydroxybutyl) urea | (500 MHz; DMSO-d₆): δ 0.90 (t, *J* = 7.3 Hz, 3H), 1.33-1.38 (m, 1H), 1.42-1.47 (m, 1H), 2.98-3.03 (m, 1H), 3.23-3.27 (m, 1H), 3.44 (d, *J* = 4.8 Hz, 1H), 4.80 (d, *J* = 4.95 Hz, 1H), 6.07 (d, *J* = 5.7 Hz, 1H), 6.29-6.40 (m, 4H), 7.38 (dd, J' = 6.8 Hz, *J"* = 10.35 Hz, 2H), 7.76-7.78 (m, 1H), 7.97 (d, *J* = 9.1 Hz, 1H), 8.15 (d, *J* = 1.85 Hz, 1H), 8.29 (s, 1H), 9.14 (s, 1H), 11.64 (s, 2H) | 446.21 | 98.75 |
| 30 | 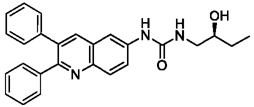 | (S)-1-(2,3-diphenylquinol in-6-yl)-3-(2-hydroxybutyl) urea | (500 MHz; DMSO-d₆): δ 0.90 (t, *J* = 7.4 Hz, 3H), 1.33-1.47 (m, 2H), 2.98-3.03 (m, 1H), 3.23-3.28 (m, 1H), 3.45 (d, *J =* 4.85 Hz, 1H), 4.80 (d, *J* = 5.1 Hz, 1H), 6.32 (t, *J* = 5.5 Hz, 1H), 7.25-7.36 (m, 10H), 7.72 (dd, *J'* = 2.15 Hz, *J" =* 9.05 Hz, 1H), 7.95 (d, *J* = 9.05 Hz, 1H), 8.12 (d, *J* = 2.15 Hz, 1H), 8.20 (s, 1H), 9.02 (s, 1H) | 412.26 | 98.98 |
| 31 | 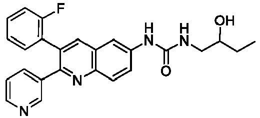 | 1-(3-(2-fluorophenyl)-2-(pyridin-3-yl)quinolin-6-yl)-3-(2-hydroxybutyl) urea | (500 MHz; DMSO-d₆): δ 0.91 (t, *J* = 7.4 Hz, 3H), 1.36-1.37 (m, 1H), 1.43-1.45 (m, 1H), 3.01 (d, *J =* 7.1 Hz, 1H), 3.24-3.27 (m, 1H), 3.45 (d, *J* = 4.85 Hz, 1H), 4.80 (d, *J* = 5.1 Hz, 1H), 6.34 (t, *J* = 5.4 Hz, 1H), 7.11-7.15 (m, 1H), 7.30-7.33 (m, 2H), 7.43 (d, *J* = 7.35 Hz, 1H), 7.53-7.56 (m, 1H), 7.73-7.79 (m, 2H), 8.01 (d, *J* = 9.1 Hz, 1H), 8.16 (s, 1H), 8.31 (s, 1H), 8.48-8.49 (m, 1H), 8.51 (d, *J* = 1.75 Hz, 1H), 9.08 (s, 1H) | 431.2 | 98.90 |
| 32 | 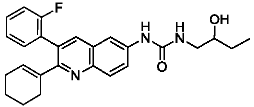 | 1-(2-(cyclohex-1-en-1-yl)-3-(2-fluorophenyl) quinolin-6-yl)-3-(2-hydroxybutyl) urea | (500 MHz; DMSO-d₆): δ 0.89 (t, *J* = 7.4 Hz, 3H), 1.33-1.36 (m, 1H), 1.42 (d, *J* = 7.5 Hz, 1H), 1.48-1.51 (m, 2H), 1.61-1.63 (m, 2H), 1.84 (s, 2H), 2.40 (s, 2H), 2.97-3.02 (m, 1H), 3.23-3.25 (m, 1H), 3.43 (d, *J* = 4.9 Hz, 1H), 4.79 (d, *J* = 5.1 Hz, 1H), 5.39 (s, 1H), 6.30 (t, *J* = 5.55 Hz, 1H), 7.25-7.32 (m, 2H), 7.42-7.7.45 (m, 2H), 7.47-7.50 (m, 1H), 7.68-7.70 (m, 1H), 7.86 (d, *J* = 9.05 Hz, 1H), 8.04 (s, 1H), 8.10 (s, 1H), 8.97 (s, 1H) | 434.26 | 99.85 |
| 33 | 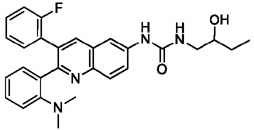 | 1-(2-(2-(dimethylamino)phenyl)-3-(2-fluorophenyl) quinolin-6-yl)-3-(2-hydroxybutyl) urea | (500 MHz; DMSO-d₆): δ 0.91 (t, *J* = 7.4 Hz, 3H), 1.36-1.39 (m, 1H), 1.42-1.46 (m, 1H), 2.08 (s, 6H), 2.99-3.03 (m, 1H), 3.23-3.28 (m, 1H), 3.44 (d, *J =* 4.9 Hz, 1H), 4.80 (d, *J =* 5.05 Hz, 1H), 6.32 (t, *J =* 5.45 Hz, 1H), 6.68 (d, *J =* 8.1 Hz, 1H), 6.92 (d, *J =* 6.35 Hz, 2H), 7.02 (t, *J =* 7.4 Hz, 1H), 7.11 (t, *J =* 9.65 Hz, 1H), 7.19-7.22 (m, 2H), 7.44 (d, *J* = 7.4 Hz, 1H), 7.71 (d, *J* = 9.15 Hz, 1H), 7.96 (d, *J* = 9.05 Hz, 1H), 8.09 (s, 1H), 8.14 (s, 1H), 9.00 (s, 1H) | 473.27 | 98.09 |
| 34 | 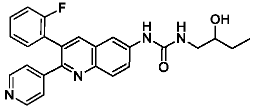 | 1-(3-(2-fluorophenyl)-2-(pyridin-4-yl)quinolin-6-yl)-3-(2-hydroxybutyl) urea | (500 MHz; DMSO-d₆): δ 0.91 (t, *J* = 7.4 Hz, 3H), 1.33-1.39 (m, 1H), 1.42-1.46 (m, 1H), 2.98-3.03 (m, 1H), 3.24-3.28 (m, 1H), 3.45 (d, *J* = 4.9 Hz, 1H), 4.80 (d, *J* = 5.1 Hz, 1H), 6.35 (t, *J* = 5.55 Hz, 1H), 7.15 (t, *J* = 9.25 Hz, 1H), 7.30-7.33 (m, 3H), 7.45 (d, *J* = 7.45 Hz, 1H), 7.52-7.55 (m, 1H), 7.77-7.79 (m, 1H), 8.02 (d, *J* = 9.1 Hz, 1H), 8.17 (d, *J* = 2.1 Hz, 1H), 8.33 (s, 1H), 8.48 (s, 2H), 9.10 (s, 1H) | 431.21 | 98.91 |
| 35 | 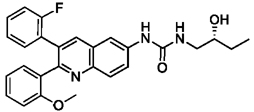 | (R)-1-(3-(2-fluorophenyl)-2-(2-methoxyphenyl)quinolin-6-yl)-3-(2-hydroxybutyl) urea | (500 MHz; DMSO-d₆): δ 0.91 (t, *J* = 7.4 Hz, 3H), 1.33-1.40 (m, 1H), 1.42-1.47 (m, 1H), 2.98-3.03 (m, 1H), 3.24-3.26 (m, 1H), 3.29 (s, 3H), 3.45 (d, *J* = 4.8 Hz, 1H), 4.80 (d, *J* = 5.1 Hz, 1H), 6.32 (t, *J =* 5.55 Hz, 1H), 6.76 (d, *J =* 8.3 Hz, 1H), 6.99 (t, *J =* 7.45 Hz, 1H), 7.05 (t, *J =* 7.4 Hz, 1H), 7.12 (t, *J =* 8.95 Hz, 2H), 7.27 (t, *J =* 7.4 Hz, 2H), 7.41 (d, *J =* 7.35 Hz, 1H), 7.71-7.73 (m, 1H), 7.93 (d, *J* = 9.05 Hz, 1H), 8.11 (s, 1H), 8.12 (s, 1H), 9.04 (s, 1H) | 460.25 | 98.12 |
| 36 | 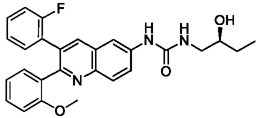 | (S)-1-(3-(2-fluorophenyl)-2-(2-methoxyphenyl)quinolin-6-yl)-3-(2-hydroxybutyl) urea | (500 MHz; DMSO-d₆): δ 0.91 (t, *J* = 7.4 Hz, 3H), 1.34-1.39 (m, 1H), 1.42-1.46 (m, 1H), 2.98-3.03 (m, 1H), 3.24-3.26 (m, 1H), 3.29 (s, 3H), 3.43-3.46 (m, 1H), 4.80 (d, *J =* 5.1 Hz, 1H), 6.32 (t, *J =* 5.55 Hz, 1H), 6.75 (d, *J =* 8.3 Hz, 1H), 6.99 (t, *J =* 7.4 Hz, 1H), 7.05 (t, *J =* 7.35 Hz, 1H), 7.12 (t, *J =* 9.25 Hz, 2H), 7.27 (t, *J =* 7.05 Hz, 2H), 7.41 (d, *J =* 7.4 Hz, 1H), 7.71-7.73 (m, 1H), 7.93 (d, *J* = 9.05 Hz, 1H), 8.12 (s, 1H), 8.15 (s, 1H), 9.01 (s, 1H) | 460.25 | 99.21 |
| 37 | 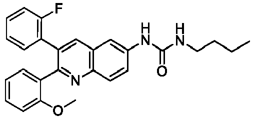 | 1-butyl-3-(3-(2-fluorophenyl)-2-(2-methoxyphenyl )quinolin-6-yl)urea | (500 MHz; DMSO-d₆): δ 0.92 (t, *J* = 7.3 Hz, 3H), 1.32-1.37 (m, 2H), 1.43-1.47 (m, 2H), 3.12-3.16 (m, 2H), 3.29 (s, 3H), 6.29 (t, *J =* 5.55 Hz, 1H), 6.75 (d, *J* = 8.25 Hz, 1H), 6.99 (t, *J =* 7.5 Hz, 1H), 7.05 (t, *J =* 7.4 Hz, 1H), 7.12 (t, *J* = 9.02 Hz, 2H), 7.26-7.29 (m, 2H), 7.41 (dd, *J' =* 1.4 Hz, *J"* = 7.45 Hz, 1H), 7.72-7.75 (m, 1H), 7.92 (d, *J* = 9.05 Hz, 1H), 8.11 (d, *J =* 2.2 Hz, 1H), 8.15 (s, 1H), 8.82 (s, 1H) | 444.24 | 99.23 |
| 38 | 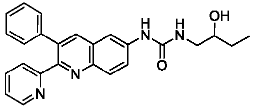 | 1-(2-hydroxybutyl)-3-(3-phenyl-2-(pyridin-2-yl)quinolin-6-yl)urea | (500 MHz; DMSO-d₆): δ 0.91 (t, *J* = 7.4 Hz, 3H), 1.35-1.39 (m, 1H), 1.42-1.45 (m, 1H), 3.00-3.04 (m, 1H), 3.24-3.28 (m, 1H), 3.45 (d, *J* = 4.8 Hz, 1H), 4.80 (d, *J* = 5.05 Hz, 1H), 6.34 (t, *J* = 5.45 Hz, 1H), 7.18 (d, *J* = 6.3 Hz, 2H), 7.24-7.32 (m, 4H), 7.71-7.75 (m, 2H), 7.83-7.86 (m, 1H), 7.97 (d, *J* = 9.05 Hz, 1H), 8.16 (s, 1H), 8.25 (s, 1H), 8.34 (d, *J* = 4.45 Hz, 1H), 9.06 (s, 1H) | 413.23 | 98.04 |
| 39 | 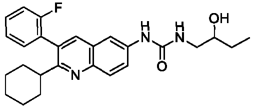 | 1-(2-cyclohexyl-3-(2-fluorophenyl) quinolin-6-yl)-3-(2-hydroxybutyl) urea | (500 MHz; DMSO-d₆): δ 0.91 (t, *J* = 7.4 Hz, 3H), 1.06 (bh, 2H), 1.19-1.24 (m, 1H), 1.32-1.37 (m, 1H), 1.39-1.46 (m, 1H), 1.61-1.74 (m, 7H), 2.64 (bs, 1H), 2.97-3.06 (m, 1H), 3.21-3.28 (m, 1H), 3.43 (d, *J=* 4.95 Hz, 1H), 4.78 (d, *J* = 5.0 Hz, 1H), 6.28 (t, *J =* 5.45 Hz, 1H), 7.34-7.39 (m, 2H), 7.44 (t, *J* = 7.4 Hz, 1H), 7.51-7.55 (m, 1H), 7.69 (t, *J* = 7.3 Hz, 1H), 7.87 (d, *J* = 9.05 Hz, 1H), 7.98 (s, 2H), 8.94 (s, 1H) | 436.27 | 98.12 |
| 40 | 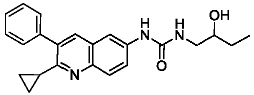 | 1-(2-cyclopropyl-3-phenylquinolin -6-yl)-3-(2-hydroxybutyl) urea | (500 MHz; DMSO-d₆): δ 0.88-0.92 (m, 5H), 1.16 (t, *J* = 6.3 Hz, 2H), 1.33-1.37 (m, 1H), 1.40-1.43 (m, 1H), 2.07-2.10 (m, 1H), 2.29-3.00 (m, 1H), 3.21-3.24 (m, 1H), 3.42 (d, *J* = 4.9 Hz, 1H), 4.78 (d, *J* = 5.05 Hz, 1H), 6.26 (t, *J* = 5.5 Hz, 1H), 7.46 (d, *J* = 7.1 Hz, 1H), 7.52-7.57 (m, 4H), 7.62-7.64 (m, 1H), 7.74 (d, *J* = 9.05 Hz, 1H), 7.95 (s, 1H), 7.98 (s, 1H), 8.88 (s, 1H) | 376.24 | 98.44 |
| 41 | 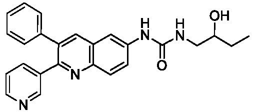 | 1-(2-hydroxybutyl)-3-(3-phenyl-2-(pyridin-3-yl)quinolin-6-yl)urea | (500 MHz; DMSO-d₆): δ 0.91 (t, *J* = 7.4 Hz, 3H), 1.34-1.39 (m, 1H), 1.42-1.46 (m, 1H), 2.98-3.03 (m, 1H), 3.23-3.28 (m, 1H), 3.45 (d, *J* = 5.05 Hz, 1H), 4.80 (d, *J* = 5.05 Hz, 1H), 6.34 (t, *J* = 5.5 Hz, 1H), 7.28-7.36 (m, 6H), 7.73-7.75 (m, 2H), 7.99 (d, *J* = 9.1 Hz, 1H), 8.15 (s, 1H), 8.26 (s, 1H), 8.47-8.50 (m, 2H), 9.06 (s, 1H) | 413.23 | 98.26 |
| 42 | 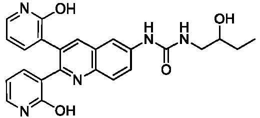 | 1-(2,3-bis(2-hydroxypyridi n-3-yl)quinolin-6-yl)-3-(2-hydroxybutyl) urea | (500 MHz; DMSO-d₆): δ 0.91 (t, *J* = 7.3 Hz, 3H), 1.32-1.38 (m, 1H), 1.42-1.47 (m, 1H), 2.97-3.02 (m, 1H), 3.22-3.27 (m, 1H), 3.44 (s, 1H), 4.82 (s, 1H), 6.10 (t, *J* = 6.6 Hz, 1H), 6.22 (t, *J* = 6.6 Hz, 1H), 6.55 (s, 1H), 7.29 (t, *J* = 6.6 Hz, 2H), 7.35 (d, *J* = 4.85 Hz, 1H), 7.54-7.56 (m, 1H), 7.66-7.68 (m, 1H), 7.86 (d, J = 9.05 Hz, 1H), 7.07-7.08 (m, 2H), 9.21 (s, 1H), 11.57 (s, 2H) | 446.15 | 98.35 |
| 43 | 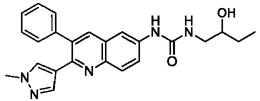 | 1-(2-hydroxybutyl)-3-(2-(1-methyl-1H-pyrazol-4-yl)-3-phenylquinolin -6-yl)urea | (500 MHz; DMSO-d₆): δ 0.91 (t, *J* = 4.3 Hz, 3H), 1.32-1.38 (m, 1H), 1.40-1.46 (m, 1H), 2.97-3.02 (, 1H), 3.22-3.27 (m, 1H), 3.44 (d, *J* = 4.8 Hz, 1H), 3.76 (s, 3H), 4.77 (d, *J =* 5.15 Hz, 1H), 6.28 (s, 1H), 6.96 (s, 1H), 7.37-7.39 (m, 2H), 7.48-7.50 (m, 3H), 7.56 (s, 1H), 7.69 (dd, *J'* = 2.25 Hz, *J"* = 9.1 Hz, 1H), 7.86 (d, *J* = 9.05 Hz, 1H), 8.00 (d, *J* = 2.3 Hz, 2H), 8.94 (s, 1H), | 416.23 | 98.83 |
| 44 | 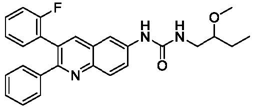 | 1-(3-(2-fluorophenyl)-2-phenylquinolin -6-yl)-3-(2-methoxybutyl) urea | (500 MHz; DMSO-d₆): δ 0.90 (t, *J* = 7.4 Hz, 3H), 1.47-1.52 (m, 2H), 2.51 (s, 1H), 3.16-3.22 (m, 2H), 3.32 (s, 3H), 6.31 (t, *J =* 5.12 Hz, 1H), 7.11 (t, J = 9.2 Hz, 1H), 7.25-7.29 (m, 4H), 7.35-7.41 (m, 3H), 7.47 (t, *J* = 7.15 Hz, 1H), 7.75 (dd, *J' =* 1.8 Hz, *J"* = 9.0 Hz, 1H), 7.98 (d, *J =* 9.05 Hz, 1H), 8.13 (d, *J =* 1.55 Hz, 1H), 8.25 (s, 1H), 8.98 (s, 1H) | 444.18 | 99.79 |
| 45 | 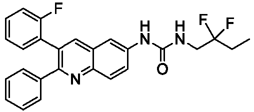 | 1-(2,2-difluorobutyl)-3-(3-(2-fluorophenyl)-2-phenylquinolin -6-yl)urea | (500 MHz; DMSO-d₆): δ 0.99 (t, *J* = 7.5 Hz. 3H), 1.86-1.97 (m, 2H), 3.59-3.66 (m, 2H), 6.71 (t, *J =* 6.2 Hz, 1H), 7.09-7.13 (m, 1H), 7.25-7.30 (m 4H), 7.35-7.42 (m 3H), 7.46-7.49 (m, 1H), 7.76-7.78 (m, 1H), 7.99 (d, *J* = 9.05 Hz, 1H), 8.15 (d, *J* = 2.2 Hz, 1H), 8.28 (s, 1H), 9.08 (s, 1H). | 450.15 | 99.86 |
| 46 | 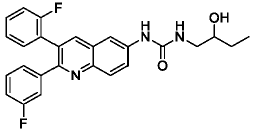 | 1-(3-(2-fluorophenyl)-2-(3-fluorophenyl) quinolin-6-yl)-3-(2-hydroxybutyl) urea | (500 MHz; DMSO-d₆): δ 0.90 (t, *J* = 7.4 Hz, 3H), 1.33-1.39 (m, 1H), 1.42-1.46 (m, 1H), 2.98-3.03 (m, 1H), 3.23-3.28 (m, 1H), 3.44 (d, *J* = 4.8 Hz, 1H), 4.80 (d, *J* = 5.15 Hz, 1H), 6.34 (t, *J* = 5.45 Hz, 1H), 7.12-7.16 (m, 4H), 7.30 (dd, *J*' = 6.05 Hz, *J"* = 13.6 Hz, 2H), 7.41-7.45 (m, 1H), 7.50-7.53 (m, 1H), 7.76 (dd, *J' =* 2.2 Hz, *J"* = 9.05 Hz, 1H), 7.99 (d, *J =* 9.05 Hz, 1H), 8.14 (d, *J =* 2.1 Hz, 1H), 8.28 (s, 1H), 9.07 (s, 1H) | 448.15 | 99.44 |
| 47 | 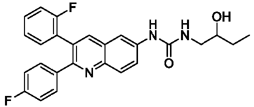 | 1-(3-(2-fluorophenyl)-2-(4-fluorophenyl)quinolin-6-yl)-3-(2-hydroxybutyl) urea | (500 MHz; DMSO-d₆): δ 0.91 (t, *J =* 7.42 Hz, 3H), 1.34-1.39 (m, 1H), 1.42-1.46 (m, 1H), 2.98-3.03 (m, 1H), 3.23-3.28 (m, 1H), 3.44 (d, *J* = 4.75 Hz, 1H), 4.80 (d, *J* = 5.05 Hz, 1H), 6.33 (t, *J* = 5.5 Hz, 1H), 7.09-7.15 (m, 3H), 7.29 (t, *J* = 7.4 Hz, 1H), 7.37-7.44 (m, 3H), 7.49 (t, *J =* 7.5 Hz, 1H), 7.75 (dd, *J'* = 2.1 Hz, *J"* = 9.05 Hz, 1H), 7.97 (d, *J* = 9.1 Hz, 1H), 8.13 (d, *J* = 2.05 Hz, 1H), 8.25 (s, 1H), 9.05 (s, 1H) | 448.15 | 99.88 |
| 48 | 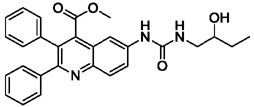 | methyl 6-(3-(2-hydroxybutyl) ureido)-2,3-diphenylquinoline-4-carboxylate | (500 MHz; DMSO-d₆): δ 0.90 (t, *J* = 7.45 Hz, 3H), 1.34-1.38 (m, 1H), 1.41-1.44 (m, 1H), 2.97-3.01 (m, 1H), 3.23-3.27 (m, 1H), 3.42-3.45 (m, 1H), 3.61 (s, 3H), 4.79 (d, *J* = 5.15 Hz, 1H), 6.31 (t, *J* = 5.5 Hz, 1H), 7.16 (dd, *J' =* 3.0 Hz, *J" =* 6.4 Hz, 2H), 7.22-7.26 (m, 3H), 7.30-7.32 (m, 5H), 7.74-7.76 (m, 1H), 8.02-8.06 (m, 2H), 9.17 (s, 1H) | 470.16 | 99.79 |
| 49 | 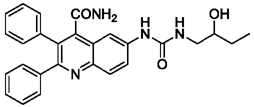 | 6-(3-(2-hydroxybutyl) ureido)-2,3-diphenylquinoline-4-carboxamide | (500 MHz; DMSO-d₆): δ 0.90 (t, *J* = 7.4 Hz, 3H), 1.32-1.38 (m, 1H), 1.40-1.45 (m, 1H), 2.96-3.01 (m, 1H), 3.23-3.28 (m, 1H), 3.44 (d, *J* = 4.9 Hz, 1H), 4.79 (d, *J* = 5.1 Hz, 1H), 6.25 (t, *J* = 5.4 Hz, 1H), 7.21-7.26 (m, 10H), 7.66 (s, 1H), 7.79-7.81 (m, 1H), 7.92 (s, 1H), 7.98 (d, *J* = 9.05 Hz, 1H), 8.10 (d, *J =* 2.0 Hz, 1H), 9.12 (s, 1H) | 455.19 | 98.04 |
| 50 | 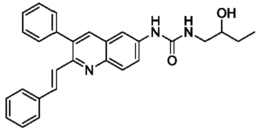 | (E)-1-(2-hydroxybutyl)-3-(3-phenyl-2-styrylquinolin-6-yl)urea | (500 MHz; DMSO-d₆): δ 0.90 (t, *J* = 7.4 Hz, 3H), 1.23 (s, 1H), 1.34-1.38 (m, 1H), 1.42-1.44 (m, 1H), 2.99-3.02 (m, 1H), 3.24-3.26 (m, 1H), 3.44 (d, *J =* 4.9 Hz, 1H), 4.80 (d, *J =* 5.1 Hz, 1H), 6.32 (t, *J* = 5.5 Hz, 1H), 7.23 (d, *J* = 15.65 Hz, 1H), 7.30 (t, *J* = 7.25 Hz, 1H), 7.37 (t, *J* = 7.4 Hz, 2H), 7.39-7.59 (m, 7H), 7.72 (dd, *J'* = 2.2 9.1 Hz, *J" =* Hz, 1H), 7.85 (d, *J* = 15.65 Hz, 1H), 7.96 (d, *J* = 9.1 Hz, 1H), 8.05 (s, 1H), 8.09 (s, 1H), 9.00 (s, 1H) | 438.17 | 98.48 |
| 51 | 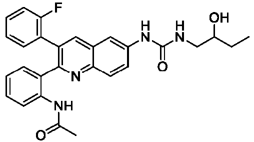 | N-(2-(3-(2-fluorophenyl)-6-(3-(2-hydroxybutyl) ureido) quinolin-2-yl)phenyl)acet amide | (500 MHz; DMSO-d₆): δ 0.90 (t, *J* = 7.4 Hz, 3H), 1.36-1.45 (m, 2H), 1.87 (s, 3H), 2.99-3.03 (m, 1H), 3.24-3.28 (m, 1H), 3.45 (d, *J =* 4.75 Hz, 1H), 4.79 (d, *J* = 5.15 Hz, 1H), 6.34 (t, *J =* 5.4 Hz, 1H), 6.97 (d, J = 7.5 Hz, 1H), 7.09-7.13 (m, 3H), 7.22-7.30 (m, 3H), 7.76 (dd, *J' =* 2.25 Hz, *J"* = 9.05 Hz, 1H), 7.80 (d, *J =* 8.1 Hz, 1H), 7.99 (d, *J* = 9.05 Hz, 1H), 8.14 (d, *J =* 2.2 Hz, 1H), 8.27 (s, 1H), 9.06 (s, 1H), 9.45 (s, 1H) | 487.16 | 99.28 |
| 52 | 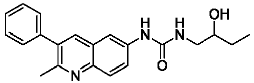 | 1-(2-hydroxybutyl)-3-(2-methyl-3-phenylquinolin -6-yl)urea | (500 MHz; DMSO-d₆): δ 0.89 (t, *J* = 7.4 Hz, 3H), 1.32-1.37 (m, 1H), 1.41-1.44 (m, 1H), 2.53 (s, 3H), 2.96-3.01 (m, 1H), 3.22-3.26 (m, 1H), 3.43 (d, *J =* 4.8 Hz, 1H), 4.78 (d, *J* = 5.1 Hz, 1H), 6.28 (t, *J =* 5.55 Hz, 1H), 7.43-7.45 (m, 1H), 7.49-7.53 (m, 4H), 7.65 (dd, *J*' = 2.1 Hz, *J"* = 9.05 Hz, 1H), 7.84 (d, *J* = 9.0 Hz, 1H), 7.97 (s, 1H), 8.01 (d, *J* = 2.0 Hz, 1H), 8.92 (s, 1H) | 350.17 | 98.98 |
| 53 | 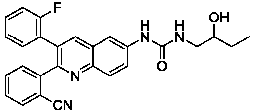 | 1-(2-(2-cyanophenyl)-3-(2-fluorophenyl) quinolin-6-yl)-3-(2-hydroxybutyl) urea | (500 MHz; DMSO-d₆): δ 0.91 (t, *J* = 7.4 Hz, 3H), 1.33-1.39 (m, 1H), 1.42-1.48 (m, 1H), 3.00-3.02 (m, 1H), 3.25-3.28 (m, 1H), 3.45 (d, *J* = 3.1 Hz, 1H), 4.80 (d, *J =* 5.05 Hz, 1H), 6.36 (s, 1H), 7.08 (t, *J* = 9.25 Hz, 1H), 7.24-7.28 (m, 2H), 7.36 (d, *J* = 6.2 Hz, 1H), 7.46-7.55 (m, 3H), 7.79 (d, *J* = 9.25 Hz, 1H), 7.86 (d, *J* = 7.6 Hz, 1H), 8.00 (d, *J* = 9.0 Hz, 1H), 8.21 (s, 1H), 8.38 (s, 1H), 9.12 (s, 1H) | 455.17 | 95.21 |
| 54 | 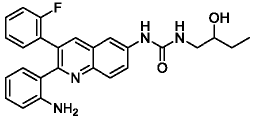 | 1-(2-(2-aminophenyl)-3-(2-fluorophenyl) quinolin-6-yl)-3-(2-hydroxybutyl) urea | (500 MHz; DMSO-d₆): δ 0.90 (t, *J* = 7.4 Hz, 3H), 1.34-1.39 (m, 1H), 1.42-1.45 (m, 1H), 2.95-3.03 (m, 1H), 3.23-3.28 (s, 1H), 3.45 (d, *J* = 4.55 Hz, 1H), 4.79 (d, *J* = 5.05 Hz, 1H), 5.32 (s, 2H), 6.27-6.33 (m, 2H), 6.63 (d, *J* = 7.35 Hz, 1H), 6.70 (d, *J* = 8.0 Hz, 1H), 6.93 (t, *J* = 7.1 Hz, 1H), 7.09-7.18 (m, 2H), 7.31-7.37 (m, 2H), 7.74 (dd, *J'* = 2.1 Hz, *J"* = 9.05 Hz, 1H), 7.92 (d, *J* = 9.1 Hz, 1H), 8.10 (d, *J* = 1.95 Hz, 1H), 8.25 (s, 1H), 9.03 (s, 1H) | 445.16 | 98.97 |
| 55 | 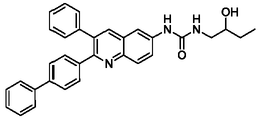 | 1-(2-([1,1'-biphenyl]-4-yl)-3-phenylquinolin -6-yl)-3-(2-hydroxybutyl) urea | (500 MHz; DMSO-d₆): δ 0.91 (t, *J* = 7.4 Hz, 3H), 1.35-1.39 (m, 1H), 1.43-1.46 (m, 1H), 3.00-3.03 (m, 1H), 3.25-3.26 (m, 1H), 3.45 (s, 1H), 4.79 (d, *J* = 4.4 Hz, 1H), 6.34 (s, 1H), 7.34-7.47 (m, 10H), 7.60 (d, *J* = 7.75 Hz, 1H), 7.68-7.74 (m, 4H), 7.97 (d, *J =* 8.9 Hz, 1H), 8.14 (s, 1H), 8.21 (s, 1H), 9.06 (s, 1H) | 488.20 | 99.64 |
| 56 | 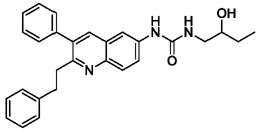 | 1-(2-hydroxybutyl)-3-(2-phenethyl-3-phenylquinolin -6-yl)urea | (500 MHz; DMSO-d₆): δ 0.90 (t, *J* = 7.4 Hz, 3H), 1.34-1.38 (m, 1H), 1.42-1.45 (m, 1H), 2.94-3.01 (m, 3H), 3.07 (d, *J* = 7.65 Hz, 2H), 3.23-3,25 (m, 1H), 3.44 (s, 1H), 4.78 (s, 1H), 6.31 (s, 1H), 7.01 (d, *J =* 6.7 Hz, 2H), 7.13 (d, *J =* 6.7 Hz, 1H), 7.19 (d, *J =* 6.8 Hz, 2H), 7.42-7.50 (m, 5H), 7.69 (d, *J* = 8.25 Hz, 1H), 7.90-8.02 (m, 3H), 8.97 (s, 1H) | 440.21 | 93.56 |
| 57 | 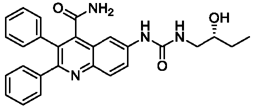 | (R)-6-(3-(2-hydroxybutyl) ureido)-2,3-diphenylquinoline-4-carboxamide | (500 MHz; DMSO-d₆): δ 0.90 (t, *J* = 7.4 Hz, 3H), 1.34-1.38 (m, 1H), 1.41-1.45 (m, 1H), 3.23-3.27 (m, 1H), 3.28-3.36 (m, 1H), 3.44 (d, *J* = 4.95 Hz, 1H), 4.79 (d, *J* = 5.05 Hz, 1H), 6.24 (t, *J* = 5.45 Hz, 1H), 7.21-7.26 (m, 10H), 7.64 (s, 1H), 7.79-7.81 (dd, *J1 =* 2.15 Hz, J2 = 9.1 Hz, 1H), 7.92 (s, 1H), 7.98 (d, *J* = 9.05 Hz, 1H), 8.10 (d, *J =* 2.05 Hz, 1H), 9.11 (s, 1H) | 455.18 | 99.16 |
| 58 | 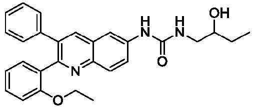 | 1-(2-(2-ethoxyphenyl)-3-phenylquinolin -6-yl)-3-(2-hydroxybutyl) urea | (500 MHz; DMSO-d₆): δ 0.66(t, J = 6.15 Hz, 5H) 1.36-1.37 (m, 1H), 1.43 (bs 1H), 1.91 (s, 1H), 3.01 (bs 1H), 3.14 (bs, 1H), 3.25 (bs 1H), 3.45 (s, 1H), 3.75 (bs 1H), 4.80 (s, 1H), 6.34 (s 1H), 6.77 (d, *J* = 6.95 Hz 1H), 7.02 (s, 1H), 7.15-7.2E (m, 5H), 7.30 (s, 1H), 7.44 (bs, 1H), 7.69 (d, *J =* 6.8 Hz 1H), 7.91 (d, *J* = 8.05 Hz 1H), 8.13 (s, 2H), 9.04 (s 1H) 7.15-7.28 | 456.19 | 99.78 |
| 59 | 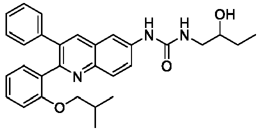 | 1-(2-hydroxybutyl)-3-(2-(2-isobutoxyphenyl)-3-phenylquinolin -6-yl)urea | (500 MHz; DMSO-d₆): δ 0.66 (d, *J* = 6.6 Hz, 6H), 0.84-0.88 (m, 2H), 0.90 (t, *J* = 7.35 Hz, 3H), 1.34-1.40 (m, 1H), 1.42-1.47 (m, 1H), 1.63 1.67 (m, 1H), 2.98-3.03 (m, 1H), 3.23-3.34 (m, 1H), 3.45 (bs, 1H), 4.80 (d, *J* = 3.95 Hz, 1H), 6.32 (t, *J* = 5.15 Hz, 1H), 6.85 (d, *J* = 8.3 Hz, 1H), 6.97 (t, *J* = 7.35 Hz, 1H), 7.22 (s, 5H), 7.28 (t, *J* = 7.4 Hz, 1H), 7.33 (d, *J* = 7.3 Hz, 1H), 7.68 (d, *J =* 7.7 Hz, 1H), 7.90 (d, *J =* 8.95 Hz, 1H), 8.15 (d, *J =* 19 Hz, 2H), 9.007 (s, 1H) | 484.22 | 99.14 |
| 60 | 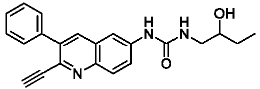 | 1-(2-ethynyl-3-phenylquinolin -6-yl)-3-(2-hydroxybutyl) | (500 MHz; DMSO-d₆): δ 0.89 *(t, J* = 7.35 Hz, 3H), 1.30-1.39 (m, 1H), 1.41-1.48 (m, 1H), 2.97-3.02 (m, 1H), 3.22-3.27 (m, 1H), 3.44-3.45 (m, 1H), 4.5 (s, 1H), 4.79 (d, *J =* 5.1 Hz, 1H), 6.34 (t, *J =* 5.55 Hz, 1H), 7.46-7.47 (m, 1H), 7.51 (t, *J* = 7.55 Hz, 2H), 7.67 (d, *J =* 7.25 Hz, 2H), 7.72 (d, *J =* 2 Hz, 1H), 7.70 (d, *J=* 9.1 Hz, 1H), 8.11 (d, *J =* 2.05 Hz, 1H), 8.21 (s, 1H), 9.09 (s, 1H) | 360.12 | 98.65 |
| 61 | 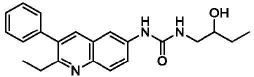 | 1-(2-ethyl-3-phenylquinolin -6-yl)-3-(2-hydroxybutyl) urea | (500 MHz; DMSO-d₆): δ 0.89 (t, *J* = 7.35 Hz, 3H), 1.15 (t, *J* = 7.45 Hz, 3H), 1.36-1.39 (m, 1H), 1.41-1.47 (m, 1H), 2.80-2.85 (dd, *J*1 = 7.4 Hz, *J*2 *=* 14.85 Hz, 2H), 2.96-3.01 (m, 1H), 3.17-3.26 (m, 1H), 3.41-3.42 (m, 1H), 4.80 (d, *J* = 3.2 Hz, 1H), 6.36 (s, 1H), 7.44-7.52 (m, 5H), 7.67 (d, *J* = 8.65 Hz, 1H), 7.86 (d, *J* = 8.95 Hz, 1H), 7.93 (s, 1H), 8.01 (s, 1H), 9.01 (s, 1H) | 364.15 | 98.04 |
| 62 | 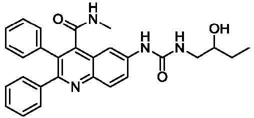 | 6-(3-(2-hydroxybutyl) ureido)-N-methyl-2,3-diphenylquinol ine-4-carboxamide | (500 MHz; DMSO-d₆): δ 0.90 (t, *J* = 7.4 Hz, 3H), 1.32-1.38 (m, 1H), 1.40-1.46 (m, 1H), 2.50 (s, 3H), 2.78 (s, 3H), 2.94- 3.01 (m, 1H), 3.21-3.35 (m, 1H), 3.43-3.44 (m, 1H), 4.80 (d, *J* = 5.15 Hz, 1H), 6.26 (t, *J* = 5.45 Hz, 1H), 7.01 (bs, 1H), 7.21-7.32 (m, 9H), 7.79-7.85 (m, 2H), 8.01 (d, *J* = 8.95 Hz, 1H), 9.13 (s, 1H) | 469.11 | 98.44 |
| 63 | 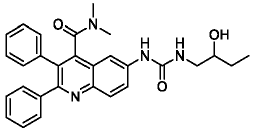 | 6-(3-(2-hydroxybutyl) ureido)-N,N-dimethyl-2,3-diphenylquinoline-4-carboxamide | (500 MHz; DMSO-d₆): δ 0.90 (t, *J* = 7.4 Hz, 3H), 1.31-1.38 (m, 1H), 1.40-1.46 (m, 1H), 2.50 (s, 3H), 2.78 (s, 3H), 2.94- 3.03 (m, 1H), 3.21-3.35 (m, 1H), 3.43-3.44 (m, 1H), 4.80 (d, *J* = 5.15 Hz, 1H), 6.26 (t, *J* = 5.40 Hz, 1H), 7.01 (bs, 1H), 7.21-7.32 (m, 9H), 7.94-7.85 (m, 2H), 8.01 (d, *J* = 8.95 Hz, 1H), 9.13 (s, 1H) | 483.14 | 98.72 |
| 64 | 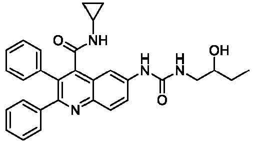 | N-cyclopropyl-6-(3-(2-hydroxybutyl) ureido)-2,3-diphenylquinoline-4-carboxamide | (500 MHz; DMSO-d₆): δ 0.08 (bs, 2H), 0.49-0.50 (m, 2H), 0.90 (t, *J* = 7.35 Hz, 3H), 1.31-1.38 (m, 1H), 1.40-1.47 (m, 1H), 2.97-3.02 (m, 1H), 3.23-3.27 (m, 1H), 3.39-3.44 (m, 2H), 4.81 (d, *J* = 5.0 Hz, 1H), 6.26 (t, *J* = 5.40 Hz, 1H), 7.21-7.26 (m, 10H), 7.81 (d, *J* = 9.15 Hz, 1H), 7.97-7.99 (m, 2H), 8.43 (d, *J* = 3.45 Hz, 1H), 9.13 (s, 1H) | 495.27 | 99.70 |
| 65 | 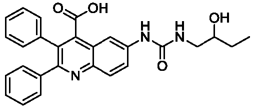 | 6-(3-(2-hydroxybutyl) ureido)-2,3-diphenylquinol ine-4-carboxylic acid | (500 MHz; DMSO-d₆): δ 0.87-.95 (m, 5H), 1.24-1.34 (m, 2H), 1.41-1.44 (m, 1H), 1.57 (bs, 1H), 2.96-2.99 (m, 1H), 3.15-3.20 (m, 2H), 3.35 (m, 2H), 4.78 (s, 1H), 6.45 (s, 1H), 7.20-7.24 (m, 10H), 7.89-8.0 (m, 3H), 9.35 (s, 1H) | 456.22 | 98.22 |
| 66 | 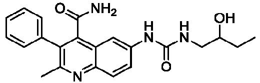 | 6-(3-(2-hydroxybutyl) ureido)-2-methyl-3-phenylquinolin e-4-carboxamide | (500 MHz; DMSO-d₆): δ 0.90 (t, *J* = 7.4 Hz, 3H), 1.31-1.38 (m, 1H), 1.40-1.44 (m, 1H), 2.33 (s, 3H), 2.94- 3.00 (m, 1H), 3.21-3.34 (m, 1H), 3.42-3.43 (m, 1H), 4.78 (d, *J* = 5.10 Hz, 1H), 6.20 (t, *J* = 5.45 Hz, 1H), 7.35-7.47 (m, 5H), 7.57 (s, 1H), 7.71-7.74 (dd, *J1* = 2.15 Hz, J2 = 9 Hz, 2H), 7.85-7.87 (m, 2H), 8.00 (s, 1H), 9.01 (s, 1H) | 393.27 | 99.38 |
| 67 | 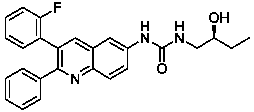 | (S)-1-(3-(2-fluorophenyl)-2-phenylquinolin -6-yl)-3-(2-hydroxybutyl) urea | (500 MHz; DMSO-d₆): δ 0.90 (t, *J* = 7.35 Hz, 3H), 1.31-1.47 (m, 2H), 2.99-3.02 (m, 1H), 3.24-3.26 (m, 1H), 3.43-3.44 (m, 1H), 4.80 (d, *J* = 5.0 Hz, 1H), 6.33 (t, *J* = 5.15 Hz, 1H), 7.13 (t, *J* = 9.025 Hz, 1H), 7.25-7.47 (m, 8H), 7.74-7.76 (m, 1H), 7.98 (d, *J* = 9.0 Hz, 1H), 8.13 (s, 1H), 8.25 (s, 1H), 9.13 (s, 1H) | 430.26 | 98.60 |
| 68 | 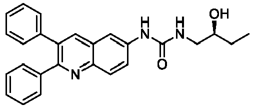 | (S)-1-(2,3-diphenylquinol in-6-yl)-3-(2-hydroxybutyl) urea | (500 MHz; DMSO-d₆): δ 0.90 (t, *J* = 7.4 Hz, 3H), 1.31-1.48 (m, 2H), 2.98-3.03 (m, 1H), 3.23-3.28 (m, 1H), 3.44-3.45 (m, 1H), 4.83 (d, *J =* 5.10 Hz, 1H), 6.34 (t, *J =* 5.35 Hz, 1H), 7.25-7.36 (m, 10H), 7.70-7.72 (dd, *J1* = 2.10 Hz, *J*2 = 9.05 Hz, 1H), 7.95 (d, *J =* 7.25 Hz, 1H), 8.13-8.21 2H), 9.01 (s, 1H) | 412.28 | 99.58 |
| 69 | 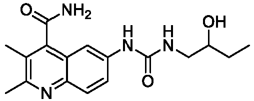 | 6-(3-(2-hydroxybutyl) ureido)-2,3-dimethylquino line-4-carboxamide | (500 MHz; DMSO-d₆): δ 0.90 (t, *J* = 7.4 Hz, 3H), 1.33-1.39 (m, 1H), 1.41-1.45 (m, 1H), 2.32 (s, 3H), 2.58 (s, 3H), 2.94- 2.99 (m, 1H), 3.20-3.25 (m, 1H), 3.36-3.42 (m, 1H), 4.80 (d, *J* = 5.0 Hz, 1H), 6.20 (t, *J* = 5.25 Hz, 1H), 7.63 (d, *J* = 8.95 Hz, 1H), 7.77 (d, *J =* 9Hz, 1H), 7.90 (s, 2H), 8.06 (s, 1H), 8.97 (s, 1H) | 331.24 | 97.70 |
| 70 | 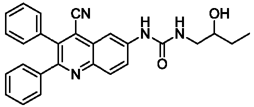 | 1-(4-cyano-2,3-diphenylquinol in-6-yl)-3-(2-hydroxybutyl)urea | (500 MHz; DMSO-d₆): δ 0.90 (t, *J* = 7.4 Hz, 3H), 1.33-1.39 (m, 1H), 1.40-1.47 (m, 1H), 2.99- 3.04 (m, 1H), 3.26-3.36 (m, 1H), 3.46-3.47 (m, 1H), 4.84 (d, *J* = 5.05 Hz, 1H), 6.41 (t, *J* = 5.35 Hz, 1H), 7.24-7.31 (m, 5H), 7.37-7.41 (m, 5H), 7.80 (d, *J =* 7.85 Hz, 1H), 8.12 (d, *J =* 9.1 Hz, 1H), 8.54 (d, *J =* 1.4 Hz, 1H), 9.40 (s, 1H) | 437.28 | 99.79 |
| 71 | 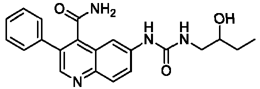 | 6-(3-(2-hydroxybutyl) ureido)-3-phenylquinoline -4-carboxamide | (500 MHz; DMSO-d₆): δ 0.89 (t, *J* = 7.4 Hz, 3H), 1.33-1.37 (m, 1H), 1.42-1.44 (m, 1H), 2.97- 2.99 (m, 1H), 3.73-3.74 (m, 2H), 4.81 (bs, 1H), 6.28 (s, 1H), 7.46-7.51 (m, 3H), 7.63 (d, J = 7.15 Hz, 2H), 7.78-7.82 (m, 2H), 7.96 (d, *J* = 9.05 Hz, 1H), 8.05 (s, 1H), 8.12 (s, 1H), 8.71 (s, 1H), 9.15 (s, 1H) | 379.28 | 86.58 |
| 72 | 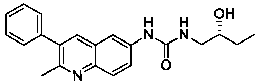 | (R)-1-(2-hydroxybutyl)-3-(2-methyl-3-phenylquinolin -6-yl)urea | (400 MHz; DMSO-d₆): δ 0.88 (t, *J* = 7.44 Hz, 3H), 1.28-1.44 (m, 2H), 2.50 (s, 3H), 2.93-2.99 (m, 1H), 3.18-3.32 (m, 1H), 3.40 (d, *J* = 4.84 Hz, 1H), 4.76 (d, *J* = 4.96 Hz, 1H), 6.25 (t, *J =* 5.72 Hz, 1H), 7.40-7.50 (m, 5H), 7.61-7.64 (m, 1H), 7.82 (d, *J* = 9.0 Hz, 1H), 7.95 (s, 1H), 7.99 (s, 1H), 8.90 (s, 1H) | 350.28 | 98.20 |
| 73 | 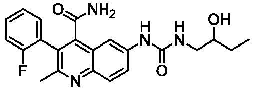 | 3-(2-fluorophenyl)-6-(3-(2-hydroxybutyl) ureido)-2-methylquinoline-4-carboxamide | (500 MHz; DMSO-d₆): δ 0.89 (t, *J* = 7.4 Hz, 3H), 1.32-1.36 (m, 1H), 1.40-1.45 (m, 1H), 2.32 (s, 3H), 2.95-2.98 (m, 1H), 3.21-3.24 (m, 1H), 3.42-3.43 (m, 1H), 4.81 (d, *J* = 4.9 Hz, 1H), 6.39 (s, 1H), 7.28-7.30 (m, 2H), 7.31-34 (m, 1H), 7.41-7.43 (m, 1H), 7.47-7.50 (m, 1H), 7.68-7.74 (m, 1H), 7.87-7.89 (m, 1H), 7.98 (s 1H), 8.07 (s, 1H), 9.20 (s, 1H) | 411.28 | 98.90 |
| 74 | 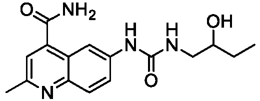 | 6-(3-(2-hydroxybutyl) ureido)-2-methylquinoli ne-4-carboxamide | (500 MHz; DMSO-d₆): δ 0.89 (t, *J* = 7.35 Hz, 3H), 1.31-1.38 (m, 1H), 1.40-1.45 (m, 1H), 2.61 (s, 3H), 2.94-2.99 (m, 1H), 3.20-3.36 (m, 1H), 3.42-3.43 (m, 1H), 4.80 (d, *J =* 5 Hz, 1H), 6.25 (s, 1H), 7.34 (s, 1H), 7.78-7.83 (m, 3H), 8.11 (s, 1H), 8.18 (s, 1H), 9.03 (s, 1H) | 317.18 | 99.10 |
| 75 | 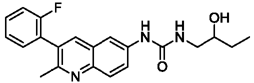 | 1-(3-(2-fluorophenyl)-2-methylquinoli n-6-yl)-3-(2-hydroxybutyl) urea | (400 MHz; DMSO-d₆): δ 0.86 (t, *J* = 7.4 Hz, 3H), 1.30-1.42 (m, 2H), 2.48 (t, *J* = 1.64 Hz, 3H), 2.94-2.99 (m, 1H), 3.18-3.31 (m, 1H), 3.41 (bs, 1H), 4.76 (bs, 1H), 6.27 (t, *J =* 5.36 Hz, 1H), 7.49-7.53 (m, 1H), 7.62-7.65 (m, 1H), 7.83 (d, *J* = 9.04 Hz, 1H), 7.93-7.96 (m, 1H), 8.02-8.04 (m, 2H), 8.61-8.69 (m, 2H), 8.93 (s, 1H) | 368.28 | 98.13 |
| 76 | 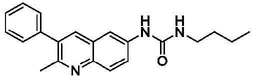 | 1-butyl-3-(2-methyl-3-phenylquinolin -6-yl)urea | (500 MHz; DMSO-d₆): δ 0.91 (t, *J* = 7.3 Hz, 3H), 1.31-1.36 (m, 2H), 1.41-1.47 (m, 2H), 2.53 (s, 3H), 3.11 (t, *J* = 6.25 Hz, 2H), 6.25 (t, *J* = 5.45 Hz, 1H), 7.44-7.45 (m, 1H), 7.50-7.53 (m, 4H), 7.65-7.67 (m, 1H), 7.84 (d, *J* = 9.05 Hz, 1H), 7.96 (s, 1H), 8.02 (m, 1H), 8.75 (s, 1H) | 334.29 | 97.40 |
| 77 | 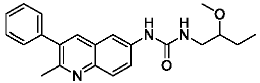 | 1-(2-methoxybutyl) -3-(2-methyl-3-phenylquinolin -6-yl)urea | (500 MHz; DMSO-d₆): δ 0.88 (t, *J* = 7.3 Hz, 3H), 1.43-1.52 (m, 2H), 2.53 (s, 3H), 3.14-3.20 (m, 2H), 3.35 (s, 4H), 6.28 (s, 1H), 7.44-7.50 (m, 5H), 7.65 (d, *J* = 8.75 Hz, 1H), 7.85 (d, *J* = 8.95 Hz, 1H), 7.98 (s, 1H), 8.03 (s, 1H), 8.89 (s, 1H) | 364.32 | 98.15 |
| 78 | 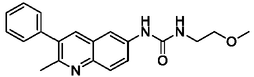 | 1-(2-methoxyethyl) -3-(2-methyl-3-phenylquinolin -6-yl)urea | (500 MHz; DMSO-d₆): δ 2.52 (d, *J* = 11.95 Hz, 3H), 3.29 (t, *J* = 8.55 Hz, 5H), 3.41 (t, *J* = 5.35 Hz, 2H), 6.33 (t, *J* = 5.35 Hz, 1H), 7.44-7.53 (m, 5H), 7.65 (d, *J =* 9.1 Hz, 1H), 7.85 (d, *J* = 9.05 Hz, 1H), 7.98 (s, 1H), 8.02 (s, 1H), 8.88 (s, 1H) | 336.25 | 98.34 |
| 79 | 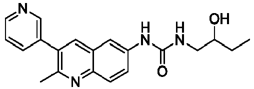 | 1-(2-hydroxybutyl)-3-(2-methyl-3-(pyridin-3-yl)quinolin-6-yl)urea | (500 MHz; DMSO-d₆): δ 0.90 (t, *J* = 7.4 Hz, 3H), 1.32-1.44 (m, 2H), 2.54 (s, 3H), 2.96-3.01 (m, 1H), 3.21-3.23 (m, 1H), 3.41 (bs, 1H), 4.81 (d, *J* = 6.45 Hz, 1H), 6.29 (t, *J* = 5.2 Hz, 1H), 7.53-7.55 (m, 1H), 7.65-7.67 (m, 1H), 7.86 (d, *J* = 8.6 Hz, 1H), 7.98 (d, *J* = 7.8 Hz, 1H), 8.06 (d, *J =* 10.1 Hz, 2H), 8.66 (d, *J =* 4.3 Hz, 1H), 8.72 (s, 1H), 8.96 (s, 1H) | 351.26 | 99.28 |
| 80 | 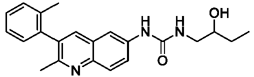 | 1-(2-hydroxybutyl)-3-(2-methyl-3-(o-tolyl)quinolin-6-yl)urea | (500 MHz; DMSO-d₆): δ 0.89 (t, *J* = 7.4 Hz, 3H), 1.32-1.44 (m, 2H), 2.04 (s, 3H), 2.31 (s, 3H), 2.97-2.99 (m, 1H), 3.21-3.23 (m, 1H), 3.41 (bs, 1H), 4.80 (d, *J* = 3.6 Hz, 1H), 6.28 (t, *J* = 5.0 Hz, 1H), 7.21 (d, *J* = 7.2 Hz, 1H), 7.29-7.36 (m, 3H), 7.65 (d, *J* = 8.85 Hz, 1H), 7.85 (d, *J* = 9.05 Hz, 1H), 7.88 (s, 1H), 7.99 (s, 1H), 8.93 (s, 1H) | 364.33 | 98.04 |
| 81 | 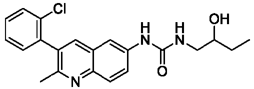 | 1-(3-(2-chlorophenyl)-2-methylquinoli n-6-yl)-3-(2-hydroxybutyl) urea | (500 MHz; DMSO-d₆): δ 0.89 (t, *J* = 7.4 Hz, 3H), 1.30-1.45 (m, 2H), 2.35 (s, 3H), 2.95-3.0 (m, 1H), 3.21-3.37 (m, 1H), 3.42-3.43 (m, 1H), 4.81 (d, *J =* 4.6 Hz, 1H), 6.30 (t, *J =* 5.25 Hz, 1H), 7.45-7.51 (m, 3H), 7.63-7.69 (m, 2H), 7.85-7.87 (m, 1H), 7.96 (s, 1H), 8.01 (s, 1H), 8.97 (s, 1H) | 384.29 | 98.44 |
| 82 | 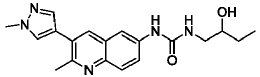 | 1-(2-hydroxybutyl)-3-(2-methyl-3-(1-methyl-1H-pyrazol-4-yl)quinolin-6-yl)urea | NA | 354.25 | 98.25 |
| 83 | 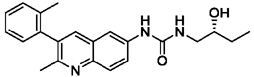 | (R)-1-(2-hydroxybutyl)-3-(2-methyl-3-(o-tolyl)quinolin-6-yl)urea | (500 MHz; DMSO-d₆): δ 0.89 (t, *J* = 7.4 Hz, 3H), 1.30-1.45 (m, 2H), 2.04 (s, 3H), 2.30 (s, 3H), 2.97-2.99 (m, 1H), 3.22-3.36 (m, 1H), 3.42-3.50 (m, 1H), 4.81 (d, *J* = 4.65 Hz, 1H), 6.28 (t, *J* = 5.25 Hz, 1H), 7.20-7.21 (m, 1H), 7.29-7.38 (m, 3H), 7.64-7.66 (m, 1H), 7.84-7.88 (m, 2H), 7.99 (s, 1H), 8.92 (s, 1H) | 364.33 | 98.37 |
| 84 | 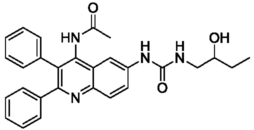 | N-(6-(3-(2-hydroxybutyl) ureido)-2,3-diphenylquinol in-4-yl)acetamide | (500 MHz; DMSO-d₆): δ 0.90 (t, *J* = 7.4 Hz, 3H), 1.32-1.38 (m, 1H), 1.40-1.47 (m, 1H), 1.88 (s, 3H), 2.89- 3.01 (m, 1H), 3.23-3.36 (m, 1H), 3.43-3.44 (m, 1H), 4.82 (s, 1H), 6.29 (s, 1H), 7.10 (d, *J* = 5.55 Hz, 2H), 7.20-7.28 (m, 8H), 7.72-7.80 (m, 1H), 7.98 (d, *J* = 9.0 Hz, 1H), 8.04 (s, 1H), 9.09 (s, 1H), 9.72 (s, 1H) | 469.40 | 98.15 |
| 85 | 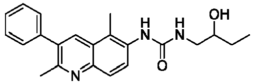 | 1-(2,5-dimethyl-3-phenylquinolin -6-yl)-3-(2-hydroxybutyl) urea | (500 MHz; DMSO-d₆): δ 0.90 (t, *J* = 7.4 Hz, 3H), 1.33-1.38 (m, 1H), 1.40-1.47 (m, 1H), 2.47 (s, 3H), 2.55 (s, 3H), 2.97-3.01 (m, 1H), 3.21-3.37 (m, 1H), 3.4-3.45 (m, 1H), 4.82 (d, *J =* 4.30 Hz, 1H), 6.62 (t, *J* = 5.35 Hz, 1H), 7.45-7.48 (m, 1H), 7.50-7.53 (m, 4H), 7.74 (d, *J* = 9.15 Hz, 1H), 8.12-8.16 (m, 2H), 8.23 (s, 1H) | 364.04 | 98.24 |
| 86 | 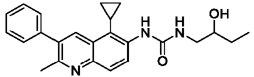 | 1-(5-cyclopropyl-2-methyl-3-phenylquinolin -6-yl)-3-(2-hydroxybutyl) urea | (500 MHz; DMSO-d₆): δ 0.50 (d, *J* = 4.3 Hz, 2H), 0.91 (t, *J* = 7.4 Hz, 3H), 1.67-1.24 (m, 2H), 1.33-1.39 (m, 1H), 1.44-1.49 (m, 1H), 1.85-1.99 (m, 1H), 2.54 (s, 3H), 3.02-3.07 (m, 1H), 3.20-3.36 (m, 1H), 3.44-3.46 (m, 1H), 4.79 (s, 1H), 6.96 (s, 1H), 7.46-7.52 (m, 5H), 7.76 (d, *J* = 9.25 Hz, 1H), 8.28 (bs, 2H), 8.44 (s, 1H) | 390.05 | 98.73 |
| 87 | 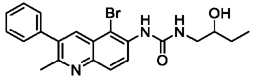 | 1-(5-bromo-2-methyl-3-phenylquinolin -6-yl)-3-(2-hydroxybutyl) urea | (500 MHz; DMSO-d₆): δ 0.90 (t, *J* = 7.4 Hz, 3H), 1.33-1.48 (m, 2H), 2.58 (s, 3H), 3.00-3.05 (m, 1H), 3.22-3.35 (m, 1H), 3.43 (bs, 1H), 4.86 (s, 1H), 7.39 (s, 1H), 7.48-7.49 (m, 1H), 7.50-7.54 (m, 4H), 7.92 (d, *J* = 9.30 Hz, 1H), 8.10 (s, 1H), 8.45 (s, 1H), 8.52 (d, *J* = 9.3 Hz, 1H) | 428.28 | 99.67 |
| 88 | 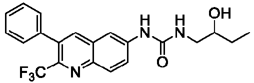 | 1-(2-hydroxybutyl)-3-(3-phenyl-2-(trifluorometh yl)quinolin-6-yl)urea | (500 MHz; DMSO-d₆): δ 0.88 (t, *J =* 7.32 Hz, 3H), 1.34-1.43 (m, 2H), 2.97-3.01 (m, 1H), 3.22-3.31 (m, 1H), 3.44 (s, 1H), 4.78 (d, *J* = 4.2 Hz, 1H), 6.37 (s, 1H), 7.44-7.48 (m, 4H), 7.85 (d, *J =* 9.52 Hz, 1H), 8.07 (d, *J =* 9.52 Hz, 1H), 8.17 (s, 1H), 8.30 (s, 1H), 9.17 (s, 1H) (one proton missing). | 404.20 | 99.43 |
| 89 | 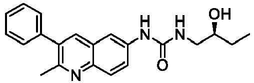 | (S)-1-(2-hydroxybutyl)-3-(2-methyl-3-phenylquinolin -6-yl)urea | (500 MHz; DMSO-d₆): δ 0.89 (t, *J* = 7.45 Hz, 3H), 1.31-1.46 (m, 2H), 2.53 (s, 3H), 2.96-3.01 (m, 1H), 3.21-3.36 (m, 1H), 3.42-3.43 (m, 1H), 4.81 (d, *J =* 5.1 Hz, 1H), 6.32 (t, *J =* 5.45 Hz, 1H), 7.43-7.50 (m, 5H), 7.63-7.66 (m, 1H), 7.97 (m, 1H), 8.02 (d, *J* = 1.85 Hz, 2H), 8.96 (s, 1H) | 350.25 | 99.23 |
| 90 | 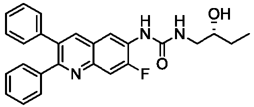 | (R)-1-(7-fluoro-2,3-diphenylquinol in-6-yl)-3-(2-hydroxybutyl) urea | (500 MHz; DMSO-d₆): δ 0.90 (t, *J* = 7.45 Hz, 3H), 1.35-1.45 (m, 2H), 3.00-3.05 (m, 1H), 3.24-3.36 (m, 1H), 3.43-3.44 (m, 1H), 4.82 (d, *J* = 3.9 Hz, 1H), 6.97 (t, *J* = 5.4 Hz, 1H), 7.25-7.35 (m, 10H), 7.85 (d, *J* = 12.6 Hz, 1H), 8.26 (s, 1H), 8.75 (d, *J* = 8.9 Hz, 1H), 8.87 (s, 1H) | 430.32 | 96.47 |
| 91 | 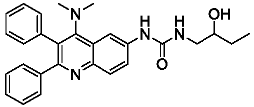 | 1-(4-(dimethylamino )-2,3-diphenylquinol in-6-yl)-3-(2-hydroxybutyl) urea | (500 MHz; DMSO-d₆): δ 0.90 (t, *J* = 7.45 Hz, 3H), 1.35-1.38 (m, 1H), 1.42-144 (m, 1H), 2.57 (s, 6H), 2.97-3.00 (m, 1H), 3.25-3.27 (m, 1H), 3.44-3.45 (m, 1H), 4.82 (d, *J* = 5.2 Hz, 1H), 6.26 (t, *J* = 5.35 Hz, 1H), 7.11-7.17 (m, 7H), 7.25-7.30 (m, 3H), 7.65-7.67 (m, 1H), 7.86 (d, *J* = 9 Hz, 1H), 8.32 (d, *J* = 2.25 Hz, 1H), 9.01 (s, 1H) | 455.40 | 99.66 |
| 92 | 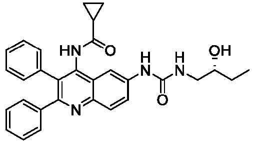 | (R)-N-(6-(3-(2-hydroxybutyl) ureido)-2,3-diphenylquinol in-4-yl)cyclopropan ecarboxamide | (500 MHz; DMSO-d₆): δ 0.62-0.66 (m, 4H), 0.90 (t, *J =* 7.4 Hz, 3H), 1.34-1.38 (m, 1H), 1.42-146 (m, 1H), 1.68-1.70 (m, 1H), 2.98- 3.02 (m, 1H), 3.22-3.36 (m, 1H), 3.44-3.45 (m, 1H), 4.82 (d, *J* = 5.1 Hz, 1H), 6.34 (t, *J* = 5.10 Hz, 1H), 7.08 (t, *J* = 3.1 Hz, 2H), 7.20-7.27 (m, 8H), 7.81-8.00 (m, 3H), 9.14 (s, 1H), 9.88 (s, 1H) | 495.41 | 98.53 |
| 93 | 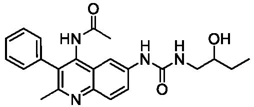 | N-(6-(3-(2-hydroxybutyl) ureido)-2-methyl-3-phenylquinolin -4-yl)acetamide | (500 MHz; DMSO-d₆): δ 0.89 (t, *J* = 7.4 Hz, 3H), 1.31-1.36 (m, 1H), 1.40-1.44 (m 1H), 1.82 (s, 3H), 2.35 (s, 3H), 2.95-3.00 (m, 1H), 3.20-3.36 (m, 1H), 3.40-3.42 (m, 1H), 4.80 (bs, 1H), 6.27 (t, *J* = 5.45 Hz, 1H), 7.26 (d, *J* = 7.05 Hz, 2H), 7.40-7.48 (m, 3H), 7.70-7.72 (m, 1H), 7.86 (d, *J* = 9.05 Hz, 1H), 7.94 (s, 1H), 9.01 (s, 1H), 9.60 (s, 1H) | 407.34 | 98.54 |
| 94 | 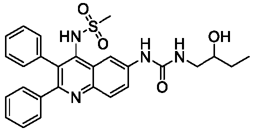 | N-(6-(3-(2-hydroxybutyl) ureido)-2,3-diphenylquinol in-4-yl)methanesulf onamide | (500 MHz; DMSO-d₆): δ 0.90 (t, *J* = 7.4 Hz, 3H), 1.34-1.46 (m, 2H), 2.24 (s, 3H), 2.98-3.03 (m, 1H), 3.24-3·35 (m, 1H), 3.45 (s, 1H), 4.81 (d, *J* = 5.1 Hz, 1H), 6.36 (d, *J* = 5.1 Hz, 1H), 7.22-7.34 (m, 10H), 7.97 (d, *J =* 9 Hz, 1H), 8.05 (d, *J* = 9.15 Hz, 1H), 8.17 (s, 1H), 9.16 (s, 1H), 9.56 (bs, 1H) | 505.37 | 98.16 |
| 95 | 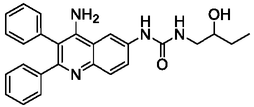 | 1-(4-amino-2,3-diphenylquinol in-6-yl)-3-(2-hydroxybutyl) urea | (500 MHz; DMSO-d₆): δ 0.90 (t, *J* = 7.4 Hz, 3H), 1.42-1.47 (m, 2H), 2.98-3.03 (m, 1H), 3.24-3.28 (m, 1H), 3.44-3.45 (m, 1H), 4.82 (d, *J* =4.95 Hz, 1H), 6.53 (s, 1H), 7.21 (d, *J* = 7.05 Hz, 2H), 7.36-7.39 (m, 10H), 7.87-7.92 (m, 2H), 8.34 (s, 1H), 8.99 (s, 1H) | 427.37 | 98.19 |
| 96 | 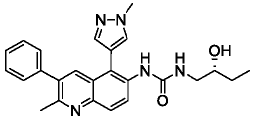 | (R)-1-(2-hydroxybutyl)-3-(2-methyl-5-(1-methyl-1H-pyrazol-4-yl)-3-phenylquinolin -6-yl)urea | (500 MHz; DMSO-d₆): δ 0.89 (t, *J* = 7.4 Hz, 3H), 1.30-1.39 (m, 1H), 1.41-1.43 (m 1H), 2.53 (s, 3H), 2.93-3.98 (m, 1H), 3.14-3.19 (m, 1H), 3.38-3.40 (m, 1H), 4.01 (s, 3H), 4.73 (d, *J =* 5.1 Hz, 1H), 6.99 (t, *J =* 5.25 Hz, 1H), 7.39-7.43 (m, 3H), 7.45-7.48 (m, 2H), 7.53 (s, 1H), 7.57 (s, 1H), 7.69 (s, 1H), 7.87 (d, *J* = 7.0 Hz, 2H), 8.52 (d, *J* = 9.3 Hz, 1H) | 430.37 | 98.23 |
| 97 | 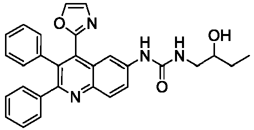 | 1-(2-hydroxybutyl)-3-(4-(oxazol-2-yl)-2,3-diphenylquinol in-6-yl)urea | (400 MHz; DMSO-d₆): δ 0.87 (t, *J* = 7.44 Hz, 3H), 1.15-1.19 (m, 1H), 1.33-1.40 (m 1H), 1.98 (s, 2H), 2.93 (bs, 1H), 3.18-3.22 (m, 1H), 4.02 (d, *J* -= 6.84 HZ, 1H), 6.23 (s, 1H), 6.99 (s, 2H), 7.15-7.23 (m, 5H), 7.29 (bs, 2H), 7.36 (s, 1H), 7.78 (s, 1H), 7.85 (d, *J* = 11.2 Hz, 1H), 8.06 (d, *J* = 8.64 Hz, 1H), 8.11 (s, 1H), 9.10 (s, 1H) | 479.20 | 98.15 |
| 98 | 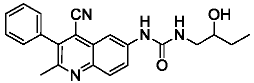 | 1-(4-cyano-2-methyl-3-phenylquinolin -6-yl)-3-(2-hydroxybutyl) urea | (400 MHz; DMSO-d₆): δ 0.87 (d, J = 7.44 Hz, 3H), 1.30-1.43 (m, 2H), 2.42 (s, 3H), 2.95-3.00 (m, 1H), 3.20-3.32 (m, 1H), 3.41-3.42 (m, 1H), 4.78 (d, *J =* 5.12 Hz, 1H), 6.33 (t, *J =* 5.32 Hz, 1H), 7.50-7.58 (m, 5H), 5.69-5.72 (dd, *J*₁ = 2.24 Hz, *J*₂ = 9.08 Hz, 1H), 7.98 (d, *J* = 9.08 Hz, 1H), 8.40 (d, *J* = 2.16 Hz, 1H), 9.27 (s, 1H) | 375.30 | 97.03 |
| 99 | 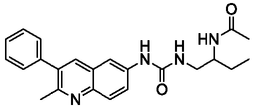 | N-(1-(3-(2-methyl-3-phenylquinolin -6-yl)ureido)buta n-2-yl)acetamide | NA | 391.36 | 95.08 |
| 100 | 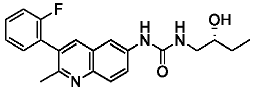 | (R)-1-(3-(2-fluorophenyl)-2-methylquinolin -6-yl)-3-(2-hydroxybutyl)urea | (500 MHz; DMSO-d₆): δ 0.89 (d, J = 7.4 Hz, 3H), 1.30-1.46 (m, 2H), 2.43 (s, 3H), 2.96-3.01 (m, 1H), 3.22-3.34 (m, 1H), 3.43-3.44 (m, 1H), 4.80 (d, *J =* 4.95 Hz, 1H), 6.29 (t, *J =* 5.05 Hz, 1H), 7.35-7.47 (m, 2H), 7.48-7.55 (m, 2H), 7.68 (d, *J* = 8.85 Hz, 1H), 7.86 (d, *J =* 9 Hz, 1H), 8.04 (d *J* = 6.8 Hz, 2H), 8.95 (s, 1H) | 368.32 | 99.15 |
| 101 | 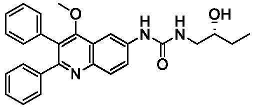 | (R)-1-(2-hydroxybutyl)-3-(4-methoxy-2,3-diphenylquinol in-6-yl)urea | (500 MHz; DMSO-d₆): δ 0.88 (t, *J* = 7.4 Hz, 3H), 1.32-1.42 (m, 2H), 2.96-3.01 (m, 1H), 3.21-3.25 (m, 1H), 3.42 (d, *J* = 4.76 Hz, 1H). 3.49 (s, 3H), 4.75 (d, *J =* 5.16 Hz, 1H), 6.26 (t, *J* = 5.48 Hz, 1H), 7.16-7.33 (m, 10H), 7.67 (dd, *J'* = 2.36 Hz, *J"* = 9.04 Hz, 1H), 7.93 (d, *J* = 9.08 Hz, 1H), 8.29 (d, *J* = 2.44 Hz, 1H), 9.04 (s, 1H) | 442.27 | 99.51 |
| 102 | 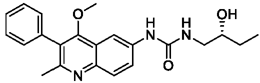 | (R)-1-(2-hydroxybutyl)-3-(4-methoxy-2-methyl-3-phenylquinolin -6-yl)urea | (500 MHz; DMSO-d₆): δ 0.90 (d, J = 7.45 Hz, 3H), 1.33-1.47 (m, 2H), 2.31 (s, 3H), 2.98-3.02 (m, 1H), 3.24-3.36 (m, 1H), 3.44-3.45 (m, 1H), 3.95 (s, 3H), 4.82 (d, *J* = 5.1 Hz, 1H), 6.29 (t, *J* = 5.3 Hz, 1H), 7.45-7.51 (m, 3H), 7.58-7.59 (m, 3H), 7.85-7.87 (m, 1H), 8.26 (d, *J* = 1.85 Hz, 1H), 9.04 (s, 1H) | 380.32 | 98.69 |
| 103 | 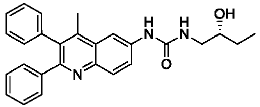 | (R)-1-(2-hydroxybutyl)-3-(4-methyl-2,3-diphenylquinol in-6-yl)urea | (400 MHz; DMSO-d₆): δ 0.90 (t, *J* = 7.4 Hz, 3H), 1.32-1.42 (m, 2H), 2.35 (s, 3H), 2.98-3.03 (m, 1H), 3.21-3.39 (m, 1H), 3.42-3.43 (m, 1H), 4.76 (d, *J =* 5.08 Hz, 1H). 3.49 (s, 3H), 6.28 (t, *J* = 5.24 Hz, 1H), 7.12-7.20 (m, 5H), 7.31-7.25 (m, 2H), 7.27-7.31 (m, 3H), 7.63-7.65 (dd, *J' =* 2.2 Hz, *J"* = 9.08 Hz, 1H), 7.91 (d, *J* = 8.96 Hz, 1H), 8.31 (d, *J* = 2.08 Hz, 1H), 9.01 (s, 1H) | 426.37 | 99.69 |
| 104 | 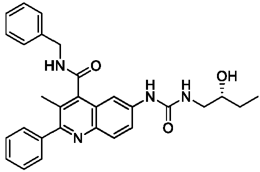 | (R)-N-benzyl-6-(3-(2-hydroxybutyl) ureido)-3-methyl-2-phenylquinoline -4-carboxamide | (400 MHz; DMSO-d₆): δ 0.89 (t, *J* = 7.4 Hz, 3H), 1.32-1.43 (m, 2H), 2.23 (s, 3H), 2.98-3.02 (m, 1H), 3.22-3.30 (m, 1H), 3.43-3.45 (m, 1H), 4.57-4.58 (m, 2H), 4.77 (d, *J* = 5.16 Hz, 1H), 6.26 (t, *J* = 5.12 Hz, 1H), 7.23-7.26 (m, 1H), 7.33-7.37 (m, 2H), 7.42-7.53 (m, 7H), 7.63-7.66 (m, 1H), 7.86 (d, *J =* 9.04 Hz, 1H), 8.03 (d, *J =* 2.16 Hz, 1H), 9.03 (s, 1H), 9.17 (t, *J* = 5.96 Hz, 1H) | 483.40 | 96.33 |
| 105 | 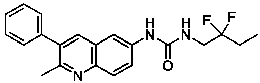 | 1-(2,2-difluorobutyl)-3-(2-methyl-3-phenylquinolin -6-yl)urea | (400 MHz; DMSO-d₆): δ 0.96 (d, J = 7.52 Hz, 3H), 1.81-1.95 (m, 2H), 2.47-2.48 (m, 3H), 3.58-3.63 (m, 2H), 6.62 (d, *J* = 6.12 Hz, 1H), 7.39-7.51 (m, 5H), 7.63-7.66 (dd, *J*1 = 2.36 Hz, *J*2 = 9.04 Hz, 1H), 7.84 (d, *J* = 9.08 Hz, 1H), 7.96 (s, 1H), 8.01 (d, *J* = 2.2 Hz, 1H), 8.89 (s, 1H) | 370.31 | 99.67 |
| 106 | 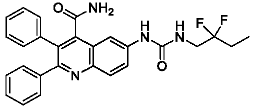 | 6-(3-(2,2-difluorobutyl)u reido)-2,3-diphenylquinol ine-4-carboxamide | (400 MHz; DMSO-d₆): δ 0.97 (d, J = 7.44 Hz, 3H), 1.81-2.06 (m, 2H), 3.60-3.64 (m, 2H), 6.66 (t, *J =* 6.16 Hz, 1H), 7.20-7.24 (m, 10H), 7.62 (s, 1H), 7.77-7.79 (dd, *J*1 = 11.4 Hz, *J*2 = 1.12 Hz, 1H), 7.89 (s, 1H), 7.97 (d, *J* = 9.08 Hz, 1H), 8.12 (d, *J* = 2.24 Hz, 1H), 9.20 (s, 1H) | 475.40 | 98.87 |
| 107 | 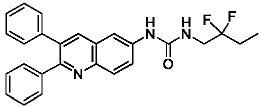 | 1-(2,2-difluorobutyl)-3-(2,3-diphenylquinol in-6-yl)urea | (400 MHz; DMSO-d₆): δ 0.97 (d, J = 7.48 Hz, 3H), 1.81-1.96 (m, 2H), 3.56-3.65 (m, 2H), 6.66 (t, *J =* 6.2 Hz, 1H), 7.22-7.34 (m, 10H), 7.70-7.73 (dd, *J*1 *=* 2.36 Hz, *J*2 = 9.08 Hz, 1H), 7.95 (d, *J* = 9.04 Hz, 1H), 8.12 (d, *J* = 2.24 Hz, 1H), 8.20 (s, 1H), 9.01 (s, 1H) | 432.38 | 98.09 |
| 108 | 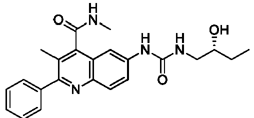 | (R)-6-(3-(2-hydroxybutyl) ureido)-N,3-dimethyl-2-phenylquinoline -4-carboxamide | (400 MHz; DMSO-d₆): δ 0.87 (t, *J* = 7.4 Hz, 3H), 1.30-1.45 (m, 2H), 2.24 (s, 3H), 2.87 (d, *J* = 4.56 Hz, 3H), 2.93-2.99 (m, 1H), 3.18-3.29 (m, 1H), 3.41 (bs, 1H), 4.75 (s, 1H), 6.27 (t, *J* = 5.56 Hz, 1H), 7.42-7.52 (m, 5H), 7.70-7.73 (dd *J*1 = 2.2 Hz, *J*2 = 9.04 Hz, 1H), 7.80-7.86 (m, 2H), 8.56 (t, *J* = 4.64 Hz, 1H), 9.07 (s, 1H) | 407.38 | 98.14 |
| 109 | 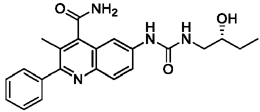 | (R)-6-(3-(2-hydroxybutyl) ureido)-3-methyl-2-phenylquinoline -4-carboxamide | (400 MHz; DMSO-d₆): δ 0.87 (t, *J* = 7.4 Hz, 3H), 1.31-1.44 (m, 2H), 2.30 (s, 3H), 2.90-2.99 (m, 1H), 3.19-3.29 (m, 1H), 3.41 (bs, 1H), 4.75 (d, *J* = 4.44 Hz, 1H), 6.24 (t, *J* = 5.40 Hz, 1H), 7.42-7.54 (m, 5H), 7.67-7.70 (dd *J*1 *=* 2.16 Hz, *J*2 = 9.08 Hz, 1H), 7.84 (d, *J* = 9.04 Hz, 1H), 7.91 (s, 1H), 7.972 (s, 1H), 7.978 (s, 1H), 8.10 (bs, 1H), 9.05 (s, 1H) | 393.37 | 98.27 |
| 110 | 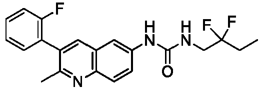 | 1-(2,2-difluorobutyl)-3-(3-(2-fluorophenyl)-2-methylquinolin -6-yl)urea | (500 MHz; DMSO-d₆): δ 0.99 (d, J = 7.45 Hz, 3H), 1.87-1.93 (m, 2H), 2.44 (s, 3H), 3.58-3.65 (m, 2H), 6.80 (t, *J* = 4.6 Hz, 1H), 7.35-7.38 (m, 2H), 7.40-7.54 (m, 2H), 7.70-7.72 (dd, *J*1 = 2.0 Hz, *J*2 = 8.95 Hz, 1H), 7.88 (d, *J* = 9.05 Hz, 1H), 8.07 (d, *J* = 2.7 Hz, 2H), 9.12 (s, 1H) | 388.35 | 98.57 |
| 111 | 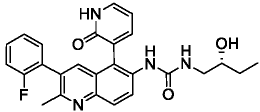 | (R)-1-(3-(2-fluorophenyl)-2-methyl-5-(2-OXO-1,2-dihydropyridin -3-yl)quinolin-6-yl)-3-(2-hydroxybutyl) urea | (500 MHz; DMSO-d₆): δ 0.88 (t, *J* = 7.4 Hz, 3H), 1.31-1.41 (m, 2H), 2.45 (s, 3H), 2.89-3.00 (m, 1H), 3.12-3.19 (m, 1H), 3.47 (bs, 2H), 4.72 (bs, 1H), 6.36 (t, *J* = 6.6 Hz, 1H), 6.93 (d, *J* = 4.9 Hz, 1H), 7.29-7.53 (m, 7H), 7.67 (s, 1H), 7.90 (d, *J* = 9.3 Hz, 1H), 8.56 (t, *J* = 9.05 Hz, 1H) | 461.44 | 95.19 |
| 112 | 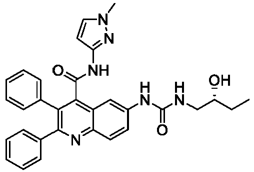 | (R)-6-(3-(2-hydroxybutyl) ureido)-N-(1-methyl-1H-pyrazol-3-yl)-2,3-diphenylquinol ine-4-carboxamide | (500 MHz; DMSO-d₆): δ 0.90 (t, *J* = 7.4 Hz, 3H), 1.31-1.43 (m, 2H), 2.92-2.97 (m, 1H), 3.19-3.34 (m, 1H), 3.40-3.41 (m, 1H), 3.70 (s, 3H), 4.77 (d, *J* = 5.15 Hz, 1H), 6.22 (t, *J* = 5.55 Hz, 1H), 6.39 (d, *J* = 2 Hz, 1H), 7.20-7.28 (m, 10H), 7.53 (d, J = 1.9 Hz, 1H), 7.87 (d, *J' =* 1.95 Hz, 1H), 7.91-7.93 (dd, *J*1 *=* 2.0 Hz, *J*2 = 9.1 Hz, 1H), 8.02 (d, *J* = 2.0 Hz, 1H), 9.12 (s, 1H), 10.85 (s, 1H) | 535.71 | 99.72 |
| 113 | 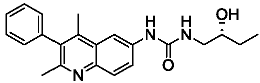 | (R)-1-(2,4-dimethyl-3-phenylquinolin -6-yl)-3-(2-hydroxybutyl) urea | (400 MHz; DMSO-d₆): δ 0.87 (d, J = 7.36 Hz, 3H), 1.30-1.44 (m, 2H), 2.21 (s, 3H), 2.24 (s, 3H), 2.93-2.99 (m, 1H), 3.19-3.32 (m, 1H), 3.41-3.42 (m, 1H), 4.76 (d, *J* = 4.8 Hz, 1H), 6.27 (d, *J =* 5 Hz, 1H), 7.24 (d, *J* = 7.16 Hz, 2H), 7.41-7.59 (m, 4H), 7.80 (d, *J* = 8.96 Hz, 1H), 7.82 (s, 1H), 8.94 (s, 1H) | 364.58 | 99.28 |
| 114 | 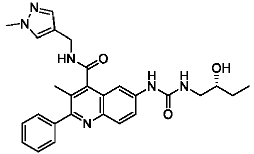 | (R)-6-(3-(2-hydroxybutyl) ureido)-3-methyl-N-((1-methyl-1H-pyrazol-4-yl)methyl)-2-phenylquinoline -4-carboxamide | (500 MHz; DMSO-d₆): δ 0.91 (bs, 3H), 1.37-1.45 (m, 2H), 2.25 (s, 3H), 3.02 (bs, 1H), 3.27 (bs, 1H), 3.46 (bs, 1H), 3.84 (s, 3H), 4.43 (bs, 2H), 4.82 (s, 1H), 6.32 (s, 1H), 7.43-7.54 (m, 7H), 7.61-7.89 (m, 2H), 8.04 (s, 1H), 9.02-9.08 (m, 2H) | 487.51 | 98.60 |
| 115 | 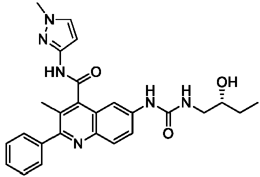 | (R)-6-(3-(2-hydroxybutyl) ureido)-3-methyl-N-(1-methyl-1H-pyrazol-3-yl)-2-phenylquinoline -4-carboxamide | (500 MHz; DMSO-d₆): δ 0.88 (bs, 3H), 1.33-1.41 (m, 2H), 2.31 (s, 3H), 2.95 (bs, 1H), 3.20 (bs, 1H), 3.41 (s, 1H), 3.79 (s, 3H), 4.77 (s, 1H), 6.20 (s, 1H), 6.72 (s, 1H), 7.49-7.57 (m, 5H), 7.68 (s, 1H), 7.79-7.82 (m, 2H), 7.91 (d, *J =* 10.1 Hz, 1H), 9.07 (s, 1H)11.12 (s, 1H). | 473.57 | 98.83 |
| 116 | 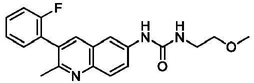 | 1-(3-(2-fluorophenyl)-2-methylquinolin-6-yl)-3-(2-methoxyethyl) urea | (500 MHz; DMSO-d₆): δ 2.43 (s, 3H), 3.29-3.50 (m, 7H), 6.32 (d, *J* = 5.4 Hz, 1H), 7.35-7.39 (m, 2H), 7.46-7.54 (m, 2H), 7.68-7.70 (dd, *J*1 = 2.15 Hz, *J*2= =9.05 Hz, 1H), 7.86 (d, *J =* 9.05 Hz, 1H), 8.03 (d, *J =* 9.1 Hz, 2H), 8.88 (s, 1H) | 354.21 | 99.68 |
| 117 | 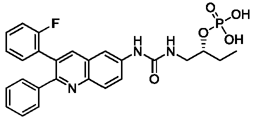 | (R)-1-(3-(3-(2-fluorophenyl)-2-phenylquinolin -6-yl)ureido)buta n-2-yl dihydrogen phosphate | (500 MHz; DMSO-d₆): δ 0.92 (bs, 3H), 1.60 (s, 2H), 3.36-3.39 (m, 3H), 4.16 (bs, 2H), 6.43 (s, 1H), 7.12-7.13 (m, 1H), 7.28-7.47 (m, 8H), 7.79 (d, *J =* 8.55 Hz, 1H), 8.01 (d, *J =* 8.75 Hz, 1H), 8.18 (s, 1H), 8.33 (s, 1H), 9.14 (s, 1H) | 510.24 | 99.66 |
| 131 | 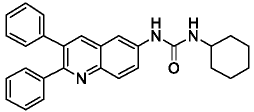 | 1-cyclohexyl-3-(2,3-diphenylquinol in-6-yl)urea | (400 MHz; DMSO-d₆): δ 1.15-1.24 (m, 3H), 1.28-1.34 (m, 2H), 1.53 (bs, 1H), 1.66 (bs, 2H), 1.84 (d, *J =* 9.52 Hz, 2H), 3.51 (bs, 1H), 6.23 (d, *J* = 7.88 Hz, 1H), 7.24-7.35 (m, 10H), 7.7-7.72 (m, 1H), 7.94 (d, *J* = 9 Hz, 1H), 8.09 (d, *J =* 1.96 Hz, 1H), 8.18 (s, 1H), 8.72 (s, 1H). | 422.42 | 99.04 |
| 132 | 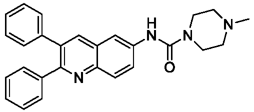 | N-(2,3-diphenylquinol in-6-yl)-4-methylpiperazi ne-1-carboxamide | (400 MHz; DMSO-d₆): δ 2.17 (s, 3H), 2.21 (s, 4H), 3.50 (s, 4H), 7.26-7.35 (m, 10H), 7.87 (d, *J =* 9.96 Hz, 1H), 7.95 (d, *J* = 9.04 Hz, 1H), 8.15 (s, 1H), 8.19 (s, 1H), 8.90 (S, 1H). | 423.28 | 99.78 |
| 133 | 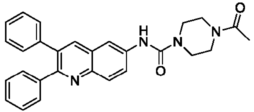 | 4-acetyl-N-(2,3-diphenylquinol in-6-yl)piperazine-1-carboxamide | (400 MHz; DMSO-d₆): δ 2.05 (s, 3H), 3.50-3.54 (m, 8H), 7.27-7.36 (m, 10H), 7.87-7.97 (q, 2H), 8.15 (s, 1H), 8.21 (s, 1H), 9.0 (s, 1H) | 451.26 | 99.44 |
| 134 | 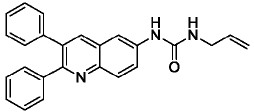 | 1-allyl-3-(2,3-diphenylquinol in-6-yl)urea | (400 MHz; DMSO-d₆): δ 3.78 (s, 2H), 5.09 (d, *J* = 10.4 Hz, 1H), 5.20 (d, *J =* 17.52 Hz, 1H), 5.87-5.93 (m, 1H), 6.44 (d, *J* = 5.18 Hz, 1H), 7.26-7.35 (m, 10H), 7.73-7.75 (m, 1H), 7.95 (d, *J* = 9.04 Hz, 1H), 8.12 (s, 1H), 8.19 (s, 1H), 8.95 (s, 1H). | 380.17 | 99.89 |
| 135 | 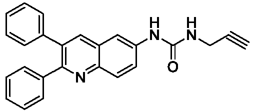 | 1-(2,3-diphenylquinol in-6-yl)-3-(prop-2-yn-1-yl)urea | (400 MHz; DMSO-d₆): δ 3.13 (s, 1H), 3.93 (d, *J* = 3.2 Hz, 2H), 6.61-6.62 (m, 1H), 7.26-7.35 (m, 10H), 7.74 (d, *J* = 7.2 Hz, 1H), 7.95 (d, *J* = 8.96 Hz, 1H), 8.13 (s, 1H), 8.21 (s, 1H), 9.02 (s, 1H). | 378.49 | 99.62 |
| 136 | 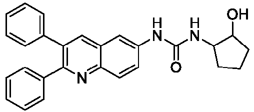 | 1-(2,3-diphenylquinol in-6-yl)-3-(2-hydroxycyclop entyl)urea | (400 MHz; DMSO-d₆): δ 1.13-1.23 (m, 1H). 1.37-1.48 (m, 1H), 1.62-1.67 (m, 2H), 1.76-1.82 (m, 1H), 1.99-2.08 (m, 1H), 3.74 (s, 1H), 3.84 (s, 1H), 6.31-6.33 (d, *J* = 6.2 Hz, 1H), 7.26-7.33 (m, 10H), 7.71 (d, *J* = 8.68 Hz, 1H), 7.94 (d, *J* = 8.64 Hz, 1H), 8.11 (s, 1H), 8.19 (s, 1H), 8.77 (s, 1H). | 424.50 | 99.74 |
| 137 | 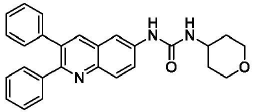 | 1-(2,3-diphenylquinol in-6-yl)-3-(tetrahydro-2H-pyran-4-yl)urea | (400 MHz; DMSO-d₆): δ 1.41-1.46 (m, 2H), 1.71 (s, 1H), 1.82 (d, *J* = 11.88 Hz, 2H), 3.37-3.42 (m, 3H), 3.73 (bs, 1H), 3.84 (d, *J* = 11.68 Hz, 2H), 6.69 (s, 1H), 7.24-7.35 (m, 8H), 7.74 (d, *J* = 9.16, 1H), 7.94 (d, *J* = 9.04 Hz, 1H), 8.12 (s, 1H), 8.17 (s, 1H), 9.11 (s, 1H). | 424.21 | 97.17 |
| 138 | 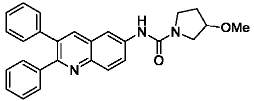 | N-(2,3-diphenylquinol in-6-yl)-3-methoxypyrrol idine-1-carboxamide | (400 MHz; DMSO-d₆): δ 2.00 (s, 2H), 3.32-3.27 (m, 3H), 3.39-3.56 (m, 4H), 4.01 (s, 1H), 7.26-7.35 (m, 10H), 7.95 (d, *J =* 9.08 Hz, 2H), 8.18-8.20 (m, 2H), 8.56 (s, 1H). | 424.40 | 99.74 |
| 139 | 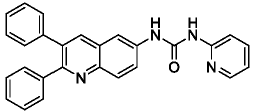 | 1-(2,3-diphenylquinol in-6-yl)-3-(pyridin-2-yl)urea | (400 MHz; DMSO-d₆): δ 7.04 (t, *J* = 5.94 Hz, 1H), 7.28-7.44 (m, 10H), 7.55 (d, *J =* 8.2 Hz, 1H), 7.78 (t, *J* = 6.92 Hz, 1H), 7.86 (d, *J* = 7.52 Hz, 1H), 8.04 (d, *J* = 9Hz, 1H), 8.30-8.33 (m, 3H), 9.60 (s, 1H), 10.90 (s, 1H). | 417.43 | 97.23 |
| 140 | 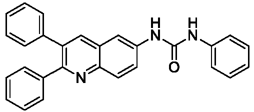 | 1-(2,3-diphenylquinol in-6-yl)-3-phenylurea | (400 MHz; DMSO-d₆): δ 6.99 (t, *J* = 7.3 Hz, 1H), 7.26-7.37 (m, 12H), 7.50 (d, *J* = 8.08 Hz, 2H), 7.77-7.80 (dd, *J'* = 2.24 Hz, *J"* = 2.2 Hz, 1H), 8.0 (d, *J* = 9 Hz, 1H), 8.21 (d, *J* = 2.08 Hz, 1H), 8.26 (s, 1H), 8.82 (s, 1H), 9.10 (s, 1H). | 416.44 | 99.47 |
| 141 | 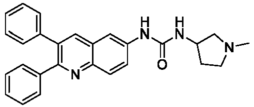 | 1-(2,3-diphenylquinol in-6-yl)-3-(1-methylpyrrolid in-3-yl)urea | (400 MHz; DMSO-d₆): δ 1.55-1.58 (m, 1H), 2.17-2.23 (m, 1H), 2.28-2.43 (m, 4H), 2.35-2.43 (m, 1H), 2.49-2.56 (m, 1H), 2.58-2.72 (m, 1H), 4.17 (s, 1H), 6.57 (d, *J* = 7.52 Hz, 1H), 7.72-7.35 (m, 10H), 7.69-7.71 (dd, *J*' = 2.12 Hz, *J"* = 1.96 Hz, 1H), 7.94 (d, *J* = 9.2 Hz, 1H), 8.10 (d, *J* = 1.88 Hz, 1H), 8.18-8.21 (m, 1H), 8.83 (s, 1H). | 423.46 | 99.13 |
| 142 | 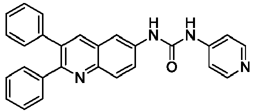 | 1-(2,3-diphenylquinol in-6-yl)-3-(pyridin-4-yl)urea | (400 MHz; DMSO-d₆): δ 7.28-7.37 (m, 10H), 7.49 (d, *J* = 5.96 Hz, 2H), 7.78-7.81 (dd, *J' =* 2.08 Hz, *J"* = 2.08 Hz, 1H), 8.03 (d, *J* = 9.04 Hz, 1H), 8.14 (s, 1H), 8.23 (d, *J* = 1.76 Hz, 1H), 8.30 (s, 1H), 8.38 (d, *J =* 5.88 Hz, 2H), 9.30 (d, *J =* 15.75 Hz, 1H). | 417.39 | 98.44 |
| 143 | 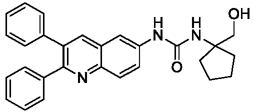 | 1-(2,3-diphenylquinol in-6-yl)-3-(1-(hydroxymethyl)cyclopentyl) urea | (400 MHz; DMSO-d₆): δ 1.54-1.59 (m, 2H), 1.65-1.72 (m, 4H), 1.82-1.89 (m, 2H), 3.49 (d, *J* = 5.44 Hz, 2H), 4.94 (t, *J* = 5.46 Hz, 1H), 6.20 (s, 1H), 7.23-7.35 (m, 10H), 7.64-7.67 (dd, *J*' = 2.32 Hz, *J" =* 2.28 Hz, 1H), 7.93 (d, *J* = 9.08 Hz, 1H), 8.12 (d, *J* = 2.24 Hz, 1H), 8.19 (s, 1H), 8.87 (s, 1H). | 438.50 | 99.25 |
| 144 | 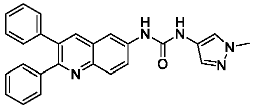 | 1-(2,3-diphenylquinol in-6-yl)-3-(1-methyl-1H-pyrazol-4-yl)urea | (400 MHz; DMSO-d₆): δ 3.79 (s, 3H), 7.25-7.36 (m, 10H), 7.41 (s, 1H), 7.75-7.77 (m, 2H), 7.98 (d, *J* = 9.04 Hz, 1H), 8.18 (d, *J =* 2.12 Hz, 1H), 8.23 (s, 1H), 8.53 (s, 1H), 9.04 (s, 1H). | 420.42 | 98.5 |
| 146 | 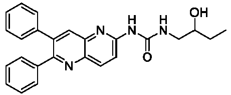 | 1-(6,7-diphenyl-1,5-naphthyridin-2-yl)-3-(2-hydroxybut yl)urea | 147 | 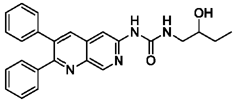 | 1-(2,3-diphenyl-1,7-naphthyridin -6-yl)-3-(2-hydroxybutyl )urea |
| 148 | 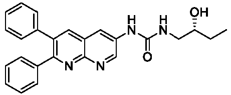 | (R)-1-(6,7-diphenyl-1,8-naphthyridin-3-yl)-3-(2-hydroxybutyl)urea | 149 | 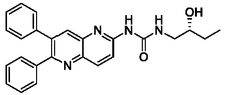 | (R)-1-(6,7-diphenyl-1,5-naphthyridin -2-yl)-3-(2-hydroxybutyl )urea |
| 150 | 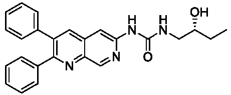 | (R)-1-(2,3-diphenyl-1,7-naphthyridin-6-yl)-3-(2-hydroxybutyl)urea | 151 | 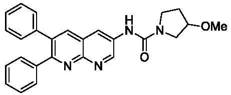 | N-(6,7-diphenyl-1,8-naphthyridin -3-yl)-3-methoxypyrr olidine-1-carboxamide |
| 152 | 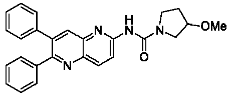 | N-(6,7-diphenyl-1,5-naphthyridin-2-yl)-3-methoxypyr rolidine-1-carboxamide | 153 | 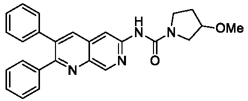 | N-(2,3-diphenyl-1,7-naphthyridin -6-yl)-3-methoxypyrr olidine-1-carboxamide |
| 154 | 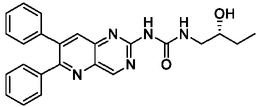 | (R)-1-(6,7-diphenylpyr ido[3,2-d]pyrimidin hydroxybut yl)urea | 155 | 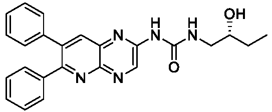 | (R)-1-(6,7-diphenylpyri do[2,3-b]pyrazin-2-yl)-3-(2-hydroxybutyl )urea |

### Example 128: N-(2,3-Bis(2-fluorophenyl)quinolin-6-yl)-4-hydroxyhexanamide

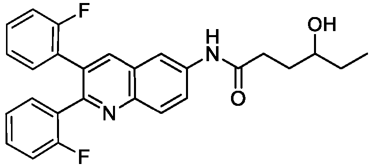

Example 128 was prepared according to the methods described in General Procedures 1, 2, 3, 7, 8, 9, 10 and the method described below.

### Preparation 15: N-(2,3-Bis(2-fluorophenyl)quinolin-6-yl)-4-oxohexanamide (Example 124)

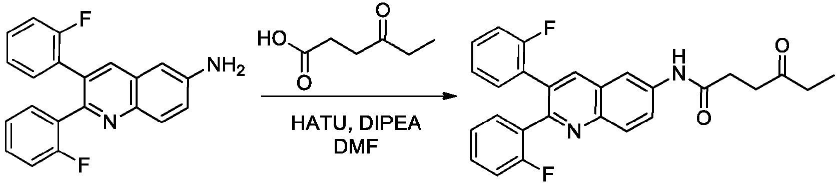

To a stirred solution of 4-oxohexanoic acid (56.2 mg, 0.432 mmol) in DMF (2 mL) was added DIPEA (0.103 mL, 0.602 mmol) and HATU (171.68 mg, 0.452 mmol). Stirring was continued for 10 min., then 2,3-bis(2-fluorophenyl)quinolin-6-amine (Preparation 7 Step 5) (100 mg, 0.301 mmol) was added. The whole was stirred at RT for 2 h. Progress of the reaction was confirmed by TLC/LCMS and after completion the reaction mixture was diluted with water and extracted with MTBE, washed with brine, dried over anhydrous Na₂SO₄ and evaporated in vacuo to give the crude product which was purified by prep-HPLC to afford the title compound (39 mg, yield 29 %) as a white solid. LCMS m/z: 445.2 [M+H]; Purity 98.04%; ¹H NMR (500 MHz; DMSO-d₆): δ 0.96 (t, *J* =7.3 Hz, 3H) 2.52 (t, *J =* 7.35 Hz, 2H), 2.66 (t, *J =* 6.65 Hz, 2H), 2.79 (t, *J =* 6.6 Hz, 2H), 7.03-7.21 (m, 4H), 7.31-7.39 (m, 3H), 7.45 (t, *J =* 7.55 Hz, 1H), 7.89 (dd, *J'* = 2.1 Hz, *J"* = 9.15 Hz, 1H), 8.04 (d, *J =* 9.1 Hz, 1H), 8.37 (s, 1H), 8.45 (d, *J =* 1.85 Hz, 1H), 10.41 (s, 1H).

### Preparation 16: N-(2,3-Bis(2-fluorophenyl)quinolin-6-yl)-4-hydroxyhexanamide (Example 128)

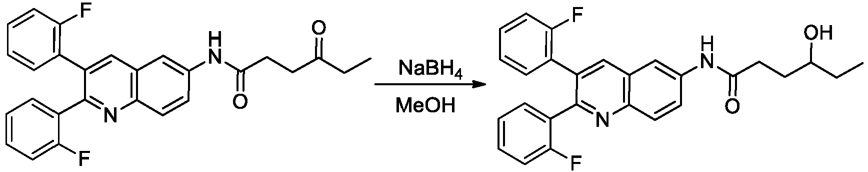

To a stirred solution of N-(2,3-bis(2-fluorophenyl)quinolin-6-yl)-4-oxohexanamide (Preparation 15) (50 mg, 0.113 mmol) in MeOH (2 mL) was added NaBH₄ (4.26 mg, 0.113 mmol) at 0-5 °C and the whole stirred at the same temperature for 1 h. After completion of the reaction (monitored by TLC and LCMS) the reaction mixture was quenched with water and extracted with EtOAc, washed with brine, dried over anhydrous Na₂SO₄ and evaporated in vacuo to give the crude product which was purified by prep-HPLC to afford the title compound (15 mg, yield 29.8%) as a white solid. LCMS m/z: 447.22 [M+H]; Purity 98.69%; ¹H NMR (500 MHz; DMSO-d₆): δ 0.89 (t, *J =* 7.35 Hz, 3H), 1.23 (s, 1H), 1.36-1.45 (m, 2H), 1.60-1.63 (m, 1H), 1.78-1.81 (m, 1H), 2.46-2.48 (m, 1H), 3.39 (d, *J =* 3.75 Hz, 1H), 4.49 (d, *J =* 5.3 Hz, 1H), 7.05 (t, *J* = 9.4 Hz, 1H), 7.10-7.21 (m, 3H), 7.31-7.37 (m, 3H), 7.45 (t, *J =* 7.25 Hz, 1H), 7.90 (t, *J =* 7.15 Hz, 1H), 7.92-7.05 (m, 1H), 8.38 (s, 1H), 8.49 (s, 1H), 10. 34 (s, 1H)

| **Ex** | **Structure** | **IUPAC Name** | **1H NMR** | **LCMS [M+H]** | **Purit y (%)** |
|---|---|---|---|---|---|
| 118 | 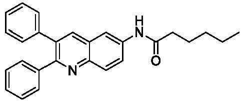 | N-(2,3-diphenylquin olin-6-yl)hexanamide | (500 MHz; DMSO-d₆): δ 0.90 (t, *J* = 6.77 Hz, 3H), 1.32-1.35 (m, 4H), 1.64-1.68 (m, 2H), 2.40 (t, *J =* 7.45 Hz, 2H), 7.26-7.37 (m, 10H), 7.84-7.87 (m, 1H), 8.01 (d, *J* = 9.0 Hz, 1H), 8.28 (s, 1H), 8.44 (d, *J* = 1.85 Hz, 1H), 10.27 (s, 1H). | 395.19 | 99.68 |
| 119 | 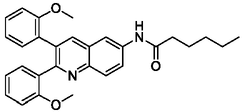 | N-(2,3-bis(2-methoxyphe nyl)quinolin-6-yl)hexanamide | (500 MHz; DMSO-d₆): δ 0.90 (t, *J* = 6.82 Hz, 3H), 1.32-1.35 (m, 4H), 1.63-1.68 (m, 2H), 2.39 (t, *J =* 7.45 Hz, 2H), 3.30 (s, 3H), 3.44 (bs, 3H), 6.74 (d, *J* =8.25 Hz, 1H), 6.85 (bs, 2H), 6.93 (t, *J* = 7.37 Hz, 1H), 7.11 (bs, 1H), 7.19-7.23 (m, 2H), 7.32 (dd, *J'* = 1.4 Hz, *J"* = 1.35 Hz, 1H), 7.82 (dd, *J'* = 2.15 Hz, *J"* = 2.1 Hz, 1H), 7.96 (d, *J =* 9.05 Hz, 1H), 8.08 (s, 1H), 8.38 (d, *J* = 1.7 Hz, 1H), 10.22 (s, 1H). | 455.23 | 98.32 |
| 120 | 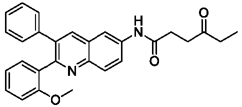 | N-(2-(2-methoxyphe nyl)-3-phenylquinol in-6-yl)-4-oxohexanamide | (500 MHz; DMSO-d₆): δ 0.96 (t, *J* = 7.3 Hz, 3H), 2.54 (s, 2H), 2.65 (t, *J* = 6.5 Hz, 2H), 2.78 (t, *J* = 6.55 Hz, 2H), 3.20 (s, 2H), 6.80 (d, *J* = 8.2 Hz, 1H), 7.02 (t, *J =* 7.4 Hz, 1H), 7.18-7.25 (m, 6H), 7.30-7.34 (m, 1H), 7.45 (dd, *J' =* 1.55 Hz, *J"* = 1.3 Hz, 1H), 7.82 (dd, *J'* = 2.1 Hz, *J"* = 1.95 Hz, 1H), 7.98 (d, *J* = 9Hz, 1H), 8.20 (s, 1H), 8.41 (d, *J =* 1.75 Hz, 1H ), 10.35 (s, 1H). | 439.23 | 72.11 |
| 121 | 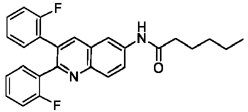 | N-(2,3-bis(2-fluorophenyl )quinolin-6-yl)hexanami de | (500 MHz; DMSO-d₆): δ 0.89 (s, 3H), 1.33 (s, 4H), 1.66 (t, *J* = 6.3 Hz, 2H), 2.41 (t, *J* = 7.4 Hz, 2H), 7.03-7.21 (m, 4H), 7.31-7.39 (m, 3H), 7.45 (t, *J* = 7.4 Hz, 1H), 7.90 (d, *J =* 9.05 Hz, 1H), 8.04 (d, *J* = 9.05 Hz, 1H), 8.38 (s, 1H), 8.49 (s, 1H), 10.32 (s, 1H). | 431.20 | 98.17 |
| 122 | 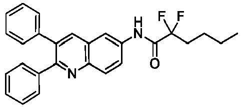 | N-(2,3-diphenylquin olin-6-yl)-2,2-difluorohexa namide | (500 MHz; DMSO-d₆): δ 0.91 (t, *J =* 7.2 Hz, 3H), 1.37-1.48 (m, 4H), 2.17-2.26 (m, 2H), 7.27-7.39 (m, 10H), 8.02-8.09 (m, 2H), 8.37 (s, 1H), 8.51 (d, *J* = 1.95 Hz, 1H), 10.92 (s, 1H). | 31.20 | 98.52 |
| 123 | 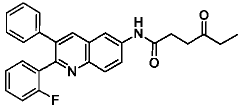 | N-(2-(2-fluorophenyl )-3-phenylquinol in-6-yl)-4-oxohexanamide | (500 MHz; DMSO-d₆): δ 0.96 (t, *J* = 7.3 Hz, 3H), 2.52 (s, 2H), 2.66 (t, *J* = 6.52 Hz, 2H), 2.79 (t, *J* = 6.52 Hz, 2H), 7.04 (t, *J* = 9.2 Hz, 1H), 7.24-7.29 (m, 6H), 7.37-7.42 (m, 1H), 7.53-7.56 (m, 1H), 7.84-7.87 (m, 1H), 8.02 (d, *J* = 9.05 Hz, 1H), 8.33 (s, 1H), 8.46 (d, *J* = 1.9 Hz, 1H), 10.39 (s, 1H). | 427.24 | 98.60 |
| 124 | 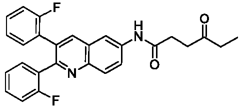 | N-(2,3-bis(2-fluorophenyl )quinolin-6-yl)-4-oxohexanamide | (500 MHz; DMSO-d₆): δ 0.96 (t, *J* =7.3 Hz, 3H) 2.52 (t, *J* = 7.35 Hz, 2H), 2.66 (t, *J =* 6.65 Hz, 2H), 2.79 (t, *J =* 6.6 Hz, 2H), 7.03-7.21 (m, 4H), 7.31-7.39 (m, 3H), 7.45 (t, *J* = 7.55 Hz, 1H), 7.89 (dd, *J' =* 2.1 Hz, *J" =* 9.15 Hz, 1H), 8.04 (d, *J =* 9.1 Hz, 1H), 8.37 (s, 1H), 8.45 (d, *J* = 1.85 Hz, 1H), 10.41 (s, 1H). | 445.20 | 98.04 |
| 125 | 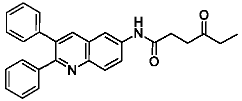 | N-(2,3-diphenylquin olin-6-yl)-4-oxohexanamide | (500 MHz; DMSO-d₆): δ 0.96 (t, *J* = 7.3 Hz, 3H), 2.54 (s, 2H), 2.65 (t, *J* = 6.55 Hz, 2H), 2.79 (t, *J* = 6.52 Hz, 2H), 7.26-7.37 (m, 10H), 7.83-7.85 (m, 1H), 8.02 (d, *J* = 9.05 Hz, 1H), 8.28 (s, 1H), 8.42 (d, *J* = 1.85 Hz, 1H), 10.37 (s, 1H). | 409.23 | 91.99 |
| 126 | 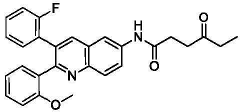 | N-(3-(2-fluorophenyl )-2-(2-methoxyphe nyl)quinolin-6-yl)-4-oxohexanami de | (500 MHz; DMSO-d₆): δ 0.96 (t, *J* = 7.27 Hz, 3H), 2.52 (s, 2H), 2.65 (t, *J* = 6.47 Hz, 2H), 2.79 (t, *J* = 6.5 Hz, 2H), 3.38 (bs, 3H), 6.77 (d, *J* = 8.35 Hz, 1H) 6.98-7.15 (m, 4H), 7.27-7.30 (m, 2H), 7.43 (d, *J* = 7.3 Hz, 1H), 7.86 (d, *J* = 9.15 Hz, 1H), 8.01 (d, *J =* 9.05 Hz, 1H), 8.26 (s, 1H), 8.41 (s, 1H), 10.38 (s, 1H). | 457.18 | 84.89 |
| 127 | 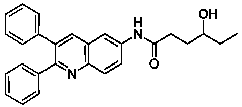 | N-(2,3-diphenylquin olin-6-yl)-4-hydroxyhexa namide | (500 MHz; DMSO-d₆): δ 0.89 (t, *J* = 7.4 Hz, 3H), 1.40-1.45 (m, 2H), 1.59-1.63 (m, 1H), 1.78-1.80 (m, 1H), 2.46-2.48 (m, 2H), 3.40 (d, *J* = 3.8 Hz, 1H), 4.49 (d, *J* = 5.35 Hz, 1H), 7.26-7.37 (m, 10H), 7.85-7.87 (m, 1H), 8.01 (d, *J* = 9.05 Hz, 1H), 8.28 (s, 1H), 8.45 (d, *J* = 1.6 Hz, 1H), 10.29 (s, 1H). | 411.20 | 98.15 |
| 128 | 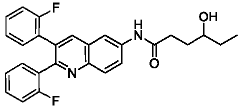 | N-(2,3-bis(2-fluorophenyl )quinolin-6-yl)-4-hydroxyhexa namide | (500 MHz; DMSO-d₆): δ 0.89 (t, *J* = 7.35 Hz, 3H), 1.23 (s, 1H), 1.36-1.45 (m, 2H), 1.60-1.63 (m, 1H), 1.78-1.81 (m, 1H), 2.46-2.48 (m, 1H), 3.39 (d, *J* = 3.75 Hz, 1H), 4.49 (d, *J =* 5.3 Hz, 1H), 7.05 (t, *J =* 9.4 Hz, 1H), 7.10-7.21 (m, 3H), 7.31-7.37 (m, 3H), 7.45 (t, *J* = 7.25 Hz, 1H), 7.90 (t, *J =* 7.15 Hz, 1H), 7.92-7.05 (m, 1H), 8.38 (s, 1H), 8.49 (s, 1H), 10. 34 (s, 1H) | 447.22 | 98.69 |
| 129 | 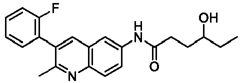 | N-(3-(2-fluorophenyl )-2-methylquinol in-6-yl)-4-hydroxyhexa namide | (500 MHz; DMSO-d₆): δ 0.88 (t, *J* = 6.17 Hz, 3H), 1.34-1.42 (m, 2H), 1.60 (bs, 1H), 1.78 (bs, 1H), 2.45 (s, 6H), 4.48 (bs, 1H), 7.37-7.40 (m, 2H), 7.47-7.54 (m, 2H), 7.81-7.93 (m, 2H), 8.14 (d, J = 21.5 Hz, 1H), 8.34 (s, 1H), 10.22 (s, 1H). | 367.28 | 98.05 |
| 130 | 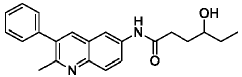 | 4-hydroxy-N-(2-methyl-3-phenylquinol in-6-yl)hexanamide | (500 MHz; DMSO-d₆): δ 1.41 (t, *J =* 6.3 Hz, 3H), 1.56-1.62 (m, 2H), 1.75-1.79 (m, 2H), 2.41-2.47 (m, 2H), 2.62 (s, 3H), 3.38-3.40 (m, 1H), 4.42 (bs, 1H), 7.48-7.53 (m, 5H), 7.98-8.02 (m, 2H), 8.30 (s, 1H), 8.43 (s, 1H), 10.29 (s, 1H). | 349.30 | 99.85 |

### Biological assays

### ACSS2 enzyme assay

Novel compounds were screened in a human ACSS2 enzyme assay. Briefly, the ACSS2 enzyme reaction was monitored by directly measuring acetyl-CoA formation. The cytosolic fraction of BT474 cells was used as a source of the ACSS2 enzyme. A short protocol for this assay is provided below.

### Preparation of cytosolic fraction from BT474 cells

9 x 10⁶ cells were harvested by trypsinization followed by centrifugation. The cell pellet was washed with PBS and dissolved in 500µl STM buffer (50mM Tris-HCl pH7.4, 250mM sucrose, 5mM MgCl₂ & protease-phosphatase inhibitor cocktail) and homogenized at 600-1000 rpm for 1 min. on ice followed by incubation for 30 min. with intermittent vortexing. Supernatant was collected by centrifugation at 800g for 15 min. at 4 °C. Cytosolic fraction was prepared by further centrifugation of the supernatant at 12000g for 15 min. at 4 °C. Total protein content of the cytosolic fraction was estimated & diluted to 1 mg/ml concentration in storage buffer (25mM Tris-HCl pH7.4, 1mM MgCl₂, 1mM DTT & 10% glycerol) and stored at -80 °C for further use.

### Assay protocol

100ng of cytosolic fraction in buffer composed of 25mM Tris-HCl pH7.4 1mM MgCl₂, 1mM DTT & 0.05% Tween20 was preincubated with desired concentrations of novel compounds for 5 min. maintaining 1% DMSO. Reaction was started with addition of substrate mix (100µM of acetate, CoA and ATP) and incubated for 2 h at 25 °C with constant shaking at 500 rpm. Post incubation the reaction was terminated by addition of 10% TCA followed by 5 fold dilution in water. The reaction mixture was centrifuged at 800g for 10 min. at RT and acetyl-CoA detected in supernatant by LC-MS/MS (QTrap5500 ABSCIEX).

### Cell based [¹⁴C] acetate incorporation assay

In the cellular assay, ACSS2 inhibitors were screened against [¹⁴C] acetate incorporation in macromolecules. 4 x 10⁴ BT474 cells were plated in 96 well plates in growth media composed of DMEM & 10% FBS and incubated overnight in a 37 °C, 5% CO₂ incubator. The following day the cells were washed in HBSS and the assay was performed in 100 µl of HBSS. 50 µl of 4x compound in 4% DMSO was added to the cells and preincubated for 10min. at 37 °C. 50 µl of acetate solution (0.1 µCi ¹⁴C acetate + 10 µM ¹²C acetate) was added to each well of the plate and incubated for 3 h in a 37 °C, 5% CO₂ incubator. Following incubation, cells were harvested with water on polyethylene imine (PEI) presoaked GF/C filtermat, air dried and radioactive count were recorded in Trilux micro beta Liquid Scintillation Counter (PerkinElmer).

In the tables below, IC₅₀ value ranges for exemplary compounds are given. The IC₅₀ ranges are indicated as "A" for values less than 100 nM, "B" for values less than or equal to 1 µM, "C" for values less than or equal to 10 µM, and "D" for values greater than 10 µM.

### ACSS2 activity

| **Ex** | **Enzyme Activity** | **Cellular Activity** | **Ex** | **Enzyme Activity** | **Cellular Activity** |
|---|---|---|---|---|---|
| 1 | A | A | 75 | A | A |
| 2 | A | A | 76 | B | A |
| 3 | A | A | 77 | A | B |
| 4 | A | A | 78 | B | B |
| 5 | A | A | 79 | A | B |
| 6 | A | A | 80 | A | A |
| 7 | A | A | 81 | A | A |
| 8 | A | A | 82 | B | D |
| 9 | D | C | 83 | A | A |
| 10 | A | A | 84 | A | A |
| 11 | B | C | 85 | A | A |
| 12 | A | A | 86 | A | A |
| 13 | B | B | 87 | A | A |
| 14 | A | A | 88 | C | D |
| 15 | A | A | 89 | B | B |
| 16 | A | A | 90 | B | B |
| 17 | A | A | 91 | A | A |
| 18 | A | A | 92 | A | A |
| 19 | A | C | 93 | A | B |
| 20 | A | A | 94 | A | A |
| 21 | C | C | 95 | A | A |
| 22 | C | D | 96 | A | A |
| 23 | A | A | 97 | A | A |
| 24 | B | C | 98 | B | A |
| 25 | B | B | 99 | B | B |
| 26 | A | A | 100 | A | A |
| 27 | B | C | 101 | A | A |
| 28 | A | A | 102 | A | A |
| 29 | D | D | 103 | A | A |
| 30 | A | A | 104 | A | A |
| 31 | A | A | 105 | A | A |
| 32 | B | B | 106 | A | A |
| 33 | A | A | 107 | A | A |
| 34 | A | A | 108 | A | A |
| 35 | A | A | 109 | A | B |
| 36 | A | A | 110 | A | A |
| 37 | A | A | 111 | A | B |
| 38 | A | B | 112 | A | A |
| 39 | D | C | 113 | A | A |
| 40 | B | C | 114 | A | B |
| 41 | A | A | 115 | A | B |
| 42 | D | D | 116 | B | A |
| 43 | A | B | 117 | A | A |
| 44 | A | A | 118 | B | B |
| 45 | A | A | 119 | A | A |
| 46 | A | A | 120 | A | A |
| 47 | A | A | 121 | A | B |
| 48 | A | A | 122 | C | D |
| 49 | A | A | 123 | A | A |
| 50 | C | D | 124 | A | A |
| 51 | A | A | 125 | A | A |
| 52 | A | A | 126 | A | A |
| 53 | A | B | 127 | A | A |
| 54 | A | A | 128 | A | A |
| 55 | C | D | 129 | A | A |
| 56 | C | C | 130 | A | B |
| 57 | A | A | 131 | B | B |
| 58 | A | A | 132 | C | D |
| 59 | A | A | 133 | C | D |
| 60 | A | A | 134 | B | B |
| 61 | A | A | 135 | B | B |
| 62 | A | A | 136 | B | B |
| 63 | A | A | 137 | B | B |
| 64 | A | A | 138 | A | A |
| 65 | A | C | 139 | D | D |
| 66 | A | B | 140 | D | D |
| 67 | A | A | 141 | C | D |
| 68 | A | A | 142 | D | D |
| 69 | C | D | 143 | D | C |
| 70 | A | A | 144 | B | A |
| 71 | B | D | 145 | B | B |
| 72 | A | A | | | |
| 73 | A | B | | | |
| 74 | C | D | | | |

## Claims

1. A compound of formula (I): 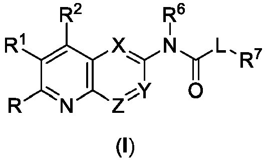
, wherein X is CR³;
Y is CR⁴;
Z is CR⁵ or N;
R is selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl;
R¹ is selected from the group consisting of mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl, wherein when R¹ is an optionally substituted alkyl, an optionally substituted alkenyl or an optionally substituted alkynyl, the alkyl, alkenyl or alkynyl is unsubstituted or substituted with one or more of a halogen, an optionally substituted C₆-C₁₂ aryl or an optionally substituted 5 to 10 membered heteroaryl;
R² is H, halogen, COOR⁹, CN, CONR⁹R¹⁰, NR⁹R¹⁰, NR⁹SO₂R¹⁰, SO₂NR⁹R¹⁰, NR⁹COR¹⁰, optionally substituted C₁-C₁₀ alkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R³ is H, halogen, optionally substituted C₁-C₁₀ alkyl, optionally substituted mono or bicyclic C₃-C₆ cycloalkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R⁴ and R⁵ are each independently selected from the group consisting of H and halogen;
R⁶ is H;
L is NR⁸ and R⁷ is optionally substituted C₃-C₁₀ alkyl, optionally substituted C₂-C₁₀ alkenyl, optionally substituted C₂-C₁₀ alkynyl, mono or bicyclic optionally substituted C₆-C₁₂ aryl, mono or bicyclic optionally substituted 5 to 10 membered heteroaryl, optionally substituted mono or bicyclic C₃-C₆ cycloalkyl, or optionally substituted mono or bicyclic 3 to 8 membered heterocycle; or L is absent and R⁷ is optionally substituted C₃-C₁₀ alkyl or optionally substituted mono or bicyclic 3 to 8 membered heterocycle, wherein when L is absent and R⁷ is optionally substituted C₃-C₁₀ alkyl, the alkyl, is unsubstituted or substituted with one or more of OH, oxo or halogen;
R⁸ is H; and
R⁹ and R¹⁰ are each independently selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, optionally substituted C₂-C₁₀ alkynyl, optionally substituted C₁-C₁₀ alkoxy and NH₂;
or a pharmaceutically acceptable complex, salt, solvate, tautomeric form or polymorphic form thereof,
wherein, unless otherwise specified:
an optionally substituted alkyl is unsubstituted or substituted with one or more of halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted alkenyl is unsubstituted or substituted with one or more of optionally substituted C₁-C₆ alkyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted alkynyl group is unsubstituted or substituted with one or more of optionally substituted C₁-C₆ alkyl, optionally substituted C₂-C₆ alkenyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted alkoxy group is unsubstituted or substituted with one or more of halogen, OH, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted aryloxy, optionally substituted heteroaryloxy, optionally substituted C₆-C₁₂ aryl, optionally substituted 5 to 10 membered heteroaryl, C₃-C₆ cycloalkyl and 3 to 8 membered heterocycle;
an optionally substituted aryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted aryloxy is the group aryl-O- where aryl is an optionally substituted C₆-C₁₂ aryl group;
an optionally substituted cycloalkyl or cycloalkenyl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted heteroaryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted heterocycle is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle; and wherein the compound is not:

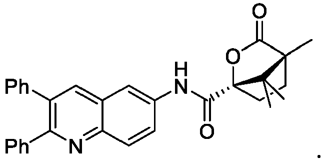

2. The compound according to claim 1, wherein R and R¹ are each independently be selected from the group consisting of mono or bicyclic optionally substituted C₆-C₁₂ aryl, mono or bicyclic optionally substituted 5 to 10 membered heteroaryl, optionally substituted C₁-C₆ alkyl, optionally substituted mono or bicyclic C₃-C₆ cycloalkyl, optionally substituted mono or bicyclic C₃-C₆ cycloalkenyl, optionally substituted C₂-C₆ alkenyl and optionally substituted C₂-C₆ alkynyl.

3. The compound according to claim 2, wherein R and R¹ are each independently selected from an optionally substituted phenyl ring, an optionally substituted C₁-C₆ alkyl, an optionally substituted C₂-C₆ alkenyl, an optionally substituted C₂-C₆ alkynyl or an optionally substituted 5 or 6 membered heteroaryl.

4. The compound according to any preceding claim, wherein R² is H, COOR⁹, CONR⁹R¹⁰, CN, NR⁹COR¹⁰, NR⁹R¹⁰, NR⁹SO₂R¹⁰, optionally substituted C₁-C₆ alkyl, optionally substituted C₁-C₆ alkoxy, mono or bicyclic optionally substituted C₆-C₁₂ aryl or a mono or bicyclic optionally substituted 5 to 10 membered heteroaryl.

5. The compound according to any preceding claim, wherein R³ is H, halogen, optionally substituted C₁-C₆ alkyl, optionally substituted mono or bicyclic C₃-C₆ cycloalkyl, optionally substituted C₁-C₆ alkoxy, mono or bicyclic optionally substituted C₆-C₁₂ aryl, or mono or bicyclic optionally substituted 5 to 10 membered heteroaryl.

6. The compound according to any preceding claim, wherein R⁴ and R⁵ are each independently selected from the group consisting of H, halogen, OH, CN, mono or bicyclic optionally substituted C₆-C₁₂ aryl, optionally substituted C₁-C₆ alkyl, optionally substituted C₁-C₆ alkoxy or optionally substituted mono or bicyclic C₃-C₆ cycloalkyl.

7. The compound according to claim 6, wherein R⁴ and R⁵ are H, halogen, OH, CN or an optionally substituted C₁-C₆ alkyl.

8. The compound according to claim 7, wherein R⁴ and R⁵ are H.

9. The compound according to any preceding claim, wherein L is NR⁸ and R⁷ is optionally substituted C₃-C₆ alkyl, optionally substituted C₂-C₆ alkenyl, optionally substituted C₂-C₆ alkynyl, mono or bicyclic optionally substituted C₆-C₁₂ aryl, optionally substituted mono or bicyclic C₃-C₆ cycloalkyl, or optionally substituted mono or bicyclic 3 to 8 membered heterocycle.

10. The compound according to claim 1, wherein the compound is:
1-(2,3-diphenylquinolin-6-yl)-3-(2-hydroxybutyl)urea;
(R)-1-(2,3-diphenylquinolin-6-yl)-3-(2-hydroxybutyl)urea;
1-butyl-3-(2,3-diphenylquinolin-6-yl)urea;
1-(2,3-bis(2-fluorophenyl)quinolin-6-yl)-3-(2-hydroxybutyl)urea;
1-butyl-3-(5-(2-hydroxypyridin-3-yl)-2,3-diphenylquinolin-6-yl)urea;
1-(2,3-bis(2-methoxyphenyl)quinolin-6-yl)-3-(2-hydroxybutyl)urea;
1-(3-(2-fluorophenyl)-2-phenylquinolin-6-yl)-3-(2-hydroxybutyl)urea;
1-(2-(2-fluorophenyl)-3-phenylquinolin-6-yl)-3-(2-hydroxybutyl)urea;
1-(2,3-bis(2-methoxypyridin-4-yl)quinolin-6-yl)-3-(2-hydroxybutyl)urea;
1-(2-hydroxybutyl)-3-(3-(2-methoxyphenyl)-2-phenylquinolin-6-yl)urea;
1-(2,3-bis(6-methoxypyridin-3-yl)quinolin-6-yl)-3-(2-hydroxybutyl)urea;
1-(2-hydroxybutyl)-3-(2-(2-methoxyphenyl)-3-phenylquinolin-6-yl)urea;
1-(2,3-bis(2-methoxypyridin-3-yl)quinolin-6-yl)-3-(2-hydroxybutyl)urea;
1-(2,3-bis(2-fluorophenyl)quinolin-6-yl)-3-butylurea;
(R)-1-(3-(2-fluorophenyl)-2-phenylquinolin-6-yl)-3-(2-hydroxybutyl)urea;
1-butyl-3-(3-(2-fluorophenyl)-2-phenylquinolin-6-yl)urea;
1-(2,3-bis(4-fluorophenyl)quinolin-6-yl)-3-(2-hydroxybutyl)urea;
1-(3-(2-fluorophenyl)-2-(2-methoxyphenyl)quinolin-6-yl)-3-(2-hydroxybutyl)urea;
1-(2,3-di(pyridin-3-yl)quinolin-6-yl)-3-(2-hydroxybutyl)urea;
(R)-1-(2,3-bis(2-fluorophenyl)quinolin-6-yl)-3-(2-hydroxybutyl)urea;
1-(2,3-bis(3-acetylphenyl)quinolin-6-yl)-3-(2-hydroxybutyl)urea;
1-(2,3-di(1H-pyrazol-4-yl)quinolin-6-yl)-3-(2-hydroxybutyl)urea;
1-butyl-3-(3-(2-methoxyphenyl)-2-phenylquinolin-6-yl)urea;
1-(2,3-di(pyridin-4-yl)quinolin-6-yl)-3-(2-hydroxybutyl)urea;
1-(2,3-bis(3-fluorophenyl)quinolin-6-yl)-3-(2-hydroxybutyl)urea;
1-(2-(2-fluorophenyl)-3-(2-methoxyphenyl)quinolin-6-yl)-3-(2-hydroxybutyl)urea;
1-(2,3-bis(6-oxo-1,6-dihydropyridin-3-yl)quinolin-6-yl)-3-(2-hydroxybutyl)urea;
(S)-1-(2,3-bis(2-fluorophenyl)quinolin-6-yl)-3-(2-hydroxybutyl)urea;
1-(2,3-bis(2-hydroxypyridin-4-yl)quinolin-6-yl)-3-(2-hydroxybutyl)urea;
(S)-1-(2,3-diphenylquinolin-6-yl)-3-(2-hydroxybutyl)urea;
1-(3-(2-fluorophenyl)-2-(pyridin-3-yl)quinolin-6-yl)-3-(2-hydroxybutyl)urea;
1-(2-(cyclohex-1-en-1-yl)-3-(2-fluorophenyl)quinolin-6-yl)-3-(2-hydroxybutyl)urea;
1-(2-(2-(dimethylamino)phenyl)-3-(2-fluorophenyl)quinolin-6-yl)-3-(2-hydroxybutyl)urea;
1-(3-(2-fluorophenyl)-2-(pyridin-4-yl)quinolin-6-yl)-3-(2-hydroxybutyl)urea;
(R)-1-(3-(2-fluorophenyl)-2-(2-methoxyphenyl)quinolin-6-yl)-3-(2-hydroxybutyl)urea;
(S)-1-(3-(2-fluorophenyl)-2-(2-methoxyphenyl)quinolin-6-yl)-3-(2-hydroxybutyl)urea;
1-butyl-3-(3-(2-fluorophenyl)-2-(2-methoxyphenyl)quinolin-6-yl)urea;
1-(2-hydroxybutyl)-3-(3-phenyl-2-(pyridin-2-yl)quinolin-6-yl)urea;
1-(2-cyclohexyl-3-(2-fluorophenyl)quinolin-6-yl)-3-(2-hydroxybutyl)urea;
1-(2-cyclopropyl-3-phenylquinolin-6-yl)-3-(2-hydroxybutyl)urea;
1-(2-hydroxybutyl)-3-(3-phenyl-2-(pyridin-3-yl)quinolin-6-yl)urea;
1-(2,3-bis(2-hydroxypyridin-3-yl)quinolin-6-yl)-3-(2-hydroxybutyl)urea;
1-(2-hydroxybutyl)-3-(2-(1-methyl-1H-pyrazol-4-yl)-3-phenylquinolin-6-yl)urea;
1-(3-(2-fluorophenyl)-2-phenylquinolin-6-yl)-3-(2-methoxybutyl)urea;
1-(2,2-difluorobutyl)-3-(3-(2-fluorophenyl)-2-phenylquinolin-6-yl)urea;
1-(3-(2-fluorophenyl)-2-(3-fluorophenyl)quinolin-6-yl)-3-(2-hydroxybutyl)urea;
1-(3-(2-fluorophenyl)-2-(4-fluorophenyl)quinolin-6-yl)-3-(2-hydroxybutyl)urea;
methyl 6-(3-(2-hydroxybutyl)ureido)-2,3-diphenylquinoline-4-carboxylate;
6-(3-(2-hydroxybutyl)ureido)-2,3-diphenylquinoline-4-carboxamide;
(E)-1-(2-hydroxybutyl)-3-(3-phenyl-2-styrylquinolin-6-yl)urea;
N-(2-(3-(2-fluorophenyl)-6-(3-(2-hydroxybutyl)ureido)quinolin-2-yl)phenyl)acetamide;
1-(2-hydroxybutyl)-3-(2-methyl-3-phenylquinolin-6-yl)urea;
1-(2-(2-cyanophenyl)-3-(2-fluorophenyl)quinolin-6-yl)-3-(2-hydroxybutyl)urea;
1-(2-(2-aminophenyl)-3-(2-fluorophenyl)quinolin-6-yl)-3-(2-hydroxybutyl)urea;
1-(2-([1,1'-biphenyl]-4-yl)-3-phenylquinolin-6-yl)-3-(2-hydroxybutyl)urea;
1-(2-hydroxybutyl)-3-(2-phenethyl-3-phenylquinolin-6-yl)urea;
(R)-6-(3-(2-hydroxybutyl)ureido)-2,3-diphenylquinoline-4-carboxamide;
1-(2-(2-ethoxyphenyl)-3-phenylquinolin-6-yl)-3-(2-hydroxybutyl)urea;
1-(2-hydroxybutyl)-3-(2-(2-isobutoxyphenyl)-3-phenylquinolin-6-yl)urea;
1-(2-ethynyl-3-phenylquinolin-6-yl)-3-(2-hydroxybutyl)urea;
1-(2-ethyl-3-phenylquinolin-6-yl)-3-(2-hydroxybutyl)urea;
6-(3-(2-hydroxybutyl)ureido)-N-methyl-2,3-diphenylquinoline-4-carboxamide;
6-(3-(2-hydroxybutyl)ureido)-N,N-dimethyl-2,3-diphenylquinoline-4-carboxamide;
N-cyclopropyl-6-(3-(2-hydroxybutyl)ureido)-2,3-diphenylquinoline-4-carboxamide;
6-(3-(2-hydroxybutyl)ureido)-2,3-diphenylquinoline-4-carboxylic acid;
6-(3-(2-hydroxybutyl)ureido)-2-methyl-3-phenylquinoline-4-carboxamide;
(S)-1-(3-(2-fluorophenyl)-2-phenylquinolin-6-yl)-3-(2-hydroxybutyl)urea;
(S)-1-(2,3-diphenylquinolin-6-yl)-3-(2-hydroxybutyl)urea;
6-(3-(2-hydroxybutyl)ureido)-2,3-dimethylquinoline-4-carboxamide;
1-(4-cyano-2,3-diphenylquinolin-6-yl)-3-(2-hydroxybutyl)urea;
6-(3-(2-hydroxybutyl)ureido)-3-phenylquinoline-4-carboxamide;
(R)-1-(2-hydroxybutyl)-3-(2-methyl-3-phenylquinolin-6-yl)urea;
3-(2-fluorophenyl)-6-(3-(2-hydroxybutyl)ureido)-2-methylquinoline-4-carboxamide;
1-(3-(2-fluorophenyl)-2-methylquinolin-6-yl)-3-(2-hydroxybutyl)urea;
1-butyl-3-(2-methyl-3-phenylquinolin-6-yl)urea;
1-(2-methoxybutyl)-3-(2-methyl-3-phenylquinolin-6-yl)urea;
1-(2-methoxyethyl)-3-(2-methyl-3-phenylquinolin-6-yl)urea;
1-(2-hydroxybutyl)-3-(2-methyl-3-(pyridin-3-yl)quinolin-6-yl)urea;
1-(2-hydroxybutyl)-3-(2-methyl-3-(o-tolyl)quinolin-6-yl)urea;
1-(3-(2-chlorophenyl)-2-methylquinolin-6-yl)-3-(2-hydroxybutyl)urea;
1-(2-hydroxybutyl)-3-(2-methyl-3-(1-methyl-1H-pyrazol-4-yl)quinolin-6-yl)urea;
(R)-1-(2-hydroxybutyl)-3-(2-methyl-3-(o-tolyl)quinolin-6-yl)urea;
N-(6-(3-(2-hydroxybutyl)ureido)-2,3-diphenylquinolin-4-yl)acetamide;
1-(2,5-dimethyl-3-phenylquinolin-6-yl)-3-(2-hydroxybutyl)urea;
1-(5-cyclopropyl-2-methyl-3-phenylquinolin-6-yl)-3-(2-hydroxybutyl)urea;
1-(5-bromo-2-methyl-3-phenylquinolin-6-yl)-3-(2-hydroxybutyl)urea;
1-(2-hydroxybutyl)-3-(3-phenyl-2-(trifluoromethyl)quinolin-6-yl)urea;
(S)-1-(2-hydroxybutyl)-3-(2-methyl-3-phenylquinolin-6-yl)urea;
(R)-1-(7-fluoro-2,3-diphenylquinolin-6-yl)-3-(2-hydroxybutyl)urea;
1-(4-(dimethylamino)-2,3-diphenylquinolin-6-yl)-3-(2-hydroxybutyl)urea;
(R)-N-(6-(3-(2-hydroxybutyl)ureido)-2,3-diphenylquinolin-4-yl)cyclopropanecarboxamide;
N-(6-(3-(2-hydroxybutyl)ureido)-2-methyl-3-phenylquinolin-4-yl)acetamide;
N-(6-(3-(2-hydroxybutyl)ureido)-2,3-diphenylquinolin-4-yl)methanesulfonamide;
1-(4-amino-2,3-diphenylquinolin-6-yl)-3-(2-hydroxybutyl)urea;
(R)-1-(2-hydroxybutyl)-3-(2-methyl-5-(1-methyl-1H-pyrazol-4-yl)-3-phenylquinolin-6-yl)urea;
1-(2-hydroxybutyl)-3-(4-(oxazol-2-yl)-2,3-diphenylquinolin-6-yl)urea;
1-(4-cyano-2-methyl-3-phenylquinolin-6-yl)-3-(2-hydroxybutyl)urea;
N-(1-(3-(2-methyl-3-phenylquinolin-6-yl)ureido)butan-2-yl)acetamide;
(R)-1-(3-(2-fluorophenyl)-2-methylquinolin-6-yl)-3-(2-hydroxybutyl)urea;
(R)-1-(2-hydroxybutyl)-3-(4-methoxy-2,3-diphenylquinolin-6-yl)urea;
(R)-1-(2-hydroxybutyl)-3-(4-methoxy-2-methyl-3-phenylquinolin-6-yl)urea;
(R)-1-(2-hydroxybutyl)-3-(4-methyl-2,3-diphenylquinolin-6-yl)urea;
(R)-N-benzyl-6-(3-(2-hydroxybutyl)ureido)-3-methyl-2-phenylquinoline-4-carboxamide;
1-(2,2-difluorobutyl)-3-(2-methyl-3-phenylquinolin-6-yl)urea;
6-(3-(2,2-difluorobutyl)ureido)-2,3-diphenylquinoline-4-carboxamide;
1-(2,2-difluorobutyl)-3-(2,3-diphenylquinolin-6-yl)urea;
(R)-6-(3-(2-hydroxybutyl)ureido)-N,3-dimethyl-2-phenylquinoline-4-carboxamide;
(R)-6-(3-(2-hydroxybutyl)ureido)-3-methyl-2-phenylquinoline-4-carboxamide;
1-(2,2-difluorobutyl)-3-(3-(2-fluorophenyl)-2-methylquinolin-6-yl)urea;
(R)-1-(3-(2-fluorophenyl)-2-methyl-5-(2-oxo-1,2-dihydropyridin-3-yl)quinolin-6-yl)-3-(2-hydroxybutyl)urea;
(R)-6-(3-(2-hydroxybutyl)ureido)-N-(1-methyl-1H-pyrazol-3-yl)-2,3-diphenylquinoline-4-carboxamide;
(R)-1-(2,4-dimethyl-3-phenylquinolin-6-yl)-3-(2-hydroxybutyl)urea;
(R)-6-(3-(2-hydroxybutyl)ureido)-3-methyl-N-((1-methyl-1H-pyrazol-4-yl)methyl)-2-phenylquinoline-4-carboxamide;
(R)-6-(3-(2-hydroxybutyl)ureido)-3-methyl-N-(1-methyl-1H-pyrazol-3-yl)-2-phenylquinoline-4-carboxamide;
1-(3-(2-fluorophenyl)-2-methylquinolin-6-yl)-3-(2-methoxyethyl)urea;
(R)-1-(3-(3-(2-fluorophenyl)-2-phenylquinolin-6-yl)ureido)butan-2-yl dihydrogen phosphate;
N-(2,3-diphenylquinolin-6-yl)hexanamide;
N-(2,3-bis(2-methoxyphenyl)quinolin-6-yl)hexanamide;
N-(2-(2-methoxyphenyl)-3-phenylquinolin-6-yl)-4-oxohexanamide;
N-(2,3-bis(2-fluorophenyl)quinolin-6-yl)hexanamide;
N-(2,3-diphenylquinolin-6-yl)-2,2-difluorohexanamide;
N-(2-(2-fluorophenyl)-3-phenylquinolin-6-yl)-4-oxohexanamide;
N-(2,3-bis(2-fluorophenyl)quinolin-6-yl)-4-oxohexanamide;
N-(2,3-diphenylquinolin-6-yl)-4-oxohexanamide;
N-(3-(2-fluorophenyl)-2-(2-methoxyphenyl)quinolin-6-yl)-4-oxohexanamide;
N-(2,3-diphenylquinolin-6-yl)-4-hydroxyhexanamide;
N-(2,3-bis(2-fluorophenyl)quinolin-6-yl)-4-hydroxyhexanamide;
N-(3-(2-fluorophenyl)-2-methylquinolin-6-yl)-4-hydroxyhexanamide;
4-hydroxy-N-(2-methyl-3-phenylquinolin-6-yl)hexanamide;
1-cyclohexyl-3-(2,3-diphenylquinolin-6-yl)urea;
N-(2,3-diphenylquinolin-6-yl)-4-methylpiperazine-1-carboxamide;
4-acetyl-N-(2,3-diphenylquinolin-6-yl)piperazine-1-carboxamide;
1-allyl-3-(2,3-diphenylquinolin-6-yl)urea;
1-(2,3-diphenylquinolin-6-yl)-3-(prop-2-yn-1-yl)urea;
1-(2,3-diphenylquinolin-6-yl)-3-(2-hydroxycyclopentyl)urea;
1-(2,3-diphenylquinolin-6-yl)-3-(tetrahydro-2H-pyran-4-yl)urea;
N-(2,3-diphenylquinolin-6-yl)-3-methoxypyrrolidine-1-carboxamide;
1-(2,3-diphenylquinolin-6-yl)-3-(pyridin-2-yl)urea;
1-(2,3-diphenylquinolin-6-yl)-3-phenylurea;
1-(2,3-diphenylquinolin-6-yl)-3-(1-methylpyrrolidin-3-yl)urea;
1-(2,3-diphenylquinolin-6-yl)-3-(pyridin-4-yl)urea;
1-(2,3-diphenylquinolin-6-yl)-3-(1-(hydroxymethyl)cyclopentyl)urea;
1-(2,3-diphenylquinolin-6-yl)-3-(1-methyl-1H-pyrazol-4-yl)urea;
1-(6,7-diphenyl-1,8-naphthyridin-3-yl)-3-(2-hydroxybutyl)urea;
(R)-1-(6,7-diphenyl-1,8-naphthyridin-3-yl)-3-(2-hydroxybutyl)urea; or
N-(6,7-diphenyl-1,8-naphthyridin-3-yl)-3-methoxypyrrolidine-1-carboxamide.

11. A pharmaceutical composition comprising a compound according to any one of claims 1 to 10, or a pharmaceutically acceptable salt, solvate, tautomeric form or polymorphic form thereof, and a pharmaceutically acceptable vehicle.

12. A compound according to any one of claims 1 to 10, or a pharmaceutically acceptable salt, solvate, tautomeric form or polymorphic form thereof, or a pharmaceutical composition according to claim 11, for use in therapy.

13. A compound of formula (I): 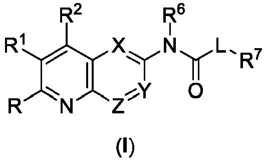
, wherein X is CR³;
Y is CR⁴;
Z is CR⁵ or N;R is selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl;
R¹ is selected from the group consisting of mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, and optionally substituted C₂-C₁₀ alkynyl, wherein when R¹ is an optionally substituted alkyl, an optionally substituted alkenyl or an optionally substituted alkynyl, the alkyl, alkenyl or alkynyl is unsubstituted or substituted with one or more of a halogen, an optionally substituted C₆-C₁₂ aryl or an optionally substituted 5 to 10 membered heteroaryl;
R² is H, halogen, COOR⁹, CN, CONR⁹R¹⁰, NR⁹R¹⁰, NR⁹SO₂R¹⁰, SO₂NR⁹R¹⁰, NR⁹COR¹⁰, optionally substituted C₁-C₁₀ alkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R³ is H, halogen, optionally substituted C₁-C₁₀ alkyl, optionally substituted mono or bicyclic C₃-C₆ cycloalkyl, optionally substituted mono or bicyclic 3 to 8 membered heterocycle, optionally substituted C₁-C₁₀ alkoxy, mono or bicyclic optionally substituted C₆-C₁₂ aryl or mono or bicyclic optionally substituted 5 to 10 membered heteroaryl;
R⁴ and R⁵ are each independently selected from the group consisting of H and halogen; R⁶ is H;
L is NR⁸ and R⁷ is optionally substituted C₃-C₁₀ alkyl, optionally substituted C₂-C₁₀ alkenyl, optionally substituted C₂-C₁₀ alkynyl, mono or bicyclic optionally substituted C₆-C₁₂ aryl, mono or bicyclic optionally substituted 5 to 10 membered heteroaryl, optionally substituted mono or bicyclic C₃-C₆ cycloalkyl, or optionally substituted mono or bicyclic 3 to 8 membered heterocycle; or L is absent and R⁷ is optionally substituted C₃-C₁₀ alkyl or optionally substituted mono or bicyclic 3 to 8 membered heterocycle, wherein when L is absent and R⁷ is optionally substituted C₃-C₁₀ alkyl, the alkyl, is unsubstituted or substituted with one or more of OH, oxo or halogen;
R⁸ is H; and
R⁹ and R¹⁰ are each independently selected from the group consisting of H, mono or bicyclic optionally substituted C₆-C₁₂ aryl, mono or bicyclic optionally substituted 5 to 10 membered heteroaryl, optionally substituted C₁-C₁₀ alkyl, optionally substituted mono or bicyclic C₃-C₆ cycloalkyl, optionally substituted mono or bicyclic C₃-C₆ cycloalkenyl, optionally substituted C₂-C₁₀ alkenyl, optionally substituted C₂-C₁₀ alkynyl, optionally substituted C₁-C₁₀ alkoxy and NH₂;
wherein, unless otherwise specified:
an optionally substituted alkyl is unsubstituted or substituted with one or more of halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted alkenyl is unsubstituted or substituted with one or more of optionally substituted C₁-C₆ alkyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted alkynyl group is unsubstituted or substituted with one or more of optionally substituted C₁-C₆ alkyl, optionally substituted C₂-C₆ alkenyl, halogen, OH, optionally substituted C₁-C₆ alkoxy, optionally substituted aryloxy, optionally substituted heteroaryloxy, NR⁹R¹⁰, C(O)R⁹, CN, oxo, azido OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted alkoxy group is unsubstituted or substituted with one or more of halogen, OH, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted aryloxy, optionally substituted heteroaryloxy, optionally substituted C₆-C₁₂ aryl, optionally substituted 5 to 10 membered heteroaryl, C₃-C₆ cycloalkyl and 3 to 8 membered heterocycle;
an optionally substituted aryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted aryloxy is the group aryl-O- where aryl is an optionally substituted C₆-C₁₂ aryl group;
an optionally substituted cycloalkyl or cycloalkenyl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted heteroaryl is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
an optionally substituted heterocycle is unsubstituted or substituted with one or more of optionally substituted C₁-C₆ alkyl, optionally substituted C₁-C₆ alkenyl, optionally substituted C₁-C₆ alkynyl, halogen, OH, optionally substituted C₁-C₆ alkoxy, optionally substituted aryloxy, optionally substituted heteroaryloxy, NR⁹R¹⁰, C(O)R⁹, CN, oxo, azido, OP(O)(OH)₂, OC(O)R⁹, COOR⁹, C₁-C₆ alkenyl, =NOR⁹, NR⁹C(O)R¹⁰, SO₂R⁹, SO₂NR⁹R¹⁰, optionally substituted C₆-C₁₂ aryl, optionally substituted 5 to 10 membered heteroaryl, optionally substituted C₃-C₆ cycloalkyl and optionally substituted 3 to 8 membered heterocycle;
or a pharmaceutically acceptable salt, solvate, tautomeric form or polymorphic form thereof, or a pharmaceutical composition comprising a compound of formula (I), or a pharmaceutically acceptable salt, solvate, tautomeric form or polymorphic form thereof, and a pharmaceutically acceptable vehicle, for use in treating, ameliorating or preventing a disease selected from cancer, bacterial infection, viral infection, parasitic infection, fungal infection, neurodegenerative disease, neurological disorder, cerebrovascular disease, cardiovascular disease, non-alcoholic fatty liver disease and obesity or for use in promoting healthy ageing.

14. A compound or pharmaceutical composition for use according to claim 13, wherein the disease is cancer, and the cancer is selected from the group consisting of colorectal cancer, aero-digestive squamous cancer, gastrointestinal stromal tumors, lung cancer, brain cancer, neuroblastoma, glial tumors, astrocytoma, glioblastoma, liver cancer, stomach cancer, sarcoma, leukaemia, lymphoma, multiple myeloma, ovarian cancer, uterine cancer, breast cancer, melanoma, prostate cancer, bladder cancer, pancreatic carcinoma or renal carcinoma, or wherein the disease is a viral infection, and the viral infection is a hepatitis C virus (HCV) infection or a human cytomegalovirus (HCMV) infection.

## Patentansprüche

1. Verbindung der Formel (**I**): 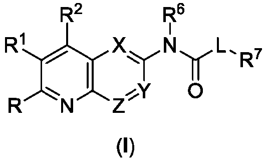
, wobei X CR³ ist;
Y CR⁴ ist;
Z CR⁵ oder N ist;
R ausgewählt ist aus der Gruppe, bestehend aus H, mono- oder bicyclischem optional substituierten C₆-C₁₂-Aryl, mono- oder bicyclischem optional substituierten 5- bis 10-gliedrigen Heteroaryl, optional substituiertem C₁-C₁₀-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₁₀-Alkenyl und optional substituiertem C₂-C₁₀-Alkinyl;
R¹ ausgewählt ist aus der Gruppe, bestehend aus mono- oder bicyclischem optional substituierten C₆-C₁₂-Aryl, mono- oder bicyclischem optional substituierten 5- bis 10-gliedrigen Heteroaryl, optional substituiertem C₁-C₁₀-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₁₀-Alkenyl und optional substituiertem C₂-C₁₀-Alkinyl, wobei, wenn R¹ ein optional substituiertes Alkyl, ein optional substituiertes Alkenyl oder ein optional substituiertes Alkinyl ist, das Alkyl, Alkenyl oder Alkinyl unsubstituiert oder mit einem oder mehreren von einem Halogen, einem optional substituierten C₆-C₁₂-Aryl oder einem optional substituierten 5-bis 10-gliedrigen Heteroaryl substituiert ist;
R² H, Halogen, COOR⁹, CN, CONR⁹R¹⁰, NR⁹R¹⁰, NR⁹SO₂R¹⁰, SO₂NR⁹R¹⁰, NR⁹COR¹⁰, optional substituiertes C₁-C₁₀-Alkyl, optional substituierter mono- oder bicyclischer 3- bis 8-gliedriger Heterocyclus, optional substituiertes C₁-C₁₀-Alkoxy, mono- oder bicyclisches optional substituiertes C₆-C₁₂-Aryl oder mono- oder bicyclisches optional substituiertes 5 bis 10-gliedriges Heteroaryl ist;
R³ H, Halogen, optional substituiertes C₁-C₁₀-Alkyl, optional substituiertes mono- oder bicyclisches C₃-C₆-Cycloalkyl, optional substituierter mono- oder bicyclischer 3- bis 8-gliedriger Heterocyclus, optional substituiertes C₁-C₁₀-Alkoxy, mono- oder bicyclisches optional substituiertes C₆-C₁₂-Aryl oder mono- oder bicyclisches optional substituiertes 5- bis 10-gliedriges Heteroaryl ist;
R⁴ und R⁵ jeweils unabhängig ausgewählt sind aus der Gruppe, bestehend aus H und Halogen;
R⁶ H ist;
L NR⁸ ist und R⁷ optional substituiertes C₃-C₁₀-Alkyl, optional substituiertes C₂-C₁₀-Alkenyl, optional substituiertes C₂-C₁₀-Alkinyl, mono- oder bicyclisches optional substituiertes C₆-C₁₂-Aryl, mono- oder bicyclisches optional substituiertes 5- bis 10-gliedriges Heteroaryl, optional substituiertes mono- oder bicyclisches C₃-C₆-Cycloalkyl oder optional substituierter mono- oder bicyclischer 3- bis 8- gliedriger Heterocyclus ist; oder L abwesend ist und R⁷ optional substituiertes C₃-C₁₀-Alkyl oder optional substituierter mono- oder bicyclischer 3- bis 8-gliedriger Heterocyclus ist, wobei, wenn L abwesend ist und R⁷ optional substituiertes C₃-C₁₀-Alkyl ist, das Alkyl unsubstituiert oder mit einem oder mehreren von OH, Oxo oder Halogen substituiert ist;
R⁸ H ist; und
R⁹ und R¹⁰ jeweils unabhängig ausgewählt sind aus der Gruppe, bestehend aus H, mono- oder bicyclischem optional substituierten C₆-C₁₂-Aryl, mono- oder bicyclischem optional substituierten 5- bis 10-gliedrigen Heteroaryl, optional substituiertem C₁-C₁₀-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₁₀-Alkenyl, optional substituiertem C₂-C₁₀-Alkinyl, optional substituiertem C₁-C₁₀-Alkoxy und NH₂;
oder pharmazeutisch unbedenklicher Komplex, pharmazeutisch unbedenkliches Salz, Solvat, pharmazeutisch unbedenkliche tautomere Form oder polymorphe Form davon, wobei, sofern nicht anders spezifiziert:
ein optional substituiertes Alkyl unsubstituiert oder substituiert ist mit einem oder mehreren von Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5- bis 10-gliedrigem Heteroaryl, optional substituiertem C₃-C₆-Cycloalkyl und optional substituiertem 3- bis 8-gliedrigem Heterocyclus;
ein optional substituiertes Alkenyl unsubstituiert oder substituiert ist mit einem oder mehreren von optional substituiertem C₁-C₆-Alkyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3- bis 8-gliedrigem Heterocyclus;
eine optional substituierte Alkinylgruppe unsubstituiert oder substituiert ist mit einem oder mehreren von optional substituiertem C₁-C₆-Alkyl, optional substituiertem C₂-C₆-Alkenyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3- bis 8-gliedrigem Heterocyclus;
eine optional substituierte Alkoxygruppe unsubstituiert oder substituiert ist mit einem oder mehreren von Halogen, OH, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem Aryloxy, optional substituiertem Heteroaryloxy, optional substituiertem C₆-C₁₂-Aryl, optional substituiertem 5- bis 10-gliedrigen Heteroaryl, C₃-C₆-Cycloalkyl und 3- bis 8-gliedrigem Heterocyclus;
ein optional substituiertes Aryl unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3- bis 8-gliedrigem Heterocyclus;
ein optional substituiertes Aryloxy ist die Gruppe Aryl-O-, wobei Aryl eine optional substituierte C₆₋C₁₂-Arylgruppe ist;
ein optional substituiertes Cycloalkyl oder Cycloalkenyl unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5- bis 10-gliedrigen Heteroaryl, optional substituiertem C₃-C₆-Cycloalkyl und optional substituiertem 3- bis 8-gliedrigen Heterocyclus;
ein optional substituiertes Heteroaryl unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3 bis 8-gliedrigen Heterocyclus;
ein optional substituierter Heterocyclus unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3- bis 8-gliedrigem Heterocyclus; und wobei die Verbindung Folgendes nicht ist:

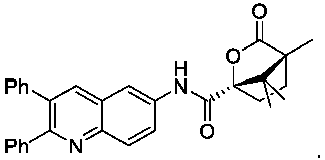

2. Verbindung gemäß Anspruch 1, wobei R und R¹ jeweils unabhängig ausgewählt sind aus der Gruppe, bestehend aus mono- oder bicyclischem optional substituierten C₆-C₁₂-Aryl, mono- oder bicyclischem optional substituierten 5- bis 10-gliedrigen Heteroaryl, optional substituiertem C₁-C₆-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₆-Alkenyl und optional substituiertem C₂-C₆-Alkinyl.

3. Verbindung gemäß Anspruch 2, wobei R und R¹ jeweils unabhängig ausgewählt sind aus einem optional substituierten Phenylring, einem optional substituierten C₁-C₆-Alkyl, einem optional substituierten C₂-C₆-Alkenyl, einem optional substituierten C₂-C₆-Alkinyl oder einem optional substituierten 5- oder 6-gliedrigen Heteroaryl.

4. Verbindung gemäß einem der vorhergehenden Ansprüche, wobei R² H, COOR⁹, CONR⁹R¹⁰, CN, NR⁹COR¹⁰, NR⁹R¹⁰, NR⁹SO₂R¹⁰, optional substituiertes C₁-C₆-Alkyl, optional substituiertes C₁-C₆-Alkoxy, mono- oder bicyclisches optional substituiertes C₆-C₁₂-Aryl oder ein mono- oder bicyclisches optional substituiertes 5- bis 10-gliedriges Heteroaryl ist.

5. Verbindung gemäß einem der vorhergehenden Ansprüche, wobei R³ H, Halogen, optional substituiertes C₁-C₆-Alkyl, optional substituiertes mono- oder bicyclisches C₃-C₆-Cycloalkyl, optional substituiertes C₁-C₆-Alkoxy, mono- oder bicyclisches optional substituiertes C₆-C₁₂-Aryl oder mono- oder bicyclisches optional substituiertes 5- bis 10-gliedriges Heteroaryl ist.

6. Verbindung gemäß einem der vorhergehenden Ansprüche, wobei R⁴ und R⁵ jeweils unabhängig ausgewählt sind aus der Gruppe, bestehend aus H, Halogen, OH, CN, mono- oder bicyclischem optional substituierten C₆-C₁₂-Aryl, optional substituiertem C₁-C₆-Alkyl, optional substituiertem C₁-C₆-Alkoxy oder optional substituiertem mono- oder bicyclischen C₃-C₆-Cycloalkyl.

7. Verbindung gemäß Anspruch 6, wobei R⁴ und R⁵ H, Halogen, OH, CN oder ein optional substituiertes C₁-C₆-Alkyl sind.

8. Verbindung gemäß Anspruch 7, wobei R⁴ und R⁵ H sind.

9. Verbindung gemäß einem der vorhergehenden Ansprüche, wobei L NR⁸ ist und R⁷ optional substituiertes C₃-C₆-Alkyl, optional substituiertes C₂-C₆-Alkenyl, optional substituiertes C₂-C₆-Alkinyl, mono- oder bicyclisches optional substituiertes C₆-C₁₂-Aryl, optional substituiertes mono- oder bicyclisches C₃-C₆-Cycloalkyl oder optional substituierter mono- oder bicyclischer 3-bis 8-gliedriger Heterocyclus ist.

10. Verbindung gemäß Anspruch 1, wobei die Verbindung wie folgt ist:
1-(2,3-Diphenylchinolin-6-yl)-3-(2-hydroxybutyl)harnstoff;
(R)-1-(2,3-Diphenylchinolin-6-yl)-3-(2-hydroxybutyl)harnstoff;
1-Butyl-3-(2,3-diphenylchinolin-6-yl)harnstoff;
1-(2,3-Bis(2-fluorphenyl)chinolin-6-yl)-3-(2-hydroxybutyl)harnstoff;
1-Butyl-3-(5-(2-hydroxypyridin-3-yl)-2,3-diphenylchinolin-6-yl)harnstoff;
1-(2,3-Bis(2-methoxyphenyl)chinolin-6-yl)-3-(2-hydroxybutyl)harnstoff;
1-(3-(2-Fluorphenyl)-2-phenylchinolin-6-yl)-3-(2-hydroxybutyl)harnstoff;
1-(2-(2-Fluorphenyl)-3-phenylchinolin-6-yl)-3-(2-hydroxybutyl)harnstoff;
1-(2,3-Bis(2-methoxypyridin-4-yl)chinolin-6-yl)-3-(2-hydroxybutyl)harnstoff;
1-(2-Hydroxybutyl)-3-(3-(2-methoxyphenyl)-2-phenylchinolin-6-yl)harnstoff;
1-(2,3-Bis(6-methoxypyridin-3-yl)chinolin-6-yl)-3-(2-hydroxybutyl)harnstoff;
1-(2-Hydroxybutyl)-3-(2-(2-methoxyphenyl)-3-phenylchinolin-6-yl)harnstoff;
1-(2,3-Bis(2-methoxypyridin-3-yl)chinolin-6-yl)-3-(2-hydroxybutyl)harnstoff;
1-(2,3-Bis(2-fluorphenyl)chinolin-6-yl)-3-butylharnstoff;
(R)-1-(3-(2-Fluorphenyl)-2-phenylchinolin-6-yl)-3-(2-hydroxybutyl)harnstoff;
1-Butyl-3-(3-(2-fluorphenyl)-2-phenylchinolin-6-yl)harnstoff;
1-(2,3-Bis(4-fluorphenyl)chinolin-6-yl)-3-(2-hydroxybutyl)harnstoff;
1-(3-(2-Fluorphenyl)-2-(2-methoxyphenyl)chinolin-6-yl)-3-(2-hydroxybutyl)harnstoff;
1-(2,3-Di(pyridin-3-yl)chinolin-6-yl)-3-(2-hydroxybutyl)harnstoff;
(R)-1-(2,3-Bis(2-fluorphenyl)chinolin-6-yl)-3-(2-hydroxybutyl)harnstoff;
1-(2,3-Bis(3-acetylphenyl)chinolin-6-yl)-3-(2-hydroxybutyl)harnstoff;
1-(2,3-Di(1H-pyrazol-4-yl)chinolin-6-yl)-3-(2-hydroxybutyl)harnstoff;
1-Butyl-3-(3-(2-methoxyphenyl)-2-phenylchinolin-6-yl)harnstoff;
1-(2,3-Di(pyridin-4-yl)chinolin-6-yl)-3-(2-hydroxybutyl)harnstoff;
1-(2,3-Bis(3-fluorphenyl)chinolin-6-yl)-3-(2-hydroxybutyl)harnstoff;
1-(2-(2-Fluorphenyl)-3-(2-methoxyphenyl)chinolin-6-yl)-3-(2-hydroxybutyl)harnstoff;
1-(2,3-Bis(6-oxo-1,6-dihydropyridin-3-yl)chinolin-6-yl)-3-(2-hydroxybutyl)harnstoff;
(S)-1-(2,3-Bis(2-fluorphenyl)chinolin-6-yl)-3-(2-hydroxybutyl)harnstoff;
1-(2,3-Bis(2-hydroxypyridin-4-yl)chinolin-6-yl)-3-(2-hydroxybutyl)harnstoff;
(S)-1-(2,3-Diphenylchinolin-6-yl)-3-(2-hydroxybutyl)harnstoff;
1-(3-(2-Fluorphenyl)-2-(pyridin-3-yl)chinolin-6-yl)-3-(2-hydroxybutyl)harnstoff;
1-(2-(Cyclohex-1-en-1-yl)-3-(2-fluorphenyl)chinolin-6-yl)-3-(2-hydroxybutyl)harnstoff;
1-(2-(2-(Dimethylamino)phenyl)-3-(2-fluorphenyl)chinolin-6-yl)-3-(2-hydroxybutyl)harnstoff;
1-(3-(2-Fluorphenyl)-2-(pyridin-4-yl)chinolin-6-yl)-3-(2-hydroxybutyl)harnstoff;
(R)-1-(3-(2-Fluorphenyl)-2-(2-methoxyphenyl)chinolin-6-yl)-3-(2-hydroxybutyl)harnstoff;
(S)-1-(3-(2-Fluorphenyl)-2-(2-methoxyphenyl)chinolin-6-yl)-3-(2-hydroxybutyl)harnstoff;
1-Butyl-3-(3-(2-fluorphenyl)-2-(2-methoxyphenyl)chinolin-6-yl)harnstoff;
1-(2-Hydroxybutyl)-3-(3-phenyl-2-(pyridin-2-yl)chinolin-6-yl)harnstoff;
1-(2-Cyclohexyl-3-(2-fluorphenyl)chinolin-6-yl)-3-(2-hydroxybutyl)harnstoff;
1-(2-Cyclopropyl-3-phenylchinolin-6-yl)-3-(2-hydroxybutyl)harnstoff;
1-(2-Hydroxybutyl)-3-(3-phenyl-2-(pyridin-3-yl)chinolin-6-yl)harnstoff;
1-(2,3-Bis(2-hydroxypyridin-3-yl)chinolin-6-yl)-3-(2-hydroxybutyl)harnstoff;
1-(2-Hydroxybutyl)-3-(2-(1-methyl-1H-pyrazol-4-yl)-3-phenylchinolin-6-yl)harnstoff;
1-(3-(2-Fluorphenyl)-2-phenylchinolin-6-yl)-3-(2-methoxybutyl)harnstoff;
1-(2,2-Difluorbutyl)-3-(3-(2-fluorphenyl)-2-phenylchinolin-6-yl)harnstoff;
1-(3-(2-Fluorphenyl)-2-(3-fluorphenyl)chinolin-6-yl)-3-(2-hydroxybutyl)harnstoff;
1-(3-(2-Fluorphenyl)-2-(4-fluorphenyl)chinolin-6-yl)-3-(2-hydroxybutyl)harnstoff;
Methyl-6-(3-(2-hydroxybutyl)ureido)-2,3-diphenylchinolin-4-carboxylat;
6-(3-(2-Hydroxybutyl)ureido)-2,3-diphenylchinolin-4-carboxamid;
(E)-1-(2-Hydroxybutyl)-3-(3-phenyl-2-styrylchinolin-6-yl)harnstoff;
N-(2-(3-(2-Fluorphenyl)-6-(3-(2-hydroxybutyl)ureido)chinolin-2-yl)phenyl)acetamid;
1-(2-Hydroxybutyl)-3-(2-methyl-3-phenylchinolin-6-yl)harnstoff;
1-(2-(2-Cyanophenyl)-3-(2-fluorphenyl)chinolin-6-yl)-3-(2-hydroxybutyl)harnstoff;
1-(2-(2-Aminophenyl)-3-(2-fluorphenyl)chinolin-6-yl)-3-(2-hydroxybutyl)harnstoff;
1-(2-([1,1'-Biphenyl]-4-yl)-3-phenylchinolin-6-yl)-3-(2-hydroxybutyl)harnstoff;
1-(2-Hydroxybutyl)-3-(2-phenethyl-3-phenylchinolin-6-yl)harnstoff;
(R)-6-(3-(2-Hydroxybutyl)ureido)-2,3-diphenylchinolin-4-carboxamid;
1-(2-(2-Ethoxyphenyl)-3-phenylchinolin-6-yl)-3-(2-hydroxybutyl)harnstoff;
1-(2-Hydroxybutyl)-3-(2-(2-isobutoxyphenyl)-3-phenylchinolin-6-yl)harnstoff;
1-(2-Ethinyl-3-phenylchinolin-6-yl)-3-(2-hydroxybutyl)harnstoff;
1-(2-Ethyl-3-phenylchinolin-6-yl)-3-(2-hydroxybutyl)harnstoff;
6-(3-(2-Hydroxybutyl)ureido)-N-methyl-2,3-diphenylchinolin-4-carboxamid;
6-(3-(2-Hydroxybutyl)ureido)-N,N-dimethyl-2,3-diphenylchinolin-4-carboxamid;
N-Cyclopropyl-6-(3-(2-hydroxybutyl)ureido)-2,3-diphenylchinolin-4-carboxamid;
6-(3-(2-Hydroxybutyl)ureido) -2,3-diphenylchinolin-4-carbonsäure;
6-(3-(2-Hydroxybutyl)ureido)-2-methyl-3-phenylchinolin-4-carboxamid;
(S)-1-(3-(2-Fluorphenyl)-2-phenylchinolin-6-yl)-3-(2-hydroxybutyl)harnstoff;
(S)-1-(2,3-Diphenylchinolin-6-yl)-3-(2-hydroxybutyl)harnstoff;
6-(3-(2-Hydroxybutyl)ureido)-2,3-dimethylchinolin-4-carboxamid;
1-(4-Cyano-2,3-diphenylchinolin-6-yl)-3-(2-hydroxybutyl)harnstoff;
6-(3-(2-Hydroxybutyl)ureido)-3-phenylchinolin-4-carboxamid;
(R)-1-(2-Hydroxybutyl)-3-(2-methyl-3-phenylchinolin-6-yl)harnstoff;
3-(2-Fluorphenyl)-6-(3-(2-hydroxybutyl)ureido)-2-methylchinolin-4-carboxamid;
1-(3-(2-Fluorphenyl)-2-methylchinolin-6-yl)-3-(2-hydroxybutyl)harnstoff;
1-Butyl-3-(2-methyl-3-phenylchinolin-6-yl)harnstoff;
1-(2-Methoxybutyl)-3-(2-methyl-3-phenylchinolin-6-yl)harnstoff;
1-(2-Methoxyethyl)-3-(2-methyl-3-phenylchinolin-6-yl)harnstoff;
1-(2-Hydroxybutyl)-3-(2-methyl-3-(pyridin-3-yl)chinolin-6-yl)harnstoff;
1-(2-Hydroxybutyl)-3-(2-methyl-3-(o-tolyl)chinolin-6-yl)harnstoff;
1-(3-(2-Chlorphenyl)-2-methylchinolin-6-yl)-3-(2-hydroxybutyl)harnstoff;
1-(2-Hydroxybutyl)-3-(2-methyl-3-(1-methyl-1H-pyrazol-4-yl)chinolin-6-yl)harnstoff;
(R)-1-(2-Hydroxybutyl)-3-(2-methyl-3-(o-tolyl)chinolin-6-yl)harnstoff;
N-(6-(3-(2-Hydroxybutyl)ureido)-2,3-diphenylchinolin-4-yl)acetamid;
1-(2,5-Dimethyl-3-phenylchinolin-6-yl)-3-(2-hydroxybutyl)harnstoff;
1-(5-Cyclopropyl-2-methyl-3-phenylchinolin-6-yl)-3-(2-hydroxybutyl)harnstoff;
1-(5-Brom-2-methyl-3-phenylchinolin-6-yl)-3-(2-hydroxybutyl)harnstoff;
1-(2-Hydroxybutyl)-3-(3-phenyl-2-(trifluormethyl)chinolin-6-yl)harnstoff;
(S)-1-(2-Hydroxybutyl)-3-(2-methyl-3-phenylchinolin-6-yl)harnstoff;
(R)-1-(7-Fluor-2,3-diphenylchinolin-6-yl)-3-(2-hydroxybutyl)harnstoff;
1-(4-(Dimethylamino)-2,3-diphenylchinolin-6-yl)-3-(2-hydroxybutyl)harnstoff;
(R)-N-(6-(3-(2-Hydroxybutyl)ureido)-2,3-diphenylchinolin-4-yl)cyclopropancarboxamid;
N-(6-(3-(2-Hydroxybutyl)ureido)-2-methyl-3-phenylchinolin-4-yl)acetamid;
N-(6-(3-(2-Hydroxybutyl)ureido)-2,3-diphenylchinolin-4-yl)methansulfonamid;
1-(4-Amino-2,3-diphenylchinolin-6-yl)-3-(2-hydroxybutyl)harnstoff;
(R)-1-(2-Hydroxybutyl)-3-(2-methyl-5-(1-methyl-1H-pyrazol-4-yl)-3-phenylchinolin-6-yl)harnstoff;
1-(2-Hydroxybutyl)-3-(4-(oxazol-2-yl)-2,3-diphenylchinolin-6-yl)harnstoff;
1-(4-Cyano-2-methyl-3-phenylchinolin-6-yl)-3-(2-hydroxybutyl)harnstoff;
N-(1-(3-(2-Methyl-3-phenylchinolin-6-yl)ureido)butan-2-yl)acetamid;
(R)-1-(3-(2-Fluorphenyl)-2-methylchinolin-6-yl)-3-(2-hydroxybutyl)harnstoff;
(R)-1-(2-Hydroxybutyl)-3-(4-methoxy-2,3-diphenylchinolin-6-yl)harnstoff;
(R)-1-(2-Hydroxybutyl)-3-(4-methoxy-2-methyl-3-phenylchinolin-6-yl)harnstoff;
(R)-1-(2-Hydroxybutyl)-3-(4-methyl-2,3-diphenylchinolin-6-yl)harnstoff;
(R)-N-Benzyl-6-(3-(2-hydroxybutyl)ureido)-3-methyl-2-phenylchinolin-4-carboxamid;
1-(2,2-Difluorbutyl)-3-(2-methyl-3-phenylchinolin-6-yl)harnstoff;
6-(3-(2,2-Difluorbutyl)ureido)-2,3-diphenylchinolin-4-carboxamid;
1-(2,2-Difluorbutyl)-3-(2,3-diphenylchinolin-6-yl)harnstoff;
(R)-6-(3-(2-Hydroxybutyl)ureido)-N,3-dimethyl-2-phenylchinolin-4-carboxamid;
(R)-6-(3-(2-Hydroxybutyl)ureido)-3-methyl-2-phenylchinolin-4-carboxamid;
1-(2,2-Difluorbutyl)-3-(3-(2-fluorphenyl)-2-methylchinolin-6-yl)harnstoff;
(R)-1-(3-(2-Fluorphenyl)-2-methyl-5-(2-oxo-1,2-dihydropyridin-3-yl)chinolin-6-yl)-3-(2-hydroxybutyl)harnstoff;
(R)-6-(3-(2-Hydroxybutyl)ureido)-N-(1-methyl-1H-pyrazol-3-yl)-2,3-diphenylchinolin-4-carboxamid;
(R)-1-(2,4-Dimethyl-3-phenylchinolin-6-yl)-3-(2-hydroxybutyl)harnstoff;
(R)-6-(3-(2-Hydroxybutyl)ureido)-3-methyl-N-((1-methyl-1H-pyrazol-4-yl)methyl)-2-phenylchinolin-4-carboxamid;
(R)-6-(3-(2-Hydroxybutyl)ureido)-3-methyl-N-(1-methyl-1H-pyrazol-3-yl)-2-phenylchinolin-4-carboxamid;
1-(3-(2-Fluorphenyl)-2-methylchinolin-6-yl)-3-(2-methoxyethyl)harnstoff;
(R)-1-(3-(3-(2-Fluorphenyl)-2-phenylchinolin-6-yl)ureido)butan-2-yldihydrogenphosphat;
N-(2,3-Diphenylchinolin-6-yl)hexanamid;
N-(2,3-Bis(2-methoxyphenyl)chinolin-6-yl)hexanamid;
N-(2-(2-Methoxyphenyl)-3-phenylchinolin-6-yl)-4-oxohexanamid;
N-(2,3-Bis(2-fluorphenyl)chinolin-6-yl)hexanamid;
N-(2,3-Diphenylchinolin-6-yl)-2,2-difluorhexanamid;
N-(2-(2-Fluorphenyl)-3-phenylchinolin-6-yl)-4-oxohexanamid;
N-(2,3-Bis(2-fluorphenyl)chinolin-6-yl)-4-oxohexanamid;
N-(2,3-Diphenylchinolin-6-yl)-4-oxohexanamid;
N-(3-(2-Fluorphenyl)-2-(2-methoxyphenyl)chinolin-6-yl)-4-oxohexanamid;
N-(2,3-Diphenylchinolin-6-yl)-4-hydroxyhexanamid;
N-(2,3-Bis(2-fluorphenyl)chinolin-6-yl)-4-hydroxyhexanamid;
N-(3-(2-Fluorphenyl)-2-methylchinolin-6-yl)-4-hydroxyhexanamid;
4-Hydroxy-N-(2-methyl-3-phenylchinolin-6-yl)hexanamid;
1-Cyclohexyl-3-(2,3-diphenylchinolin-6-yl)harnstoff;
N-(2,3-Diphenylchinolin-6-yl)-4-methylpiperazin-1-carboxamid;
4-Acetyl-N-(2,3-diphenylchinolin-6-yl)piperazin-1-carboxamid;
1-Allyl-3-(2,3-diphenylchinolin-6-yl)harnstoff;
1-(2,3-Diphenylchinolin-6-yl)-3-(prop-2-in-1-yl)harnstoff;
1-(2,3-Diphenylchinolin-6-yl)-3-(2-hydroxycyclopentyl)harnstoff;
1-(2,3-Diphenylchinolin-6-yl)-3-(tetrahydro-2H-pyran-4-yl)harnstoff;
N-(2,3-Diphenylchinolin-6-yl)-3-methoxypyrrolidin-1-carboxamid;
1-(2,3-Diphenylchinolin-6-yl)-3-(pyridin-2-yl)harnstoff;
1-(2,3-Diphenylchinolin-6-yl)-3-phenylharnstoff;
1-(2,3-Diphenylchinolin-6-yl)-3-(1-methylpyrrolidin-3-yl)harnstoff;
1-(2,3-Diphenylchinolin-6-yl)-3-(pyridin-4-yl)harnstoff;
1-(2,3-Diphenylchinolin-6-yl)-3-(1-(hydroxymethyl)cyclopentyl)harnstoff;
1-(2,3-Diphenylchinolin-6-yl)-3-(1-methyl-1H-pyrazol-4-yl)harnstoff;
1-(6,7-Diphenyl-1,8-naphthyridin-3-yl)-3-(2-hydroxybutyl)harnstoff;
(R)-1-(6,7-Diphenyl-1,8-naphthyridin-3-yl)-3-(2-hydroxybutyl)harnstoff; oder
N-(6,7-Diphenyl-1,8-naphthyridin-3-yl)-3-methoxypyrrolidin-1-carboxamid.

11. Pharmazeutische Zusammensetzung, umfassend eine Verbindung gemäß einem der Ansprüche 1 bis 10, oder ein pharmazeutisch unbedenkliches Salz, Solvat, eine pharmazeutisch unbedenkliche tautomere Form oder polymorphe Form davon und ein pharmazeutisch unbedenkliches Vehikel.

12. Verbindung gemäß einem der Ansprüche 1 bis 10 oder ein pharmazeutisch unbedenkliches Salz, Solvat, eine pharmazeutisch unbedenkliche tautomere Form oder polymorphe Form davon, oder eine pharmazeutische Zusammensetzung gemäß Anspruch 11 zur Verwendung in der Therapie.

13. Verbindung der Formel (**I**): 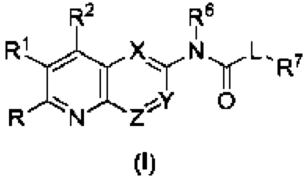
, wobei X CR³ ist;
Y CR⁴ ist;
Z CR⁵ oder N ist; R ausgewählt ist aus der Gruppe, bestehend aus H, mono- oder bicyclischem optional substituierten C₆-C₁₂-Aryl, mono- oder bicyclischem optional substituierten 5- bis 10-gliedrigen Heteroaryl, optional substituiertem C₁-C₁₀-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₁₀-Alkenyl und optional substituiertem C₂-C₁₀-Alkinyl; R¹ ausgewählt ist aus der Gruppe, bestehend aus mono- oder bicyclischem optional substituierten C₆-C₁₂-Aryl, mono- oder bicyclischem optional substituierten 5- bis 10-gliedrigen Heteroaryl, optional substituiertem C₁-C₁₀-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₁₀-Alkenyl und optional substituiertem C₂-C₁₀-Alkinyl, wobei, wenn R¹ ein optional substituiertes Alkyl, ein optional substituiertes Alkenyl oder ein optional substituiertes Alkinyl ist, das Alkyl, Alkenyl oder Alkinyl unsubstituiert oder mit einem oder mehreren von einem Halogen, einem optional substituierten C₆₋C₁₂-Aryl oder einem optional substituierten 5-bis 10-gliedrigen Heteroaryl substituiert ist;
R² H, Halogen, COOR⁹, CN, CONR⁹R¹⁰, NR⁹R¹⁰, NR⁹SO₂R¹⁰, SO₂NR⁹R¹⁰, NR⁹COR¹⁰, optional substituiertes C₁-C₁₀-Alkyl, optional substituierter mono- oder bicyclischer 3- bis 8-gliedriger Heterocyclus, optional substituiertes C₁-C₁₀-Alkoxy, mono- oder bicyclisches optional substituiertes C₆-C₁₂-Aryl oder mono- oder bicyclisches optional substituiertes 5 bis 10-gliedriges Heteroaryl ist;
R³ H, Halogen, optional substituiertes C₁-C₁₀-Alkyl, optional substituiertes mono- oder bicyclisches C₃-C₆-Cycloalkyl, optional substituierter mono- oder bicyclischer 3- bis 8-gliedriger Heterocyclus, optional substituiertes C₁-C₁₀-Alkoxy, mono- oder bicyclisches optional substituiertes C₆₋C₁₂-Aryl oder mono- oder bicyclisches optional substituiertes 5- bis 10-gliedriges Heteroaryl ist;
R⁴ und R⁵ jeweils unabhängig ausgewählt sind aus der Gruppe, bestehend aus H und Halogen; R⁶ H ist;
L NR⁸ ist und R⁷ optional substituiertes C₃-C₁₀-Alkyl, optional substituiertes C₂-C₁₀-Alkenyl, optional substituiertes C₂-C₁₀-Alkinyl, mono- oder bicyclisches optional substituiertes C₆₋C₁₂-Aryl, mono- oder bicyclisches optional substituiertes 5- bis 10-gliedriges Heteroaryl, optional substituiertes mono- oder bicyclisches C₃-C₆-Cycloalkyl oder optional substituierter mono- oder bicyclischer 3- bis 8-gliedriger Heterocyclus; oder L abwesend ist und R⁷ optional substituiertes C₃-C₁₀-Alkyl oder optional substituierter mono- oder bicyclischer 3- bis 8-gliedriger Heterocyclus ist, wobei, wenn L abwesend ist und R⁷ optional substituiertes C₃-C₁₀-Alkyl ist, das Alkyl unsubstituiert ist oder mit einem oder mehreren von OH, Oxo oder Halogen substituiert ist;
R⁸ H ist; und
R⁹ und R¹⁰ jeweils unabhängig ausgewählt sind aus der Gruppe, bestehend aus H, mono- oder bicyclischem optional substituierten C₆-C₁₂-Aryl, mono- oder bicyclischem optional substituierten 5- bis 10-gliedrigen Heteroaryl, optional substituiertem C₁-C₁₀-Alkyl, optional substituiertem mono- oder bicyclischen C₃-C₆-Cycloalkyl, optional substituiertem mono- oder bicyclischen C₃-C₆-Cycloalkenyl, optional substituiertem C₂-C₁₀-Alkenyl, optional substituiertem C₂-C₁₀-Alkinyl, optional substituiertem C₁-C₁₀-Alkoxy und NH₂;
wobei, sofern nicht anders spezifiziert:
ein optional substituiertes Alkyl unsubstituiert oder substituiert ist mit einem oder mehreren von Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆₋C₁₂-Aryl, optional substituiertem 5- bis 10-gliedrigen Heteroaryl, optional substituiertem C₃-C₆-Cycloalkyl und optional substituiertem 3- bis 8-gliedrigen Heterocyclus;
ein optional substituiertes Alkenyl unsubstituiert oder substituiert ist mit einem oder mehreren von optional substituiertem C₁-C₆-Alkyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido OP(O)(OH)₂, OC(O)R⁹, COOR⁹, C₁-C₆-Alkenyl, =NOR⁹, NR⁹C(O)R^{1O}, SO₂R⁹, SO₂NR⁹R¹⁰, optional substituiertem C₆-C₁₂-Aryl, optional substituiertem 5- bis 10-gliedrigen Heteroaryl, optional substituiertem C₃-C₆-Cycloalkyl und optional substituiertem 3- bis 8-gliedrigem Heterocyclus;
eine optional substituierte Alkinylgruppe unsubstituiert oder substituiert ist mit einem oder mehreren von optional substituiertem C₁-C₆-Alkyl, optional substituiertem C₂-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5- bis 10-gliedrigen Heteroaryl, optional substituiertem C₃-C₆-Cycloalkyl und optional substituiertem 3- bis 8-gliedrigem Heterocyclus;
eine optional substituierte Alkoxygruppe unsubstituiert oder substituiert ist mit einem oder mehreren von Halogen, OH, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem Aryloxy, optional substituiertem Heteroaryloxy, optional substituiertem C₆₋C₁₂-Aryl, optional substituiertem 5- bis 10-gliedrigem Heteroaryl, C₃-C₆-Cycloalkyl und 3- bis 8-gliedrigem Heterocyclus;
ein optional substituiertes Aryl unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3- bis 8-gliedrigem Heterocyclus;
ein optional substituiertes Aryloxy ist die Gruppe Aryl-O-, wobei Aryl eine optional substituierte C₆₋C₁₂-Arylgruppe ist;
ein optional substituiertes Cycloalkyl oder Cycloalkenyl unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5- bis 10-gliedrigen Heteroaryl, optional substituiertem C₃-C₆-Cycloalkyl und optional substituiertem 3- bis 8-gliedrigen Heterocyclus;
ein optional substituiertes Heteroaryl unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5 bis 10-gliedrigen Heteroaryl, optional substituiertem C₃-C₆-Cycloalkyl und optional substituiertem 3 bis 8-gliedrigen Heterocyclus;
ein optional substituierter Heterocyclus unsubstituiert oder substituiert ist mit einem oder mehreren von optional substituiertem C₁-C₆-Alkyl, optional substituiertem C₁-C₆-Alkenyl, optional substituiertem C₁-C₆-Alkinyl, Halogen, OH, optional substituiertem C₁-C₆-Alkoxy, optional substituiertem Aryloxy, optional substituiertem Heteroaryloxy, NR⁹R¹⁰, C(O)R⁹, CN, Oxo, Azido, OP(O)(OH)₂, OC(O)R⁹, COOR⁹, C₁-C₆-Alkenyl, =NOR⁹, NR⁹C(O)R¹⁰, SO₂R⁹, SO₂NR⁹R¹⁰, optional substituiertem C₆-C₁₂-Aryl, optional substituiertem 5- bis 10-gliedrigen Heteroaryl, optional substituiertem C₃-C₆-Cycloalkyl und optional substituiertem 3- bis 8-gliedrigem Heterocyclus;
oder ein pharmazeutisch unbedenkliches Salz, Solvat, eine pharmazeutisch unbedenkliche tautomere Form oder polymorphe Form davon oder eine pharmazeutische Zusammensetzung, umfassend eine Verbindung der Formel (I) oder ein pharmazeutisch unbedenkliches Salz, Solvat, eine pharmazeutisch unbedenkliche tautomere Form oder polymorphe Form davon und ein pharmazeutisch unbedenkliches Vehikel zur Verwendung beim Behandeln, Lindern oder Verhindern einer Erkrankung, ausgewählt aus Krebs, bakterieller Infektion, viraler Infektion, parasitärer Infektion, Pilzinfektion, neurodegenerativer Erkrankung, neurologischer Störung, zerebrovaskulärer Erkrankung, kardiovaskulärer Erkrankung, nichtalkoholischer Fettlebererkrankung und Fettleibigkeit oder zur Verwendung beim Fördern eines gesunden Alterns.

14. Verbindung oder pharmazeutische Zusammensetzung zur Verwendung gemäß Anspruch 13, wobei die Erkrankung Krebs ist und der Krebs ausgewählt ist aus der Gruppe, bestehend aus Kolorektalkrebs, aero-digestivem Plattenepithelkrebs, gastrointestinalen Stromatumoren, Lungenkrebs, Hirnkrebs, Neuroblastom, Glia-Tumoren, Astrozytom, Glioblastom, Leberkrebs, Magenkrebs, Sarkom, Leukämie, Lymphom, multiplem Myelom, Eierstockkrebs, Gebärmutterkrebs, Brustkrebs, Melanom, Prostatakrebs, Blasenkrebs, Bauchspeicheldrüsenkrebs oder Nierenkrebs, oder wobei die Erkrankung eine virale Infektion ist und die virale Infektion eine Hepatitis-C-Virus- (HCV-)Infektion oder eine humane Cytomegalievirus- (HCMV-)Infektion ist.

## Revendications

1. Composé de formule (I) : 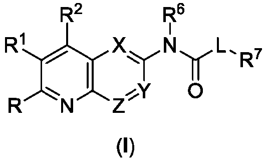
où X est CR³ ;
Y est CR⁴ ;
Z est CR⁵ ou N ;
R est choisi dans le groupe constitué par H, un aryle en C₆-C₁₂ mono- ou bicyclique éventuellement substitué, un hétéroaryle à 5 à 10 chaînons mono- ou bicyclique éventuellement substitué, un alkyle en C₁-C₁₀ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₁₀ éventuellement substitué et un alcynyle en C₂-C₁₀ éventuellement substitué ;
R¹ est choisi dans le groupe constitué par un aryle en C₆-C₁₂ mono- ou bicyclique éventuellement substitué, un hétéroaryle à 5 à 10 chaînons mono- ou bicyclique éventuellement substitué, un alkyle en C₁-C₁₀ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₁₀ éventuellement substitué et un alcynyle en C₂-C₁₀ éventuellement substitué, où lorsque R¹ est un alkyle éventuellement substitué, un alcényle éventuellement substitué ou un alcynyle éventuellement substitué, l'alkyle, l'alcényle ou l'alcynyle est non substitué ou substitué par un ou plusieurs parmi un halogène, un aryle en C₆-C₁₂ éventuellement substitué ou un hétéroaryle à 5 à 10 chaînons éventuellement substitué ;
R² est H, un halogène, COOR⁹, CN, CONR⁹R¹⁰, NR⁹R¹⁰, NR⁹SO₂R¹⁰, SO₂NR⁹R¹⁰, NR⁹COR¹⁰, un alkyle en C₁-C₁₀ éventuellement substitué, un hétérocycle à 3 à 8 chaînons mono- ou bicyclique éventuellement substitué, un alcoxy en C₁-C₁₀ éventuellement substitué, un aryle en C₆-C₁₂ mono- ou bicyclique éventuellement substitué ou un hétéroaryle à 5 à 10 chaînons mono- ou bicyclique éventuellement substitué ;
R³ est H, un halogène, un alkyle en C₁-C₁₀ éventuellement substitué, un cycloalkyle en C₃-C₆ mono- ou bicyclique éventuellement substitué, un hétérocycle à 3 à 8 chaînons mono- ou bicyclique éventuellement substitué, un alcoxy en C₁-C₁₀ éventuellement substitué, un aryle en C₆-C₁₂ mono- ou bicyclique éventuellement substitué ou un hétéroaryle à 5 à 10 chaînons mono- ou bicyclique éventuellement substitué ;
R⁴ et R⁵ sont chacun indépendamment choisis dans le groupe constitué par H et un halogène ;
R⁶ est H ;
L est NR⁸ et R⁷ est un alkyle en C₃-C₁₀ éventuellement substitué, un alcényle en C₂-C₁₀ éventuellement substitué, un alcynyle en C₂-C₁₀ éventuellement substitué, un aryle en C₆-C₁₂ mono- ou bicyclique éventuellement substitué, un hétéroaryle à 5 à 10 chaînons mono- ou bicyclique éventuellement substitué, un cycloalkyle en C₃-C₆ mono- ou bicyclique éventuellement substitué ou un hétérocycle à 3 à 8 chaînons mono- ou bicyclique éventuellement substitué ; ou L est absent et R⁷ est un alkyle en C₃-C₁₀ éventuellement substitué ou un hétérocycle à 3 à 8 chaînons mono- ou bicyclique éventuellement substitué, où lorsque L est absent et R⁷ est un alkyle en C₃-C₁₀ éventuellement substitué, l'alkyle est non substitué ou substitué par un ou plusieurs parmi OH, un oxo ou un halogène ;
R⁸ est H ; et
R⁹ et R¹⁰ sont chacun indépendamment choisis dans le groupe constitué par H, un aryle en C₆-C₁₂ mono- ou bicyclique éventuellement substitué, un hétéroaryle à 5 à 10 chaînons mono- ou bicyclique éventuellement substitué, un alkyle en C₁-C₁₀ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₁₀ éventuellement substitué, un alcynyle en C₂-C₁₀ éventuellement substitué, un alcoxy en C₁-C₁₀ éventuellement substitué et NH₂ ;
ou un complexe, un sel, un solvate, une forme tautomère ou une forme polymorphe acceptable pharmaceutiquement de celui-ci,
où, sauf indication contraire :
un alkyle éventuellement substitué est non substitué ou substitué par un ou plusieurs parmi un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R^{1O}, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un alcényle éventuellement substitué est non substitué ou substitué par un ou plusieurs parmi un alkyle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R^{1O}, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un groupe alcynyle éventuellement substitué est non substitué ou substitué par un ou plusieurs parmi un alkyle en C₁-C₆ éventuellement substitué, un alcényle en C₂-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un groupe alcoxy éventuellement substitué est non substitué ou substitué par un ou plusieurs parmi un halogène, OH, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryloxy éventuellement substitué, un hétéroaryloxy éventuellement substitué, un aryle en C₆-C₁₂ éventuellement substitué, un hétéroaryle à 5 à 10 chaînons éventuellement substitué, un cycloalkyle en C₃-C₆ et un hétérocycle à 3 à 8 chaînons ;
un aryle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un azido, OP(O)(OH)₂, OC(O)R⁹, COOR⁹, un alcényle en C₁-C₆, =NOR⁹, NR⁹C(O)R^{1O}, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un aryloxy éventuellement substitué est le groupe aryl-O- où aryle est un groupe aryle en C₆-C₁₂ éventuellement substitué ;
un cycloalkyle ou un cycloalcényle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un hétéroaryle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un hétérocycle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ; et où le composé n'est pas :

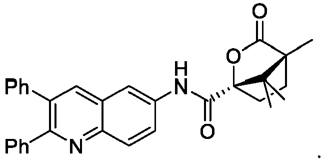

2. Composé selon la revendication 1, dans lequel R et R¹ sont chacun indépendamment choisis dans le groupe constitué par un aryle en C₆-C₁₂ mono- ou bicyclique éventuellement substitué, un hétéroaryle à 5 à 10 chaînons mono- ou bicyclique éventuellement substitué, un alkyle en C₁-C₆ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₆ éventuellement substitué et un alcynyle en C₂-C₆ éventuellement substitué.

3. Composé selon la revendication 2, dans lequel R et R¹ sont chacun indépendamment choisis parmi un cycle phényle éventuellement substitué, un alkyle en C₁-C₆ éventuellement substitué, un alcényle en C₂-C₆ éventuellement substitué, un alcynyle en C₂-C₆ éventuellement substitué ou un hétéroaryle à 5 ou 6 chaînons éventuellement substitué.

4. Composé selon une quelconque revendication précédente, dans lequel R² est **H,** COOR⁹, CONR⁹R¹⁰, CN, NR⁹COR¹⁰, NR⁹R¹⁰, NR⁹SO₂R¹⁰, un alkyle en C₁-C₆ éventuellement substitué, un alcoxy en C₁-C₆ éventuellement substitué, un aryle en C₆-C₁₂ mono- ou bicyclique éventuellement substitué ou un hétéroaryle à 5 à 10 chaînons mono- ou bicyclique éventuellement substitué.

5. Composé selon une quelconque revendication précédente, dans lequel R³ est H, un halogène, un alkyle en C₁-C₆ éventuellement substitué, un cycloalkyle en C₃-C₆ mono- ou bicyclique éventuellement substitué, un alcoxy en C₁-C₆ éventuellement substitué, un aryle en C₆-C₁₂ mono- ou bicyclique éventuellement substitué, ou un hétéroaryle à 5 à 10 chaînons mono- ou bicyclique éventuellement substitué.

6. Composé selon une quelconque revendication précédente, dans lequel R⁴ et R⁵ sont chacun indépendamment choisis dans le groupe constitué par H, un halogène, OH, CN, un aryle en C₆-C₁₂ mono- ou bicyclique éventuellement substitué, un alkyle en C₁-C₆ éventuellement substitué, un alcoxy en C₁-C₆ éventuellement substitué ou un cycloalkyle en C₃-C₆ mono- ou bicyclique éventuellement substitué.

7. Composé selon la revendication 6, dans lequel R⁴ et R⁵ sont H, un halogène, OH, CN ou un alkyle en C₁-C₆ éventuellement substitué.

8. Composé selon la revendication 7, dans lequel R⁴ et R⁵ sont H.

9. Composé selon une quelconque revendication précédente, dans lequel L est NR⁸ et R⁷ est un alkyle en C₃-C₆ éventuellement substitué, un alcényle en C₂-C₆ éventuellement substitué, un alcynyle en C₂-C₆ éventuellement substitué, un aryle en C₆-C₁₂ mono- ou bicyclique éventuellement substitué, un cycloalkyle en C₃-C₆ mono- ou bicyclique éventuellement substitué ou un hétérocycle à 3 à 8 chaînons mono- ou bicyclique éventuellement substitué.

10. Composé selon la revendication 1, ledit composé étant :
1-(2,3-diphénylquinolin-6-yl)-3-(2-hydroxybutyl)urée ;
(R)-1-(2,3-diphénylquinolin-6-yl)-3-(2-hydroxybutyl)urée ;
1-butyl-3-(2,3-diphénylquinolin-6-yl)urée ;
1-(2,3-bis(2-fluorophényl)quinolin-6-yl)-3-(2-hydroxybutyl)urée ;
1-butyl-3-(5-(2-hydroxypyridin-3-yl)-2,3-diphénylquinolin-6-yl)urée ;
1-(2,3-bis(2-méthoxyphényl)quinolin-6-yl)-3-(2-hydroxybutyl)urée ;
1-(3-(2-fluorophényl)-2-phénylquinolin-6-yl)-3-(2-hydroxybutyl)urée ;
1-(2-(2-fluorophényl)-3-phénylquinolin-6-yl)-3-(2-hydroxybutyl)urée ;
1-(2,3-bis(2-méthoxypyridin-4-yl)quinolin-6-yl)-3-(2-hydroxybutyl)urée ;
1-(2-hydroxybutyl)-3-(3-(2-méthoxyphényl)-2-phénylquinolin-6-yl)urée ;
1-(2,3-bis(6-méthoxypyridin-3-yl)quinolin-6-yl)-3-(2-hydroxybutyl)urée ;
1-(2-hydroxybutyl)-3-(2-(2-méthoxyphényl)-3-phénylquinolin-6-yl)urée ;
1-(2,3-bis(2-méthoxypyridin-3-yl)quinolin-6-yl)-3-(2-hydroxybutyl)urée ;
1-(2,3-bis(2-fluorophényl)quinolin-6-yl)-3-butylurée ;
(R)-1-(3-(2-fluorophényl)-2-phénylquinolin-6-yl)-3-(2-hydroxybutyl)urée ;
1-butyl-3-(3-(2-fluorophényl)-2-phénylquinolin-6-yl)urée ;
1-(2,3-bis(4-fluorophényl)quinolin-6-yl)-3-(2-hydroxybutyl)urée ;
1-(3-(2-fluorophényl)-2-(2-méthoxyphényl)quinolin-6-yl)-3-(2-hydroxybutyl)urée ;
1-(2,3-di(pyridin-3-yl)quinolin-6-yl)-3-(2-hydroxybutyl)urée ;
(R)-1-(2,3-bis(2-fluorophényl)quinolin-6-yl)-3-(2-hydroxybutyl)urée ;
1-(2,3-bis(3-acétylphényl)quinolin-6-yl)-3-(2-hydroxybutyl)urée ;
1-(2,3-di(1H-pyrazol-4-yl)quinolin-6-yl)-3-(2-hydroxybutyl)urée ;
1-butyl-3-(3-(2-méthoxyphényl)-2-phénylquinolin-6-yl)urée ;
1-(2,3-di(pyridin-4-yl)quinolin-6-yl)-3-(2-hydroxybutyl)urée ;
1-(2,3-bis(3-fluorophényl)quinolin-6-yl)-3-(2-hydroxybutyl)urée ;
1-(2-(2-fluorophényl)-3-(2-méthoxyphényl)quinolin-6-yl)-3-(2-hydroxybutyl)urée ;
1-(2,3-bis(6-oxo-1,6-dihydropyridin-3-yl)quinolin-6-yl)-3-(2-hydroxybutyl)urée ;
(S)-1-(2,3-bis(2-fluorophényl)quinolin-6-yl)-3-(2-hydroxybutyl)urée ;
1-(2,3-bis(2-hydroxypyridin-4-yl)quinolin-6-yl)-3-(2-hydroxybutyl)urée ;
(S)-1-(2,3-diphénylquinolin-6-yl)-3-(2-hydroxybutyl)urée ;
1-(3-(2-fluorophényl)-2-(pyridin-3-yl)quinolin-6-yl)-3-(2-hydroxybutyl)urée ;
1-(2-(cyclohex-1-én-1-yl)-3-(2-fluorophényl)quinolin-6-yl)-3-(2-hydroxybutyl)urée ;
1-(2-(2-(diméthylamino)phényl)-3-(2-fluorophényl)quinolin-6-yl)-3-(2-hydroxybutyl)urée ;
1-(3-(2-fluorophényl)-2-(pyridin-4-yl)quinolin-6-yl)-3-(2-hydroxybutyl)urée ;
(R)-1-(3-(2-fluorophényl)-2-(2-méthoxyphényl)quinolin-6-yl)-3-(2-hydroxybutyl)urée ;
(S)-1-(3-(2-fluorophényl)-2-(2-méthoxyphényl)quinolin-6-yl)-3-(2-hydroxybutyl)urée ;
1-butyl-3-(3-(2-fluorophényl)-2-(2-méthoxyphényl)quinolin-6-yl)urée ;
1-(2-hydroxybutyl)-3-(3-phényl-2-(pyridin-2-yl)quinolin-6-yl)urée ;
1-(2-cyclohexyl-3-(2-fluorophényl)quinolin-6-yl)-3-(2-hydroxybutyl)urée ;
1-(2-cyclopropyl-3-phénylquinolin-6-yl)-3-(2-hydroxybutyl)urée ;
1-(2-hydroxybutyl)-3-(3-phényl-2-(pyridin-3-yl)quinolin-6-yl)urée ;
1-(2,3-bis(2-hydroxypyridin-3-yl)quinolin-6-yl)-3-(2-hydroxybutyl)urée ;
1-(2-hydroxybutyl)-3-(2-(1 -méthyl-1H-pyrazol-4-yl)-3-phénylquinolin-6-yl)urée ;
1-(3-(2-fluorophényl)-2-phénylquinolin-6-yl)-3-(2-méthoxybutyl)urée ;
1-(2,2-difluorobutyl)-3-(3-(2-fluorophényl)-2-phénylquinolin-6-yl)urée ;
1-(3-(2-fluorophényl)-2-(3-fluorophényl)quinolin-6-yl)-3-(2-hydroxybutyl)urée ;
1-(3-(2-fluorophényl)-2-(4-fluorophényl)quinolin-6-yl)-3-(2-hydroxybutyl)urée ;
6-(3-(2-hydroxybutyl)uréido)-2,3-diphénylquinoléine-4-carboxylate de méthyle ;
6-(3-(2-hydroxybutyl)uréido)-2,3-diphénylquinoléine-4-carboxamide ;
(E)-1-(2-hydroxybutyl)-3-(3-phényl-2-styrylquinolin-6-yl)urée ;
N-(2-(3-(2-fluorophényl)-6-(3-(2-hydroxybutyl)uréido)quinolin-2-yl)phényl)acétamide ;
1-(2-hydroxybutyl)-3-(2-méthyl-3-phénylquinolin-6-yl)urée ;
1-(2-(2-cyanophényl)-3-(2-fluorophényl)quinolin-6-yl)-3-(2-hydroxybutyl)urée ;
1-(2-(2-aminophényl)-3-(2-fluorophényl)quinolin-6-yl)-3-(2-hydroxybutyl)urée ;
1-(2-([1,1'-biphényl]-4-yl)-3-phénylquinolin-6-yl)-3-(2-hydroxybutyl)urée ;
1-(2-hydroxybutyl)-3-(2-phénéthyl-3-phénylquinolin-6-yl)urée ;
(R)-6-(3-(2-hydroxybutyl)uréido)-2,3-diphénylquinoléine-4-carboxamide ;
1-(2-(2-éthoxyphényl)-3-phénylquinolin-6-yl)-3-(2-hydroxybutyl)urée ;
1-(2-hydroxybutyl)-3-(2-(2-isobutoxyphényl)-3-phénylquinolin-6-yl)urée ;
1-(2-éthynyl-3-phénylquinolin-6-yl)-3-(2-hydroxybutyl)urée ;
1-(2-éthyl-3-phénylquinolin-6-yl)-3-(2-hydroxybutyl)urée ;
6-(3-(2-hydroxybutyl)uréido)-N-méthyl-2,3-diphénylquinoléine-4-carboxamide ;
6-(3-(2-hydroxybutyl)uréido)-N,N-diméthyl-2,3-diphénylquinoléine-4-carboxamide ;
N-cyclopropyl-6-(3-(2-hydroxybutyl)uréido)-2,3-diphénylquinoléine-4-carboxamide ;
Acide 6-(3-(2-hydroxybutyl)uréido)-2,3-diphénylquinoléine-4-carboxylique ;
6-(3-(2-hydroxybutyl)uréido)-2-méthyl-3-phénylquinoléine-4-carboxamide ;
(S)-1-(3-(2-fluorophényl)-2-phénylquinolin-6-yl)-3-(2-hydroxybutyl)urée ;
(S)-1-(2,3-diphénylquinolin-6-yl)-3-(2-hydroxybutyl)urée ;
6-(3-(2-hydroxybutyl)uréido)-2,3-diméthylquinoléine-4-carboxamide ;
1-(4-cyano-2,3-diphénylquinolin-6-yl)-3-(2-hydroxybutyl)urée ;
6-(3-(2-hydroxybutyl)uréido)-3-phénylquinoléine-4-carboxamide ;
(R)-1-(2-hydroxybutyl)-3-(2-méthyl-3-phénylquinolin-6-yl)urée ;
3-(2-fluorophényl)-6-(3-(2-hydroxybutyl)uréido)-2-méthylquinoléine-4-carboxamide ;
1-(3-(2-fluorophényl)-2-méthylquinolin-6-yl)-3-(2-hydroxybutyl)urée ;
1-butyl-3-(2-méthyl-3-phénylquinolin-6-yl)urée ;
1-(2-méthoxybutyl)-3-(2-méthyl-3-phénylquinolin-6-yl)urée ;
1-(2-méthoxyéthyl)-3-(2-méthyl-3-phénylquinolin-6-yl)urée ;
1-(2-hydroxybutyl)-3-(2-méthyl-3-(pyridin-3-yl)quinolin-6-yl)urée ;
1-(2-hydroxybutyl)-3-(2-méthyl-3-(o-tolyl)quinolin-6-yl)urée ;
1-(3-(2-chlorophényl)-2-méthylquinolin-6-yl)-3-(2-hydroxybutyl)urée ;
1-(2-hydroxybutyl)-3-(2-méthyl-3-(1-méthyl-1H-pyrazol-4-yl)quinolin-6-yl)urée ;
(R)-1-(2-hydroxybutyl)-3-(2-méthyl-3-(o-tolyl)quinolin-6-yl)urée ;
N-(6-(3-(2-hydroxybutyl)uréido)-2,3-diphénylquinolin-4-yl)acétamide ;
1-(2,5-diméthyl-3-phénylquinolin-6-yl)-3-(2-hydroxybutyl)urée ;
1-(5-cyclopropyl-2-méthyl-3-phénylquinolin-6-yl)-3-(2-hydroxybutyl)urée ;
1-(5-bromo-2-méthyl-3-phénylquinolin-6-yl)-3-(2-hydroxybutyl)urée ;
1-(2-hydroxybutyl)-3-(3-phényl-2-(trifluorométhyl)quinolin-6-yl)urée ;
(S)-1-(2-hydroxybutyl)-3-(2-méthyl-3-phénylquinolin-6-yl)urée ;
(R)-1-(7-fluoro-2,3-diphénylquinolin-6-yl)-3-(2-hydroxybutyl)urée ;
1-(4-(diméthylamino)-2,3-diphénylquinolin-6-yl)-3-(2-hydroxybutyl)urée ;
(R)-N-(6-(3-(2-hydroxybutyl)uréido)-2,3-diphénylquinolin-4-yl)cyclopropanecarboxamide ;
N-(6-(3-(2-hydroxybutyl)uréido)-2-méthyl-3-phénylquinolin-4-yl)acétamide ;
N-(6-(3-(2-hydroxybutyl)uréido)-2,3 -diphénylquinolin-4-yl)méthanesulfonamide ;
1-(4-amino-2,3-diphénylquinolin-6-yl)-3-(2-hydroxybutyl)urée ;
(R)-1-(2-hydroxybutyl)-3-(2-méthyl-5-(1-méthyl-1H-pyrazol-4-yl)-3-phénylquinolin-6-yl)urée ;
1-(2-hydroxybutyl)-3-(4-(oxazol-2-yl)-2,3-diphénylquinolin-6-yl)urée ;
1-(4-cyano-2-méthyl-3-phénylquinolin-6-yl)-3-(2-hydroxybutyl)urée ;
N-(1-(3-(2-méthyl-3-phénylquinolin-6-yl)uréido)butan-2-yl)acétamide ;
(R)-1-(3-(2-fluorophényl)-2-méthylquinolin-6-yl)-3-(2-hydroxybutyl)urée ;
(R)-1-(2-hydroxybutyl)-3-(4-méthoxy-2,3-diphénylquinolin-6-yl)urée ;
(R)-1-(2-hydroxybutyl)-3-(4-méthoxy-2-méthyl-3-phénylquinolin-6-yl)urée ;
(R)-1-(2-hydroxybutyl)-3-(4-méthyl-2,3-diphénylquinolin-6-yl)urée ;
(R)-N-benzyl-6-(3-(2-hydroxybutyl)uréido)-3-méthyl-2-phénylquinoléine-4-carboxamide ;
1-(2,2-difluorobutyl)-3-(2-méthyl-3-phénylquinolin-6-yl)urée ;
6-(3-(2,2-difluorobutyl)uréido)-2,3-diphénylquinoléine-4-carboxamide ;
1-(2,2-difluorobutyl)-3-(2,3-diphénylquinolin-6-yl)urée ;
(R)-6-(3-(2-hydroxybutyl)uréido)-N,3-diméthyl-2-phénylquinoléine-4-carboxamide ;
(R)-6-(3-(2-hydroxybutyl)uréido)-3-méthyl-2-phénylquinoléine-4-carboxamide ;
1-(2,2-difluorobutyl)-3-(3-(2-fluorophényl)-2-méthylquinolin-6-yl)urée ;
(R)-1-(3-(2-fluorophényl)-2-méthyl-5-(2-oxo-1,2-dihydropyridin-3-yl)quinolin-6-yl)-3-(2-hydroxybutyl)urée ;
(R)-6-(3-(2-hydroxybutyl)uréido)-N-(1-methyl-1H-pyrazol-3-yl)-2,3-diphénylquinoléine-4-carboxamide ;
(R)-1-(2,4-diméthyl-3-phénylquinolin-6-yl)-3-(2-hydroxybutyl)urée ;
(R)-6-(3-(2-hydroxybutyl)uréido)-3-méthyl-N-((1-méthyl-1H-pyrazol-4-yl)méthyl)-2-phénylquinoléine-4-carboxamide ;
(R)-6-(3-(2-hydroxybutyl)uréido)-3-méthyl-N-(1-méthyl-1H-pyrazol-3-yl)-2-phénylquinoléine-4-carboxamide ;
1-(3-(2-fluorophényl)-2-méthylquinolin-6-yl)-3-(2-méthoxyéthyl)urée ;
dihydrogénophosphate de (R)-1-(3-(3-(2-fluorophényl)-2-phénylquinoléin-6-yl)uréido)butan-2-yle ;
N-(2,3-diphénylquinolin-6-yl)hexanamide ;
N-(2,3-bis(2-méthoxyphényl)quinolin-6-yl)hexanamide ;
N-(2-(2-méthoxyphényl)-3-phénylquinolin-6-yl)-4-oxohexanamide ;
N-(2,3-bis(2-fluorophényl)quinolin-6-yl)hexanamide ;
N-(2,3-diphénylquinolin-6-yl)-2,2-difluorohexanamide ;
N-(2-(2-fluorophényl)-3-phénylquinolin-6-yl)-4-oxohexanamide ;
N-(2,3-bis(2-fluorophényl)quinolin-6-yl)-4-oxohexanamide ;
N-(2,3-diphénylquinolin-6-yl)-4-oxohexanamide ;
N-(3-(2-fluorophényl)-2-(2-méthoxyphényl)quinolin-6-yl)-4-oxohexanamide ;
N-(2,3-diphénylquinolin-6-yl)-4-hydroxyhexanamide ;
N-(2,3-bis(2-fluorophényl)quinolin-6-yl)-4-hydroxyhexanamide ;
N-(3-(2-fluorophényl)-2-méthylquinolin-6-yl)-4-hydroxyhexanamide ;
4-hydroxy-N-(2-méthyl-3-phénylquinolin-6-yl)hexanamide ;
1-cyclohexyl-3-(2,3-diphénylquinolin-6-yl)urée ;
N-(2,3-diphénylquinolin-6-yl)-4-méthylpipérazine-1-carboxamide ;
4-acétyl-N-(2,3-diphénylquinolin-6-yl)pipérazine-1-carboxamide ;
1-allyl-3-(2,3-diphénylquinolin-6-yl)urée ;
1-(2,3-diphénylquinolin-6-yl)-3-(prop-2-yn-1-yl)urée ;
1-(2,3-diphénylquinolin-6-yl)-3-(2-hydroxycyclopentyl)urée ;
1-(2,3-diphénylquinolin-6-yl)-3-(tétrahydro-2H-pyran-4-yl)urée ;
N-(2,3-diphénylquinolin-6-yl)-3-méthoxypyrrolidine-1-carboxamide ;
1-(2,3-diphénylquinolin-6-yl)-3-(pyridin-2-yl)urée ;
1-(2,3-diphénylquinolin-6-yl)-3-phénylurée ;
1-(2,3-diphénylquinolin-6-yl)-3-(1-méthylpyrrolidin-3-yl)urée ;
1-(2,3-diphénylquinolin-6-yl)-3-(pyridin-4-yl)urée ;
1-(2,3-diphénylquinolin-6-yl)-3-(1-(hydroxyméthyl)cyclopentyl)urée ;
1-(2,3-diphénylquinolin-6-yl)-3-(1-méthyl-1H-pyrazol-4-yl)urée ;
1-(6,7-diphényl-1,8-naphthyridin-3-yl)-3-(2-hydroxybutyl)urée ;
(R)-1-(6,7-diphényl-1,8-naphthyridin-3-yl)-3-(2-hydroxybutyl)urée ; ou
N-(6,7-diphényl-1,8-naphtyridin-3-yl)-3-méthoxypyrrolidine-1-carboxamide.

11. Composition pharmaceutique comprenant un composé selon l'une quelconque des revendications 1 à 10, ou un sel, un solvate, une forme tautomère ou une forme polymorphe acceptable pharmaceutiquement de celui-ci et un véhicule acceptable pharmaceutiquement.

12. Composé selon l'une quelconque des revendications 1 à 10, ou sel, solvate, forme tautomère ou forme polymorphe acceptable pharmaceutiquement de celui-ci, ou composition pharmaceutique selon la revendication 11, destiné(e) à être utilisé(e) en thérapie.

13. Composé de formule (1) : 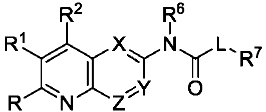
où X est CR³ ;
Y est CR⁴ ;
Z est CR⁵ ou N ; R est choisi dans le groupe constitué par H, un aryle en C₆-C₁₂ mono- ou bicyclique éventuellement substitué, un hétéroaryle à 5 à 10 chaînons mono- ou bicyclique éventuellement substitué, un alkyle en C₁-C₁₀ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₁₀ éventuellement substitué et un alcynyle en C₂-C₁₀ éventuellement substitué ;
R¹ est choisi dans le groupe constitué par un aryle en C₆-C₁₂ mono- ou bicyclique éventuellement substitué, un hétéroaryle à 5 à 10 chaînons mono- ou bicyclique éventuellement substitué, un alkyle en C₁-C₁₀ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₁₀ éventuellement substitué et un alcynyle en C₂-C₁₀ éventuellement substitué, où lorsque R¹ est un alkyle éventuellement substitué, un alcényle éventuellement substitué ou un alcynyle éventuellement substitué, l'alkyle, l'alcényle ou l'alcynyle est non substitué ou substitué par un ou plusieurs parmi un halogène, un aryle en C₆-C₁₂ éventuellement substitué ou un hétéroaryle à 5 à 10 chaînons éventuellement substitué ;
R² est H, un halogène, COOR⁹, CN, CONR⁹R¹⁰, NR⁹R¹⁰, NR⁹SO₂R¹⁰, SO₂NR⁹R¹⁰, NR⁹COR¹⁰, un alkyle en C₁-C₁₀ éventuellement substitué, un hétérocycle à 3 à 8 chaînons mono- ou bicyclique éventuellement substitué, un alcoxy en C₁-C₁₀ éventuellement substitué, un aryle en C₆-C₁₂ mono- ou bicyclique éventuellement substitué ou un hétéroaryle à 5 à 10 chaînons mono- ou bicyclique éventuellement substitué ;
R³ est H, un halogène, un alkyle en C₁-C₁₀ éventuellement substitué, un cycloalkyle en C₃-C₆ mono- ou bicyclique éventuellement substitué, un hétérocycle à 3 à 8 chaînons mono- ou bicyclique éventuellement substitué, un alcoxy en C₁-C₁₀ éventuellement substitué, un aryle en C₆-C₁₂ mono- ou bicyclique éventuellement substitué ou un hétéroaryle à 5 à 10 chaînons mono- ou bicyclique éventuellement substitué ;
R⁴ et R⁵ sont chacun indépendamment choisis dans le groupe constitué par H et un halogène ;
R⁶ est H ;
L est NR⁸ et R⁷ est un alkyle en C₃-C₁₀ éventuellement substitué, un alcényle en C₂-C₁₀ éventuellement substitué, un alcynyle en C₂-C₁₀ éventuellement substitué, un aryle en C₆-C₁₂ mono- ou bicyclique éventuellement substitué, un hétérocycle à 5 à 10 chaînons mono- ou bicyclique éventuellement substitué, un cycloalkyle en C₃-C₆ mono- ou bicyclique éventuellement substitué, un hétérocycle à 3 à 8 chaînons mono- ou bicyclique éventuellement substitué ; ou L est absent et R⁷ est un alkyle en C₃-C₁₀ éventuellement substitué ou un hétérocycle à 3 à 8 chaînons mono- ou bicyclique éventuellement substitué, où lorsque L est absent et R⁷ est un alkyle en C₃-C₁₀ éventuellement substitué, l'alkyle est non substitué ou substitué par un ou plusieurs parmi OH, un oxo ou un halogène ;
R⁸ est H ; et
R⁹ et R¹⁰ sont chacun indépendamment choisis dans le groupe constitué par H, un aryle en C₆-C₁₂ mono- ou bicyclique éventuellement substitué, un hétéroaryle à 5 à 10 chaînons mono- ou bicyclique éventuellement substitué, un alkyle en C₁-C₁₀ éventuellement substitué, un cycloalkyle en C₃-C₆ mono- ou bicyclique éventuellement substitué, un cycloalcényle en C₃-C₆ mono- ou bicyclique éventuellement substitué, un alcényle en C₂-C₁₀ éventuellement substitué, un alcynyle en C₂-C₁₀ éventuellement substitué, un alcoxy en C₁-C₁₀ éventuellement substitué et NH₂ ;
où, sauf indication contraire :
un alkyle éventuellement substitué est non substitué ou substitué par un ou plusieurs parmi un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R^{1O}, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un alcényle éventuellement substitué est non substitué ou substitué par un ou plusieurs parmi un alkyle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un groupe alcynyle éventuellement substitué est non substitué ou substitué par un ou plusieurs parmi un alkyle en C₁-C₆ éventuellement substitué, un alcényle en C₂-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un groupe alcoxy éventuellement substitué est non substitué ou substitué par un ou plusieurs parmi un halogène, OH, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryloxy éventuellement substitué, un hétéroaryloxy éventuellement substitué, un aryle en C₆-C₁₂ éventuellement substitué, un hétéroaryle à 5 à 10 chaînons éventuellement substitué, un cycloalkyle en C₃-C₆ et un hétérocycle à 3 à 8 chaînons ;
un aryle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un azido, OP(O)(OH)₂, OC(O)R⁹, COOR⁹, un alcényle en C₁-C₆, =NOR⁹, NR⁹C(O)R^{1O}, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un aryloxy éventuellement substitué est le groupe aryl-O- où aryle est un groupe aryle en C₆-C₁₂ éventuellement substitué ;
un cycloalkyle ou un cycloalcényle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un hétéroaryle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
un hétérocycle éventuellement substitué est non substitué ou substitué par un ou plusieurs parmi un alkyle en C₁-C₆ éventuellement substitué, un alcényle en C₁-C₆ éventuellement substitué, un alcynyle en C₁-C₆ éventuellement substitué, un halogène, OH, un alcoxy en C₁-C₆ éventuellement substitué, un aryloxy éventuellement substitué, un hétéroaryloxy éventuellement substitué, NR⁹R¹⁰, C(O)R⁹, CN, un oxo, un azido, OP(O)(OH)₂, OC(O)R⁹, COOR⁹, un alcényle en C₁-C₆, =NOR⁹, NR⁹C(O)R¹⁰, SO₂R⁹, SO₂NR⁹R¹⁰, un aryle en C₆-C₁₂ éventuellement substitué, un hétéroaryle à 5 à 10 chaînons éventuellement substitué, un cycloalkyle en C₃-C₆ éventuellement substitué et un hétérocycle à 3 à 8 chaînons éventuellement substitué ;
ou un sel, un solvate, une forme tautomère ou une forme polymorphe acceptable pharmaceutiquement de celui-ci, ou une composition pharmaceutique comprenant un composé de formule (1), ou un sel, un solvate, une forme tautomère ou une forme polymorphe acceptable pharmaceutiquement de celui-ci, et un véhicule acceptable pharmaceutiquement, destiné(e) à être utilisé(e) dans le traitement, l'amélioration ou la prévention d'une maladie choisie parmi le cancer, une infection bactérienne, une infection virale, une infection parasitaire, une infection fongique, une maladie neurodégénérative, un trouble neurologique, une maladie cérébrovasculaire, une maladie cardiovasculaire, la stéatose hépatique non alcoolique et l'obésité ou destiné(e) à être utilisé(e) pour favoriser un vieillissement en bonne santé.

14. Composé ou composition pharmaceutique destiné(e) à être utilisé(e) selon la revendication 13, ladite maladie étant le cancer, et ledit cancer étant choisi dans le groupe constitué par le cancer colorectal, le cancer épidermoïde des voies aérodigestives, les tumeurs stromales gastro-intestinales, le cancer du poumon, le cancer du cerveau, le neuroblastome, les tumeurs gliales, l'astrocytome, le glioblastome, le cancer du foie, le cancer de l'estomac, le sarcome, la leucémie, le lymphome, le myélome multiple, le cancer de l'ovaire, le cancer de l'utérus, le cancer du sein, le mélanome, le cancer de la prostate, le cancer de la vessie, le carcinome pancréatique ou le carcinome rénal, ou ladite maladie étant une infection virale, et ladite infection virale étant une infection par le virus de l'hépatite C (VHC) ou une infection par le cytomégalovirus humain (HCMV).
